# EUROPEAN PATENT APPLICATION

(11) **EP 4 707 277 A1**
(43) Date of publication of application: **11.03.2026**
(21) Application number: 24800261.0
(22) Date of filing: 03.05.2024
(51) Int. Cl.: C07D 471/04, A61K 31/437, A61K 31/506, A61K 31/444, A61P 29/00, A61P 37/00, A61P 35/00

(54) **NOVEL HETEROCYCLIC AMINE DERIVATIVES AND PHARMACEUTICAL COMPOSITION COMPRISING SAME**

(30) Priority: 04.05.2023 KR 20230058527
(71) Applicant: Daewoong Pharmaceutical Co., Ltd., Hwaseong-si, Gyenoggi-do 18623 (KR)
(72) Inventor: JEONG, Jaehun, Seongnam-si, Gyeonggi-do 13581 (KR); LEE, Doohyun, Daegu 41561 (KR); JO, Hyeim, Suwon-si, Gyeonggi-do 16687 (KR); JUNG, Sooyeon, Seoul 05507 (KR); LEE, Eunhye, Gyeonggi-do 17027 (KR); LEE, Choongmin, Anyang-si, Gyeonggi-do 13994 (KR); LEE, Ahreum, Seoul 07562 (KR); PARK, Joon Seok, Gyeonggi-do 17000 (KR); HYUN, Hyae Jung, Seongnam-si, Gyeonggi-do 13568 (KR)
(74) Representative: Germain Maureau
(86) International application number: PCT/KR2024/006004
(87) International publication number: WO 2024/228580

(57) **Abstract**

The present disclosure relates to a novel heterocyclic amine derivative represented by the following Chemical Formula 1 or a pharmaceutical composition comprising the same. The compound according to the present disclosure can be effectively used for the prevention or treatment of inflammatory diseases, autoimmune diseases, or cancers. 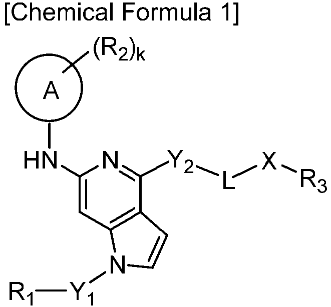 in Chemical Formula 1, Y₁, R₁, A, R₂, k, Y₂, L, X and R₃ are as defined in the detailed description.

## Description

### [TECHNICAL FIELD]

The present disclosure relates to a novel heterocyclic amine useful as BTK (Bruton's Tyrosine Kinase) and ITK (Interleukin-2 Tyrosine Kinase) inhibitors, and a pharmaceutical composition comprising the same.

### [BACKGROUND]

BTK (Bruton's Tyrosine Kinase) and ITK (Interleukin-2 Tyrosine Kinase) is a type of TEC (tyrosine kinase expressed in hepatocellular carcinoma)-family kinase, and acts on various immune responses.

BTK acts as a regulator of early B-cell development as well as of mature B-cell activation, signaling and survival. The B-cell is signaled by a B cell receptor (BCR) that recognizes an antigen attached to the surface of an antigen-presenting cell, and is activated into a mature antibody-producing cell. However, aberrant signaling via BCR leads to abnormal B-cell proliferation and the formation of pathological autoantibodies, and thereby can induce cancer, autoimmune and/or inflammatory diseases. Thus, in the abnormal B-cell proliferation, signaling via BCR may be blocked when BTK is deficient. Accordingly, inhibition of BTK can block B-cell mediated disease processes, and thus, the use of BTK inhibitors may be a useful approach for the treatment of B-cell mediated diseases.

Furthermore, BTK can be expressed by other cells that may be associated with disease besides B-cells. As an example, BTK is important components for Fc-gamma signaling in microglia cells. Specifically, BTK is known to be useful in regulating diseases such as multiple sclerosis through the regulation of B-cells and microglia cells (J Kramer et al. Nature Reviews Neurology. 2023). In addition, it is known that monocytes from XLA patients, in which BTK activity is absent, show decreased TNF alpha production following stimulation and thus TNF alpha-mediated inflammation could be inhibited by BTK inhibitors (see, Horwood et al., J. Exp. Med. 197: 1603, 2003).

In addition, ITK is known to be an important component of TCR signaling in T-cells. T cells are activated by TCR signaling, and the activated T cells produce inflammatory cytokines, and activate B cells and macrophages, causing autoimmune diseases (Sahu N. et al. Curr Top Med Chem. 2009, 9, 690). Further, recently, it has been reported to regulate the development of Th17 and Treg cells via ITK-/- mice, and it has ample potential as a therapeutic target for autoimmune diseases (Gomez-Rodriguez J. et al. J. Exp. Med. 2014, 211, 529). Recently, the expression of Th1 and Th17 - associated cytokines has been confirmed in autoimmune diseases such as multiple sclerosis, and the Th17-associated cytokines IL-17 and IL-23 were confirmed to play a major role in the pathogenesis of diseases (Shi-Rong Wen. et al. J. Neuroimmunol. 2012. 244, 94-96).

Therefore, substances that inhibit BTK and ITK have been developed, respectively. As the BTK inhibitor, WO2008/039218 discloses 4-aminopyrazolo[3,4-d]pyrimidinylpiperidine derivatives, WO2015/061247 discloses hetero compounds such as pyridine, pyrimidine, pyrazine and pyridazine compounds, and WO2014/055934 discloses pyrimidinylphenylacrylamide derivatives. In addition, as the ITK inhibitor, WO2005/066335 discloses aminobenzimidazole derivatives, WO2005/056785 discloses pyridone derivatives, WO2002/050071 discloses aminothiazole derivatives, and WO2014/036016 discloses benzimidazole derivatives.

Meanwhile, since autoimmune diseases such as multiple sclerosis are caused by complex factors of B-cells and T-cells (Kathrine E. Attfield. et al. Nature Reviews Immunology. 2022. 734-750), suppressing B cells and T cells simultaneously rather than suppressing each of them alone may be more effective in treating the diseases.

However, as BTK acts on B-cells as a kinase expressed on the B-cell receptor subtype, and ITK acts on T-cells as a kinase expressed on the T-cell receptor subtype, BTK and ITK are different target substances from each other. Therefore, it was not possible to predict the ITK inhibitory effect of a BTK inhibitory substance, and thus it was not easy to develop a substance that dually inhibits BTK and ITK.

In view of the above, as a result of studying novel compounds, the present inventors has found that compounds having different chemical structures from the BTK and ITK inhibitors reported to date have excellent BTK and ITK dual activity inhibitory effects, thereby completing the present disclosure. The compounds belonging to the present disclosure mainly have BTK and ITK inhibitory activities on their own, but do not exclude a possibility of exhibiting a pharmacological action as an efficacious agent by a special body environment or by products of metabolic process, after absorption into the body.

### [DETAILED DESCRIPTION OF THE INVENTION]

### [Technical Problem]

It is an object of the present disclosure to provide a novel heterocyclic amine useful as BTK and/or ITK inhibitors and a pharmaceutical composition comprising the same.

### [Technical Solution]

In order to achieve the above object, according to the present disclosure, there is provided a compound represented by the following Chemical Formula 1, or a pharmaceutically acceptable salt thereof: 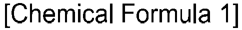 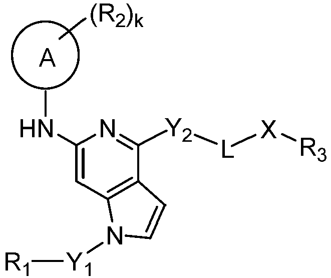 in Chemical Formula 1,
Y₁ is a single bond, a C₁₋₄ alkylene, or a C₁₋₄ haloalkylene,
R₁ is a C₁₋₁₀ alkyl, a C₁₋₁₀ alkoxy, a C₃₋₁₀ cycloalkyl, a C₆₋₂₀ aryl, a N-containing 6-membered heteroaryl, tetrahydrofuranyl, or tetrahydropyranyl,
wherein R₁ is unsubstituted, or substituted with one or more substituents selected from the group consisting of halogen, cyano, hydroxy, a C₁₋₄ alkyl and a C₁₋₄ haloalkyl,
A is benzene ring; or a 5- or 6-membered heterocycle containing 1 to 3 heteroatoms selected from the group consisting of N, O and S substituted or unsubstituted with oxo(=O), provided that the 5-membered or 6-membered heterocycle contains at least one N,
R₂ is each independently hydrogen, halogen, cyano, a C₁₋₄ alkyl, a C₁₋₄ haloalkyl, a C₁₋₄ alkoxy, a C₁₋₄ haloalkoxy, or -CONH(C₁₋₄ alkyl),
k is 1 or 2,
Y₂ is a single bond, -O-, or -O-(C₁₋₄ alkylene),
L is any one of the linking groups represented by the following Chemical Formulas 2a to 2k, 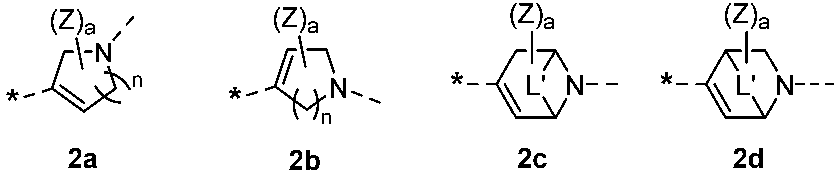 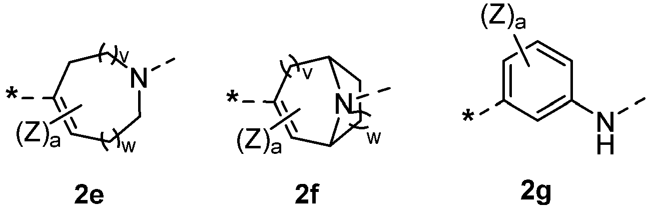 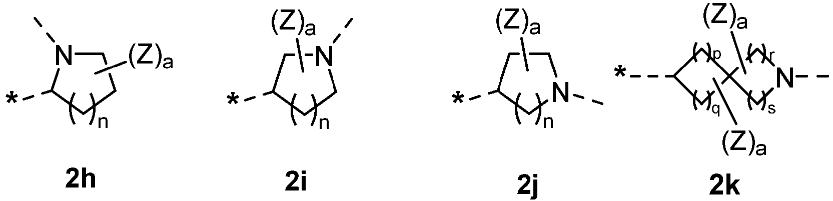
in Chemical Formulas 2a to 2k,
n is 0, 1, 2, or 3,
Z is each independently halogen, a C₁₋₄ alkyl, a C₁₋₄ haloalkyl, a C₁₋₄ alkoxy, or a C₁₋₄ haloalkoxy,
a is each independently 0, 1, or 2,
L' is methylene, ethylene, or methylene-O-methylene,
v and w are each independently 0, 1, or 2,
provided that v+w is an integer from 0 to 3,
p to s are each independently 0, 1, 2, 3, or 4,
provided that p+q and r+s are each independently an integer from 2 to 4,
* means a point linked to Y₂ in Chemical Formula 1,
X is CO or SO₂, and
R₃ is a C₁₋₄ alkyl, a C₂₋₄ alkenyl, or a C₂₋₄ alkynyl,
wherein R₃ is unsubstituted, or substituted with one or more substituents selected from the group consisting of halogen, NH₂, NH(C₁₋₄ alkyl) and N(C₁₋₄ alkyl)₂.

Preferably, Y₁ may be a single bond, methylene, or ethylene.

Preferably, R₁ may be unsubstituted, or substituted with 1 to 3 substituents selected from the group consisting of halogen, cyano, hydroxy, a C₁₋₄ alkyl and a C₁₋₄ haloalkyl.

More preferably, R₁ may be unsubstituted, or substituted with 1 to 3 substituents selected from the group consisting of chloro, fluoro, cyano, hydroxy, methyl, monofluoromethyl, difluoromethyl and trifluoromethyl.

For example, R₁ is methyl, ethyl, n-propyl, isopropyl, n-butyl, isobutyl, sec-butyl, tert-butyl, n-pentyl, isopentyl, n-hexyl, isohexyl, n-heptyl, isoheptyl, n-oxyl, isoxyl, n-nonyl, isononyl, n-decyl, isodecyl, methoxy, ethoxy, propoxy, cyclopropyl, cyclobutyl, cyclopentyl, cyclohexyl, cycloheptyl, cyclooctyl, phenyl, biphenylyl, terphenylyl, naphthyl, pyridinyl, pyrimidinyl, tetrahydrofuranyl, or tetrahydropyranyl,
wherein R₁ may be unsubstituted, or substituted with 1 to 3 substituents selected from the group consisting of halogen, cyano, hydroxy, a C₁₋₄ alkyl and a C₁₋₄ haloalkyl.

Further, for example, R₁ is methyl, ethyl, n-propyl, isopropyl, n-butyl, isobutyl, sec-butyl, tert-butyl, n-pentyl, isopentyl, n-hexyl, isohexyl, methoxy, ethoxy, propoxy, cyclopropyl, cyclobutyl, cyclopentyl, cyclohexyl, phenyl, pyridinyl, tetrahydrofuranyl, or tetrahydropyranyl,
wherein R₁ may be unsubstituted, or substituted with 1 to 3 substituents selected from the group consisting of chloro, fluoro, cyano, hydroxy, methyl, monofluoromethyl, difluoromethyl and trifluoromethyl.

Further, preferably, when Y₁ is a single bond, R₁ is a C₁₋₁₀ alkyl, or a C₃₋₁₀ cycloalkyl,
wherein R₁ may be unsubstituted, or substituted with one or more substituents selected from the group consisting of halogen, cyano, hydroxy, a C₁₋₄ alkyl, and a C₁₋₄ haloalkyl.

For example, when Y₁ is a single bond, R₁ is methyl, ethyl, n-propyl, isopropyl, n-butyl, isobutyl, sec-butyl, tert-butyl, n-pentyl, isopentyl, n-hexyl, isohexyl, cyclopropyl, cyclobutyl, cyclopentyl, or cyclohexyl,
wherein R₁ may be unsubstituted, or substituted with 1 to 3 substituents selected from the group consisting of chloro, fluoro, cyano, hydroxy, methyl, monofluoromethyl, difluoromethyl and trifluoromethyl.

Further, preferably, when Y₁ is a C₁₋₄ alkylene or a C₁₋₄ haloalkylene, R₁ is a C₁₋₁₀ alkoxy, a C₃₋₁₀ cycloalkyl, a C₆₋₂₀ aryl, a N-containing 6-membered heteroaryl, tetrahydrofuranyl, or tetrahydropyranyl,
wherein R₁ may be unsubstituted, or substituted with one or more substituents selected from the group consisting of halogen, cyano, hydroxy, a C₁₋₄ alkyl, and a C₁₋₄ haloalkyl.

For example, when Y₁ is a C₁₋₄ alkylene or a C₁₋₄ haloalkylene, specifically, for example, when Y₁ is methylene or ethylene, R₁ is methoxy, ethoxy, propoxy, cyclopropyl, cyclobutyl, cyclopentyl, cyclohexyl, phenyl, pyridinyl, tetrahydrofuranyl, or tetrahydropyranyl,
wherein R₁ may be unsubstituted, or substituted with 1 to 3 substituents selected from the group consisting of chloro, fluoro, cyano, hydroxy, methyl, monofluoromethyl, difluoromethyl and trifluoromethyl.

Preferably, A may be benzene, thiazole, thiadiazole, imidazole, pyrazole, pyrazine, pyridine, pyrimidine, or pyridin-2(1H)-one ring.

More preferably, 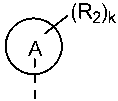 may be represented by any one of the following Chemical Formulas A1 to A12: 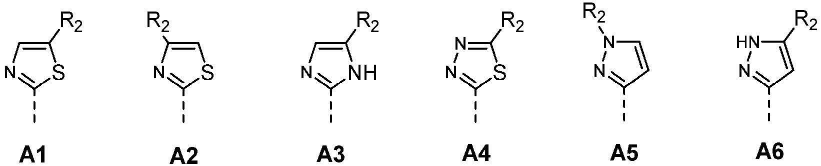 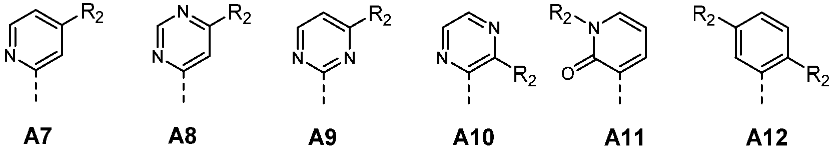 in Chemical Formulas A1 to A12,
R₂ is as defined in Chemical Formula 1.

Preferably, R₂ may be each independently hydrogen, chloro, fluoro, cyano, methyl, ethyl, monofluoromethyl, difluoromethyl, trifluoromethyl, methoxy, or -CONH(methyl).

Meanwhile, k means the number of R₂, and when k is 2, the two R₂s may be the same or different from each other.

More preferably,
A is benzene, thiazole, thiadiazole, pyrazine, pyridine, or pyridin-2(1H)-one ring, and R₂ is each independently halogen, cyano, a C₁₋₄ alkyl, a C₁₋₄ haloalkyl, a C₁₋₄ alkoxy, a C₁₋₄ haloalkoxy, or -CONH(methyl); or
A is an imidazole ring, and R₂ is hydrogen; or
A is pyrazole or pyrimidine ring, and R₂ is each independently hydrogen, halogen, a C₁₋₄ alkyl, a C₁₋₄ haloalkyl, a C₁₋₄ alkoxy, or a C₁₋₄ haloalkoxy.

Preferably, Y₂ may be a single bond, -O-, or -O-(methylene).

Wherein, when Y₂ is -O-(methylene), (methylene) may be linked to L.

Preferably, when Y₂ is a single bond, L may be any one of the linking groups represented by Chemical Formulas 2a to 2g.

For example, when Y₂ is a single bond, L may be any one of the linking groups represented by Chemical Formulas 2a, 2b, 2c, 2e, 2f and 2g.

Further, preferably, when Y₂ is -O- or -O-(C₁₋₄ alkylene), L may be any one of the linking groups represented by Chemical Formulas 2h to 2k.

For example, when Y₂ is -O-, L may be a linking group represented by Chemical Formula 2i or 2k.

Further, for example, when Y₂ is -O-(C₁₋₄ alkylene), L may be a linking group represented by Chemical Formula 2h or 2i.

Meanwhile, a means the number of Z, and when a is 2, the two Zs may be the same or different from each other.

Preferably, L may be any one of the linking groups represented by Chemical Formulas 2a, 2b, 2c, 2e, 2f, 2g, 2h, 2i, and 2k.

For example, in Chemical Formula 2a, n may be 2, and a may be 0.

Further, for example, in Chemical Formula 2b, n may be 1 or 2, and a may be 0 or 1.

Further, for example, in Chemical Formula 2c, L' may be methylene-O-methylene, and a may be 0.

Further, for example, in Chemical Formula 2e, v and w may each be 1, and a may be 0.

Further, for example, in Chemical Formula 2f, v and w may each be 1; or v may be 1, w may be 2, and a may be 0.

Further, for example, in Chemical Formula 2g, a may be 0, 1, or 2.

Further, for example, in Chemical Formula 2h, n may be 0, 1, or 2, and a may be 0, 1, or 2.

Further, for example, in Chemical Formula 2i, n may be 0, 1, or 2, and a may be 0, 1, or 2.

Further, for example, in Chemical Formula 2k, p and q may be each independently 1 or 2, r may be 0 or 1, and s may be 2, 3, or 4.

In this regard, p+q is 2 or 3, r+s is 2, 3, or 4.

Further, preferably, L may be any one linking group selected from the group represented by the following: 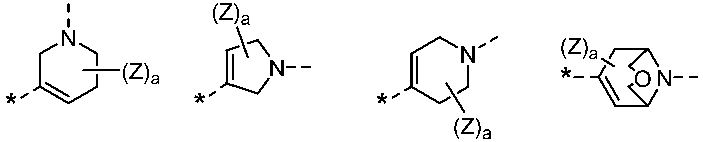 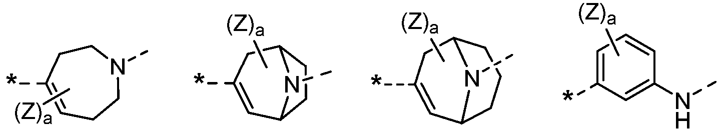 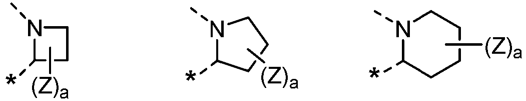 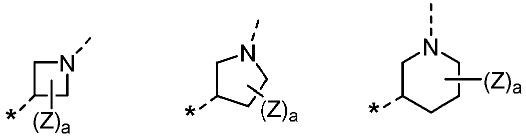 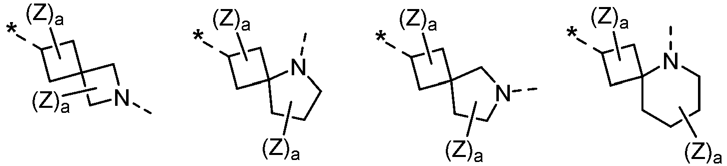 wherein,
Z and a are as defined in in Chemical Formula 1, and
* means a point linked to Y₂ in Chemical Formula 1.

Preferably, **-Y₂-L- may be any one of the linking groups represented by the following Chemical Formulas 3a to 3r: 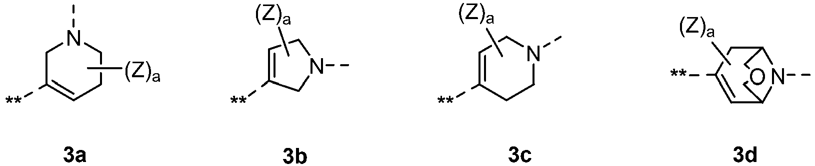 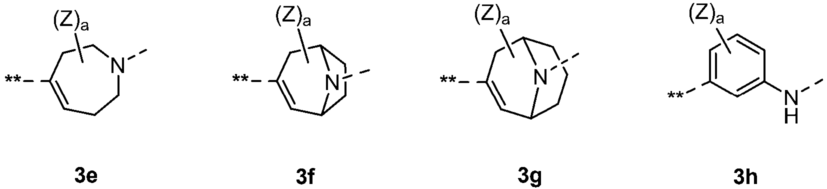 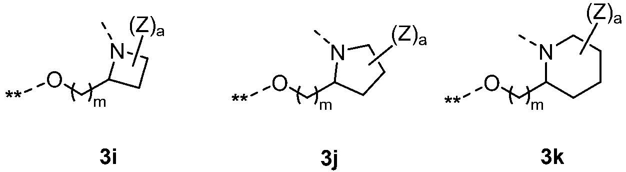 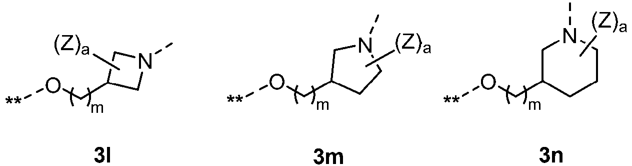 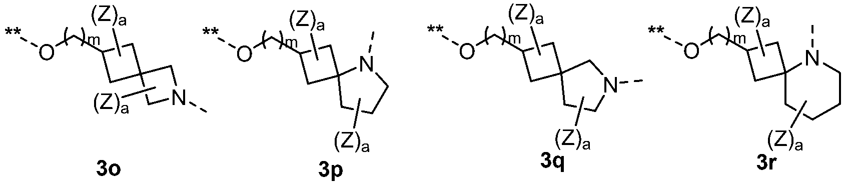 in Chemical Formulas 3a to 3r,
m is 0 or 1,
Z and a are as defined in Chemical Formula 1, and
** means a point linked to carbon number 4 of the pyrrolo[3,2-c]pyridine ring in Chemical Formula 1.

Further, for example, when Y₂ is -O-, the compound may be a stereoisomer represented by the following Chemical Formula 1A or 1B, or a mixture thereof: 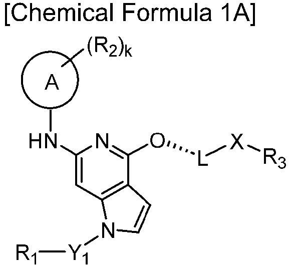 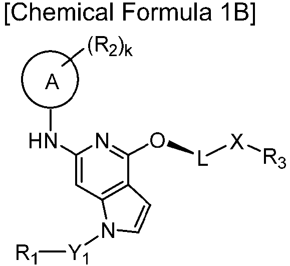 in Chemical Formulas1A and 1B,
Y₁, R₁, A, R₂, k, L, X and R₃ are as defined in Chemical Formula 1.

Further, for example, when Y₂ is -O-(methylene), the compound may be a stereoisomer represented by the following Chemical Formula 1C or 1D, or a mixture thereof: 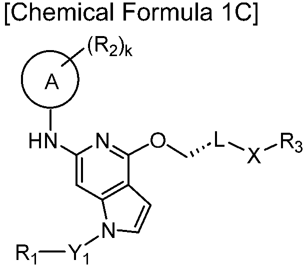 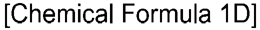 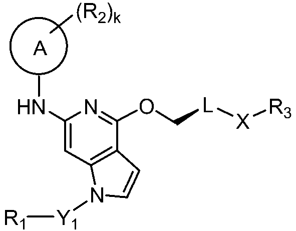 in Chemical Formulas1C and 1D,
Y₁, R₁, A, R₂, k, L, X and R₃ are as defined in Chemical Formula 1.

Preferably, Z may be chloro, fluoro, methyl, or methoxy.

Preferably, R₃ may be unsubstituted, or substituted with one or two substituents selected from the group consisting of chloro, fluoro, NH₂, NH(methyl), and N(methyl)₂.

More preferably, R₃ may be -CH₃, -CH=CH₂, -CH=CHCH₃, -C=CH, or -C≡CCH₃,
wherein R₃ may be unsubstituted, or substituted with one or two substituents selected from the group consisting of chloro, fluoro, NH₂, NH(methyl), and N(methyl)₂.

For example, R₃ may be -CH₃, -CH₂Cl, -CH=CH₂, -CF=CH₂, -CH=CHCH₃, - CH=CHCH₂N(CH₃)₂, -C=CH, or -C≡CCH₃.

Meanwhile, the compound represented by Chemical Formula 1 may be represented by any one of the following Chemical Formulas 1-1 to 1-3: 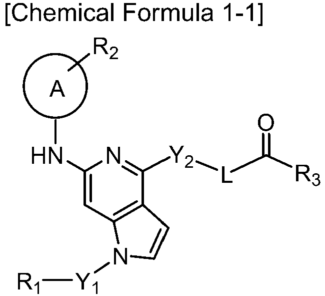 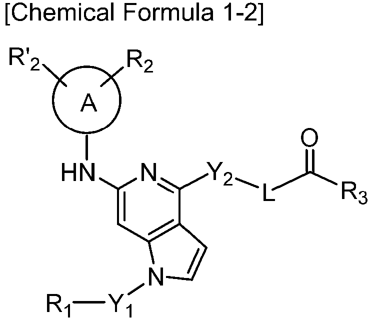 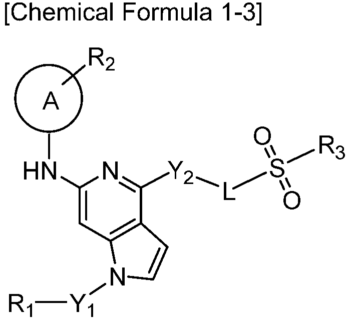 in Chemical Formulas 1-1 to 1-3,
R'₂ is halogen, cyano, a C₁₋₄ alkyl, a C₁₋₄ haloalkyl, a C₁₋₄ alkoxy, a C₁₋₄ haloalkoxy, or -CONH(C₁₋₄ alkyl),
Y₁, R₁, A, R₂, Y₂, L and R₃ are as defined in Chemical Formula 1.

For example, R'₂ may be chloro, fluoro, cyano, methyl, ethyl, monofluoromethyl, difluoromethyl, trifluoromethyl, methoxy, or -CONH(methyl).

Preferably, in Chemical Formula 1-1,
A is thiazole, thiadiazole, imidazole, pyrazole, pyrazine, pyridine, pyrimidine, or pyridin-2(1H)-one ring, and
L is any one of the linking groups represented by Chemical Formulas 2a, 2b, 2c, 2e, 2f, 2g, 2h, 2i, and 2k.

Preferably, in Chemical Formula 1-2,
A is benzene ring, and
L is a linking group represented by Chemical Formula 2i.

Preferably, in Chemical Formula 1-3,
A is thiazole ring, and
L is a linking group represented by Chemical Formula 2i.

Preferably, A is the Chemical Formula A1,
R₂ is halogen, cyano, a C₁₋₄ alkyl, a C₁₋₄ haloalkyl, or -CONH(C₁₋₄ alkyl),
Y₂ is a single bond, -O-, or -O-(C₁₋₄ alkylene), and
L is any one of the linking groups represented by Chemical Formulas 2a to 2c and 2e to 2k.

Preferably, A is the Chemical Formula A2,
R₂ is a C₁₋₄ alkyl or a C₁₋₄ haloalkyl,
Y₂ is a single bond, and
L is a linking group represented by Chemical Formula 2b.

Preferably, A is the Chemical Formula A3,
R₂ is hydrogen,
Y₂ is a single bond, and
L is a linking group represented by Chemical Formula 2b.

Preferably, A is the Chemical Formula A4,
R₂ is a C₁₋₄ alkyl or a C₁₋₄ haloalkyl,
Y₂ is a single bond, and
L is a linking group represented by Chemical Formula 2b.

Preferably, A is the Chemical Formulas A5 or A6,
R₂ is hydrogen, halogen, a C₁₋₄ alkyl, a C₁₋₄ haloalkyl, a C₁₋₄ alkoxy, or a C₁₋₄ haloalkoxy,
Y₂ is -O-, and
L is a linking group represented by Chemical Formula 2i.

Preferably, A is the Chemical Formula A7,
R₂ is halogen, a C₁₋₄ alkyl or a C₁₋₄ haloalkyl,
Y₂ is a single bond, and
L is a linking group represented by Chemical Formula 2b.

Preferably, A is the Chemical Formula A8,
R₂ is hydrogen, halogen, a C₁₋₄ alkyl, a C₁₋₄ haloalkyl, a C₁₋₄ alkoxy, or a C₁₋₄ haloalkoxy,
Y₂ is a single bond or -O-, and
L is a linking group represented by Chemical Formulas 2b or 2i.

Preferably, A is the Chemical Formula A9,
R₂ is hydrogen, halogen, a C₁₋₄ alkyl, a C₁₋₄ haloalkyl, a C₁₋₄ alkoxy, or a C₁₋₄ haloalkoxy,
Y₂ is -O-, and
L is a linking group represented by Chemical Formula 2i.

Preferably, A is the Chemical Formula A10,
R₂ is a C₁₋₄ alkoxy or a C₁₋₄ haloalkoxy,
Y₂ is -O-, and
L is a linking group represented by Chemical Formula 2i.

Preferably, A is the Chemical Formula A11,
R₂ is a C₁₋₄ alkyl or a C₁₋₄ haloalkyl,
Y₂ is -O-, and
L is a linking group represented by Chemical Formula 2i.

Preferably, A is the Chemical Formula A12,
R₂ is each independently halogen, a C₁₋₄ alkyl, or a C₁₋₄ haloalkyl,
Y₂ is -O-, and
L is a linking group represented by Chemical Formula 2i.

Meanwhile, representative examples of the compound represented by Chemical Formula 1 are as follows:
1) 1-(4-(1-methyl-6-((5-methylthiazol-2-yl)amino)-1H-pyrrolo[3,2-c]pyridin-4-yl)-3,6-dihydropyridin-1(2H)-yl)prop-2-en-1-one,
2) 1-(4-(1-isopropyl-6-((5-methylthiazol-2-yl)amino)-1H-pyrrolo[3,2-c]pyridin-4-yl)-3,6-dihydropyridin-1(2H)-yl)prop-2-en-1-one,
3) 1-(3-(1-methyl-6-((5-methylthiazol-2-yl)amino)-1H-pyrrolo[3,2-c]pyridin-4-yl)-2,5-dihydro-1H-pyrrol-1-yl)prop-2-en-1-one,
4) 1-(4-(1-(cyclopropylmethyl)-6-((5-methylthiazol-2-yl)amino)-1H-pyrrolo[3,2-c]pyridin-4-yl)-3,6-dihydropyridin-1(2H)-yl)prop-2-en-1-one,
5) 1-(4-(1-cyclopentyl-6-((5-methylthiazol-2-yl)amino)-1H-pyrrolo[3,2-c]pyridin-4-yl)-3,6-dihydropyridin-1(2H)-yl)prop-2-en-1-one,
6) 1-(4-(1-isobutyl-6-((5-methylthiazol-2-yl)amino)-1H-pyrrolo[3,2-c]pyridin-4-yl)-3,6-dihydropyridin-1(2H)-yl)prop-2-en-1-one,
7) 1-(4-(1-(cyclobutylmethyl)-6-((5-methylthiazol-2-yl)amino)-1H-pyrrolo[3,2-c]pyridin-4-yl)-3,6-dihydropyridin-1(2H)-yl)prop-2-en-1-one,
8) 1-(4-(6-((5-methylthiazol-2-yl)amino)-1-((tetrahydrofuran-3-yl)methyl)-1H-pyrrolo[3,2-c]pyridin-4-yl)-3,6-dihydropyridin-1(2H)-yl)prop-2-en-1-one,
9) 1-(4-(1-(cyclohexylmethyl)-6-((5-methylthiazol-2-yl)amino)-1H-pyrrolo[3,2-c]pyridin-4-yl)-3,6-dihydropyridin-1(2H)-yl)prop-2-en-1-one,
10) 1-(4-(1-(cyclopentylmethyl)-6-((5-methylthiazol-2-yl)amino)-1H-pyrrolo[3,2-c]pyridin-4-yl)-3,6-dihydropyridin-1(2H)-yl)prop-2-en-1-one,
11) 1-(4-(1-((4,4-difluorocyclohexyl)methyl)-6-((5-methylthiazol-2-yl)amino)-1H-pyrrolo[3,2-c]pyridin-4-yl)-3,6-dihydropyridin-1 (2H)-yl)prop-2-en-1-one,
12) 1-(4-(1-(2,2-difluoroethyl)-6-((5-methylthiazol-2-yl)amino)-1H-pyrrolo[3,2-c]pyridin-4-yl)-3,6-dihydropyridin-1(2H)-yl)prop-2-en-1-one,
13) (S)-1-(4-(1-(sec-butyl)-6-((5-methylthiazol-2-yl)amino)-1H-pyrrolo[3,2-c]pyridin-4-yl)-3,6-dihydropyridin-1(2H)-yl)prop-2-en-1-one,
14) (R)-1-(4-(1-(sec-butyl)-6-((5-methylthiazol-2-yl)amino)-1H-pyrrolo[3,2-c]pyridin-4-yl)-3,6-dihydropyridin-1(2H)-yl)prop-2-en-1-one,
15) 1-(4-(1-benzyl-6-((5-methylthiazol-2-yl)amino)-1H-pyrrolo[3,2-c]pyridin-4-yl)-3,6-dihydropyridin-1(2H)-yl)prop-2-en-1-one,
16) 1-(4-(6-((5-methylthiazol-2-yl)amino)-1-phenethyl-1H-pyrrolo[3,2-c]pyridin-4-yl)-3,6-dihydropyridin-1(2H)-yl)prop-2-en-1-one,
17) 1-(4-(6-((5-methylthiazol-2-yl)amino)-1-((1-(trifluoromethyl)cyclopropyl)methyl)-1H-pyrrolo[3,2-c]pyridin-4-yl)-3,6-dihydropyridin-1 (2H)-yl)prop-2-en-1-one,
18) 1-(4-(1-(3,3-dimethylbutyl)-6-((5-methylthiazol-2-yl)amino)-1H-pyrrolo[3,2-c]pyridin-4-yl)-3,6-dihydropyridin-1(2H)-yl)prop-2-en-1-one,
19) 1-(4-(6-((5-methylthiazol-2-yl)amino)-1-(2,2,2-trifluoroethyl)-1H-pyrrolo[3,2-c]pyridin-4-yl)-3,6-dihydropyridin-1(2H)-yl)prop-2-en-1-one,
20) 1-(4-(1-(2,2-difluorocyclopropyl)-6-((5-methylthiazol-2-yl)amino)-1H-pyrrolo[3,2-c]pyridin-4-yl)-3,6-dihydropyridin-1(2H)-yl)prop-2-en-1-one,
21) 1-(4-(1-(difluoromethyl)-6-((5-methylthiazol-2-yl)amino)-1H-pyrrolo[3,2-c]pyridin-4-yl)-3,6-dihydropyridin-1(2H)-yl)prop-2-en-1-one,
22) 2-(4-(1-acryloyl-1,2,3,6-tetrahydropyridin-4-yl)-6-((5-methylthiazol-2-yl)amino)-1H-pyrrolo[3,2-c]pyridin-1-yl)-2-methylpropanenitrile,
23) 2-(4-(1-acryloyl-1,2,5,6-tetrahydropyridin-3-yl)-6-((5-methylthiazol-2-yl)amino)-1H-pyrrolo[3,2-c]pyridin-1-yl)-2-methylpropanenitrile,
24) (R)-1-(4-(6-((5-methylthiazol-2-yl)amino)-1-(1,1,1-trifluoropropan-2-yl)-1H-pyrrolo[3,2-c]pyridin-4-yl)-3,6-dihydropyridin-1 (2H)-yl)prop-2-en-1-one,
25) 1-(4-(1-(2,2-difluoroethyl)-6-((5-methylthiazol-2-yl)amino)-1H-pyrrolo[3,2-c]pyridin-4-yl)-3-methyl-3,6-dihydropyridin-1(2H)-yl)prop-2-en-1-one,
26) 1-(4-(1-isopropyl-6-((5-methylthiazol-2-yl)amino)-1H-pyrrolo[3,2-c]pyridin-4-yl)-2,3,6,7-tetrahydro-1H-azepin-1-yl)prop-2-en-1-one,
27) 1-(7-(1-isopropyl-6-((5-methylthiazol-2-yl)amino)-1H-pyrrolo[3,2-c]pyridin-4-yl)-3-oxa-9-azabicyclo[3.3.1]non-6-en-9-yl)prop-2-en-1-one,
28) 1-(3-(1-methyl-6-((5-methylthiazol-2-yl)amino)-1H-pyrrolo[3,2-c]pyridin-4-yl)-8-azabicyclo[3.2.1]oct-2-en-8-yl)prop-2-en-1-one,
29) 1-(3-(1-isopropyl-6-((5-methylthiazol-2-yl)amino)-1H-pyrrolo[3,2-c]pyridin-4-yl)-8-azabicyclo[3.2.1]oct-2-en-8-yl)prop-2-en-1-one,
30) 1-(3-(6-((5-methylthiazol-2-yl)amino)-1-((1-(trifluoromethyl)cyclopropyl)methyl)-1H-pyrrolo[3,2-c]pyridin-4-yl)-8-azabicyclo[3.2.1]oct-2-en-8-yl)prop-2-en-1-one,
31) 1-(3-(1-(2,2-difluorocyclopropyl)-6-((5-methylthiazol-2-yl)amino)-1H-pyrrolo[3,2-c]pyridin-4-yl)-8-azabicyclo[3.2.1]oct-2-en-8-yl)prop-2-en-1-one,
32) 1-(3-(1-isobutyl-6-((5-methylthiazol-2-yl)amino)-1H-pyrrolo[3,2-c]pyridin-4-yl)-8-azabicyclo[3.2.1]oct-2-en-8-yl)prop-2-en-1-one,
33) 1-(3-(1-cyclopentyl-6-((5-methylthiazol-2-yl)amino)-1H-pyrrolo[3,2-c]pyridin-4-yl)-8-azabicyclo[3.2.1]oct-2-en-8-yl)prop-2-en-1-one,
34) 1-(3-(1-(2,2-difluoroethyl)-6-((5-methylthiazol-2-yl)amino)-1H-pyrrolo[3,2-c]pyridin-4-yl)-8-azabicyclo[3.2.1]oct-2-en-8-yl)prop-2-en-1-one,
35) 1-(3-(6-((5-methylthiazol-2-yl)amino)-1-(2,2,2-trifluoroethyl)-1H-pyrrolo[3,2-c]pyridin-4-yl)-8-azabicyclo[3.2.1]oct-2-en-8-yl)prop-2-en-1-one,
36) 1-(3-(1-methyl-6-((5-methylthiazol-2-yl)amino)-1H-pyrrolo[3,2-c]pyridin-4-yl)-9-azabicyclo[3.3.1]non-2-en-9-yl)prop-2-en-1-one,
37) 1-(3-(1-isopropyl-6-((5-methylthiazol-2-yl)amino)-1H-pyrrolo[3,2-c]pyridin-4-yl)-9-azabicyclo[3.3.1]non-2-en-9-yl)prop-2-en-1-one,
38) 1-(3-(1-isobutyl-6-((5-methylthiazol-2-yl)amino)-1H-pyrrolo[3,2-c]pyridin-4-yl)-9-azabicyclo[3.3.1]non-2-en-9-yl)prop-2-en-1-one,
39) 1-(3-(1-(cyclopropylmethyl)-6-((5-methylthiazol-2-yl)amino)-1H-pyrrolo[3,2-c]pyridin-4-yl)-8-azabicyclo[3.2.1]oct-2-en-8-yl)prop-2-en-1-one,
40) 1-(4-(6-((1H-imidazol-2-yl)amino)-1-methyl-1H-pyrrolo[3,2-c]pyridin-4-yl)-3,6-dihydropyridin-1(2H)-yl)prop-2-en-1-one,
41) 1-(4-(1-isopropyl-6-((5-methyl-1,3,4-thiadiazol-2-yl)amino)-1H-pyrrolo[3,2-c]pyridin-4-yl)-3,6-dihydropyridin-1(2H)-yl)prop-2-en-1-one,
42) 1-(4-(1-isopropyl-6-((4-methylthiazol-2-yl)amino)-1H-pyrrolo[3,2-c]pyridin-4-yl)-3,6-dihydropyridin-1(2H)-yl)prop-2-en-1-one,
43) 1-(4-(1-isopropyl-6-(pyrimidin-4-ylamino)-1H-pyrrolo[3,2-c]pyridin-4-yl)-3,6-dihydropyridin-1(2H)-yl)prop-2-en-1-one,
44) 1-(4-(1-isopropyl-6-((6-methylpyrimidin-4-yl)amino)-1H-pyrrolo[3,2-c]pyridin-4-yl)-3,6-dihydropyridin-1(2H)-yl)prop-2-en-1-one,
45) 1-(4-(1-isopropyl-6-((6-(trifluoromethyl)pyrimidin-4-yl)amino)-1H-pyrrolo[3,2-c]pyridin-4-yl)-3,6-dihydropyridin-1(2H)-yl)prop-2-en-1-one,
46) 1-(4-(1-isopropyl-6-((6-methoxypyrimidin-4-yl)amino)-1H-pyrrolo[3,2-c]pyridin-4-yl)-3,6-dihydropyridin-1(2H)-yl)prop-2-en-1-one,
47) 1-(4-(6-((4-fluoropyridin-2-yl)amino)-1-isopropyl-1H-pyrrolo[3,2-c]pyridin-4-yl)-3,6-dihydropyridin-1(2H)-yl)prop-2-en-1-one,
48) 1-(4-(1-isopropyl-6-((4-(trifluoromethyl)pyridin-2-yl)amino)-1H-pyrrolo[3,2-c]pyridin-4-yl)-3,6-dihydropyridin-1(2H)-yl)prop-2-en-1-one,
49) 1-(4-(1-isopropyl-6-((4-methylpyridin-2-yl)amino)-1H-pyrrolo[3,2-c]pyridin-4-yl)-3,6-dihydropyridin-1(2H)-yl)prop-2-en-1-one,
50) N-(3-(1-methyl-6-((5-methylthiazol-2-yl)amino)-1H-pyrrolo[3,2-c]pyridin-4-yl)phenyl)acrylamide,
51) N-(2-fluoro-3-(1-methyl-6-((5-methylthiazol-2-yl)amino)-1H-pyrrolo[3,2-c]pyridin-4-yl)phenyl)acrylamide,
52) N-(2-methoxy-3-(1-methyl-6-((5-methylthiazol-2-yl)amino)-1H-pyrrolo[3,2-c]pyridin-4-yl)phenyl)acrylamide,
53) N-(3-methyl-5-(1-methyl-6-((5-methylthiazol-2-yl)amino)-1H-pyrrolo[3,2-c]pyridin-4-yl)phenyl)acrylamide,
54) N-(2-fluoro-5-(1-methyl-6-((5-methylthiazol-2-yl)amino)-1H-pyrrolo[3,2-c]pyridin-4-yl)phenyl)acrylamide,
55) N-(2-methyl-5-(1-methyl-6-((5-methylthiazol-2-yl)amino)-1H-pyrrolo[3,2-c]pyridin-4-yl)phenyl)acrylamide,
56) N-(2,6-dimethyl-3-(1-methyl-6-((5-methylthiazol-2-yl)amino)-1H-pyrrolo[3,2-c]pyridin-4-yl)phenyl)acrylamide,
57) N-(2,6-difluoro-3-(1-methyl-6-((5-methylthiazol-2-yl)amino)-1H-pyrrolo[3,2-c]pyridin-4-yl)phenyl)acrylamide,
58) N-(3-(1-isopropyl-6-((5-methylthiazol-2-yl)amino)-1H-pyrrolo[3,2-c]pyridin-4-yl)-2-methylphenyl)acrylamide,
59) N-(4-fluoro-3-(1-isopropyl-6-((5-methylthiazol-2-yl)amino)-1H-pyrrolo[3,2-c]pyridin-4-yl)phenyl)acrylamide,
60) N-(2,4-difluoro-5-(1-isopropyl-6-((5-methylthiazol-2-yl)amino)-1H-pyrrolo[3,2-c]pyridin-4-yl)phenyl)acrylamide,
61) N-(3-(1-(cyclopropylmethyl)-6-((5-methylthiazol-2-yl)amino)-1H-pyrrolo[3,2-c]pyridin-4-yl)-4-fluorophenyl)acrylamide,
62) N-(3-(1-isobutyl-6-((5-methylthiazol-2-yl)amino)-1H-pyrrolo[3,2-c]pyridin-4-yl)-2-methylphenyl)acrylamide,
63) N-(4-fluoro-3-(1-isobutyl-6-((5-methylthiazol-2-yl)amino)-1H-pyrrolo[3,2-c]pyridin-4-yl)phenyl)acrylamide,
64) N-(5-(1-(cyclopropylmethyl)-6-((5-methylthiazol-2-yl)amino)-1H-pyrrolo[3,2-c]pyridin-4-yl)-2-fluorophenyl)acrylamide,
65) N-(5-(1-(cyclopropylmethyl)-6-((5-methylthiazol-2-yl)amino)-1H-pyrrolo[3,2-c]pyridin-4-yl)-2-methylphenyl)acrylamide,
66) N-(5-(1-(cyclopropylmethyl)-6-((5-methylthiazol-2-yl)amino)-1H-pyrrolo[3,2-c]pyridin-4-yl)-2,4-difluorophenyl)acrylamide,
67) N-(2,4-difluoro-5-(1-isobutyl-6-((5-methylthiazol-2-yl)amino)-1H-pyrrolo[3,2-c]pyridin-4-yl)phenyl)acrylamide,
68) (S)-N-(3-(1-(sec-butyl)-6-((5-methylthiazol-2-yl)amino)-1H-pyrrolo[3,2-c]pyridin-4-yl)-4-fluorophenyl)acrylamide,
69) N-(5-(1-(3,3-dimethylbutyl)-6-((5-methylthiazol-2-yl)amino)-1H-pyrrolo[3,2-c]pyridin-4-yl)-2,4-difluorophenyl)acrylamide,
70) N-(3-(1-cyclopentyl-6-((5-methylthiazol-2-yl)amino)-1H-pyrrolo[3,2-c]pyridin-4-yl)-4-fluorophenyl)acrylamide,
71) N-(4-fluoro-3-(6-((5-methylthiazol-2-yl)amino)-1-((tetrahydrofuran-3-yl)methyl)-1H-pyrrolo[3,2-c]pyridin-4-yl)phenyl)acrylamide,
72) N-(2-fluoro-5-(6-((5-methylthiazol-2-yl)amino)-1-((tetrahydrofuran-3-yl)methyl)-1H-pyrrolo[3,2-c]pyridin-4-yl)phenyl)acrylamide,
73) N-(2-fluoro-3-(6-((5-methylthiazol-2-yl)amino)-1-((tetrahydro-2H-pyran-4-yl)methyl)-1H-pyrrolo[3,2-c]pyridin-4-yl)phenyl)acrylamide,
74) N-(5-(1-(2,2-difluoroethyl)-6-((5-methylthiazol-2-yl)amino)-1H-pyrrolo[3,2-c]pyridin-4-yl)-2-methylphenyl)acrylamide,
75) N-(3-(1-(2,2-difluoroethyl)-6-((5-methylthiazol-2-yl)amino)-1H-pyrrolo[3,2-c]pyridin-4-yl)-4-fluorophenyl)acrylamide,
76) N-(5-(1-(2,2-difluoroethyl)-6-((5-methylthiazol-2-yl)amino)-1H-pyrrolo[3,2-c]pyridin-4-yl)-2-fluorophenyl)acrylamide,
77) N-(2,4-difluoro-5-(6-((5-methylthiazol-2-yl)amino)-1-(2,2,2-trifluoroethyl)-1H-pyrrolo[3,2-c]pyridin-4-yl)phenyl)acrylamide,
78) N-(4-fluoro-3-(6-((5-methylthiazol-2-yl)amino)-1-(2,2,2-trifluoroethyl)-1H-pyrrolo[3,2-c]pyridin-4-yl)phenyl)acrylamide,
79) (S)-1-(3-((1-isopropyl-6-((5-methylthiazol-2-yl)amino)-1H-pyrrolo[3,2-c]pyridin-4-yl)oxy)pyrrolidin-1-yl)prop-2-en-1-one,
80) 1-(3-((1-isopropyl-6-((5-methylthiazol-2-yl)amino)-1H-pyrrolo[3,2-c]pyridin-4-yl)oxy)azetidin-1-yl)prop-2-en-1-one,
81) (S)-1-(3-((1-isobutyl-6-((5-methylthiazol-2-yl)amino)-1H-pyrrolo[3,2-c]pyridin-4-yl)oxy)pyrrolidin-1-yl)prop-2-en-1-one,
82) (R)-1-(3-((1-isobutyl-6-((5-methylthiazol-2-yl)amino)-1H-pyrrolo[3,2-c]pyridin-4-yl)oxy)pyrrolidin-1-yl)prop-2-en-1-one,
83) 1-((3S,4R)-3-fluoro-4-((1-isopropyl-6-((5-methylthiazol-2-yl)amino)-1H-pyrrolo[3,2-c]pyridin-4-yl)oxy)pyrrolidin-1-yl)prop-2-en-1-one,
84) (S)-1-(3-((1-isopropyl-6-((5-methylthiazol-2-yl)amino)-1H-pyrrolo[3,2-c]pyridin-4-yl)oxy)piperidin-1-yl)prop-2-en-1-one,
85) (S)-1-(2-(((1-isobutyl-6-((5-methylthiazol-2-yl)amino)-1H-pyrrolo[3,2-c]pyridin-4-yl)oxy)methyl)pyrrolidin-1-yl)prop-2-en-1-one,
86) (S)-1-(2-(((1-isopropyl-6-((5-methylthiazol-2-yl)amino)-1H-pyrrolo[3,2-c]pyridin-4-yl)oxy)methyl)azetidin-1-yl)prop-2-en-1-one,
87) 1-((2R,4S)-4-((1-isopropyl-6-((5-methylthiazol-2-yl)amino)-1H-pyrrolo[3,2-c]pyridin-4-yl)oxy)-2-methylpyrrolidin-1-yl)prop-2-en-1-one,
88) 1-((2S,4S)-4-((1-isopropyl-6-((5-methylthiazol-2-yl)amino)-1H-pyrrolo[3,2-c]pyridin-4-yl)oxy)-2-methylpyrrolidin-1-yl)prop-2-en-1-one,
89) (R)-1-(2-(((1-isopropyl-6-((5-methylthiazol-2-yl)amino)-1H-pyrrolo[3,2-c]pyridin-4-yl)oxy)methyl)pyrrolidin-1-yl)prop-2-en-1-one,
90) (R)-1-(2-(((1-isopropyl-6-((5-methylthiazol-2-yl)amino)-1H-pyrrolo[3,2-c]pyridin-4-yl)oxy)methyl)azetidin-1-yl)prop-2-en-1-one,
91) 1-((3R,4R)-3-fluoro-4-((1-isopropyl-6-((5-methylthiazol-2-yl)amino)-1H-pyrrolo[3,2-c]pyridin-4-yl)oxy)pyrrolidin-1-yl)prop-2-en-1-one,
92) rac-1-((3S,4R)-3-((1-isopropyl-6-((5-methylthiazol-2-yl)amino)-1H-pyrrolo[3,2-c]pyridin-4-yl)oxy)-4-methylpyrrolidin-1-yl)prop-2-en-1-one,
93) (R)-1-(4,4-difluoro-2-(((1-isopropyl-6-((5-methylthiazol-2-yl)amino)-1H-pyrrolo[3,2-c]pyridin-4-yl)oxy)methyl)pyrrolidin-1-yl)prop-2-en-1-one,
94) 1-((2R,4S)-2-(((1-isopropyl-6-((5-methylthiazol-2-yl)amino)-1H-pyrrolo[3,2-c]pyridin-4-yl)oxy)methyl)-4-methoxypyrrolidin-1-yl)prop-2-en-1-one,
95) (S)-1-(3-((1-isopropyl-6-((6-(trifluoromethyl)pyrimidin-4-yl)amino)-1H-pyrrolo[3,2-c]pyridin-4-yl)oxy)pyrrolidin-1-yl)prop-2-en-1-one,
96) 1-((2R,4S)-4-((1-isobutyl-6-((5-methylthiazol-2-yl)amino)-1H-pyrrolo[3,2-c]pyridin-4-yl)oxy)-2-methylpyrrolidin-1-yl)prop-2-en-1-one,
97) (S)-1-(3-((1-(cyclopropylmethyl)-6-((5-methylthiazol-2-yl)amino)-1H-pyrrolo[3,2-c]pyridin-4-yl)oxy)pyrrolidin-1-yl)prop-2-en-1-one,
98) 1-((2R,4S)-4-((1-(cyclopropylmethyl)-6-((5-methylthiazol-2-yl)amino)-1H-pyrrolo[3,2-c]pyridin-4-yl)oxy)-2-methylpyrrolidin-1-yl)prop-2-en-1-one,
99) (S)-1-(3-((1-cyclopentyl-6-((5-methylthiazol-2-yl)amino)-1H-pyrrolo[3,2-c]pyridin-4-yl)oxy)pyrrolidin-1-yl)prop-2-en-1-one,
100) 1-((2R,4S)-4-((1-cyclopentyl-6-((5-methylthiazol-2-yl)amino)-1H-pyrrolo[3,2-c]pyridin-4-yl)oxy)-2-methylpyrrolidin-1-yl)prop-2-en-1-one,
101) (S)-1-(3-((1-isopropyl-6-((6-methylpyrimidin-4-yl)amino)-1H-pyrrolo[3,2-c]pyridin-4-yl)oxy)pyrrolidin-1-yl)prop-2-en-1-one,
102) (S)-1-(3-((6-((5-methylthiazol-2-yl)amino)-1-((1-(trifluoromethyl)cyclopropyl)methyl)-1H-pyrrolo[3,2-c]pyridin-4-yl)oxy)pyrrolidin-1-yl)prop-2-en-1-one,
103) (S)-1-(3-((1-isopropyl-6-((6-methoxypyrimidin-4-yl)amino)-1H-pyrrolo[3,2-c]pyridin-4-yl)oxy)pyrrolidin-1-yl)prop-2-en-1-one,
104) (S)-1-(3-((1-isobutyl-6-((6-methylpyrimidin-4-yl)amino)-1H-pyrrolo[3,2-c]pyridin-4-yl)oxy)pyrrolidin-1-yl)prop-2-en-1-one,
105) 1-((3S,4R)-3-fluoro-4-((1-isobutyl-6-((5-methylthiazol-2-yl)amino)-1H-pyrrolo[3,2-c]pyridin-4-yl)oxy)pyrrolidin-1-yl)prop-2-en-1-one,
106) 1-((3R,4R)-3-fluoro-4-((1-isobutyl-6-((5-methylthiazol-2-yl)amino)-1H-pyrrolo[3,2-c]pyridin-4-yl)oxy)pyrrolidin-1-yl)prop-2-en-1-one,
107) 1-((3R,4S)-3-((1-(cyclopropylmethyl)-6-((5-methylthiazol-2-yl)amino)-1H-pyrrolo[3,2-c]pyridin-4-yl)oxy)-4-fluoropyrrolidin-1-yl)prop-2-en-1-one,
108) 1-((3R,4R)-3-((1-(cyclopropylmethyl)-6-((5-methylthiazol-2-yl)amino)-1H-pyrrolo[3,2-c]pyridin-4-yl)oxy)-4-fluoropyrrolidin-1-yl)prop-2-en-1-one,
109) 1-((3R,4S)-3-((1-cyclopentyl-6-((5-methylthiazol-2-yl)amino)-1H-pyrrolo[3,2-c]pyridin-4-yl)oxy)-4-fluoropyrrolidin-1-yl)prop-2-en-1-one,
110) 1-((3R,4R)-3-((1-cyclopentyl-6-((5-methylthiazol-2-yl)amino)-1H-pyrrolo[3,2-c]pyridin-4-yl)oxy)-4-fluoropyrrolidin-1-yl)prop-2-en-1-one,
111) 1-((S)-3-((1-((S)-sec-butyl)-6-((5-methylthiazol-2-yl)amino)-1H-pyrrolo[3,2-c]pyridin-4-yl)oxy)pyrrolidin-1-yl)prop-2-en-1-one,
112) 1-((3R,4S)-3-((1-((S)-sec-butyl)-6-((5-methylthiazol-2-yl)amino)-1H-pyrrolo[3,2-c]pyridin-4-yl)oxy)-4-fluoropyrrolidin-1-yl)prop-2-en-1-one,
113) 1-((3R,4R)-3-((1-((S)-sec-butyl)-6-((5-methylthiazol-2-yl)amino)-1H-pyrrolo[3,2-c]pyridin-4-yl)oxy)-4-fluoropyrrolidin-1-yl)prop-2-en-1-one,
114) (S)-1-(3-((1-isopentyl-6-((5-methylthiazol-2-yl)amino)-1H-pyrrolo[3,2-c]pyridin-4-yl)oxy)pyrrolidin-1-yl)prop-2-en-1-one,
115) 1-((3R,4R)-3-fluoro-4-((1-isopentyl-6-((5-methylthiazol-2-yl)amino)-1H-pyrrolo[3,2-c]pyridin-4-yl)oxy)pyrrolidin-1-yl)prop-2-en-1-one,
116) 1-(3-((1-isobutyl-6-((5-methylthiazol-2-yl)amino)-1H-pyrrolo[3,2-c]pyridin-4-yl)oxy)-4-methylpyrrolidin-1-yl)prop-2-en-1-one,
117) (S)-1-(3-((6-((5-methylthiazol-2-yl)amino)-1-(pyridin-2-ylmethyl)-1H-pyrrolo[3,2-c]pyridin-4-yl)oxy)pyrrolidin-1-yl)prop-2-en-1-one,
118) 1-(3-((1-isobutyl-6-((5-methylthiazol-2-yl)amino)-1H-pyrrolo[3,2-c]pyridin-4-yl)oxy)azetidin-1-yl)prop-2-en-1-one,
119) 1-(3-((1-isobutyl-6-((5-methylthiazol-2-yl)amino)-1H-pyrrolo[3,2-c]pyridin-4-yl)oxy)-3-methylazetidin-1-yl)prop-2-en-1-one,
120) 1-((3R,4R)-3-fluoro-4-((6-((5-methylthiazol-2-yl)amino)-1-((1-(trifluoromethyl)cyclopropyl)methyl)-1H-pyrrolo[3,2-c]pyridin-4-yl)oxy)pyrrolidin-1-yl)prop-2-en-1-one,
121) (S)-1-(3-((1-benzyl-6-((5-methylthiazol-2-yl)amino)-1H-pyrrolo[3,2-c]pyridin-4-yl)oxy)pyrrolidin-1-yl)prop-2-en-1-one,
122) (S)-1-(3-((6-((5-ethylthiazol-2-yl)amino)-1-isobutyl-1H-pyrrolo[3,2-c]pyridin-4-yl)oxy)pyrrolidin-1-yl)prop-2-en-1-one,
123) 1-((3S)-3-((1-(2-methylbutyl)-6-((5-methylthiazol-2-yl)amino)-1H-pyrrolo[3,2-c]pyridin-4-yl)oxy)pyrrolidin-1-yl)prop-2-en-1-one,
124) (S)-1-(3-((1-butyl-6-((5-methylthiazol-2-yl)amino)-1H-pyrrolo[3,2-c]pyridin-4-yl)oxy)pyrrolidin-1-yl)prop-2-en-1-one,
125) (S)-1-(3-((1-ethyl-6-((5-methylthiazol-2-yl)amino)-1H-pyrrolo[3,2-c]pyridin-4-yl)oxy)pyrrolidin-1-yl)prop-2-en-1-one,
126) (S)-1-(3-((6-((5-methylthiazol-2-yl)amino)-1-propyl-1H-pyrrolo[3,2-c]pyridin-4-yl)oxy)pyrrolidin-1-yl)prop-2-en-1-one,
127) (S)-1-(3-((1-(2-methoxyethyl)-6-((5-methylthiazol-2-yl)amino)-1H-pyrrolo[3,2-c]pyridin-4-yl)oxy)pyrrolidin-1-yl)prop-2-en-1-one,
128) (S)-1-(3-((1-(cyclopentylmethyl)-6-((5-methylthiazol-2-yl)amino)-1H-pyrrolo[3,2-c]pyridin-4-yl)oxy)pyrrolidin-1-yl)prop-2-en-1-one,
129) (S)-1-(3-((1-(cyclohexylmethyl)-6-((5-methylthiazol-2-yl)amino)-1H-pyrrolo[3,2-c]pyridin-4-yl)oxy)pyrrolidin-1-yl)prop-2-en-1-one,
130) (S)-1-(3-((1-methyl-6-((5-methylthiazol-2-yl)amino)-1H-pyrrolo[3,2-c]pyridin-4-yl)oxy)pyrrolidin-1-yl)prop-2-en-1-one,
131) 1-((3S,4R)-3-fluoro-4-((1-methyl-6-((5-methylthiazol-2-yl)amino)-1H-pyrrolo[3,2-c]pyridin-4-yl)oxy)pyrrolidin-1-yl)prop-2-en-1-one,
132) 1-((3R,4S)-3-((1-(cyclopentylmethyl)-6-((5-methylthiazol-2-yl)amino)-1H-pyrrolo[3,2-c]pyridin-4-yl)oxy)-4-fluoropyrrolidin-1-yl)prop-2-en-1-one,
133) (S)-1-(3-((6-((5-methylthiazol-2-yl)amino)-1-((tetrahydro-2H-pyran-4-yl)methyl)-1H-pyrrolo[3,2-c]pyridin-4-yl)oxy)pyrrolidin-1-yl)prop-2-en-1-one,
134) 1-((3S,4S)-3-fluoro-4-((1-isobutyl-6-((5-methylthiazol-2-yl)amino)-1H-pyrrolo[3,2-c]pyridin-4-yl)oxy)pyrrolidin-1-yl)prop-2-en-1-one,
135) 1-((3R,4S)-3-fluoro-4-((1-isobutyl-6-((5-methylthiazol-2-yl)amino)-1H-pyrrolo[3,2-c]pyridin-4-yl)oxy)pyrrolidin-1-yl)prop-2-en-1-one,
136) 1-(6-((1-methyl-6-((5-methylthiazol-2-yl)amino)-1H-pyrrolo[3,2-c]pyridin-4-yl)oxy)-1-azaspiro[3.3]heptan-1-yl)prop-2-en-1-one,
137) 1-(6-((1-isobutyl-6-((5-methylthiazol-2-yl)amino)-1H-pyrrolo[3,2-c]pyridin-4-yl)oxy)-1-azaspiro[3.3]heptan-1-yl)prop-2-en-1-one,
138) (S)-1-(3-((6-((5-fluoro-2-methylphenyl)amino)-1-methyl-1H-pyrrolo[3,2-c]pyridin-4-yl)oxy)pyrrolidin-1-yl)prop-2-en-1-one,
139) 1-((3S,4R)-3-fluoro-4-((6-((5-fluoro-2-methylphenyl)amino)-1-methyl-1H-pyrrolo[3,2-c]pyridin-4-yl)oxy)pyrrolidin-1-yl)prop-2-en-1-one,
140) (S)-1-(3-((1-(2-hydroxy-2-methylpropyl)-6-((5-methylthiazol-2-yl)amino)-1H-pyrrolo[3,2-c]pyridin-4-yl)oxy)pyrrolidin-1-yl)prop-2-en-1-one,
141) 1-((3S,4R)-3-fluoro-4-((1-(2-hydroxy-2-methylpropyl)-6-((5-methylthiazol-2-yl)amino)-1H-pyrrolo[3,2-c]pyridin-4-yl)oxy)pyrrolidin-1-yl)prop-2-en-1-one,
142) 1-(3-((1-cyclopentyl-6-((5-methylthiazol-2-yl)amino)-1H-pyrrolo[3,2-c]pyridin-4-yl)oxy)azetidin-1-yl)prop-2-en-1-one,
143) (R)-1-(3-((1-cyclopentyl-6-((5-methylthiazol-2-yl)amino)-1H-pyrrolo[3,2-c]pyridin-4-yl)oxy)pyrrolidin-1-yl)prop-2-en-1-one,
144) (R)-1-(3-((1-(cyclopropylmethyl)-6-((5-methylthiazol-2-yl)amino)-1H-pyrrolo[3,2-c]pyridin-4-yl)oxy)pyrrolidin-1-yl)prop-2-en-1-one,
145) 1-(3-((1-(cyclopropylmethyl)-6-((5-methylthiazol-2-yl)amino)-1H-pyrrolo[3,2-c]pyridin-4-yl)oxy)azetidin-1-yl)prop-2-en-1-one,
146) 1-(3-((1-(2-methoxyethyl)-6-((5-methylthiazol-2-yl)amino)-1H-pyrrolo[3,2-c]pyridin-4-yl)oxy)azetidin-1-yl)prop-2-en-1-one,
147) 1-((3S,4R)-3-fluoro-4-((1-(2-methoxyethyl)-6-((5-methylthiazol-2-yl)amino)-1H-pyrrolo[3,2-c]pyridin-4-yl)oxy)pyrrolidin-1-yl)prop-2-en-1-one,
148) (S)-1-(3-((1-(2-methoxyethyl)-6-((5-methylthiazol-2-yl)amino)-1H-pyrrolo[3,2-c]pyridin-4-yl)oxy)piperidin-1-yl)prop-2-en-1-one,
149) (S)-1-(3-((1-(cyclopropylmethyl)-6-((5-methylthiazol-2-yl)amino)-1H-pyrrolo[3,2-c]pyridin-4-yl)oxy)piperidin-1-yl)prop-2-en-1-one,
150) (S)-1-(3-((1-isobutyl-6-((5-methylthiazol-2-yl)amino)-1H-pyrrolo[3,2-c]pyridin-4-yl)oxy)piperidin-1-yl)prop-2-en-1-one,
151) (S)-1-(3-((1-cyclopentyl-6-((5-methylthiazol-2-yl)amino)-1H-pyrrolo[3,2-c]pyridin-4-yl)oxy)piperidin-1-yl)prop-2-en-1-one,
152) 1-((3R,4S)-3-((1-ethyl-6-((5-methylthiazol-2-yl)amino)-1H-pyrrolo[3,2-c]pyridin-4-yl)oxy)-4-fluoropyrrolidin-1-yl)prop-2-en-1-one,
153) (S)-1-(3-((1-ethyl-6-((5-methylthiazol-2-yl)amino)-1H-pyrrolo[3,2-c]pyridin-4-yl)oxy)piperidin-1-yl)prop-2-en-1-one,
154) 1-((3R,4R)-3-((1-ethyl-6-((5-methylthiazol-2-yl)amino)-1H-pyrrolo[3,2-c]pyridin-4-yl)oxy)-4-fluoropyrrolidin-1-yl)prop-2-en-1-one,
155) 1-(3-((1-ethyl-6-((5-methylthiazol-2-yl)amino)-1H-pyrrolo[3,2-c]pyridin-4-yl)oxy)-3-methylazetidin-1-yl)prop-2-en-1-one,
156) (R)-1-(3-((1-methyl-6-((5-methylthiazol-2-yl)amino)-1H-pyrrolo[3,2-c]pyridin-4-yl)oxy)pyrrolidin-1-yl)prop-2-en-1-one,
157) 1-((3R,4R)-3-fluoro-4-((1-methyl-6-((5-methylthiazol-2-yl)amino)-1H-pyrrolo[3,2-c]pyridin-4-yl)oxy)pyrrolidin-1-yl)prop-2-en-1-one,
158) (S)-1-(3-((1-methyl-6-((5-methylthiazol-2-yl)amino)-1H-pyrrolo[3,2-c]pyridin-4-yl)oxy)piperidin-1-yl)prop-2-en-1-one,
159) 1-((3S,4R)-3-fluoro-4-((6-((5-methylthiazol-2-yl)amino)-1-propyl-1H-pyrrolo[3,2-c]pyridin-4-yl)oxy)pyrrolidin-1-yl)prop-2-en-1-one,
160) (S)-1-(3-((6-((5-methylthiazol-2-yl)amino)-1-propyl-1H-pyrrolo[3,2-c]pyridin-4-yl)oxy)piperidin-1-yl)prop-2-en-1-one,
161) 1-((3R,4R)-3-fluoro-4-((6-((5-methylthiazol-2-yl)amino)-1-propyl-1H-pyrrolo[3,2-c]pyridin-4-yl)oxy)pyrrolidin-1-yl)prop-2-en-1-one,
162) (R)-1-(3-((1-isopropyl-6-((5-methylthiazol-2-yl)amino)-1H-pyrrolo[3,2-c]pyridin-4-yl)oxy)pyrrolidin-1-yl)prop-2-en-1-one,
163) 1-(3-((1-isopropyl-6-((5-methylthiazol-2-yl)amino)-1H-pyrrolo[3,2-c]pyridin-4-yl)oxy)-3-methylazetidin-1-yl)prop-2-en-1-one,
164) 1-((2S,4S)-4-((1-cyclopentyl-6-((5-methylthiazol-2-yl)amino)-1H-pyrrolo[3,2-c]pyridin-4-yl)oxy)-2-methylpyrrolidin-1-yl)prop-2-en-1-one,
165) 1-(3-((1-cyclopentyl-6-((5-methylthiazol-2-yl)amino)-1H-pyrrolo[3,2-c]pyridin-4-yl)oxy)-3-methylazetidin-1-yl)prop-2-en-1-one,
166) 1-(3-methyl-3-((1-methyl-6-((5-methylthiazol-2-yl)amino)-1H-pyrrolo[3,2-c]pyridin-4-yl)oxy)azetidin-1-yl)prop-2-en-1-one,
167) 1-(3-((1-(cyclopropylmethyl)-6-((5-methylthiazol-2-yl)amino)-1H-pyrrolo[3,2-c]pyridin-4-yl)oxy)-3-methylazetidin-1-yl)prop-2-en-1-one,
168) 1-(3-((1-(2-methoxyethyl)-6-((5-methylthiazol-2-yl)amino)-1H-pyrrolo[3,2-c]pyridin-4-yl)oxy)-3-methylazetidin-1-yl)prop-2-en-1-one,
169) (S)-1-(3-((1-(cyclopentylmethyl)-6-((5-methylthiazol-2-yl)amino)-1H-pyrrolo[3,2-c]pyridin-4-yl)oxy)piperidin-1-yl)prop-2-en-1-one,
170) 1-((3R,4R)-3-((1-(cyclopentylmethyl)-6-((5-methylthiazol-2-yl)amino)-1H-pyrrolo[3,2-c]pyridin-4-yl)oxy)-4-fluoropyrrolidin-1-yl)prop-2-en-1-one,
171) 1-((3S,4R)-3-fluoro-4-((1-(2-methylbutyl)-6-((5-methylthiazol-2-yl)amino)-1H-pyrrolo[3,2-c]pyridin-4-yl)oxy)pyrrolidin-1-yl)prop-2-en-1-one,
172) 1-((3S)-3-((1-(2-methylbutyl)-6-((5-methylthiazol-2-yl)amino)-1H-pyrrolo[3,2-c]pyridin-4-yl)oxy)piperidin-1-yl)prop-2-en-1-one,
173) 1-((3R,4R)-3-fluoro-4-((1-(2-methylbutyl)-6-((5-methylthiazol-2-yl)amino)-1H-pyrrolo[3,2-c]pyridin-4-yl)oxy)pyrrolidin-1-yl)prop-2-en-1-one,
174) 1-((3R,4R)-3-fluoro-4-((1-((R)-2-methylbutyl)-6-((5-methylthiazol-2-yl)amino)-1H-pyrrolo[3,2-c]pyridin-4-yl)oxy)pyrrolidin-1-yl)prop-2-en-1-one,
175) 1-((3R,4R)-3-fluoro-4-((1-((S)-2-methylbutyl)-6-((5-methylthiazol-2-yl)amino)-1H-pyrrolo[3,2-c]pyridin-4-yl)oxy)pyrrolidin-1-yl)prop-2-en-1-one,
176) 1-(3-methyl-3-((6-((5-methylthiazol-2-yl)amino)-1-propyl-1H-pyrrolo[3,2-c]pyridin-4-yl)oxy)azetidin-1-yl)prop-2-en-1-one,
177) 1-(3-((1-isopentyl-6-((5-methylthiazol-2-yl)amino)-1H-pyrrolo[3,2-c]pyridin-4-yl)oxy)-3-methylazetidin-1-yl)prop-2-en-1-one,
178) 1-(3-((1-(cyclopentylmethyl)-6-((5-methylthiazol-2-yl)amino)-1H-pyrrolo[3,2-c]pyridin-4-yl)oxy)-3-methylazetidin-1-yl)prop-2-en-1-one,
179) 1-((2S,4S)-4-((1-isobutyl-6-((5-methylthiazol-2-yl)amino)-1H-pyrrolo[3,2-c]pyridin-4-yl)oxy)-2-methylpyrrolidin-1-yl)prop-2-en-1-one,
180) 1-((2S,4S)-4-((1-(cyclopropylmethyl)-6-((5-methylthiazol-2-yl)amino)-1H-pyrrolo[3,2-c]pyridin-4-yl)oxy)-2-methylpyrrolidin-1-yl)prop-2-en-1-one,
181) 2-((4-(((3R,4S)-1-acryloyl-4-fluoropyrrolidin-3-yl)oxy)-1-isobutyl-1H-pyrrolo[3,2-c]pyridin-6-yl)amino)thiazole-5-carbonitrile,
182) 2-((4-(((3R,4S)-1-acryloyl-4-fluoropyrrolidin-3-yl)oxy)-1-isobutyl-1H-pyrrolo[3,2-c]pyridin-6-yl)amino)-N-methylthiazole-5-carboxamide,
183) (S)-1-(3-((1-isopropyl-6-((4-methoxypyrimidin-2-yl)amino)-1H-pyrrolo[3,2-c]pyridin-4-yl)oxy)pyrrolidin-1-yl)prop-2-en-1-one,
184) (S)-1-(3-((1-isopropyl-6-((3-methoxypyrazin-2-yl)amino)-1H-pyrrolo[3,2-c]pyridin-4-yl)oxy)pyrrolidin-1-yl)prop-2-en-1-one,
185) 1-((2S,4S)-2-methyl-4-((1-methyl-6-((5-methylthiazol-2-yl)amino)-1H-pyrrolo[3,2-c]pyridin-4-yl)oxy)pyrrolidin-1-yl)prop-2-en-1-one,
186) 1-((2S,4S)-4-((1-ethyl-6-((5-methylthiazol-2-yl)amino)-1H-pyrrolo[3,2-c]pyridin-4-yl)oxy)-2-methylpyrrolidin-1-yl)prop-2-en-1-one,
187) 1-((2S,4S)-2-methyl-4-((6-((5-methylthiazol-2-yl)amino)-1-propyl-1H-pyrrolo[3,2-c]pyridin-4-yl)oxy)pyrrolidin-1-yl)prop-2-en-1-one,
188) (S)-1-(3-((1-(2-fluoroethyl)-6-((5-methylthiazol-2-yl)amino)-1H-pyrrolo[3,2-c]pyridin-4-yl)oxy)pyrrolidin-1-yl)prop-2-en-1-one,
189) 1-((3S,4R)-3-fluoro-4-((1-(2-fluoroethyl)-6-((5-methylthiazol-2-yl)amino)-1H-pyrrolo[3,2-c]pyridin-4-yl)oxy)pyrrolidin-1-yl)prop-2-en-1-one,
190) (S)-1-(3-((1-(3-fluoropropyl)-6-((5-methylthiazol-2-yl)amino)-1H-pyrrolo[3,2-c]pyridin-4-yl)oxy)pyrrolidin-1-yl)prop-2-en-1-one,
191) 1-((3S,4R)-3-fluoro-4-((1-(3-fluoropropyl)-6-((5-methylthiazol-2-yl)amino)-1H-pyrrolo[3,2-c]pyridin-4-yl)oxy)pyrrolidin-1-yl)prop-2-en-1-one,
192) (S)-1-(3-((6-((1H-pyrazol-3-yl)amino)-1-isopropyl-1H-pyrrolo[3,2-c]pyridin-4-yl)oxy)pyrrolidin-1-yl)prop-2-en-1-one,
193) (S)-1-(3-((1-isopropyl-6-((1-methyl-1H-pyrazol-3-yl)amino)-1H-pyrrolo[3,2-c]pyridin-4-yl)oxy)pyrrolidin-1-yl)prop-2-en-1-one,
194) (S)-1-(3-((1-isopropyl-6-((5-methyl-1H-pyrazol-3-yl)amino)-1H-pyrrolo[3,2-c]pyridin-4-yl)oxy)pyrrolidin-1-yl)prop-2-en-1-one,
195) 1-((3R,4S)-3-((6-((1H-pyrazol-3-yl)amino)-1-isobutyl-1H-pyrrolo[3,2-c]pyridin-4-yl)oxy)-4-fluoropyrrolidin-1-yl)prop-2-en-1-one,
196) 1-((3S,4R)-3-fluoro-4-((1-isobutyl-6-((5-methyl-1H-pyrazol-3-yl)amino)-1H-pyrrolo[3,2-c]pyridin-4-yl)oxy)pyrrolidin-1-yl)prop-2-en-1-one,
197) 1-((3S,4R)-3-fluoro-4-((1-isobutyl-6-((1-methyl-1H-pyrazol-3-yl)amino)-1H-pyrrolo[3,2-c]pyridin-4-yl)oxy)pyrrolidin-1-yl)prop-2-en-1-one,
198) (S)-3-((4-((1-acryloylpyrrolidin-3-yl)oxy)-1-isopropyl-1H-pyrrolo[3,2-c]pyridin-6-yl)amino)-1-methylpyridin-2(1H)-one,
199) 3-((4-(((3R,4S)-1-acryloyl-4-fluoropyrrolidin-3-yl)oxy)-1-isobutyl-1H-pyrrolo[3,2-c]pyridin-6-yl)amino)-1-methylpyridin-2(1H)-one,
200) 1-(2-((1-isobutyl-6-((5-methylthiazol-2-yl)amino)-1H-pyrrolo[3,2-c]pyridin-4-yl)oxy)-5-azaspiro[3.4]octan-5-yl)prop-2-en-1-one,
201) 1-(7-((1-isobutyl-6-((5-methylthiazol-2-yl)amino)-1H-pyrrolo[3,2-c]pyridin-4-yl)oxy)-1-azaspiro[4.4]nonan-1-yl)prop-2-en-1-one,
202) 1-(2-((1-isobutyl-6-((5-methylthiazol-2-yl)amino)-1H-pyrrolo[3,2-c]pyridin-4-yl)oxy)-6-azaspiro[3.4]octan-6-yl)prop-2-en-1-one,
203) 1-(2-((1-isobutyl-6-((5-methylthiazol-2-yl)amino)-1H-pyrrolo[3,2-c]pyridin-4-yl)oxy)-5-azaspiro[3.5]nonan-5-yl)prop-2-en-1-one,
204) 1-(7-((1-isobutyl-6-((5-methylthiazol-2-yl)amino)-1H-pyrrolo[3,2-c]pyridin-4-yl)oxy)-2-azaspiro[4.4]nonan-2-yl)prop-2-en-1-one,
205) (S)-1-(3-((6-((5-methylthiazol-2-yl)amino)-1-(2,2,2-trifluoroethyl)-1H-pyrrolo[3,2-c]pyridin-4-yl)oxy)pyrrolidin-1-yl)prop-2-en-1-one,
206) (S)-1-(3-((1-(2,2-difluoroethyl)-6-((5-methylthiazol-2-yl)amino)-1H-pyrrolo[3,2-c]pyridin-4-yl)oxy)pyrrolidin-1-yl)prop-2-en-1-one,
207) (R)-1-(2-(((6-((5-methylthiazol-2-yl)amino)-1-(2,2,2-trifluoroethyl)-1H-pyrrolo[3,2-c]pyridin-4-yl)oxy)methyl)pyrrolidin-1-yl)prop-2-en-1-one,
208) (R)-1-(2-(((6-((5-methylthiazol-2-yl)amino)-1-(2,2,2-trifluoroethyl)-1H-pyrrolo[3,2-c]pyridin-4-yl)oxy)methyl)azetidin-1-yl)prop-2-en-1-one,
209) (R)-1-(2-(((1-(2,2-difluoroethyl)-6-((5-methylthiazol-2-yl)amino)-1H-pyrrolo[3,2-c]pyridin-4-yl)oxy)methyl)azetidin-1-yl)prop-2-en-1-one,
210) (R)-1-(2-(((1-(2,2-difluoroethyl)-6-((5-methylthiazol-2-yl)amino)-1H-pyrrolo[3,2-c]pyridin-4-yl)oxy)methyl)pyrrolidin-1-yl)prop-2-en-1-one,
211) 1-((3S,4R)-3-fluoro-4-((6-((5-methylthiazol-2-yl)amino)-1-(2,2,2-trifluoroethyl)-1H-pyrrolo[3,2-c]pyridin-4-yl)oxy)pyrrolidin-1-yl)prop-2-en-1-one,
212) 1-((2R,4S)-2-methyl-4-((6-((5-methylthiazol-2-yl)amino)-1-(2,2,2-trifluoroethyl)-1H-pyrrolo[3,2-c]pyridin-4-yl)oxy)pyrrolidin-1-yl)prop-2-en-1-one,
213) 1-((S)-3-((6-((5-methylthiazol-2-yl)amino)-1-((R)-1,1,1-trifluoropropan-2-yl)-1H-pyrrolo[3,2-c]pyridin-4-yl)oxy)pyrrolidin-1-yl)prop-2-en-1-one,
214) (R)-1-(3-(((1-isobutyl-6-((5-methylthiazol-2-yl)amino)-1H-pyrrolo[3,2-c]pyridin-4-yl)oxy)methyl)piperidin-1-yl)prop-2-en-1-one,
215) (S)-1-(3-(((1-isobutyl-6-((5-methylthiazol-2-yl)amino)-1H-pyrrolo[3,2-c]pyridin-4-yl)oxy)methyl)piperidin-1-yl)prop-2-en-1-one,
216) (S)-1-(3-(((1-isobutyl-6-((5-methylthiazol-2-yl)amino)-1H-pyrrolo[3,2-c]pyridin-4-yl)oxy)methyl)pyrrolidin-1-yl)prop-2-en-1-one,
217) 1-(2-(((1-cyclopentyl-6-((5-methylthiazol-2-yl)amino)-1H-pyrrolo[3,2-c]pyridin-4-yl)oxy)methyl)pyrrolidin-1-yl)prop-2-en-1-one,
218) 1-(2-(((1-cyclopentyl-6-((5-methylthiazol-2-yl)amino)-1H-pyrrolo[3,2-c]pyridin-4-yl)oxy)methyl)azetidin-1-yl)prop-2-en-1-one,
219) (S)-1-(2-(((1-isopropyl-6-((5-methylthiazol-2-yl)amino)-1H-pyrrolo[3,2-c]pyridin-4-yl)oxy)methyl)pyrrolidin-1-yl)prop-2-en-1-one,
220) (S)-1-(2-(((1-(cyclopentylmethyl)-6-((5-methylthiazol-2-yl)amino)-1H-pyrrolo[3,2-c]pyridin-4-yl)oxy)methyl)pyrrolidin-1-yl)prop-2-en-1-one,
221) 1-((2S)-2-(((1-(2-methylbutyl)-6-((5-methylthiazol-2-yl)amino)-1H-pyrrolo[3,2-c]pyridin-4-yl)oxy)methyl)pyrrolidin-1-yl)prop-2-en-1-one,
222) (S)-1-(2-(((1-cyclopentyl-6-((5-methylthiazol-2-yl)amino)-1H-pyrrolo[3,2-c]pyridin-4-yl)oxy)methyl)pyrrolidin-1-yl)prop-2-en-1-one,
223) (R)-1-(2-(((1-cyclopentyl-6-((5-methylthiazol-2-yl)amino)-1H-pyrrolo[3,2-c]pyridin-4-yl)oxy)methyl)azetidin-1-yl)prop-2-en-1-one,
224) (S)-2-fluoro-1-(3-((1-isobutyl-6-((5-methylthiazol-2-yl)amino)-1H-pyrrolo[3,2-c]pyridin-4-yl)oxy)pyrrolidin-1-yl)prop-2-en-1-one,
225) (S)-1-(3-((1-isobutyl-6-((5-methylthiazol-2-yl)amino)-1H-pyrrolo[3,2-c]pyridin-4-yl)oxy)pyrrolidin-1-yl)prop-2-yn-1-one,
226) (S,E)-1-(3-((1-isobutyl-6-((5-methylthiazol-2-yl)amino)-1H-pyrrolo[3,2-c]pyridin-4-yl)oxy)pyrrolidin-1-yl)but-2-en-1-one,
227) (S,E)-4-(dimethylamino)-1-(3-((1-isobutyl-6-((5-methylthiazol-2-yl)amino)-1H-pyrrolo[3,2-c]pyridin-4-yl)oxy)pyrrolidin-1-yl)but-2-en-1-one,
228) N-(4-(((3R,4S)-4-fluoro-1-(vinylsulfonyl)pyrrolidin-3-yl)oxy)-1-isobutyl-1H-pyrrolo[3,2-c]pyridin-6-yl)-5-methylthiazol-2-amine,
229) 2-chloro-1-((3S,4R)-3-fluoro-4-((1-isobutyl-6-((5-methylthiazol-2-yl)amino)-1H-pyrrolo[3,2-c]pyridin-4-yl)oxy)pyrrolidin-1-yl)ethan-1-one,
230) 1-((3S,4R)-3-fluoro-4-((1-isobutyl-6-((5-methylthiazol-2-yl)amino)-1H-pyrrolo[3,2-c]pyridin-4-yl)oxy)pyrrolidin-1-yl)but-2-yn-1-one, and
231) 1-((3S,4R)-3-fluoro-4-((1-isobutyl-6-((5-methylthiazol-2-yl)amino)-1H-pyrrolo[3,2-c]pyridin-4-yl)oxy)pyrrolidin-1-yl)prop-2-yn-1-one.

Meanwhile, the compound represented by Chemical Formula 1 may be understood as a concept of encompassing all possible stereoisomers, mixtures of stereoisomers and isotopic derivatives of the compounds.

Specifically, "stereoisomers" of the compound may be understood as including all possible configurations of diastereomers and enantiomers when the compound contains one or more stereogenic centers.

Further, "isotopic derivatives" of the compound mean that one or more atoms in the compound are replaced by a naturally occurring or non-naturally occurring isotope. For example, isotopic derivatives of the compounds include compounds in which at least one hydrogen in the compound is replaced by deuterium.

In addition, the compounds of the present disclosure may exist in the form of salts, especially pharmaceutically acceptable salts. As salts, salts commonly used in the art, such as acid addition salts formed by pharmaceutically acceptable free acids can be used without limitation. The term "pharmaceutically acceptable salt" as used herein refers to any organic or inorganic addition salt of the compound represented by Chemical Formula 1, whose concentration is relatively non-toxic and harmless to ae patient and activates effectively and whose side effects do not degrade the beneficial efficacy of the above compound.

As the free acid, an organic acid and an inorganic acid can be used. Examples of the inorganic acids include hydrochloric acid, phosphoric acid, sulfuric acid, nitric acid, tartaric acid and the like. Examples of the organic acids include methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycollic acid, gluconic acid, galacturonic acid, glutamic acid, glutaric acid, glucuronic acid, aspartic acid, ascorbic acid, carbonic acid, vanillic acid, hydroiodic acid and the like, but are not limited thereto. Preferably, the salt may be hydrochloride salt.

In addition, a pharmaceutically acceptable metal salt can be obtained by a conventional method using a base. For example, a compound represented by Chemical Formula 1 is dissolved in an excessive amount of an alkali metal hydroxide or an alkaline earth metal hydroxide solution, the non-soluble salt is filtered, and the filtrate is evaporated and dried to obtain a pharmaceutically acceptable metal salt. At this time, it is particularly preferable to prepare a sodium salt, a potassium salt or a calcium salt as the metal salt.

A pharmaceutically unacceptable salt or solvate of the compound represented by Chemical Formula 1 may be used as an intermediate when preparing the compound represented by Chemical Formula 1, or the pharmaceutically acceptable salt or the solvate thereof.

Further, the compound represented by Chemical Formula 1 according to the present disclosure includes not only pharmaceutically acceptable salts thereof, but also solvates such as hydrates thereof that can be prepared therefrom, or prodrugs thereof, but are not limited thereto.

The solvate of the compound represented by Chemical Formula 1 may be prepared from the compound represented by Chemical Formula 1 using common methods known in the art.

Further, unless otherwise indicated, a "prodrug" of the compound may be understood as referring to all derivatives of a compound that can hydrolyze, oxidize, or otherwise react under biological conditions (in vitro or in vivo) to provide a compound of the present disclosure, a stereoisomer thereof, a mixture of stereoisomers thereof, an isotopic derivative thereof, or a pharmaceutically acceptable salt thereof.

In addition, in the present disclosure, the compound represented by Chemical Formula 1 may not only include a stoichiometric hydrate, but also include a compound containing various amounts of water. The solvate of the compound represented by Chemical Formula 1 according to the present disclosure includes both stoichiometric solvates and non-stoichiometric solvates.

Furthermore, the compound represented by Chemical Formula 1 can be prepared through the following Reaction Scheme 1, for example, when L is any one of the linking groups represented by Chemical Formulas 2a to 2f. 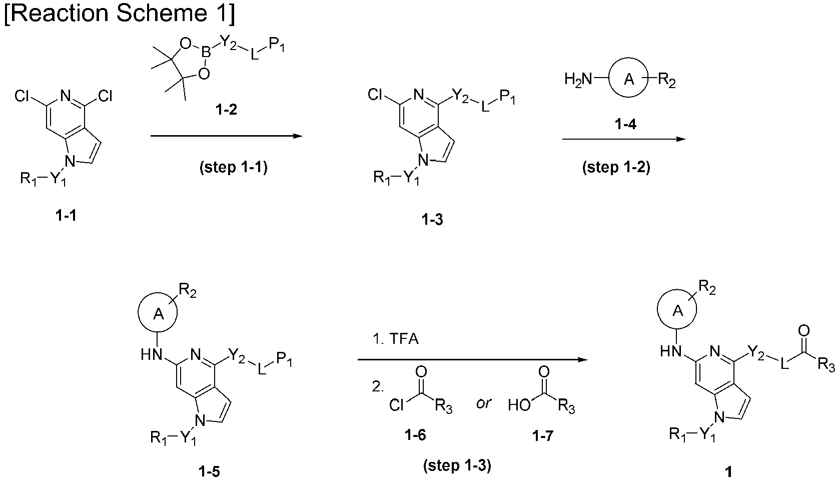 in Reaction Scheme 1, P₁ means a protecting group, and the remaining substituents are as defined in Chemical Formula 1.

For example, P₁ may be any one protecting group selected from the group consisting of tert-butyloxycarbonyl (Boc), carbobenzyloxy (Cbz), para-methoxybenzylcarbonyl (Moz), 9-fluorenylmethyloxycarbonyl (Fmoc), acetyl (Ac), benzoyl (Bz), benzyl (Bn) and para-methoxybenzyl (PMB).

Step 1-1 is a step of reacting the compound represented by Chemical Formula 1-1 with the compound represented by Chemical Formula 1-2 to prepare the compound represented by Chemical Formula 1-3. The above reaction is a Suzuki-coupling reaction, which is preferably carried out in the presence of a palladium catalyst and a base.

Step 1-2 is a step of reacting the compound represented by Chemical Formula 1-3 with the compound represented by Chemical Formula 1-4 to prepare a compound represented by Chemical Formula 1-5. The above reaction is an amine substitution reaction, which is preferably carried out in the presence of a palladium catalyst and a base.

Step 1-3 is a step of removing a protecting group from the compound represented by Chemical Formula 1-5, and then reacting the compound represented by Chemical Formula 1-6 or 1-7 to prepare the compound represented by Chemical Formula 1. The removal of the protecting group is preferably carried out under acid conditions that can remove the protecting group, such as trifluoroacetic acid (TFA), and the reaction with the compound represented by Chemical Formula 1-6 or 1-7 is an amidation reaction, which is preferably carried out in the presence of a base.

In addition, the compound represented by Chemical Formula 1 can be prepared through the following Reaction Scheme 2, for example, when L is a linking group represented by Chemical Formula 2g. 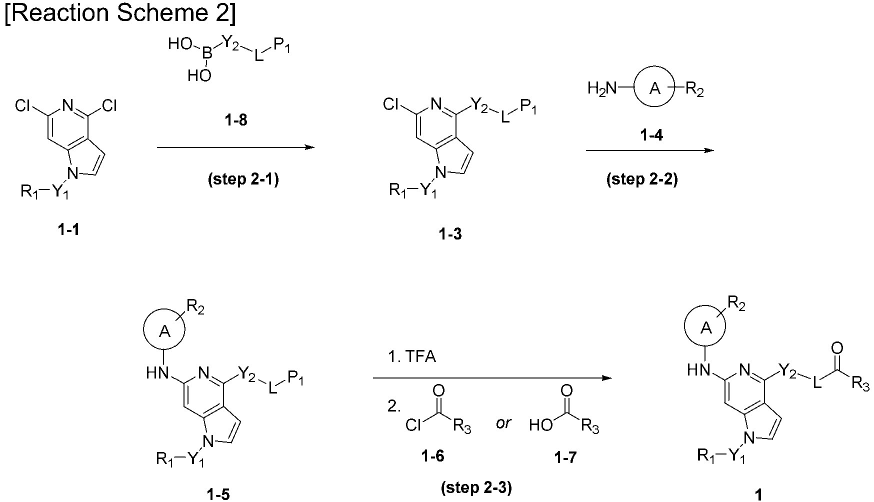 in Reaction Scheme 2, P₁ means a protecting group and is as defined in Reaction Scheme 1, and the remaining substituents are as defined in Chemical Formula 1.

Step 2-1 is a step of reacting the compound represented by Chemical Formula 1-1 with the compound represented by Chemical Formula 1-8 to prepare the compound represented by Chemical Formula 1-3, and can be carried out in the same manner as in step 1-1 of Reaction Scheme 1, except that the reactive group for the Suzuki coupling reaction is different.

Further, steps 2-2 and 2-3 may be carried out in the same manner as in steps 1-2 and 1-3 of Reaction Scheme 1, respectively.

In addition, the compound represented by Chemical Formula 1 can be prepared through the following Reaction Scheme 3, for example, when L is any one of the linking groups represented by Chemical Formulas 2h to 2j. 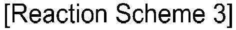 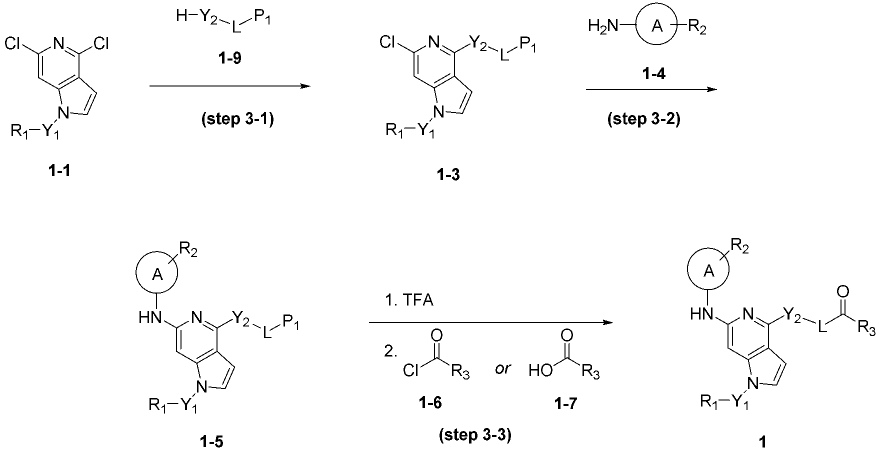 in Reaction Scheme 3, P₁ means a protecting group and is as defined in Reaction Scheme 1, and the remaining substituents are as defined in Chemical Formula 1.

Step 3-1 is a step of reacting the compound represented by Chemical Formula 1-1 with the compound represented by Chemical Formula 1-9 to prepare the compound represented by Chemical Formula 1-3. The above reaction is a nucleophilic substitution reaction, which is preferably carried out in the presence of a base.

Further, steps 3-2 and 3-3 may be carried out in the same manner as in steps 1-2 and 1-3 of Reaction Scheme 1, respectively.

The preparation method of each of the above steps can be more specifically described in Examples described below.

According to another embodiment of the present disclosure, there is provided a pharmaceutical composition comprising the compound represented by Chemical Formula 1 or a pharmaceutically acceptable salt thereof as an active ingredient.

More specifically, the present disclosure can be understood as providing a pharmaceutical composition comprising, as an active ingredient, the compound represented by Chemical Formula 1, a stereoisomer thereof, a mixture of stereoisomers thereof, an isotopic derivative thereof, a pharmaceutically acceptable salt thereof, or a prodrug thereof.

According to yet another embodiment of the present disclosure, there is provided a pharmaceutical composition for the prevention or treatment of inflammatory diseases, autoimmune diseases, or cancers, comprising the compound represented by Chemical Formula 1 or a pharmaceutically acceptable salt thereof as an active ingredient.

More specifically, the present disclosure is understood as providing a pharmaceutical composition for the prevention or treatment of inflammatory diseases, autoimmune diseases, or cancers which are associated with ITK and BTK inhibitory actions, comprising the compound represented by Chemical Formula 1, a stereoisomer thereof, a mixture of stereoisomers thereof, an isotopic derivative thereof, a pharmaceutically acceptable salt thereof, or a prodrug thereof as an active ingredient.

In this case, the autoimmune diseases include rheumatoid arthritis, systemic lupus erythematosus, childhood diabetes, psoriasis, aphthous stomatitis, chronic thyroiditis, some acquired aplastic anemia, primary cirrhosis, ulcerative colitis, Behcet's disease, Crohn's disease, Silicosis, asbestosis, Sjogren's syndrome, Guillain-Barre syndrome, dermatomyositis, polymyositis, multiple sclerosis, anti-MOG antibody disease, autoimmune hemolytic anemia, autoimmune encephalomyelitis, myasthenia gravis, Graves thyroid hyperplasia, nodular polyarteritis, ankylosing spondylitis, fibrositis, temporal arteritis, Wilson's disease, asthma, neuromyelitis optica, Fanconi syndrome, or degenerative neurological diseases such as Alzheimer's disease, Parkinson's disease, Lou Gehrig's disease, and the like.

The cancer includes blood cancer, extranodal marginal zone B-cell lymphoma, glioblastoma, lymphoplasmacytic lymphoma, acute myelogenous leukemia, macroglobulinemia, B cell lymphoma, chronic lymphocytic leukemia, follicular lymphoma, non-hodgkin lymphoma, diffuse large B cell lymphoma, hairy cell leukemia, mantle cell lymphoma, glioblastoma, bladder cancer, pancreatic cancer, ovarian cancer, colorectal cancer, renal cancer, gastric cancer, transitional cell carcinoma, carcinoid tumor, breast cancer, non-small cell lung cancer, or multiple myeloma.

As used herein, the term "prevention" refers to any act to delay or inhibit occurrence, spread or recurrence of the above-mentioned diseases by administration of the composition of the present disclosure, and "treatment" refers to any act to improve or change the symptoms of the above diseases for the better by administration of the composition of the present disclosure.

The pharmaceutical composition according to the present disclosure can be formulated in types for oral or parenteral administrations according to a standard pharmaceutical practice. These formulations may contain additives such as pharmaceutically acceptable carrier, adjuvant or diluent in addition to the active ingredient.

Suitable carriers include, for example, physiological saline, polyethylene glycol, ethanol, vegetable oil, and isopropyl myristate and the like. Diluents include, for example, lactose, dextrose, sucrose, mannitol, sorbitol, cellulose and/or glycine and the like, but are not limited thereto. Further, the compounds of the present disclosure can be dissolved in oils, propylene glycol or other solvents commonly used in the preparation of injection solutions. Furthermore, the compounds of the present disclosure can be formulated in ointments or creams for topical application.

A preferred dose of the compound of the present disclosure may be varied according to the condition and weight of a patient, the severity of a disease, the type of a drug, and the route and duration of administration, but it may be suitably selected by those skilled in the art. In order to achieve the desirable effects, however, the compound of the present disclosure may be administrated daily at a dose of 0.0001 to 100 mg/kg (body weight), and preferably 0.001 to 100 mg/kg (body weight). The administration may be performed once a day or in divided doses each day through an oral or parenteral route.

Depending on the method of administration, the pharmaceutical composition may contain the compound of the present disclosure in an amount of 0.001 to 99% by weight, preferably 0.01 to 60% by weight.

The pharmaceutical composition according to the present disclosure may be administered to mammals such as a rat, a mouse, a domestic animal, a human, through various routes. The administration may be carried out through all possible methods, for example, oral, rectal, intravenous, intramuscular, subcutaneous, intra-endometrial, intracerebroventricular injection.

On the other hand, the notation ------ as used herein means a bond linked to another substituent.

In the present disclosure, a halogen group means fluoro, chloro, bromo, or iodo.

In the present disclosure, the alkyl group may be straight-chain or branched-chain. According to one embodiment, the carbon number of the alkyl group is 1 to 10. According to another embodiment, the carbon number of the alkyl group is 1 to 4. Specific examples of the alkyl group include methyl, ethyl, propyl, n-propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl, 1-ethyl-propyl, 1,1-dimethylpropyl, hexyl, n-hexyl, 1-methylpentyl, 2-methylpentyl, 4-methyl-2-pentyl, 3,3-dimethylbutyl, 2-ethylbutyl, heptyl, n-heptyl, isohexyl, 1-methylhexyl, 2-methylhexyl, 3-methylhexyl, 4-methylhexyl, 5-methylhexyl, octyl, n-octyl, tert-octyl, 1-methylheptyl, 2-ethylhexyl, 2,4,4-trimethyl-1-pentyl, 2,4,4-trimethyl-2-pentyl, 2-propylpentyl, n-nonyl, 2,2-dimethylheptyl, or n-decyl, and the like, but are not limited thereto. In addition, the explanation regarding the alkyl group may be applied to the haloalkyl group, except that at least one carbon of the alkyl group is substituted with a halogen group. Further, the explanation regarding the alkyl group can be applied except that the alkylene group is a divalent group.

In the present disclosure, the alkenyl group may be straight-chain or branched-chain. According to one embodiment, the carbon number of the alkenyl group is 1 to 4. Specific examples of the alkenyl group include vinyl, 1-propenyl, isopropenyl, 1-butenyl, 2-butenyl, 3-butenyl, and the like, but are not limited thereto.

In the present disclosure, the alkynyl group may be straight-chain. According to one embodiment, the carbon number of the alkynyl group is 1 to 4. Specific examples of the alkynyl group include ethynyl, 1-propynyl, 2-propynyl, 1-butynyl, 3-butynyl, or 3-butynyl, and the like, but are not limited thereto.

In the present disclosure, the alkoxy group refers to an alkyl group which is singularly bonded to oxygen, and may be straight-chain or branched-chain. According to one embodiment, the carbon number of the alkoxy group is 1 to 10. According to another embodiment, the carbon number of the alkoxy group is 1 to 4. Specific examples of the alkoxy group include methoxy, ethoxy, propoxy, n-propoxy, isopropoxy, butoxy, n-butoxy, isobutoxy, tert-butoxy, sec-butoxy, 1-methylbutoxy, 1-ethylbutoxy, pentyloxy, n-pentyloxy, isopentyloxy, neopentyloxy, tert-pentyloxy, 1-ethyl-propoxy, 1,1-dimethylpropoxy, hexyloxy, n-hexyloxy, 1-methylpentyloxy, 2-methylpentyloxy, 4-methyl-2-pentyloxy, 3,3-dimethylbutoxy, 2-ethylbutoxy, heptyloxy, n-heptyloxy, isohexyloxy, 1-methylhexyloxy, 2-methylhexyloxy, 3-methylhexyloxy, 4-methylhexyloxy, 5-methylhexyloxy, octyloxy, n-octyloxy, tert-octyloxy, 1-methylheptyloxy, 2-ethylhexyloxy, 2,4,4-trimethyl-1-pentyloxy, 2,4,4-trimethyl-2-pentyloxy, 2-propylpentyloxy, n-nonyloxy, 2,2-dimethylheptyloxy, n-decyloxy, or the like, but are not limited thereto. Further, the explanation regarding the alkoxy group may be applied to the haloalkoxy group, except that at least one carbon of the alkoxy group is substituted with a halogen group.

In the present disclosure, the cycloalkyl group is understood as referring to a monovalent substituent derived from a saturated or unsaturated hydrocarbon ring compound that contains only carbon as a ring-forming atom but does not have aromaticity. According to one embodiment, the carbon number of the cycloalkyl group is 3 to 10. According to another embodiment, the carbon number of the cycloalkyl group is 3 to 8. According to yet another embodiment, the carbon number of the cycloalkyl group is 3 to 7. Specific examples of the cycloalkyl group include cyclopropyl, cyclobutyl, cyclopentyl, cycloheptyl, cyclooctyl, cyclononyl, cyclodecyl, or the like, but are not limited thereto.

In the present disclosure, the aryl group is understood as referring to a monovalent substituent derived from a monocyclic or fused polycyclic compound that contains only carbon as a ring-forming atom and has aromaticity. According to one embodiment, the carbon number of the aryl group is 6 to 20. According to another embodiment, the carbon number of the aryl group is 6 to 12. According to yet another embodiment, the carbon number of the aryl group is 6 to 10. Specific examples of the monocyclic aryl group include phenyl, biphenylyl, or terphenylyl, but are not limited thereto. Further, examples of the fused polycyclic aryl group include a naphthyl group, an anthracenyl group, a phenanthryl group, a pyrenyl group, a perylenyl group, or a chrysenyl group, but are not limited thereto.

In the present disclosure, the N-containing 6-membered heteroaryl is understood as referring to a monovalent substituent derived from a monocyclic compound that has aromaticity, further contains N in addition to carbon as a ring-forming atom, and has 6 ring-forming atoms. Specific examples of the N-containing 6-membered heteroaryl include pyridinyl, pyrimidinyl, pyridazinyl, pyrazinyl, triazinyl, or tetrazinyl, but are not limited thereto.

In the present disclosure, the 5- or 6-membered heterocyclic group is understood as referring to a substituent derived from a monocyclic compound that has 5 or 6 ring-forming atoms, further contains 1 to 3 heteroatoms selected from O, N and S in addition to carbon as a ring-forming atom. Further, the 5- or 6-membered heterocyclic group is understood to encompass all structures with or without aromaticity. Accordingly, the 5- or 6-membered heterocyclic group can be understood to include both heterocycles that has aromaticity and their hydrogenated derivatives. In addition, the 5- or 6-membered heterocyclic group is substituted or unsubstituted with oxo (=O), where "substituted with oxo (=O)" means "containing carbon substituted with oxo (C=O)" instead of carbon as a ring-forming atom. Specific examples of the 5- or 6-membered heterocyclic group containing 1 to 3 heteroatoms selected from the group consisting of N, O and S substituted or unsubstituted with oxo(=O) include imidazole, pyrazole, thiazole, isothiazole, oxazole, isoxazole, thiadiazole, oxadiazole, pyridine, pyrimidine, pyridazine, pyrazine, triazine, tetrazine ring, pyridin-2(1H)-one, or the like, but are not limited thereto.

### [ADVANTAGEOUS EFFECTS]

The compound represented by Chemical Formula 1 according to the present disclosure or a pharmaceutically acceptable salt thereof can be effectively used for the prevention or treatment of inflammatory diseases, autoimmune diseases, or cancers.

### [DETAILED DESCRIPTION OF THE EMBODIMENTS]

Below, the present disclosure will be described in more detail with reference to the following Examples. However, these examples are for illustrative purposes only, and the scope of the present disclosure is not limited thereby.

### Preparation Example 1. Preparation of 4,6-dichloro-1-isopropyl-1H-pyrrolo[3,2-c]pyridine

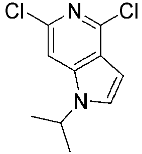

4,6-Dichloro-1*H*-pyrrolo[3,2-c]pyridine (5.61 g, 30 mmol) was dissolved in DMF (60 mL), and then 2-iodopropane (3.59 mL, 36 mmol) and Cs₂CO₃ (24 g, 75 mmol) were added thereto. The reaction mixture was gradually heated, and stirred at 60°C for 1 hour. The mixture was cooled to room temperature, and then diluted with ethyl acetate and washed with distilled water. The organic layer was dried over MgSO₄, filtered, and concentrated. The resulting residue was purified by a silica gel chromatography (0-50% dichloromethane/hexane) to synthesize 4,6-dichloro-1-isopropyl-1*H*-pyrrolo[3,2-c]pyridine as a white solid (5.1 g, yield 74%).
¹H NMR (500 MHz, CDCl₃) δ 7.28-7.24 (m, 2), 6.64 (d, *J* = 3.0 Hz, 1H), 4.61-4.53 (m, 1H), 1.54 (d, *J* = 7.0, 6H).; LC-MS (ESI) *m*/*z :* 229[M+H]⁺

### Preparation Example 2. Preparation of 4,6-dichloro-1-methyl-1H-pyrrolo[3,2-c]pyridine

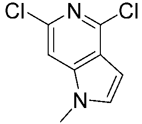

The target compound (yield: 82%, white solid) was prepared in the same manner as in Preparation Example 1, except that iodomethane was used instead of 2-iodopropane used in Preparation Example 1.
¹H NMR (500 MHz, CDCl₃) δ 7.21 (s, 1H), 7.10 (d, *J =* 3.2 Hz, 1H), 6.60 (d, *J =* 3.2 Hz, 1H), 3.78 (s, 3H).; LC-MS (ESI) *m*/*z :* 201 [M+H]⁺

### Preparation Example 3. Preparation of 4,6-dichloro-1-ethyl-1H-pyrrolo[3,2-c]pyridine

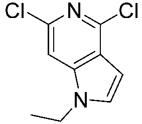

The target compound (yield: 80%, white solid) was prepared in the same manner as in Preparation Example 1, except that iodoethane was used instead of 2-iodopropane used in Preparation Example 1.
LC-MS (ESI) *m*/*z :* 215[M+H]⁺

### Preparation Example 4. Preparation of 4,6-dichloro-1-propyl-1H-pyrrolo[3,2-c]pyridine

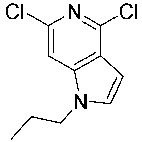

The target compound (yield: 81%, white solid) was prepared in the same manner as in Preparation Example 1, except that 1-iodopropane was used instead of 2-iodopropane used in Preparation Example 1.
LC-MS (ESI) *m*/*z :* 229[M+H]⁺

### Preparation Example 5. Preparation of 1-butyl-4,6-dichloro-1H-pyrrolo[3,2-c]pyridine

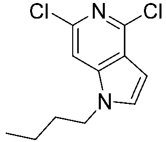

The target compound (yield: 77%, yellow oil) was prepared in the same manner as in Preparation Example 1, except that 1-iodopropane was used instead of 2-iodopropane used in Preparation Example 1.
LC-MS (ESI) *m*/*z* : 243[M+H]⁺

### Preparation Example 6. Preparation of 4,6-dichloro-1-isobutyl-1H-pyrrolo[3,2-c]pyridine

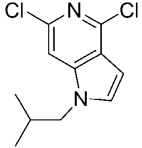

The target compound (yield: 84%, colorless oil) was prepared in the same manner as in Preparation Example 1, except that 1-bromo-2-methylpropane was used instead of 2-iodopropane used in Preparation Example 1.
LC-MS (ESI) *m*/*z :* 243[M+H]⁺

### Preparation Example 7. Preparation of (S)-1-(sec-butyl)-4,6-dichloro-1H-pyrrolo[3,2-c]pyridine

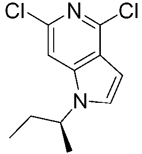

The target compound (yield: 80%, colorless oil) was prepared in the same manner as in Preparation Example 1, except that (*R*)-*sec*-butyl 4-methylbenzenesulfonate was used instead of 2-iodopropane used in Preparation Example 1.
LC-MS (ESI) *m*/*z :* 243[M+H]⁺

### Preparation Example 8. Preparation of (R)-1-(sec-butyl)-4,6-dichloro-1H-pyrrolo[3,2-c]pyridine

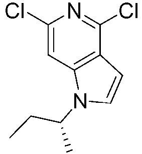

The target compound (yield: 86%, colorless oil) was prepared in the same manner as in Preparation Example 1, except that (*S*)-*sec*-butyl 4-methylbenzenesulfonate was used instead of 2-iodopropane used in Preparation Example 1.
LC-MS (ESI) *m*/*z :* 243[M+H]⁺

### Preparation Example 9. Preparation of 4,6-dichloro-1-(cyclopropylmethyl)-1H-pyrrolo[3,2-c]pyridine

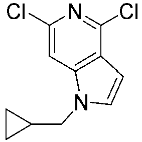

The target compound (yield: 90%, white solid) was prepared in the same manner as in Preparation Example 1, except that (bromomethyl)cyclopropane was used instead of 2-iodopropane used in Preparation Example 1.
LC-MS (ESI) *m*/*z :* 241[M+H]⁺

### Preparation Example 10. Preparation of 4,6-dichloro-1-(2-methylbutyl)-1H-pyrrolo[3,2-c]pyridine

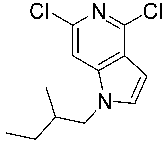

The target compound (yield: 80%, colorless oil) was prepared in the same manner as in Preparation Example 1, except that 1-bromo-2-methylbutane was used instead of 2-iodopropane used in Preparation Example 1.
LC-MS (ESI) *m*/*z :* 257[M+H]⁺

### Preparation Example 11. Preparation of 4,6-dichloro-1-cyclopentyl-1H-pyrrolo[3,2-c]pyridine

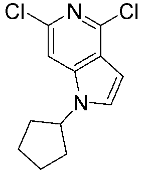

The target compound (yield: 30%, white solid) was prepared in the same manner as in Preparation Example 1, except that bromocyclopentane was used instead of 2-iodopropane used in Preparation Example 1.
LC-MS (ESI) *m*/*z* : 255[M+H]⁺

### Preparation Example 12. Preparation of 4,6-dichloro-1-(cyclobutylmethyl)-1H-pyrrolo[3,2-c]pyridine

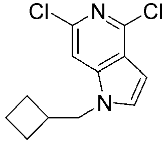

The target compound (yield: 30%, yellow solid) was prepared in the same manner as in Preparation Example 1, except that (bromomethyl)cyclobutane was used instead of 2-iodopropane used in Preparation Example 1.
LC-MS (ESI) *m*/*z :* 255[M+H]⁺

### Preparation Example 13. Preparation of 4,6-dichloro-1-(cyclopentylmethyl)-1H-pyrrolo[3,2-c]pyridine

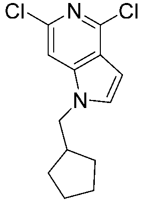

The target compound (yield: 69%, white solid) was prepared in the same manner as in Preparation Example 1, except that (bromomethyl)cyclopentane was used instead of 2-iodopropane used in Preparation Example 1.
LC-MS (ESI) *m*/*z :* 269 [M+H]⁺

### Preparation Example 14. Preparation of 4,6-dichloro-1-((tetrahydrofuran-3-yl)methyl)-1H-pyrrolo[3,2-c]pyridine

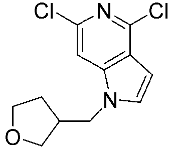

The target compound (yield: 48%, yellow solid) was prepared in the same manner as in Preparation Example 1, except that 3-(bromomethyl)tetrahydrofuran was used instead of 2-iodopropane used in Preparation Example 1.
LC-MS (ESI) *m*/*z* : 271[M+H]⁺

### Preparation Example 15. Preparation of 4,6-dichloro-1-(3,3-dimethylbutyl)-1H-pyrrolo[3,2-c]pyridine

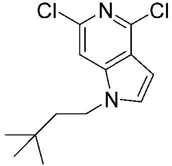

The target compound (yield: 88%, yellow solid) was prepared in the same manner as in Preparation Example 1, except that 1-bromo-3,3-dimethylbutane was used instead of 2-iodopropane used in Preparation Example 1.
LC-MS (ESI) *m*/*z :* 271[M+H]⁺

### Preparation Example 16. Preparation of 4,6-dichloro-1-(cyclohexylmethyl)-1H-pyrrolo[3,2-c]pyridine

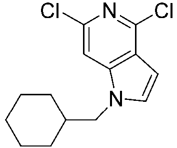

The target compound (yield: 73%, white solid) was prepared in the same manner as in Preparation Example 1, except that (bromomethyl)cyclohexane was used instead of 2-iodopropane used in Preparation Example 1.
LC-MS (ESI) *m*/*z :* 283[M+H]⁺

### Preparation Example 17. Preparation of 4,6-dichloro-1-((tetrahydro-2H-pyran-4-yl)methyl)-1H-pyrrolo[3,2-c]pyridine

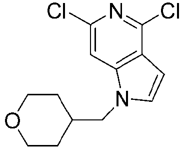

The target compound (yield: 51%, white solid) was prepared in the same manner as in Preparation Example 1, except that 4-(bromomethyl)tetrahydro-2H-pyran was used instead of 2-iodopropane used in Preparation Example 1.
LC-MS (ESI) *m*/*z :* 285[M+H]⁺

### Preparation Example 18. Preparation of 4,6-dichloro-1-(2-methoxyethyl)-1H-pyrrolo[3,2-c]pyridine

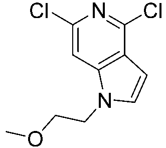

The target compound (yield: 80%, yellow solid) was prepared in the same manner as in Preparation Example 1, except that 1-bromo-2-methoxyethane was used instead of 2-iodopropane used in Preparation Example 1.
LC-MS (ESI) *m*/*z :* 245[M+H]⁺

### Preparation Example 19. Preparation of 1-benzyl-4,6-dichloro-1H-pyrrolo[3,2-c]pyridine

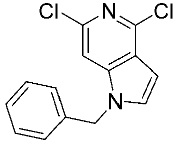

The target compound (yield: 67%, yellow syrup) was prepared in the same manner as in Preparation Example 1, except that (chloromethyl)benzene was used instead of 2-iodopropane used in Preparation Example 1.
LC-MS (ESI) *m*/*z* : 277[M+H]⁺

### Preparation Example 20. Preparation of 4,6-dichloro-1-phenethyl-1H-pyrrolo[3,2-c]pyridine

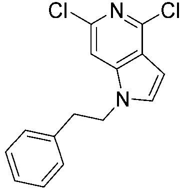

The target compound (yield: 77%, colorless syrup) was prepared in the same manner as in Preparation Example 1, except that (2-chloroethyl)benzene was used instead of 2-iodopropane used in Preparation Example 1.
LC-MS (ESI) *m*/*z :* 291[M+H]⁺

### Preparation Example 21. Preparation of 4,6-dichloro-1-(difluoromethyl)-1H-pyrrolo[3,2-c]pyridine

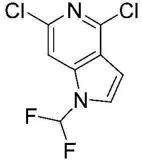

The target compound (yield: 30%, white solid) was prepared in the same manner as in Preparation Example 1, except that methyl 2-chloro-2,2-difluoroacetate was used instead of 2-iodopropane used in Preparation Example 1.
LC-MS (ESI) *m*/*z :* 237[M+H]⁺

### Preparation Example 22. Preparation of 4,6-dichloro-1-(2,2,2-trifluoroethyl)-1H-pyrrolo[3,2-c]pyridine

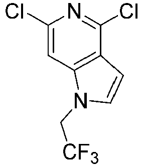

The target compound (yield: 85%, white solid) was prepared in the same manner as in Preparation Example 1, except that 2,2,2-trifluoroethyl trifluoromethanesulfonate was used instead of 2-iodopropane used in Preparation Example 1.
LC-MS (ESI) *m*/*z :* 269[M+H]⁺

### Preparation Example 23. Preparation of 4,6-dichloro-1-(2,2-difluoroethyl)-1H-pyrrolo[3,2-c]pyridine

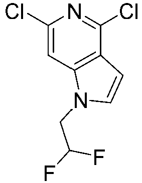

The target compound (yield: 93%, white solid) was prepared in the same manner as in Preparation Example 1, except that 2-bromo-1,1-difluoroethane was used instead of 2-iodopropane used in Preparation Example 1.
LC-MS (ESI) *m*/*z :* 251[M+H]⁺

### Preparation Example 24. Preparation of 4,6-dichloro-1-(2,2-difluorocyclopropyl)-1H-pyrrolo[3,2-c]pyridine

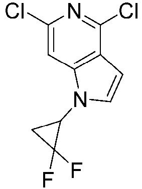

The target compound (yield: 76%, yellow solid) was prepared in the same manner as in Preparation Example 1, except that 2-bromo-1,1-difluorocyclopropane was used instead of 2-iodopropane used in Preparation Example 1.
LC-MS (ESI) *m*/*z :* 263[M+H]⁺

### Preparation Example 25. Preparation of (R)-4,6-dichloro-1-(1,1,1-trifluoropropan-2-yl)-1H-pyrrolo[3,2-c]pyridine

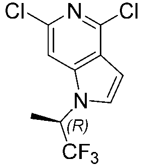

The target compound (yield: 39%, white solid) was prepared in the same manner as in Preparation Example 1, except that (S)-1,1,1-trifluoropropan-2-yl trifluoromethanesulfonate was used instead of 2-iodopropane used in Preparation Example 1.
LC-MS (ESI) *m*/*z :* 283[M+H]⁺

### Preparation Example 26. Preparation of 4,6-dichloro-1-((1-(trifluoromethyl)cyclopropyl)methyl)-1H-pyrrolo[3,2-c]pyridine

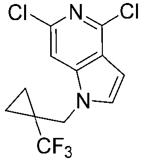

The target compound (yield: 48%, white solid) was prepared in the same manner as in Preparation Example 1, except that 1-(bromomethyl)-1-(trifluoromethyl)cyclopropane was used instead of 2-iodopropane used in Preparation Example 1.
LC-MS (ESI) *m*/*z :* 309[M+H]⁺

### Preparation Example 27. Preparation of 4,6-dichloro-1-((4,4-difluorocyclohexyl)methyl)-1H-pyrrolo[3,2-c]pyridine

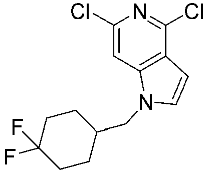

The target compound (yield: 59%, white solid) was prepared in the same manner as in Preparation Example 1, except that 4-(bromomethyl)-1,1-difluorocyclohexane was used instead of 2-iodopropane used in Preparation Example 1.
LC-MS (ESI) *m*/*z* : 319[M+H]⁺

### Preparation Example 28. Preparation of 2-(4,6-dichloro-1H-pyrrolo[3,2-c]pyridin-1-yl)-2-methylpropanenitrile

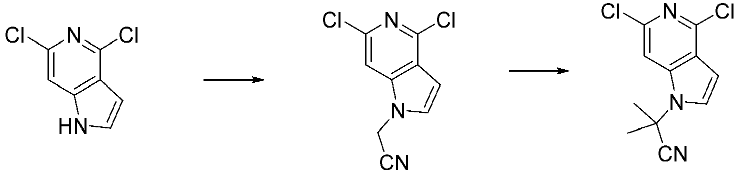

### Step 1. Preparation of 2-(4,6-dichloro-1H-pyrrolo[3,2-c]pyridin-1-yl)acetonitrile

4,6-Dichloro-1*H*-pyrrolo[3,2-c]pyridine (2.0 g, 10.7 mmol) was dissolved in DMF (20 mL), and then 2-bromoacetonitrile (0.89 mL, 12.4 mmol) and Cs₂CO₃ (5.23 g, 16.0 mmol) were added thereto. The reaction mixture was gradually heated, and stirred at 60°C for 1 hour. The mixture was cooled to room temperature, and then diluted with ethyl acetate and washed with distilled water. The organic layer was dried over MgSO₄, filtered, and concentrated. The resulting residue was purified by a silica gel chromatography (0-50% ethyl acetate/hexane) to synthesize 2-(4,6-dichloro-1*H*-pyrrolo[3,2-c]pyridin-1-yl)acetonitrile as a light brown solid (1.78 g, yield: 74%).
¹H NMR (500 MHz, DMSO-*d*₆) δ 7.96 (s, 1H), 7.74 (d, *J* = 3.4 Hz, 1H), 6.73 (d, *J* = 3.2 Hz, 1H), 5.60 (s, 2H).;LC-MS (ESI) *m*/*z :* 226[M+H]⁺

### Step 2. Preparation of Preparation of 2-(4,6-dichloro-1H-pyrrolo[3,2-c]pyridin-1-yl)-2-methylpropanenitrile

2-(4,6-Dichloro-1H-pyrrolo[3,2-c]pyridin-1-yl)acetonitrile (1.78 g, 7.9 mmol) prepared in step 1 was dissolved in DMF (20 mL), and then 60% NaH (0.79 g, 19.8 mmol) was slowly added thereto at 0°C. The reaction mixture was stirred at 0°C for 5 minutes, and Mel (1.5 mL, 24 mmol) was slowly added thereto, and allowed react at room temperature for 30 minutes. The reaction mixture was neutralized by adding 1M HCl aqueous solution, diluted with ethyl acetate and then washed with distilled water. The organic layer was dried over MgSO₄, filtered, and concentrated. The resulting residue was purified by a silica gel chromatography (20-30% ethyl acetate/hexane) to obtain the target compound as a pale white solid (1.0 g, yield: 50%).
¹H NMR (500 MHz, CDCl₃) δ 7.62 (s, 1H), 7.27 (d, *J* = 3.6 Hz, 1H), 6.70 (d, *J* = 3.4 Hz, 1H), 2.09 (s, 7H).; LC-MS (ESI) *m*/*z :* 226[M+H]⁺

### Preparation Example 29. Preparation of 4,6-dichloro-1-isopentyl-1H-pyrrolo[3,2-c]pyridine

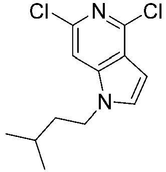

The target compound (yield: 73%, yellow solid) was prepared in the same manner as in Preparation Example 1, except that 1-bromo-3-methylbutane was used instead of 2-iodopropane used in Preparation Example 1.
LC-MS (ESI) *m*/*z :* 257[M+H]⁺

### Preparation Example 30. Preparation of 4,6-dichloro-1-(pyridin-2-ylmethyl)-1H-pyrrolo[3,2-c]pyridine

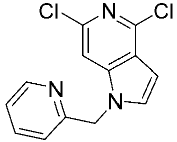

The target compound (yield: 77%, yellow solid) was prepared in the same manner as in Preparation Example 1, except that 2-(chloromethyl)pyridine was used instead of 2-iodopropane used in Preparation Example 1.
LC-MS (ESI) *m*/*z :* 278[M+H]⁺

### Preparation Example 31. Preparation of 1-(4,6-dichloro-1H-pyrrolo[3,2-c]pyridin-1-yl)-2-methylpropan-2-ol

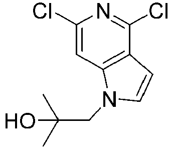

The target compound (yield: 80%, white solid) was prepared in the same manner as Preparation Example 1, except that 2,2-dimethyloxirane was used instead of the iodopropane used in Preparation Example 1.
LC-MS (ESI) m/z: 259[M+H] ⁺

### Preparation Example 32. Preparation of 4,6-dichloro-1-(2-fluoroethyl)-1H-pyrrolo[3,2-c]pyridine

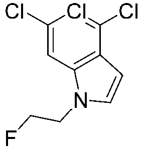

The target compound (yield: 72%, white solid) was prepared in the same manner as in Preparation Example 1, except that 1-fluoro-2-iodoethane was used instead of the iodopropane used in Preparation Example 1.
LC-MS (ESI) m/z: 233[M+H] ⁺

### Preparation Example 33. Preparation of 4,6-dichloro-1-(3-fluoropropyl)-1H-pyrrolo[3,2-c]pyridine

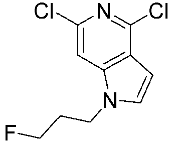

The target compound (yield: 78%, white solid) was prepared in the same manner as in Preparation Example 1, except that 1-iodo-3-fluoropropane was used instead of the iodopropane used in Preparation Example 1.
LC-MS (ESI) m/z: 247[M+H] ⁺

### Preparation Example 34. Preparation of (R)-4,6-dichloro-1-(2-methylbutyl)-1H-pyrrolo[3,2-c]pyridine

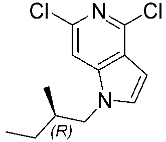

The target compound (yield: 80%, colorless oil) was prepared in the same manner as in Preparation Example 1, except that (2R)-1-bromo-2-methylbutane was used instead of 2-iodopropane used in Preparation Example 1.
LC-MS (ESI) m/z: 257[M+H]⁺

### Preparation Example 35. Preparation of (S)-4,6-dichloro-1-(2-methylbutyl)-1H-pyrrolo[3,2-c]pyridine

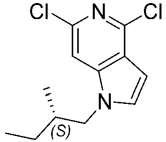

The target compound (yield: 80%, colorless oil) was prepared in the same manner as in Preparation Example 1, except that (2S)-1-bromo-2-methylbutane was used instead of 2-iodopropane used in Preparation Example 1.
LC-MS (ESI) m/z: 257[M+H]⁺

### Example 1. Preparation of 1-(4-(1-methyl-6-((5-methylthiazol-2-yl)amino)-1H-pyrrolo[3,2-c]pyridin-4-yl)-3,6-dihydropyridin-1(2H)-yl)prop-2-en-1-one

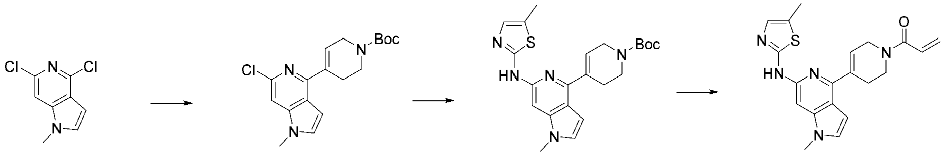

### Step 1. Preparation of tert-butyl 4-(6-chloro-1-methyl-1H-pyrrolo[3,2-c]pyridin-4-yl)-3,6-dihydropyridine-1(2H)-carboxylate

4,6-Dichloro-1H-pyrrolo[3,2-c]pyridine (201 mg, 1.0 mmol) was dissolved in 1,4-dioxane (3.0 mL), and then tert-butyl 4-(4,4,5,5-tetramethyl-1,3,2-dioxaborolan-2-yl)-3,6-dihydropyridine-1(2H)-carboxylate (371mg, 1.2mmol), Pd(PPh₃)₄ (116 mg, 0.10 mmol) and 1M Na₂CO₃ aqueous solution (2.0 mL) were added thereto at room temperature. The reaction mixture was gradually heated, and stirred at 80°C for 1 hour. The mixture was cooled to room temperature, and then diluted with ethyl acetate and washed with distilled water. The organic layer was dried over MgSO₄, filtered, and concentrated. The resulting residue was purified by a silica gel chromatography (0-40% ethyl acetate/hexane) to synthesize tert-butyl 4-(6-chloro-1-methyl-1*H*-pyrrolo[3,2-c]pyridin-4-yl)-3,6-dihydropyridine-1(2H)-carboxylate as a white solid (238 mg, yield: 68%).
¹H NMR (500 MHz, CDCl₃) δ 7.15 (s, 1H), 7.07 (d, *J =* 3.2 Hz, 1H), 6.67 (d, *J =* 3.0 Hz, 1H), 6.48 (br s, 1H), 4.17 (br s, 2H), 3.76 (s, 3H), 3.67 (br s, 2H), 2.80 (br s, 2H), 1.50 (s, 9H).; LC-MS (ESI) *m*/*z :* 348[M+H]⁺

### Step 2. Preparation of tert-butyl 4-(1-methyl-6-((5-methylthiazol-2-yl)amino)-1H-pyrrolo[3,2-c]pyridin-4-yl)-3,6-dihydropyridine-1(2H)-carboxylate

tert-Butyl 4-(6-chloro-1-methyl-1H-pyrrolo[3,2-c]pyridin-4-yl)-3,6-dihydropyridine-1(2*H*)-carboxylate (235 mg, 0.68 mmol) was dissolved in 1,4-dioxane (4.0 mL), and then 5-methylthiazol-2-amine (117 mg, 1.0 mmol), Pd₂(dba)₃ (62 mg), Xantphos (79 mg) and Cs₂CO₃ (553 mg) were added thereto at room temperature. The reaction mixture was reacted at 160°C for 2 hours using a microwave reactor. The mixture was diluted with ethyl acetate and then washed with distilled water. The organic layer was dried over MgSO₄, filtered, and concentrated. The resulting residue was purified by a silica gel chromatography (0~70% ethyl acetate/hexane) to synthesize tert-butyl 4-(1-methyl-6-((5-methylthiazol-2-yl)amino)-1*H*-pyrrolo[3,2-c]pyridin-4-yl)-3,6-dihydropyridine-1(2*H*)-carboxylate as a yellow solid (30 mg, yield: 10%).
¹H NMR (500 MHz, CDCl₃) δ 8.92 (br s, 1H), 7.01 (d, *J* = 1.0 Hz, 1H), 6.96 (d, *J* = 3.3 Hz, 1H), 6.68 (br s, 1H), 6.63 (d, *J* = 3.1 Hz, 1H), 6.56 (m, 1H), 4.21 (br s, 2H), 3.74 (br s, 2H), 3.72 (s, 3H), 2.95 (br s, 2H), 2.41 (s, 3H), 1.52 (s, 9H).; LC-MS (ESI) *m*/*z : 426* [M+H]⁺

### Step 3. Preparation of 1-(4-(1-methyl-6-((5-methylthiazol-2-yl)amino)-1H-pyrrolo[3,2-c]pyridin-4-yl)-3,6-dihydropyridin-1(2H)-yl)prop-2-en-1-one

*tert*-butyl 4-(1-methyl-6-((5-methylthiazol-2-yl)amino)-1*H*-pyrrolo[3,2-c]pyridin-4-yl)-3,6-dihydropyridine-1(2*H*)-carboxylate (30 mg, 0.070 mmol) was dissolved in CH₂Cl₂(3.0 mL), and then TFA (2.0 mL) was added thereto, and stirred at room temperature for 1 hour. After completion of the reaction, the mixture was concentrated under reduced pressure to synthesize a salt compound. The resulting salt compound was dissolved in water (2.0 mL) and THF (4.0 mL), and then NaHCO₃ (60 mg, 0.72 mmol) was added thereto, and stirred at room temperature for 10 minutes. The mixture was cooled to 0°C, and then 1.0M acryloyl chloride (0.091 mL, 1.3 eq) diluted in THF was slowly added dropwise thereto. The reaction mixture was stirred at room temperature for 30 minutes, diluted with dichloromethane, and washed with distilled water and salt water. The organic layer was dried over MgSO₄, filtered, and concentrated. The resulting residue was purified by a silica gel chromatography (0-10% methanol/ dichloromethane) to synthesize 1-(4-(1-methyl-6-((5-methylthiazol-2-yl)amino)-1*H*-pyrrolo[3,2-c]pyridin-4-yl)-3,6-dihydropyridin-1(2*H*)-yl)prop-2-en-1-one as a while solid (14 mg, yield: 52%).
¹H NMR (500 MHz, DMSO-*d*₆) δ 10.66 (s, 1H), 7.29 (d, *J* = 2.9 Hz, 1H), 6.96 (s, 1H), 6.89 (m, 1H), 687 (s, 1H), 6.65 (m, 1H), 6.61 (br s, 1H), 6.17 (dd, *J* = 16.7, 4.9 Hz, 1H), 5.73 (m, 1H), 4.35 (d, *J* = 52.5 Hz, 2H), 3.84 (dt, *J* = 17.8, 5.2 Hz, 2H), 3.68 (s, 3H), 2.89 (d, *J* = 37.3 Hz, 2H), 2.31 (s, 3H).; LC-MS (ESI) *m*/*z :* 380 [M+H]⁺

Examples 2 to 49 were prepared in a similar manner to that of Example 1, and the structure and analysis results of the compounds are summarized in Table 1 below.

**[Table 1]**

| Exam ple | Structure | Compound name | ¹H NMR; LC-MS (ESI) *m*/*z* |
|---|---|---|---|
| 2 | 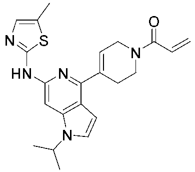 | 1-(4-(1-isopropyl-6-((5-methylthiazol-2-yl)amino)-1*H*-pyrrolo[3,2-*c*]pyridin-4-yl)-3,6-dihydropyridin-1*(2H)-*yl)prop-2-en-1-one | ¹H NMR (500 MHz, DMSO-*d*₆) δ 10.59 (s, 1H), 7.46 (d, *J =* 3.1 Hz, 1H), 7.00-6.80 (m, 3H), 6.69 (m, 1H), 6.59 (br s, 1H), 6.17 (dd, *J* = 16.6, 4.8 Hz, 1H), 5.73 (m, 1H), 4.52 (m, 1H), 4.35 (d, *J* = 53.1 Hz, 2H), 3.84 (dt, *J* = 18.9, 5.4 Hz, 2H), 2.88 (d, *J* = 37.3 Hz, 2H), 2.31 (s, 3H), 1.44 (d, *J* = 6.6 Hz, 6H).; 408 [M+H]⁺ |
| 3 | 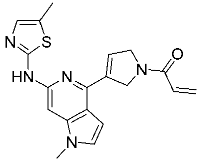 | 1-(3-(1-methyl-6-((5-methylthiazol-2-yl)amino)-1*H*-pyrrolo[3,2-c]pyridin-4-yl)-2,5-dihydro-1H-pyrrol-1-yl)prop-2-en-1-one | ¹H NMR (500 MHz, DMSO-*d*₆) δ 10.78 (d, *J* = 8.7 Hz, 1H), 7.39 (m, 1H), 6.99 (s, 1H), 6.93 (d, *J* = 15.4 Hz, 1H), 6.90-6.64 (m, 3H)., 5.13 (s, 1H), 4.89 (s, 1H), 4.72 (s, 1H), 4.50 (s, 1H), 3.71 (s, 3H), 2.34 (d, *J =* 8.0 Hz, 3H).; 366 [M+H]⁺ |
| 4 | 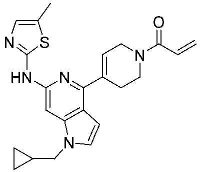 | 1-(4-(1-(cyclopropylmethyl)-6-((5-methylthiazol-2-yl)amino)-1*H*-pyrrolo[3,2-*c*]pyridin-4-yl)-3,6-dihydropyridin-1(2*H*)-yl)prop-2-en-1-one | ¹H NMR (500 MHz, CDCl₃) δ 7.12 (d, *J* = 3.3 Hz, 1H), 7.06 (d, *J* = 1.0 Hz, 1H), 6.80- 6.54 (m, 4H), 6.37 (dd, *J* = 16.7, 6.6 Hz, 1H), 5.74 (dd, *J =* 10.6, 1.0 Hz, 1H), 4.40 (d, *J* = 33.9 Hz, 2H), 3.99 (t, *J* = 5.3 Hz, 1H), 3.88 (d, *J* = 6.8 Hz, 3H), 3.02 (d, *J* = 12.7 Hz, 2H), 2.42 (s, 3H), 1.27-1.22 (m, 1H), 0.69-0.59 (m, 2H), 0.36 (q, *J* = 5.0 Hz, 2H).; 420 [M+H]⁺ |
| 5 | 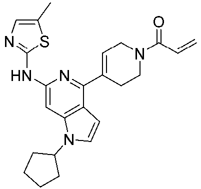 | 1-(4-(1-cyclopentyl-6-((5-methylthiazol-2-yl)amino)-1*H*-pyrrolo[3,2-c]pyridin-4-yl)-3,6-dihydropyridin-1*(2H)-*yl)prop-2-en-1-one | ¹H NMR (500 MHz, CDCl₃) δ 10.54 (d, *J* = 14.9 Hz, 1H), 7.11-7.03 (m, 2H), 6.77-6.54 (m, 4H), 6.36 (dd, *J* = 16.8, 6.2 Hz, 1H), 5.74 (d, *J* = 10.6 Hz, 1H), 4.63 (dt, *J* = 13.9, 6.9 Hz, 1H), 4.43 (d, *J* = 1.7 Hz, 1H), 4.36 (s, 1H), 3.99 (t, *J* = 5.3 Hz, 1H), 3.87 (t, *J* = 5.4 Hz, 1H), 3.03 (d, *J* = 12.3 Hz, 2H), 2.41 (s, 3H), 2.24-2.15 (m, 2H), 1.96-1.75 (m, 6H).; 434 [M+H]⁺ |
| 6 | 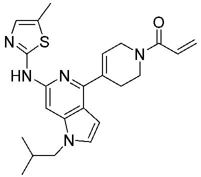 | 1-(4-(1-isobutyl-6-((5-methylthiazol-2-yl)amino)-1H-pyrrolo[3,2-c]pyridin-4-yl)-3,6-dihydropyridin-1*(2H)-*yl)prop-2-en-1-one | ¹H NMR (500 MHz, DMSO-*d*₆) δ 10.55 (s, 1H), 7.32 (d, *J =* 2.8 Hz, 1H), 7.02-6.77 (m, 3H), 6.64 (d, *J* = 27.4 Hz, 2H), 6.17 (dd, *J* = 3, 4.2 Hz, 1H), 5.73 (d, *J* = 10.1 Hz, 1H), 4.36 (d, *J* = 51.6 Hz, 2H), 3.85 (dd, *J* = 15.6, 6.3 Hz, 4H), 2.89 (d, *J* = 37.3 Hz, 2H), 2.31 (s, 3H), 2.11 (dt, *J* = 13.6, 6.7 Hz, 1H), 0.85 (d, *J* = 6.6 Hz, 6H).; 422 [M+H]⁺ |
| 7 | 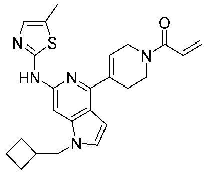 | 1-(4-(1-(cyclobutylmethyl)-6-((5-methylthiazol-2-yl)amino)-1*H*-pyrrolo[3,2-*c*]pyridin-4-yl)-3,6-dihydropyridin-1(2*H*)-yl)prop-2-en-1-one | ¹H NMR (500 MHz, CDCl₃) δ 7.03-6.97 (m, 2H), 6.77-6.60 (m, 2H), 6.58 (d, *J* = 17.7 Hz, 2H), 6.37 (dd, *J* = 16.8, 7.1 Hz, 1H), 5.75 (dd, *J* = 10.6, 1.3 Hz, 1H), 4.41 (d, *J* = 30.9 Hz, 2H), 4.01 (dd, *J* = 16.2, 6.3 Hz, 3H), 3.88 (t, *J* = 5.5 Hz, 1H), 3.02 (d, *J* = 11.0 Hz, 2H), 2.40 (s, 3H), 2.08- 2.05 (m, 1H), 1.97-1.75 (m, 6H).; 434 [M+H]⁺ |
| 8 | 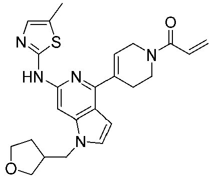 | 1-(4-(6-((5-methylthiazol-2-yl)amino)-1-((tetrahydrofuran-3-yl)methyl)-1*H*-pyrrolo[3,2-*c*]pyridin-4-yl)-3,6-dihydropyridin-1(2*H*)-yl)prop-2-en-1-one | ¹H NMR (500 MHz, CDCl₃) δ 9.29 (s, 1H), 7.06-6.94 (m, 2H), 6.76-6.53 (m, 4H), 6.37 (dd, *J* = 16.7, 7.2 Hz, 1H), 5.75 (d, *J* = 10.6 Hz, 1H), 4.41 (d, *J* = 32.1 Hz, 2H), 4.03-3.95 (m, 4H), 3.90-3.85 (m, 1H), 3.82-3.67 (m, 3H), 3.61 (dd, *J* = 8.9, 4.4 Hz, 1H), 3.03 (s, 2H), 2.41 (s, 3H), 2.06-1.98 (m, 2H).; 450 [M+H]⁺ |
| 9 | 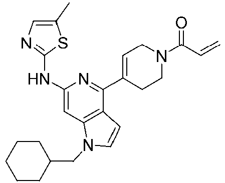 | 1-(4-(1-(cyclohexylmethyl)-6-((5-methylthiazol-2-yl)amino)-1*H*-pyrrolo[3,2-*c*]pyridin-4-yl)-3,6-dihydropyridin-1(2*H*)-yl)prop-2-en-1-one | ¹H NMR (500 MHz, DMSO-*d*₆) δ 10.52 (s, 1H), 7.30 (d, *J* = 2.9 Hz, 1H), 6.94 (dd, *J =* 18.1, 10.1 Hz, 3H), 6.63 (d, *J* = 24.8 Hz, 2H), 6.17 (dd, *J* = 16.6, 4.1 Hz, 1H), 5.73 (d, *J* = 10.3 Hz, 1H), 4.35 (d, *J* = 51.9 Hz, 2H), 3.9-3..84 (m, 4H), 2.89 (d, *J* = 37.1 Hz, 2H), 2.31 (s, 3H), 1.70-1.40 (m, 7H), 1.14 (t, *J* = 9.6 Hz, 4H).; 462 [M+H]⁺ |
| 10 | 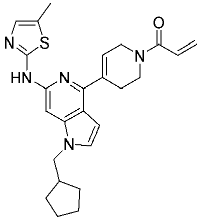 | 1-(4-(1-(cyclopentylmethyl)-6-((5-methylthiazol-2-yl)amino)-1*H*-pyrrolo[3,2-*c*]pyridin-4-yl)-3,6-dihydropyridin-1(2*H*)-yl)prop-2-en-1-one | ¹H NMR (500 MHz, DMSO-*d*₆) δ 10.55 (s, 1H), 7.37 (s, 1H), 6.95 (d, *J* = 7.6 Hz, 3H), 6.64 (d, *J* = 27.0 Hz, 2H), 6.18 (d, *J* = 16.3 Hz, 1H), 5.74 (d, *J =* 9.7 Hz, 1H), 4.41 (s, 1H), 4.31 (s, 1H), 3.97 (d, *J* = 7.4 Hz, 2H), 3.85 (d, *J* = 18.7 Hz, 2H), 2.90 (d, *J* = 38.2 Hz, 2H), 2.32 (s, 3H), 1.68-1.41 (m, 9H).; 448 [M+H]⁺ |
| 11 | 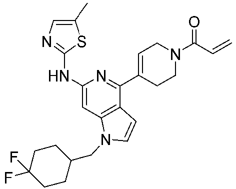 | 1-(4-(1-((4,4-difluorocyclohexyl)methyl)-6-((5-methylthiazol-2-yl)amino)-1*H*-pyrrolo[3,2-*c*]pyridin-4-yl)-3,6-dihydropyridin-1(2*H*)-yl)prop-2-en-1-one, | ¹H NMR (500 MHz, CDCl₃) δ 7.05-6.97 (m, 2H), 6.65 (dd, *J* = 40.0, 16.7 Hz, 4H), 6.40 (dd, *J* = 16.7, 7.1 Hz, 1H), 5.78 (d, *J = 10.5* Hz, 1H), 4.44 (d, *J =* 31.9 Hz, 2H), 4.07-3.87 (m, 4H), 3.04 (d, *J* = 13.9 Hz, 2H), 2.43 (s, 3H), 2.14 (s, 1H), 1.69 (dd, *J* = 34.5, 13.2 Hz, 5H), 1.40 (dt, *J* = 21.7, 10.9 Hz, 3H).; 498 [M+H]⁺ |
| 12 | 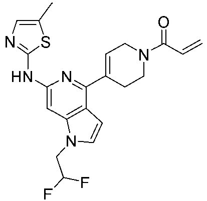 | 1-(4-(1-(2,2-difluoroethyl)-6-((5-methylthiazol-2-yl)amino)-1*H*-pyrrolo[3,2-*c*]pyridin-4-yl)-3,6-dihydropyridin-1(2*H*)-yl)prop-2-en-1-one | ¹H NMR (500 MHz, CDCl₃) δ 7.04-6.96 (m, 2H), 6.78-6.52 (m, 4H), 6.37 (dd, *J* = 16.8, 7.7 Hz, 1H), 6.01 (tt, *J* = 55.0, 3.7 Hz, 1H), 5.78-5.71 (m, 1H), 4.48-4.31 (m, 4H), 4.00 (t, *J* = 5.2 Hz, 1H), 3.88 (t, *J =* 5.3 Hz, 1H), 3.01 (d, *J* = 14.9 Hz, 2H), 2.40 (d, *J* = 6.0 Hz, 3H), 2.28-2.10 (m, 1H).; 430 [M+H]⁺ |
| 13 | 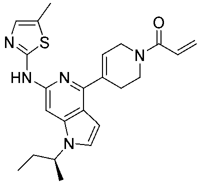 | (*S*)-1-(4-(1-(*sec*-butyl)-6-((5-methylthiazol-2-yl)amino)-1*H*-pyrrolo[3,2-c]pyridin-4-yl)-3,6-dihydropyridin-1*(2H)-*yl)prop-2-en-1-one | ¹H NMR (500 MHz, CDCl₃) δ 9.54 (br s, 1H), 7.09 (d, *J* = 3.3 Hz, 1H), 7.02 (s, 1H), 6.76-6.55 (m, 4H), 6.37 (dd, *J* = 16.8, 7.1 Hz, 1H), 5.75 (dd, *J* = 10.6, 1.5 Hz, 1H), 4.47-4.34 (m, 2H), 4.26 (m, 1H), 4.03-3.85 (m, 2H), 3.03 (m, 2H), 2.40 (s, 3H), 1.85 (m, 2H), 1.50 (d, *J* = 6.7 Hz, 3H), 0.84 (t, *J* = 7.4 Hz, 3H).; 422 [M+H]⁺ |
| 14 | 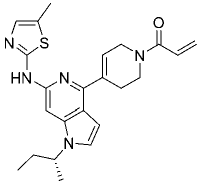 | (*R*)-1-(4-(1-(sec-butyl)-6-((5-methylthiazol-2-yl)amino)-1*H*-pyrrolo[3,2-*c*]pyridin-4-yl)-3,6-dihydropyridin-1*(2H)-*yl)prop-2-en-1-one, | ¹H NMR (500 MHz, CDCl₃) δ 9.54 (br s, 1H), 7.09 (d, *J* = 3.3 Hz, 1H), 7.02 (s, 1H), 6.76-6.55 (m, 4H), 6.37 (dd, *J* = 16.8, 7.1 Hz, 1H), 5.75 (dd, *J* = 10.6, 1.5 Hz, 1H), 4.47-4.34 (m, 2H), 4.26 (m, 1H), 4.03-3.85 (m, 2H), 3.03 (m, 2H), 2.40 (s, 3H), 1.85 (m, 2H), 1.50 (d, *J* = 6.7 Hz, 3H), 0.84 (t, *J* = 7.4 Hz, 3H).; 422 [M+H]⁺ |
| 15 | 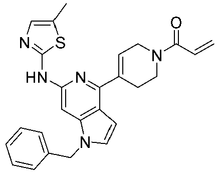 | 1-(4-(1-benzyl-6-((5-methylthiazol-2-yl)amino)-1*H*-pyrrolo[3,2-c]pyridin-4-yl)-3,6-dihydropyridin-1*(2H)-*yl)prop-2-en-1-one, | ¹H NMR (500 MHz, DMSO-*d*₆) δ 10.57 (s, 1H), 7.48 (d, *J =* 3.3 Hz, 1H), 7.33 (t, *J =* 7.4 Hz, 2H), 7.26 (t, *J* = 7.3 Hz, 1H), 7.17 (d, *J* = 7.2 Hz, 2H), 7.00-6.79 (m, 3H), 6.73 (m, 1H), 6.62 (m, 1H), 6.17 (m, 1H), 5.73 (m, 1H), 5.32 (s, 2H), 4.36 (d, *J* = 50.9 Hz, 2H), 3.84 (dt, *J* = 17.8, 5.5 Hz, 2H), 2.89 (d, *J* = 36.8 Hz, 2H), 2.31 (s, 3H).; 456 [M+H]⁺ |
| 16 | 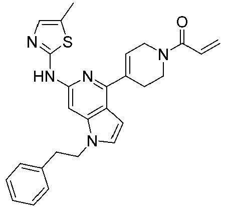 | 1-(4-(6-((5-methylthiazol-2-yl)amino)-1-phenethyl-1*H-*pyrrolo[3,2-*c*]pyridin-4-yl)-3,6-dihydropyridin-1(2*H*)-yl)prop-2-en-1-one | ¹H NMR (500 MHz, DMSO-*d₆*) δ 10.69 (s, 1H), 7.31-7.18 (*m*, 6H), 7.01-6.79 (m, 3H), 6.66-6.57 (m, 2H), 6.17 (m, 1H), 5.73 (m, 1H), 4.45-4.23 (m, 4H), 3.84 (dt, *J* = 17.9, 5.6 Hz, 2H), 3.05 (t, *J* = 7.4 Hz, 2H), 2.88 (d, *J* = 37.2 Hz, 2H), 2.32 (s, 3H).; 470 [M+H]⁺ |
| 17 | 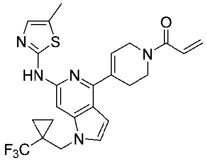 | 1-(4-(6-((5-methylthiazol-2-yl)amino)-1-((1-(trifluoromethyl)cyclopropyl) methyl)-1*H*-pyrrolo[3,2-*c*]pyridin-4-yl)-3,6-dihydropyridin-1(2*H*)-yl)prop-2-en-1-one | ¹H NMR (500 MHz, CDCl₃) δ 10.71 (s, 1H), 7.09 (s, 1H), 6.96 (d, *J* = 3.3 Hz, 1H), 6.79-6.53 (m, 4H), 6.37 (dd, *J =* 16.7, 6.7 Hz, 1H), 5.75 (d, *J* = 10.6 Hz, 1H), 4.41 (d, *J* = 28.0 Hz, 4H), 4.00 (t, *J* = 5.3 Hz, 1H), 3.88 (t, *J* = 5.5 Hz, 1H), 3.02 (d, *J* = 16.7 Hz, 2H), 2.42 (s, 3H), 1.06 (t, *J* = 6.1 Hz, 2H), 0.65 (s, 2H).; 488 [M+H]⁺ |
| 18 | 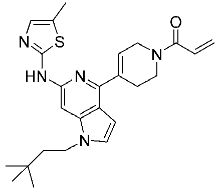 | 1-(4-(1-(3,3-dimethylbutyl)-6-((5-methylthiazol-2-yl)amino)-1*H*-pyrrolo[3,2-c]pyridin-4-yl)-3,6-dihydropyridin-1(2H)-yl)prop-2-en-1-one | ¹H NMR (500 MHz, CDCl₃) δ 7.06 (s, 1H), 7.00 (d, *J* = 3.1 Hz, 1H), 6.77-6.54 (m, 4H), 6.37 (dd, *J* = 16.7, 6.9 Hz, 1H), 5.75 (d, *J* = 10.7 Hz, 1H), 4.41 (d, *J* = 31.5 Hz, 2H), 4.07-3.98 (m, 3H), 3.88 (t, *J* = 5.4 Hz, 1H), 3.03 (d, *J* = 13.8 Hz, 2H), 2.40 (s, 3H), 1.70 (dd, *J* = 7.5, 4.3 Hz, 2H), 1.03 (s, 9H).; 450 [M+H]⁺ |
| 19 | 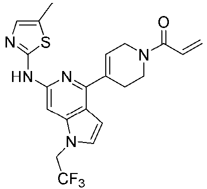 | 1-(4-(6-((5-methylthiazol-2-yl)amino)-1-(2,2,2-trifluoroethyl)-1H-pyrrolo[3,2-c]pyridin-4-yl)-3,6-dihydropyridin-1(2H)-yl)prop-2-en-1-one, | ¹H NMR (500 MHz, CDCl₃) δ 9.32 (s, 1H), 7.03 (s, 1H), 6.88-6.52 (m, 2H), 6.38 (dd, *J* = 16.7, 8.5 Hz, 1H), 5.76 (d, *J* = 10.5 Hz, 1H), 4.57 (dd, *J* = 16.7, 8.3 Hz, 1H), 4.42 (d, *J* = 32.1 Hz, 1H), 3.95 (dt, *J =* 58.8, 5.3 Hz, 1H), 3.01 (d, *J* = 16.4 Hz, 1H), 2.42 (d, *J* = 3.0 Hz, 2H).; 448 [M+H]⁺ |
| 20 | 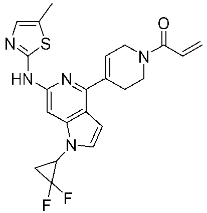 | 1-(4-(1-(2,2-difluorocyclopropyl)-6-((5-methylthiazol-2-yl)amino)-1*H*-pyrrolo[3,2-*c*]pyridin-4-yl)-3,6-dihydropyridin-1*(2H)-*yl)prop-2-en-1-one | ¹H NMR (500 MHz, CDCl₃) δ 9.92 (s, 1H), 7.10 (s, 1H), 7.02 (s, 1H), 6.90-6.52 (m, 4H), 6.38 (dd, *J* = 16.4, 7.4 Hz, 1H), 5.76 (d, *J* = 10.5 Hz, 1H), 4.42 (d, *J* = 31.8 Hz, 4H), 4.09-3.78 (m, 3H), 3.02 (d, *J =* 16.5 Hz, 1H), 2.43 (s, 3H), 2.30-1.90(m, 2H).; 442 [M+H]⁺ |
| 21 | 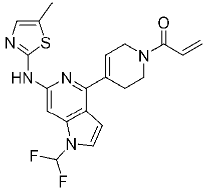 | 1-(4-(1-(difluoromethyl)-6-((5-methylthiazol-2-yl)amino)-1H-pyrrolo[3,2-c]pyridin-4-yl)-3,6-dihydropyridin-1(2H)-yl)prop-2-en-1-one, | ¹H NMR (500 MHz, DMSO-*d₆*) δ 10.88 (s, 1H), 7.89 (t, *J* = 59.4 Hz, 1H), 7.55 (d, *J* = 3.3 Hz, 1H), 7.18 (s, 1H), 7.03-6.77 (m, 3H), 6.58 (s, 1H), 6.18 (dd, *J* = 16.7, 4.3 Hz, 1H), 5.74 (dd, *J* = 11.2, 4.4 Hz, 1H), 4.36 (d, *J* = 51.4 Hz, 2H), 3.85 (dt, *J* = 18.3, 5.3 Hz, 2H), 2.88 (d, *J* = 38.3 Hz, 2H), 2.33 (s, 3H).; 416 [M+H]⁺ |
| 22 | 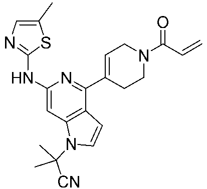 | 2-(4-(1-acryloyl-1,2,3,6-tetrahydropyridin-4-yl)-6-((5-methylthiazol-2-yl)amino)-1*H*-pyrrolo[3,2-c]pyridin-1-yl)-2-methylpropanenitrile | ¹H NMR (500 MHz, DMSO-*d₆*) δ 10.74 (s, 1H), 7.45 (d, *J* = 3.1 Hz, 1H), 7.32 (s, 1H), 7.01-6.75 (m, 3H), 6.57 (s, 1H), 6.18 (dd, *J* = 16.7, 4.9 Hz, 1H), 5.77-5.70 (m, 1H), 4.37 (d, *J* = 50.9 Hz, 2H), 3.85 (dt, *J* = 19.0, 5.4 Hz, 2H), 2.88 (d, *J* = 37.9 Hz, 2H), 2.33 (s, 3H), 2.04 (s, 6H).; 433 [M+H]⁺ |
| 23 | 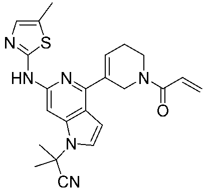 | 2-(4-(1-acryloyl-1,2,5,6-tetrahydropyridin-3-yl)-6-((5-methylthiazol-2-yl)amino)-1*H*-pyrrolo[3,2-*c*]pyridin-1-yl)-2-methylpropanenitrile | Rotamer(major) ¹H NMR (500 MHz, DMSO-*d₆*) δ 10.82 (s, 1H), 7.47 (m, 1H), 7.32 (s, 1H), 7.06-6.88 (m, 2H), 6.81-6.61 (m, 2H), 6.18 (dd, *J =* 22.8, 16.8 Hz, 1H), 5.74 (m, 1H), 4.81 (d, *J* = 27.9 Hz, 2H), 3.78 (dt, *J* = 15.8, 5.4 Hz, 2H), 2.50-2.38 (m, 2H), 2.35 (s, 3H), 2.05 (s, 6H).; 433 [M+H]⁺ |
| 24 | 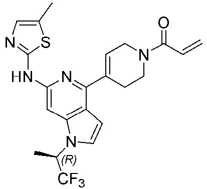 | (R)-1-(4-(6-((5-methylthiazol-2-yl)amino)-1-(1,1,1-trifluoropropan-2-yl)-1H-pyrrolo[3,2-c]pyridin-4-yl)-3,6-dihydropyridin-1(2H)-yl)prop-2-en-1-one | ¹H NMR (500 MHz, CDCl₃) δ 7.14 (d, *J* = 3.3 Hz, 1H), 7.04 (s, 1H), 6.81-6.51 (m, 4H), 6.38 (dd, *J* = 16.6, 7.6 Hz, 1H), 5.76 (dd, *J* = 10.5, 1.4 Hz, 1H), 4.84 (m, 1H), 4.41 (d, *J* = 32.7 Hz, 2H), 4.05-3.83 (m, 2H), 3.10-2.90 (m, 2H), 2.41 (s, 3H), 1.78 (d, *J* = 7.1 Hz, 3H).; 462 [M+H]⁺ |
| 25 | 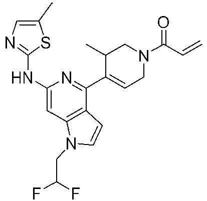 | 1-(4-(1-(2,2-difluoroethyl)-6-((5-methylthiazol-2-yl)amino)-1H-pyrrolo[3,2-c]pyridin-4-yl)-3-methyl-3,6-dihydropyridin-1(2H)-yl)prop-2-en-1-one | ¹H NMR (500 MHz, CDCl₃) δ 10.00 (s, 1H), 7.06 (d, *J* = 0.8 Hz, 1H), 7.00 (d, *J* = 3.3 Hz, 1H), 6.77-6.62 (m, 3H), 6.35 (dd, *J* = 43.0, 27.4 Hz, 2H), 6.15-5.89 (m, 1H), 5.75 (d, *J =* 10.5 Hz, 1H), 4.37 (td, *J =* 14.1, 3.7 Hz, 2H), 3.94 (dt, *J =* 21.7, 7.0 Hz, 1H), 3.84-3.64 (m, 2H), 2.41 (s, 3H), 1.79 (ddd, *J* = 18.8, 13.4, 7.1 Hz, 1H), 1.05 (s, 3H).; 444 [M+H]⁺ |
| 26 | 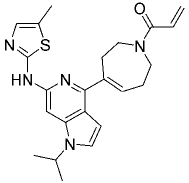 | 1-(4-(1-isopropyl-6-((5-methylthiazol-2-yl)amino)-1H-pyrrolo[3,2-c]pyridin-4-yl)-2,3,6,7-tetrahydro-1*H-*azepin-1-yl)prop-2-en-1-one | ¹H NMR (500 MHz, CDCl₃) δ 9.14 (br s, 1H), 7.14-7.09 (m, 1H), 6.99 (s, 1H), 6.75-6.49 (m, 4H), 6.35 (d, *J* = 18.1 Hz, 1H), 5.70 (d, *J* = 9.1 Hz, 1H), 4.59-4.49 (m, 1H), 4.41 (d, *J* = 5.2 Hz, 1H), 4.29 (d, *J* = 5.1 Hz, 1H), 3.95 (t, *J* = 6.0 Hz, 1H), 3.85 (t, *J* = 5.9 Hz, 1H), 3.14-3.02 (m, 2H), 2.38 (s, 3H), 2.18-2.05 (m, 2H), 1.51 (d, *J* = 6.4 Hz, 6H).; 422 [M+H]⁺ |
| 27 | 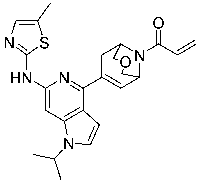 | 1-(7-(1-isopropyl-6-((5-methylthiazol-2-yl)amino)-1H-pyrrolo[3,2-c]pyridin-4-yl)-3-oxa-9-azabicyclo[3.3.1]non-6-en-9-yl)prop-2-en-1-one | ¹H NMR (500 MHz, CDCl₃) δ 9.08 (br s, 1H), 7.13 (t, *J =* 3.5 Hz, 1H), 7.00 (s, 1H), 6.75 (s, 1H), 6.70-6.57 (m, 3H), 6.38 (ddd, *J* = 16.7, 12.8, 1.4 Hz, 1H), 5.77 (d, *J* = 10.5 Hz, 2H), 5.34 (d, *J =* 5.0 Hz, 0.6H), 5.04 (d, *J* = 6.6 Hz, 0.4H), 4.66 (d, J = 4.8 Hz, 0.4H), 4.55 (m, 2H), 4.38 (d, *J* = 6.3 Hz, 0.6H), 4.08-4.01 (m, 1H), 3.91-3.73 (m, 3H), 3.43-3.09 (m, 2H), 2.40 (s, 1H), 2.38 (s, 2H), 1.57-1.45 (m, 6H).; 450 [M+H]⁺ |
| 28 | 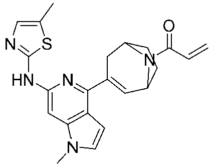 | 1-(3-(1-methyl-6-((5-methylthiazol-2-yl)amino)-1*H*-pyrrolo[3,2-c]pyridin-4-yl)-8-azabicyclo[3.2.1]oct-2-en-8-yl)prop-2-en-1-one | ¹H NMR (500 MHz, DMSO-*d₆*) δ 10.65 (s, 1H), 7.29 (d, *J* = 3.2 Hz, 1H), 6.96 (s, 1H), 6.91-6.75 (m, 3H), 6.58 (d, *J* = 3.1 Hz, 1H), 6.18 (td, *J* = 16.5, 2.2 Hz, 1H), 5.70 (ddd, *J =* 15.6, 10.3, 2.2 Hz, 1H), 4.93 (dd, *J* = 10.6, 5.2 Hz, 1H), 4.84 (dd, *J* = 11.9, 7.3 Hz, 1H), 3.67 (s, 3H), 3.17 (t, *J* = 18.8 Hz, 1H), 2.90-2.79 (m, 1H), 2.31 (s, 3H), 2.21-1.71 (m, 4H).; 406 [M+H]⁺ |
| 29 | 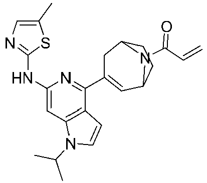 | 1-(3-(1-isopropyl-6-((5-methylthiazol-2-yl)amino)-1*H*-pyrrolo[3,2-c]pyridin-4-yl)-8-azabicyclo[3.2.1]oct-2-en-8-yl)prop-2-en-1-one | ¹H NMR (500 MHz, DMSO-*d₆*) δ 10.57 (s, 1H), 7.44 (d, *J =* 3.0 Hz, 1H), 6.99-6.75 (m, 4H), 6.61 (d, *J* = 3.3 Hz, 1H), 6.18 (td, *J* = 16.8, 2.2 Hz, 1H), 5.70 (ddd, *J* = 15.4, 10.3, 2.2 Hz, 1H), 4.93 (dd, *J* = 9.9, 4.9 Hz, 1H), 4.87-4.81 (m, 1H), 4.50 (m, 1H), 3.24-3.09 (m, 1H), 2.89-2.77 (m, 1H), 2.32 (s, 3H), 2.22-1.70 (m, 4H).; 434 [M+H]⁺ |
| 30 | 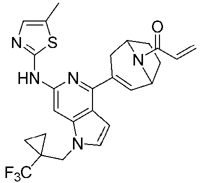 | 1-(3-(6-((5-methylthiazol-2-yl)amino)-1-((1-(trifluoromethyl)cyclopropyl) methyl)-1*H*-pyrrolo[3,2-*c*]pyridin-4-yl)-8-azabicyclo[3.2.1]oct-2-en-8-yl)prop-2-en-1-one | ¹H NMR (500 MHz, DMSO-*d₆*) δ 7.36 (d, *J* = 2.8 Hz, 1H), 6.99-6.94 (m, 2H), 6.90-6.75 (m, 2H), 6.64 (d, *J* = 3.3 Hz, 1H), 6.18 (td, *J* = 16.6, 2.2 Hz, 1H), 5.70 (ddd, *J* = 15.0, 10.3, 2.2 Hz, 1H), 4.93 (dd, J = 10.2, 5.1 Hz, 1H), 4.85 (d, *J* = 6.3 Hz, 1H), 4.34 (s, 2H), 3.17 (dd, *J* = 23.9, 18.1 Hz, 1H), 2.84 (dd, *J* = 25.8, 17.5 Hz, 1H), 2.32 (d, *J* = 7.7 Hz, 3H), 2.17-1.74 (m, 4H), 1.04 (s, 4H).; 514 [M+H]⁺ |
| 31 | 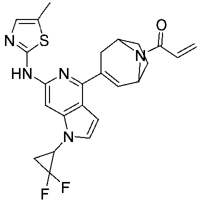 | 1-(3-(1-(2,2-difluorocyclopropyl)-6-((5-methylthiazol-2-yl)amino)-1*H*-pyrrolo[3,2-*c*]pyridin-4-yl)-8-azabicyclo[3.2.1]oct-2-en-8-yl)prop-2-en-1-one | ¹H NMR (500 MHz, DMSO-*d*₆) δ 10.73 (s, 1H), 7.33 (d, *J =* 3.4 Hz, 1H), 7.04-6.74 (m, 4H), 6.64 (d, *J* = 3.3 Hz, 1H), 6.19 (dt, *J* = 17.0, 8.7 Hz, 1H), 5.75-5.65 (m, 1H), 4.93 (dd, *J* = 11.1, 5.5 Hz, 1H), 4.84 (d, *J* = 4.6 Hz, 1H), 4.25 (dd, *J =* 15.6, 9.1 Hz, 1H), 3.18 (m, 1H), 2.2 (m, 1H), 2.41-2.24 (m, 5H), 2.22-2.72 (m, 4H).; 468 [M+H]⁺ |
| 32 | 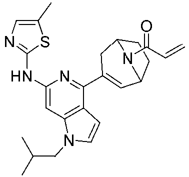 | 1-(3-(1-isobutyl-6-((5-methylthiazol-2-yl)amino)-1*H*-pyrrolo[3,2-c]pyridin-4-yl)-8-azabicyclo[3.2.1]oct-2-en-8-yl)prop-2-en-1-one | ¹H NMR (500 MHz, DMSO-*d*₆) δ 10.52 (s, 1H), 7.32 (d, *J =* 3.1 Hz, 1H), 6.97-6.77 (m, 4H), 6.61 (d, *J* = 2.9 Hz, 1H), 6.20 (dd, *J* = 23.7, 9.0 Hz, 1H), 5.78-5.66 (m, 1H), 4.95-4.82 (m, 2H), 3.86 (d, *J* = 7.1 Hz, 2H), 3.18 (dd, *J* = 27.7, 14.5 Hz, 1H), 2.85 (dd, *J =* 24.5, 17.4 Hz, 1H), 2.32 (s, 3H), 2.12-2.06 (m, 2H), 1.97-1.77 (m, 2H), 0.86-0.84 (m, 6H).; 448 [M+H]+ |
| 33 | 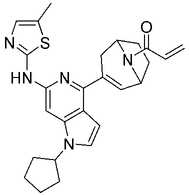 | 1-(3-(1-cyclopentyl-6-((5-methylthiazol-2-yl)amino)-1*H*-pyrrolo[3,2-c]pyridin-4-yl)-8-azabicyclo[3.2.1]oct-2-en-8-yl)prop-2-en-1-one | ¹H NMR (500 MHz, CDCl₃) δ 11.10 (s, 1H), 7.06-6.95 (m, 2H), 6.83-6.64 (m, 2H), 6.64-6.43 (m, 2H), 6.33 (dd, *J =* 27.0, 16.4 Hz, 1H), 5.64 (dd, *J* = 14.6, 11.0 Hz, 1H), 5.11 (d, *J* = 27.6 Hz, 1H), 4.70-4.46 (m, 2H), 3.46-2.83 (m, 2H), 2.37 (s, 3H), 2.16-2.05 (m, 4H), 1.81 (s, 6H), 1.75-1.62 (m, 2H).; 460 [M+H]⁺ |
| 34 | 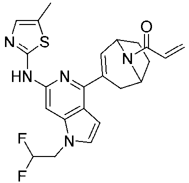 | 1-(3-(1-(2,2-difluoroethyl)-6-((5-methylthiazol-2-yl)amino)-1*H*-pyrrolo[3,2-*c*]pyridin-4-yl)-8-azabicyclo[3.2.1]oct-2-en-8-yl)prop-2-en-1-one | ¹H NMR (500 MHz, CDCl₃) δ 7.00 (d, *J* = 2.5 Hz, 2H), 6.82-6.53 (m, 4H), 6.39 (ddd, *J =* 22.2, 16.8, 1.6 Hz, 1H), 5.99 (t, *J* = 55.0 Hz, 1H), 5.75-5.67 (m, 1H), 5.21-5.08 (m, 1H), 4.73-4.63 (m, 1H), 4.36 (td, *J* = 14.1, 3.3 Hz, 2H), 2.41 (d, *J* = 6.5 Hz, 3H), 2.29-1.89 (m, 6H).; 456 [M+H]⁺ |
| 35 | 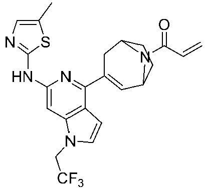 | 1-(3-(6-((5-methylthiazol-2-yl)amino)-1-(2,2,2-trifluoroethyl)-1H-pyrrolo[3,2-c]pyridin-4-yl)-8-azabicyclo[3.2.1]oct-2-en-8-yl)prop-2-en-1-one | ¹H NMR (500 MHz, DMSO-*d₆*) δ 10.72 (s, 1H), 7.35 (d, *J =* 3.4 Hz, 1H), 7.02-6.76 (m, 4H), 6.71 (d, *J* = 3.3 Hz, 1H), 6.18 (td, *J* = 16.7, 2.2 Hz, 1H), 5.76-5.66 (m, 1H), 5.10 (q, *J* = 9.1 Hz, 2H), 4.95-4.80 (m, 2H), 3.18 (t, *J* = 18.7 Hz, 1H), 2.82 (t, *J* = 18.0 Hz, 1H), 2.32 (d, *J* = 7.9 Hz, 3H), 2.21-1.70 (m, 4H).474 [M+H]⁺ |
| 36 | 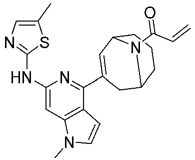 | 1-(3-(1-methyl-6-((5-methylthiazol-2-yl)amino)-1H-pyrrolo[3,2-c]pyridin-4-yl)-9-azabicyclo[3.3.1]non-2-en-9-yl)prop-2-en-1-one | ¹H NMR (500 MHz, CDCl₃) δ 7.03 (s, 1H), 6.95 (d, J = 3.2 Hz, 1H), 6.74-6.62 (m, 2H), 6.57 (dd, *J* = 21.6, 3.1 Hz, 1H), 6.47 (m, 1H), 6.32 (d, *J* = 16.8 Hz, 1H), 5.70 (td, J = 16.8, 2.0 Hz, 1H), 5.50 (s, 0.55H), 5.27 (s, 0.45H), 4.82 (s, 0.45H), 4.59 (s, 0.55H), 3.70 (s, 3H), 3.35-2.85(m, 2H), 2.40 (s, 1.35H), 2.39 (s, 1.65H), 2.10 (m, 1H), 1.91-1.59 (m, 5H).; 420 [M+H]⁺ |
| 37 | 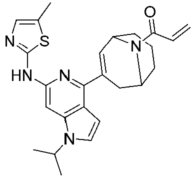 | 1-(3-(1-isopropyl-6-((5-methylthiazol-2-yl)amino)-1*H*-pyrrolo[3,2-c]pyridin-4-yl)-9-azabicyclo[3.3.1]non-2-en-9-yl)prop-2-en-1-one | ¹H NMR (500 MHz, CDCl₃) δ 7.15 (d, *J* = 2.9 Hz, 1H), 6.99 (s, 1H), 6.78 (s, 1H), 6.72-6.57 (m, 2H), 6.48 (t, J = 5.0 Hz, 1H), 6.33 (m, 1H), 5.71 (d, *J = 10.6* Hz, 1H), 5.51 (s, 0.55H), 5.27 (s, 0.45H), 4.83 (s, 0.45H), 4.62-4.51 (m, 1.55H), 3.34-2.84 (m, 2H), 2.40 (s, 1.35H), 2.39 (s, 1.65H), 2.10 (m, 1H), 1.95-1.60 (m, 7H), 1.51 (d, *J* = 6.4 Hz, 11H).; 448 [M+H]⁺ |
| 38 | 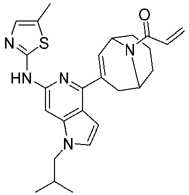 | 1-(3-(1-isobutyl-6-((5-methylthiazol-2-yl)amino)-1*H*-pyrrolo[3,2-c]pyridin-4-yl)-9-azabicyclo[3.3.1]non-2-en-9-yl)prop-2-en-1-one | ¹H NMR (500 MHz, CDCl₃) δ 7.08-6.93 (m, 2H), 6.71-6.45 (m, 4H), 6.33 (ddd, *J* = 16.8, 4.4, 1.6 Hz, 1H), 5.71 (d, *J* = 10.9 Hz, 1H), 5.39 (d, *J* = 116.5 Hz, 1H), 4.72 (d, *J* = 115.7 Hz, 1H), 3.82 (d, *J* = 7.3 Hz, 2H), 3.16 (s, 1H), 2.41 (d, *J* = 6.2 Hz, 3H), 2.26-1.97 (m, 3H), 1.97-1.57 (m, 5H), 0.99-0.83 (m, 6H).; 462 [M+H]⁺ |
| 39 | 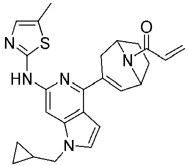 | 1-(3-(1-(cyclopropylmethyl)-6-((5-methylthiazol-2-yl)amino)-1*H*-pyrrolo[3,2-*c*]pyridin-4-yl)-8-azabicyclo[3.2.1]oct-2-en-8-yl)prop-2-en-1-one | ¹H NMR (500 MHz, CDCl₃) δ 11.24 (br s, 1H), 7.04 (s, 1H), 7.01 (d, *J* = 3.3 Hz, 1H), 6.79 (dd, *J* = 11.3, 5.2 Hz, 1H), 6.67-6.49 (m, 3H), 6.36 (ddd, *J* = 27.8, 16.8, 1.6 Hz, 1H), 5.66 (ddd, *J* = 18.4, 10.4, 1.6 Hz, 1H), 5.14 (m, 1H), 4.66 (m, 1H), 3.77 (d, *J* = 6.7 Hz, 2H), 3.50-2.85 (m, 2H), 2.40 (s, 1.5H), 2.39 (s, 1.5H), 2.34-1.82 (m, 6H), 1.13 (m, 1H), 0.58-0.51 (m, 2H), 0.30-0.23 (m, 2H).; 446 [M+H]⁺ |
| 40 | 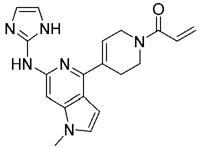 | 1-(4-(6-((1*H*-imidazol-2-yl)amino)-1-methyl-1*H-*pyrrolo[3,2-c]pyridin-4-yl)-3,6-dihydropyridin-1*(2H)-*yl)prop-2-en-1-one | ¹H NMR (500 MHz, CDCl₃) δ 6.96 (d, *J* = 3.3 Hz, 1H), 6.86 (s, 2H), 6.76-6.47 (m, 4H), 6.38 (d, *J* = 16.8 Hz, 1H), 5.77 (d, *J* = 10.5 Hz, 1H), 4.41 (d, *J* = 33.8 Hz, 2H), 4.05-3.855 (m, 2H), 3.72 (s, 3H), 2.87 (s, 2H).; 349 [M+H]⁺ |
| 41 | 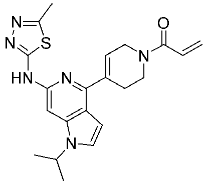 | 1-(4-(1-isopropyl-6-((5-methyl-1,3,4-thiadiazol-2-yl)amino)-1*H*-pyrrolo[3,2-*c*]pyridin-4-yl)-3,6-dihydropyridin-1(2*H*)-yl)prop-2-en-1-one | ¹H NMR (500 MHz, CDCl₃) δ 7.23 (d, *J* = 9.0 Hz, 1H), 7.17 (d, *J* = 3.3 Hz, 1H), 6.78-6.52 (m, 3H), 6.41- 6.34 (m, 1H), 5.76 (d, *J* = 10.5 Hz, 1H), 4.71-4.63 (m, 1H), 4.45 (s, 1H), 4.39 (s, 1H), 4.01 (t, *J* = 5.4 Hz, 1H), 3.89 (t, *J =* 5.5 Hz, 1H), 3.05-2.95 (m, 2H), 2.71 (m, 3H), 1.57 (d, *J* = 6.7 Hz, 6H).; 409 [M+H]⁺ |
| 42 | 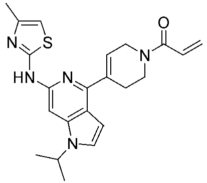 | 1-(4-(1-isopropyl-6-((4-methylthiazol-2-yl)amino)-1*H*-pyrrolo[3,2-*c*]pyridin-4-yl)-3,6-dihydropyridin-1*(2H)-*yl)prop-2-en-1-one | ¹H NMR (500 MHz, CDCl₃) δ 8.53 (br s, 1H), 7.15 (d, *J =* 3.1 Hz, 1H), 6.76-6.55 (m, 4H), 6.41-6.27 (m, 2H), 5.75 (d, *J* = 10.6 Hz, 1H), 4.54 (m, 1H), 4.46-4.35 (m, 2H), 4.02-3.85 (m, 2H), 3.08-2.96 (m, 2H), 2.34 (s, 3H), 1.51 (d, *J* = 6.7 Hz, 6H).; 408 [M+H]⁺ |
| 43 | 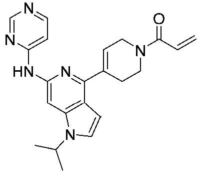 | 1-(4-(1-isopropyl-6-(pyrimidin-4-ylamino)-1*H-*pyrrolo[3,2-*c*]pyridin-4-yl)-3,6-dihydropyridin-1*(2H)-*yl)prop-2-en-1-one | ¹H NMR (500 MHz, CDCl₃) δ 8.78 (s, 1H), 8.35 (d, *J* = 5.8 Hz, 1H), 7.78-7.52 (m, 2H), 7.24-7.16 (m, 2H), 6.73-6.45 (m, 3H), 6.36 (dd, *J* = 16.6, 9.0 Hz, 1H), 5.74 (dd, *J =* 10.6, 1.7 Hz, 1H), 4.67 (m, 1H), 4.46-4.31 (m, 2H), 3.99-3.80 (m, 2H), 2.90 (m, 2H), 1.55 (d, *J* = 6.7 Hz, 6H).; 389 [M+H]⁺ |
| 44 | 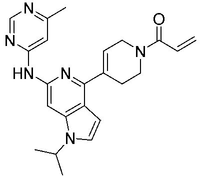 | 1-(4-(1-isopropyl-6-((6-methylpyrimidin-4-yl)amino)-1*H*-pyrrolo[3,2-*c*]pyridin-4-yl)-3,6-dihydropyridin-1(2*H*)-yl)prop-2-en-1-one | ¹H NMR (500 MHz, CDCl₃) δ 8.70 (s, 1H), 7.87 (m, 1H), 7.30-7.00 (m, 3H), 6.74-6.42 (m, 3H), 6.37 (dd, *J* = 16.6, 10.6 Hz, 1H), 5.75 (d, *J* = 10.6 Hz, 1H), 4.69 (m, 1H), 4.40 (m, 2H), 3.90 (m, 2H), 2.89 (m, 2H), 2.44 (s, 3H), 1.55 (d, *J* = 6.7 Hz, 6H).; 403 [M+H]⁺ |
| 45 | 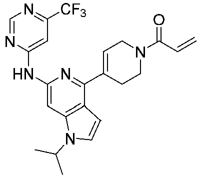 | 1-(4-(1-isopropyl-6-((6-(trifluoromethyl)pyrimidin-4-yl)amino)-1*H*-pyrrolo[3,2-*c*]pyridin-4-yl)-3,6-dihydropyridin-1(2*H*)-yl)prop-2-en-1-one | ¹H NMR (500 MHz, DMSO-*d*₆) δ 10.58 (s, 1H), 8.83 (s, 1H), 8.26 (br s, 1H), 7.60 (s, 1H), 6.99-6.55 (m, 3H), 6.17 (dd, *J* = 15.7, 10.0 Hz, 1H), 5.74 (d, *J* = 10.3 Hz, 1H), 4.63 (m, 1H), 4.42-4.25 (m, 2H), 3.86-3.75 (m, 2H), 2.85-2.70 (m, 2H), 1.48 (d, *J* = 6.6 Hz, 5H).; 457 [M+H]⁺ |
| 46 | 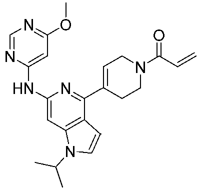 | 1-(4-(1-isopropyl-6-((6-methoxypyrimidin-4-yl)amino)-1*H*-pyrrolo[3,2-*c*]pyridin-4-yl)-3,6-dihydropyridin-1(2*H*)-yl)prop-2-en-1-one | ¹H NMR (500 MHz, CDCl₃) δ 8.46 (s, 1H), 7.57 (br s, 1H), 7.26-7.20 (m, 1H), 6.83-6.49 (m, 4H), 6.37 (dd, *J* = 16.3, 9.2 Hz, 1H), 5.76 (d, *J* = 11.5 Hz, 1H), 4.66 (m, 1H), 4.47-4.35 (m, 2H), 4.00-3.81 (m, 5H), 2.94-2.84 (m, 2H), 1.54 (d, *J* = 6.7 Hz, 6H).; 419 [M+H]⁺ |
| 47 | 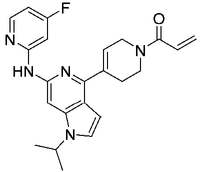 | 1-(4-(6-((4-fluoropyridin-2-yl)amino)-1-isopropyl-1*H-*pyrrolo[3,2-*c*]pyridin-4-yl)-3,6-dihydropyridin-1*(2H)-*yl)prop-2-en-1-one | ¹H NMR (500 MHz, CDCl₃) δ 8.20 (dd, *J* = 9.0, 5.8 Hz, 1H), 7.50-7.34 (m, 2H), 7.25-7.13 (m, 2H), 6.73-6.30 (m, 5H), 5.74 (dd, *J* = 10.6, 1.7 Hz, 1H), 4.62 (m, 1H), 4.44-4.30 (m, 2H), 3.98-3.80 (m, 2H), 2.90 (s, 2H), 1.53 (d, *J* = 6.7 Hz, 6H).; 406 [M+H]⁺ |
| 48 | 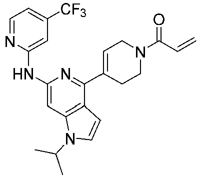 | 1-(4-(1-isopropyl-6-((4-(trifluoromethyl)pyridin-2-yl)amino)-1*H*-pyrrolo[3,2-*c*]pyridin-4-yl)-3,6-dihydropyridin-1(2*H*)-yl)prop-2-en-1-one | ¹H NMR (500 MHz, CDCl₃) δ 8.38 (d, *J* = 4.8 Hz, 1H), 8.05-7.25 (m, 3H), 7.18 (d, *J* = 3.1 Hz, 1H), 6.96 (d, *J* = 5.0 Hz, 1H), 6.73-6.47 (m, 3H), 6.35 (t, *J =* 15.1 Hz, 1H), 5.74 (d, *J* = 10.6 Hz, 1H), 4.62 (m, 1H), 4.45-4.30 (m, 2H), 3.98-3.79 (m, 2H), 2.95-2.85 (m, 2H), 1.54 (d, *J* = 6.6 Hz, 6H).; 456 [M+H]⁺ |
| 49 | 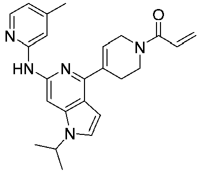 | 1-(4-(1-isopropyl-6-((4-methylpyridin-2-yl)amino)-1*H*-pyrrolo[3,2-c]pyridin-4-yl)-3,6-dihydropyridin-1*(2H)-*yl)prop-2-en-1-one | ¹H NMR (500 MHz, CDCl₃) δ 8.13 (d, *J* = 5.2 Hz, 1H), 7.77 (s, 1H), 7.48 (d, *J =* 16.1 Hz, 1H), 7.13 (d, *J* = 2.7 Hz, 1H), 6.99 (d, *J =* 19.1 Hz, 1H), 6.63 (ddd, *J* = 12.0, 11.4, 6.3 Hz, 3H), 6.48 (d, *J* = 30.6 Hz, 1H), 6.34 (t, *J* = 14.6 Hz, 1H), 5.72 (d, *J* = 10.5 Hz, 1H), 4.63 (m, 1H), 4.42-4.28 (m, 2H), 3.85 (dt, *J* = 71.7, 5.4 Hz, 2H), 2.89 (s, 2H), 2.27 (s, 3H), 1.51 (d, *J* = 6.7 Hz, 6H).; 402 [M+H]⁺ |

### Example 50. Preparation of N-(3-(1-methyl-6-((5-methylthiazol-2-yl)amino)-1H-pyrrolo[3,2-c]pyridin-4-yl)phenyl)acrylamide

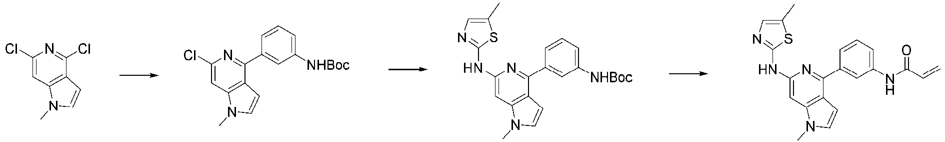

### Step 1. Preparation of tert-butyl (3-(6-chloro-1-methyl-1H-pyrrolo[3,2-c]pyridin-4-yl)phenyl)carbamate

*tert*-Butyl (3-(6-chloro-1-methyl-1*H*-pyrrolo[3,2-*c*]pyridin-4-yl)phenyl)carbamate (yield: 52%) was synthesized in the same manner as in step 1 of Example 1, except that (3-((tert-butoxycarbonyl)amino)phenyl)boronic acid was used instead of *tert*-butyl 4-(4,4,5,5-tetramethyl-1,3,2-dioxaborolan-2-yl)-3,6-dihydropyridine-1(2H)-carboxylate used in the synthesis step 1 of Example 1.
LC-MS (ESI) *m*/*z :* 358 [M+H]⁺

### Step 2. Preparation of tert-butyl (3-(1-methyl-6-((5-methylthiazol-2-yl)amino)-1H-pyrrolo[3,2-c]pyridin-4-yl)phenyl)carbamate

*tert-Butyl(3-(1-methyl-6-((5-methylthiazol-2-yl)amino)-1H-pyrrolo[3,2-c]pyridin-4-*yl)phenyl)carbamate (yield: 27%) was synthesized in the same manner as in step 2 of Example 1, except that tert-butyl (3-(6-chloro-1-methyl-1*H*-pyrrolo[3,2-c]pyridin-4-yl)phenyl)carbamate was used instead of tert-butyl 4-(6-chloro-1-methyl-1H-pyrrolo[3,2-c]pyridin-4-yl)-3,6-dihydropyridine-1(2*H*)-carboxylate used in the synthesis step 2 of Example 1.
¹H NMR (500 MHz, CDCl₃) δ 8.53 (s, 1H), 8.12 (s, 1H), 7.82 (d, *J* = 6.9 Hz, 1H), 7.46 (m, 2H), 7.01 (m, 2H), 6.79 (m, 2*H*), 6.63 (s, 1H), 3.75 (s, 3*H*), 2.39 (s, 3H), 1.54 (s, 9H).; LC-MS (ESI) *m*/*z :* 436 [M+H]⁺

### Step 3. Preparation of N-(3-(1-methyl-6-((5-methylthiazol-2-yl)amino)-1H-pyrrolo[3,2-c]pyridin-4-yl)phenyl)acrylamide

*N*-(3-(1-methyl-6-((5-methylthiazol-2-yl)amino)-1*H*-pyrrolo[3,2-c]pyridin-4-yl)phenyl)acrylamide (yield: 77%) was synthesized in the same manner as in step 3 of Example 1, except that tert-butyl (3-(1-methyl-6-((5-methylthiazol-2-yl)amino)-1*H-*pyrrolo[3,2-c]pyridin-4-yl)phenyl)carbamate was used instead of tert-butyl 4-(1-methyl-6-((5-methylthiazol-2-yl)amino)-1*H*-pyrrolo[3,2-c]pyridin-4-yl)-3,6-dihydropyridine-1(2*H*)-carboxylate used in the synthesis step 3 of Example 1.
¹H NMR (500 MHz, DMSO-*d*₆) δ 11.28 (br s, 1H), 10.36 (s, 1H), 8.66 (s, 1H), 7.89 (d, *J* = 7.7 Hz, 1H), 7.66 (d, *J* = 7.9 Hz, 1H), 7.54 (t, *J* = 7.9 Hz, 1H), 7.45 (d, *J =* 3.2 Hz, 1H), 7.09 (s, 1H), 7.03 (s, 1H), 6.82 (d, *J* = 3.1 Hz, 1H), 6.50 (dd, *J* = 16.9, 10.1 Hz, 1H), 6.31 (dd, *J* = 16.9, 1.6 Hz, 1H), 5.80 (dd, *J* = 10.2, 1.6 Hz, 1H), 3.76 (s, 3H), 2.34 (s, 3H).; LC-MS (ESI) *m*/*z :* 390 [M+H]⁺

Examples 51 to 78 were prepared in a similar manner to that of Example 50, and the structure and analysis results of the compounds are summarized in Table 2 below.

**[Table 2]**

| Example | Structure | Compound name | ¹H NMR; LC-MS (ESI) *m*/*z* |
|---|---|---|---|
| 51 | 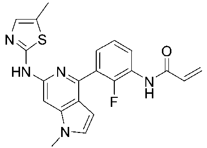 | *N*-(2-fluoro-3-(1-methyl-6-((5-methylthiazol-2-yl)amino)-1*H*-pyrrolo[3,2-*c*]pyridin-4-yl)phenyl)acrylamide | ¹H NMR (500 MHz, DMSO-*d₆*) δ 10.85 (s, 1H), 10.10 (s, 1H), 8.06 (t, *J* = 7.2 Hz, 1H), 7.60 (t, *J* = 6.8 Hz, 1H), 7.36 (t, *J =* 7.9 Hz, 1H), 7.33 (d, *J* = 3.3 Hz, 1H), 7.05 (s, 1H), 6.97 (s, 1H), 6.66 (dd, *J =* 17.0, 10.2 Hz, 1H), 6.36-6.29 (m, 2H), 5.80 (dd, *J =* 10.2, 1.7 Hz, 1H), 3.73 (s, 3H), 2.27 (s, 3H).; 408 [M+H]⁺ |
| 52 | 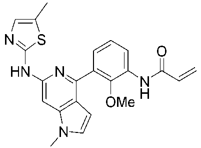 | N-(2-methoxy-3-(1-methyl-6-((5-methylthiazol-2-yl)amino)-1*H*-pyrrolo[3,2-*c*]pyridin-4-yl)phenyl)acrylamide | ¹H NMR (500 MHz, DMSO-*d₆*) δ 10.90 (s, 1H), 9.68 (s, 1H), 8.22 (d, *J* = 8.1 Hz, 1H), 7.37 (d, *J* = 7.5 Hz, 1H), 7.30-7.22 (m, 2H), 7.06 (s, 1H), 6.98 (s, 1H), 6.79 (dd, *J* = 16.9, 10.1 Hz, 1H), 6.33-6.22 (m, 2H), 5.76 (d, *J =* 10.1 Hz, 1H), 3.73 (s, 3H), 3.25 (s, 3H), 2.26 (s, 3H).; 420 [M+H]⁺ |
| 53 | 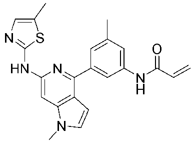 | *N*-(3-methyl-5-(1-methyl-6-((5-methylthiazol-2-yl)amino)-1*H*-pyrrolo[3,2-*c*]pyridin-4-yl)phenyl)acrylamide | ¹H NMR (500 MHz, DMSO-*d₆*) δ 10.77 (s, 1H), 10.25 (s, 1H), 8.39 (s, 1H), 7.76 (s, 1H), 7.53 (s, 1H), 7.39 (d, *J* = 3.2 Hz, 1H), 6.98 (s, 2H), 6.79 (d, *J* = 3.2 Hz, 1H), 6.49 (dd, *J* = 16.9, 10.1 Hz, 1H), 6.30 (dd, *J* = 17.0, 1.8 Hz, 1H), 5.78 (dd, *J* = 10.1, 1.8 Hz, 1H), 3.73 (s, 3H), 2.43 (s, 3H), 2.32 (s, 3H).; 404 [M+H]⁺ |
| 54 | 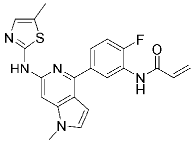 | *N*-(2-fluoro-5-(1-methyl-6-((5-methylthiazol-2-yl)amino)-1*H*-pyrrolo[3,2-*c*]pyridin-4-yl)phenyl)acrylamide | ¹H NMR (500 MHz, DMSO-*d₆*) δ 12.00 (s, 1H), 10.18 (s, 1H), 8.99 (d, *J* = 6.6 Hz, 1H), 7.93 (m, 1H), 7.56-7.44 (m, 2H), 7.23 (s, 1H), 7.10 (s, 1H), 6.86 (d, *J* = 2.9 Hz, 1H), 6.71 (dd, *J* = 17.0, 10.2 Hz, 1H), 6.34 (dd, *J* = 17.0, 1.4 Hz, 1H), 5.83 (dd, *J* = 10.3, 1.4 Hz, 1H), 3.79 (s, 3H), 2.37 (s, 3H).; 408 [M+H]⁺ |
| 55 | 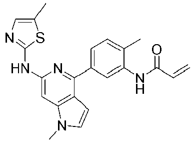 | *N*-(2-methyl-5-(1-methyl-6-((5-methylthiazol-2-yl)amino)-1*H*-pyrrolo[3,2-*c*]pyridin-4-yl)phenyl)acrylamide | ¹H NMR (500 MHz, DMSO-*d₆*) δ 10.76 (s, 1H), 9.61 (s, 1H), 8.41 (s, 1H), 7.92 (dd, *J* = 7.9, 1.3 Hz, 1H), 7.43 (d, *J* = 8.0 Hz, 1H), 7.38 (d, *J =* 3.2 Hz, 1H), 6.98 (d, *J* = 1.0 Hz, 1H), 6.94 (s, 1H), 6.81 (d, *J* = 3.1 Hz, 1H), 6.62 (dd, *J* = 16.9, 10.2 Hz, 1H), 6.31 (dd, *J* = 17.0, 1.8 Hz, 1H), 5.79 (dd, *J* = 10.2, 1.7 Hz, 1H), 3.72 (s, 3H), 2.33 (s, 3H), 2.31 (s, 3H).404 [M+H]⁺ |
| 56 | 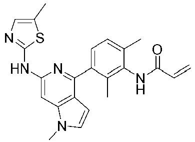 | *N*-(2,6-dimethyl-3-(1-methyl-6-((5-methylthiazol-2-yl)amino)-1H-pyrrolo[3,2-c]pyridin-4-yl)phenyl)acrylamide | ¹H NMR (500 MHz, DMSO-*d₆*) δ 12.46 (s, 1H), 10.77 (s, 1H), 9.61 (s, 1H), 7.33-7.21 (m, 3H), 6.99 (s, 1H), 6.95 (s, 1H), 6.52 (dd, *J* = 17.1, 10.3 Hz, 1H), 6.25 (dd, *J =* 17.1, 1.6 Hz, 1H), 6.07 (d, *J* = 3.0 Hz, 1H), 3.72 (s, 3H), 2.25 (s, 3H), 2.23 (s, 3H), 2.06 (s, 3H).; 418 [M+H]⁺ |
| 57 | 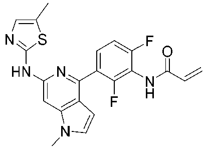 | N-(2,6-difluoro-3-(1-methyl-6-((5-methylthiazol-2-yl)amino)-1H-pyrrolo[3,2-c]pyridin-4-yl)phenyl)acrylamide | ¹H NMR (500 MHz, DMSO-*d₆*) δ 10.83 (s, 1H), 10.11 (s, 1H), 7.81 (dd, *J* = 14.4, 7.6 Hz, 1H), 7.40 (m, 1H), 7.34 (d, *J* = 3.2 Hz, 1H), 7.04 (s, 1H), 6.97 (s, 1H), 6.53 (dd, *J* = 17.0, 10.1 Hz, 1H), 6.35-6.28 (m, 2H), 5.84 (d, *J =* 11.7 Hz, 1H), 3.73 (s, 3H), 2.28 (s, 3H).; 426 [M+H]⁺ |
| 58 | 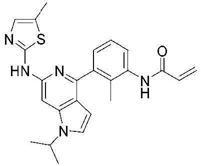 | N-(3-(1-isopropyl-6-((5-methylthiazol-2-yl)amino)-1*H*-pyrrolo[3,2-*c*]pyridin-4-yl)-2-methylphenyl)acrylamide | ¹H NMR (500 MHz, CDCl₃) δ 8.05 (s, 1H), 7.40-7.31 (m, 2H), 7.12 (d, *J* = 3.4 Hz, 1H), 7.02-6.95 (m, 2H), 6.48 (d, *J* = 16.7 Hz, 1H), 6.38-6.21 (m, 2H), 5.80 (d, *J* = 9.5 Hz, 1H), 4.61 (m, 1H), 2.34 (s, 3H), 2.23 (s, 3H), 1.54 (d, *J* = 6.7 Hz, 6H).; 432 [M+H]⁺ |
| 59 | 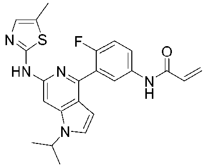 | *N*-(4-fluoro-3-(1-isopropyl-6-((5-methylthiazol-2-yl)amino)-1H-pyrrolo[3,2-c]pyridin-4-yl)phenyl)acrylamide | ¹H NMR (500 MHz, DMSO-*d₆*) δ 10.71 (s, 1H), 10.33 (s, 1H), 8.20 (dd, *J* = 6.4, 2.4 Hz, 1H), 7.78 (m, 1H), 7.50 (d, *J* = 3.4 Hz, 1H), 7.36 (t, *J* = 9.4 Hz, 1H), 7.12 (s, 1H), 6.96 (s, 1H), 6.45 (dd, *J =* 17.0, 10.1 Hz, 1H), 6.36 (t, *J* = 3.3 Hz, 1H), 6.27 (dd, *J =* 16.9, 1.6 Hz, 1H), 5.77 (m, 1H), 4.58 (m, 1H), 2.26 (s, 3H), 1.48 (d, *J* = 6.7 Hz, 6H).; 436 [M+H]⁺ |
| 60 | 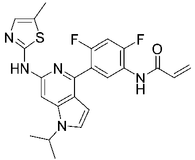 | N-(2,4-difluoro-5-(1-isopropyl-6-((5-methzylthiazol-2-yl)amino)-1H-pyrrolo[3,2-c]pyridin-4-yl)phenyl)acrylamide | ¹H NMR (500 MHz, CDCl₃) δ 9.33 (br s, 1H), 8.90 (s, 1H), 7.46 (s, 1H), 7.16 (d, *J* = 3.4 Hz, 1H), 7.07-6.95 (m, 3H), 6.52 (t, *J* = 3.4 Hz, 1H), 6.46 (d, *J* = 16.8 Hz, 1H), 6.30 (dd, *J* = 16.8, 10.2 Hz, 1H), 5.80 (d, *J* = 10.4 Hz, 1H), 4.58 (m, 1H), 2.38 (s, 3H), 1.53 (d, J = 6.7 Hz, 6H).; LC-MS (ESI) *m*/*z :* 454 [M+H]⁺ |
| 61 | 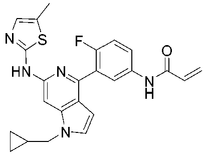 | N-(3-(1-(cyclopropylmethyl)-6-((5-methylthiazol-2-yl)amino)-1H-pyrrolo[3,2-c]pyridin-4-yl)-4-fluorophenyl)acrylamide | ¹H NMR (500 MHz, MeOD-*d₄*) δ 8.23 (dd, *J* = 6.3, 2.6 Hz, 1H), 7.76-7.72 (m, 1H), 7.31-7.22 (m, 2H), 7.14-6.82 (m, 3H), 6.50-6.32 (m, 3H), 5.78 (dd, *J* = 9.9, 1.7 Hz, 1H), 4.00 (t, *J* = 6.8 Hz, 2H), 2.32 (s, 3H), 1.28 (s, 1H), 0.63 (q, *J* = 5.6 Hz, 2H), 0.43 (d, *J* = 5.2 Hz, 2H).; 448 [M+H]⁺ |
| 62 | 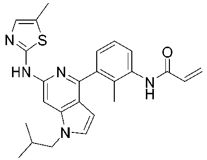 | *N*-(3-(1-isobutyl-6-((5-methylthiazol-2-yl)amino)-1H-pyrrolo[3,2-c]pyridin-4-yl)-2-methylphenyl)acrylamide | ¹H NMR (500 MHz, CDCl₃) δ 9.55 (s, 1H), 8.06 (s, 1H), 7.43-7.31 (m, 2H), 6.95 (dd, *J* = 38.9, 35.8 Hz, 3H), 6.55-6.18 (m, 3H), 5.80 (d, *J* = 9.6 Hz, 1H), 3.86 (d, *J* = 7.3 Hz, 2H), 2.34 (s, 3H), 2.23 (d, *J* = 14.1 Hz, 3H), 1.83 (s, 1H), 0.95 (t, *J* = 12.2 Hz, 6H).; 446 [M+H]⁺ |
| 63 | 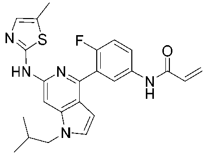 | *N*-(4-fluoro-3-(1-isobutyl-6-((5-methylthiazol-2-yl)amino)-1H-pyrrolo[3,2-c]pyridin-4-yl)phenyl)acrylamide | ¹H NMR (500 MHz, MeOD-*d₄*) δ 8.23 (dd, *J* = 6.3, 2.6 Hz, 1H), 7.79-7.71 (m, 1H), 7.29-7.18 (m, 2H), 7.00 (s, 1H), 6.91 (d, *J* = 0.9 Hz, 1H), 6.52-6.34 (m, 3H), 5.78 (dd, *J* = 10.0, 1.7 Hz, 1H), 3.95 (d, *J* = 7.3 Hz, 2H), 2.32 (s, 3H), 2.25-2.22 (m, 1H), 0.96 (d, *J* = 6.6 Hz, 6H).; 450 [M+H]⁺ |
| 64 | 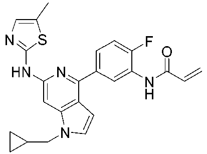 | N-(5-(1-(cyclopropylmethyl)-6-((5-methylthiazol-2-yl)amino)-1H-pyrrolo[3,2-c]pyridin-4-yl)-2-fluorophenyl)acrylamide | ¹H NMR (500 MHz, CDCl₃) δ 9.16 (s, 1H), 8.01-7.92 (m, 1H), 7.28 (s, 1H), 7.19 (d, *J* = 3.3 Hz, 2H), 6.86 (d, *J =* 38.6 Hz, 4H), 6.49-6.30 (m, 2H), 5.80 (d, *J* = 10.2 Hz, 1H), 3.91 (d, *J* = 6.8 Hz, 2H), 2.34 (d, *J* = 10.2 Hz, 3H), 2.00 (d, *J* = 12.3 Hz, 1H), 0.63 (q, *J* = 5.4 Hz, 2H), 0.36 (q, *J* = 5.1 Hz, 2H).; 448 [M+H]⁺ |
| 65 | 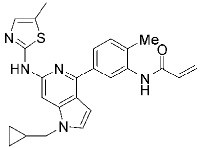 | *N*-(5-(1-(cyclopropylmethyl)-6-((5-methylthiazol-2-yl)amino)-1*H*-pyrrolo[3,2-*c*]pyridin-4-yl)-2-methylphenyl)acrylamide | ¹H NMR (500 MHz, CDCl₃) δ 8.59 (s, 1H), 7.95 (s, 1H), 7.37 (d, *J* = 7.7 Hz, 1H), 7.17 (d, *J* = 3.3 Hz, 1H), 6.85 (d, *J* = 31.8 Hz, 3H), 6.516.26 (m, 3H), 5.77 (d, *J* = 9.7 Hz, 1H), 3.93-3.83 (m, 2H), 2.33 (s, 3H), 2.15 (t, *J* = 7.5 Hz, 3H), 0.62 (dd, *J =* 13.1, 5.2 Hz, 2H), 0.36 (d, *J* = 5.0 Hz, 2H).; 444 [M+H]⁺ |
| 66 | 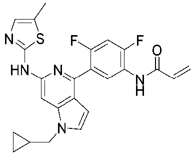 | *N*-(5-(1-(cyclopropylmethyl)-6-((5-methylthiazol-2-yl)amino)-1*H*-pyrrolo[3,2-*c*]pyridin-4-yl)-2,4-difluorophenyl)acrylamide | ¹H NMR (500 MHz, CDCl₃) δ 8.93 (s, 1H), 7.46 (d, *J* = 8.9 Hz, 1H), 7.18 (d, *J* = 3.3 Hz, 1H), 7.10-6.92 (m, 3H), 6.57-6.43 (m, 2H), 6.31 (dd, *J =* 16.8, 10.2 Hz, 1H), 5.82 (d, *J* = 10.4 Hz, 1H), 3.93 (d, *J* = 6.8 Hz, 2H), 2.36 (s, 3H), 2.27 (dd, *J* = 16.7, 5.4 Hz, 1H), 0.67 (q, *J* = 5.4 Hz, 2H), 0.39 (q, *J* = 5.0 Hz, 2H).; 466 [M+H]⁺ |
| 67 | 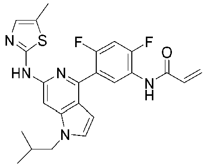 | *N*-(2,4-difluoro-5-(1-isobutyl-6-((5-methylthiazol-2-yl)amino)-1*H*-pyrrolo[3,2-*c*]pyridin-4-yl)phenyl)acrylamide | ¹H NMR (500 MHz, CDCl₃) δ 8.93 (s, 1H), 7.46-7.35 (m, 1H), 7.10-6.94 (m, 3H), 6.90 (s, 1H), 6.48 (dt, *J* = 36.6, 11.5 Hz, 2H), 6.37-6.23 (m, 1H), 5.82 (d, *J* = 10.4 Hz, 1H), 3.84 (t, *J* = 10.9 Hz, 2H), 2.37 (s, 3H), 2.24-2.15 (m, 1H), 0.96 (d, *J* = 6.6 Hz, 6H).; 468 [M+H]⁺ |
| 68 | 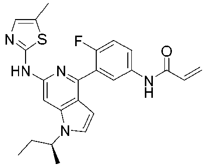 | (*S*)-*N*-(3-(1-(sec-butyl)-6-((5-methylthiazol-2-yl)amino)-1H-pyrrolo[3,2-c]pyridin-4-yl)-4-fluorophenyl)acrylamide | ¹H NMR (500 MHz, CDCl₃) δ 13.72 (s, 1H), 8.57 (s, 1H), 8.25 (s, 1H), 7.68 (s, 1H), 7.22-7.10 (m, 2H), 6.99 (s, 1H), 6.82 (s, 1H), 6.55-6.35 (m, 3H), 5.74 (d, *J* = 8.7 Hz, 1H), 4.25 (m, 1H), 2.30 (s, 3H), 1.88-1.66 (m, 2H), 1.36 (d, *J* = 5.2 Hz, 3H), 0.73 (t, *J* = 7.5 Hz, 3H).; 450 [M+H]⁺ |
| 69 | 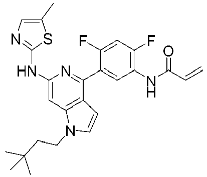 | *N*-(5-(1-(3,3-dimethylbutyl)-6-((5-methylthiazol-2-yl)amino)-1*H*-pyrrolo[3,2-*c*]pyridin-4-yl)-2,4-difluorophenyl)acrylamide | ¹H NMR (500 MHz, DMSO-*d₆*) δ 7.63-7.55 (m, 2H), 7.28 (s, 2H), 6.70-6.23 (m, 4H), 5.85-5.76 (m, 1H), 4.22-4.14 (m, 2H), 2.37-2.30 (m, 3H), 1.70-1.64 (m, 2H), 1.03-0.97 (m, 9H).; 496 [M+H]⁺ |
| 70 | 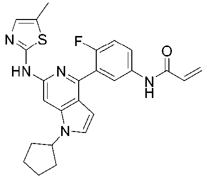 | *N*-(3-(1-cyclopentyl-6-((5-methylthiazol-2-yl)amino)-1*H*-pyrrolo[3,2-c]pyridin-4-yl)-4-fluorophenyl)acrylamide | ¹H NMR (500 MHz, DMSO-*d₆*) δ 10.73 (s, 1H), 10.34 (s, 1H), 8.20 (dd, *J* = 6.4, 2.4 Hz, 1H), 7.78 (m, 1H), 7.45 (d, *J* = 3.3 Hz, 1H), 7.36 (t, *J* = 9.4 Hz, 1H), 7.14 (s, 1H), 6.96 (s, 1H), 6.45 (dd, *J =* 16.9, 10.1 Hz, 1H), 6.35 (t, *J* = 3.3 Hz, 1H), 6.27 (dd, *J =* 16.9, 1.6 Hz, 1H), 5.77 (m, 1H), 4.73 (m, 1H), 2.26 (s, 3H), 2.21-2.12 (m, 2H), 1.95-1.80 (m, 4H), 1.80-1.66 (m, 2H).; 462 [M+H]⁺ |
| 71 | 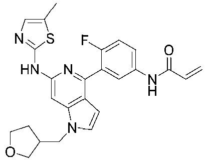 | N-(4-fluoro-3-(6-((5-methylthiazol-2-yl)amino)-1-((tetrahydrofuran-3-yl)methyl)-1*H*-pyrrolo[3,2-c]pyridin-4-yl)phenyl)acrylamide, | ¹H NMR (500 MHz, CDCl₃) δ 7.90 (d, *J* = 49.0 Hz, 2H), 7.57 (s, 1H), 7.01 (dd, *J =* 26.8, 5.2 Hz, 2H), 6.55-6.41 (m, 2H), 6.28 (dd, *J* = 16.6, 10.2 Hz, 1H), 5.79 (d, *J* = 10.2 Hz, 1H), 4.01 (dd, *J* = 17.1, 6.7 Hz, 2H), 3.80-3.55 (m, 4H), 2.82 (d, *J* = 6.7 Hz, 1H), 2.35 (s, 3H), 2.03 (dt, *J* = 14.1, 7.0 Hz, 2H).; 478 [M+H]⁺ |
| 72 | 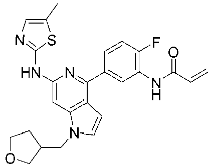 | *N*-(2-fluoro-5-(6-((5-methylthiazol-2-yl)amino)-1-((tetrahydrofuran-3-yl)methyl)-1*H*-pyrrolo[3,2-*c*]pyridin-4-yl)phenyl)acrylamide | ¹H NMR (500 MHz, DMSO-*d₆*) δ 10.18 (s, 1H), 8.93 (d, *J* = 6.4 Hz, 1H), 8.00-7.92 (m, 1H), 7.55-7.44 (m, 2H), 7.10-7.01 (m, 2H), 6.85 (d, *J* = 3.2 Hz, 1H), 6.73-6.62 (m, 1H), 6.34 (dd, *J* = 17.0, 1.5 Hz, 1H), 5.83 (d, *J* = 11.7 Hz, 2H), 4.12 (dd, *J* = 7.5, 3.4 Hz, 2H), 2.77 (d, *J* = 6.4 Hz, 1H), 2.33 (s, 3H), 2.01-1.85 (m, 4H), 1.64-1.55 (m, 2H).; 478 [M+H]⁺ |
| 73 | 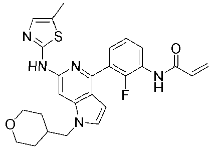 | N-(2-fluoro-3-(6-((5-methylthiazol-2-yl)amino)-1-((tetrahydro-2H-pyran-4-yl)methyl)-1H-pyrrolo[3,2-c]pyridin-4-yl)phenyl)acrylamide | ¹H NMR (500 MHz, CDCl₃) δ 8.54 (s, 1H), 7.61 (d, *J* = 6.9 Hz, 1H), 7.32 (dd, *J* = 21.0, 13.1 Hz, 1H), 7.00 (d, *J =* 3.4 Hz, 2H), 6.91 (s, 1H), 6.55-6.21 (m, 3H), 5.82 (dd, *J* = 16.4, 10.7 Hz, 1H), 4.02-3.85 (m, 4H), 3.35 (t, *J =* 11.1 Hz, 2H), 2.39-2.29 (m, 3H), 2.14-2.08 (m, 1H), 1.57-1.37 (m, 4H).; 492 [M+H]⁺ |
| 74 | 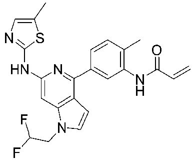 | N-(5-(1-(2,2-difluoroethyl)-6-((5-methylthiazol-2-yl)amino)-1H-pyrrolo[3,2-c]pyridin-4-yl)-2-methylphenyl)acrylamide, | ¹H NMR (500 MHz, MeOD-*d₄*) δ 8.00 (d, *J* = 26.0 Hz, 1H), 7.66 (d, *J* = 8.7 Hz, 1H), 7.38 (dd, *J* = 57.1, 25.8 Hz, 2H), 6.95 (d, *J* = 18.5 Hz, 2H), 6.61-6.46 (m, 1H), 6.46-6.01 (m, 3H), 5.86-5.77 (m, 1H), 4.57 (d, *J* = 9.9 Hz, 2H), 2.36 (dd, *J* = 20.6, 9.6 Hz, 3H), 1.29 (s, 3H).; 454 [M+H]⁺ |
| 75 | 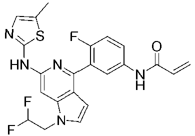 | *N*-(3-(1-(2,2-difluoroethyl)-6-((5-methylthiazol-2-yl)amino)-1*H*-pyrrolo[3,2-*c*]pyridin-4-yl)-4-fluorophenyl)acrylamide, | ¹H NMR (500 MHz, DMSO-*d₆*) δ 8.39 (d, *J* = 4.0 Hz, 1H), 7.91 (s, 1H), 7.67 (dd, *J* = 8.6, 3.5 Hz, 1H), 7.58 (d, *J* = 3.2 Hz, 1H), 7.45-7.18 (m, 3H), 6.53-6.41 (m, 2H), 6.27 (dd, *J* = 17.0, 1.6 Hz, 1H), 5.82-5.71 (m, 1H), 3.88-3.77 (m, 2H), 2.28 (d, *J* = 19.9 Hz, 3H).; 458 [M+H]⁺ |
| 76 | 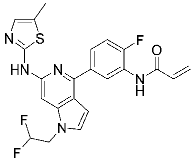 | *N*-(5-(1-(2,2-difluoroethyl)-6-((5-methylthiazol-2-yl)amino)-1*H*-pyrrolo[3,2-c]pyridin-4-yl)-2-fluorophenyl)acrylamide | ¹H NMR (500 MHz, CDCl₃) δ 9.24 (s, 1H), 8.00-7.92 (m, 1H), 7.55 (s, 1H), 7.33-7.28 (m, 1H), 7.10 (d, *J* = 3.3 Hz, 1H), 6.95 (d, *J* = 52.5 Hz, 2H), 6.51 (d, *J* = 16.8 Hz, 1H), 6.34 (dd, *J* = 16.8, 10.2 Hz, 1H), 6.03 (t, *J* = 55.0 Hz, 2H), 5.85 (d, *J* = 10.3 Hz, 1H), 4.41 (td, *J* = 14.2, 3.6 Hz, 2H), 2.44-2.33 (m, 3H). 458 [M+H]⁺ |
| 77 | 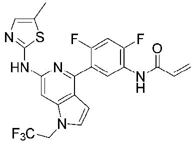 | *N*-(2,4-difluoro-5-(6-((5-methylthiazol-2-yl)amino)-1-(2,2,2-trifluoroethyl)-1*H-*pyrrolo[3,2-c]pyridin-4-yl)phenyl)acrylamide | ¹H NMR (500 MHz, CDCl₃) δ 8.93 (s, 1H), 8.76 (br s, 1H), 7.41 (s, 1H), 7.12-6.97 (m, 3H), 6.62 (t, *J* = 3.2 Hz, 1H), 6.48 (d, *J* = 16.8 Hz, 1H), 6.31 (dd, *J* = 16.8, 10.2 Hz, 1H), 5.83 (d, *J* = 10.3 Hz, 1H), 4.60 (dd, *J* = 16.9, 8.4 Hz, 2H), 2.37 (s, 3H).; LC-MS (ESI) *m*/*z :* 494 [M+H]⁺ |
| 78 | 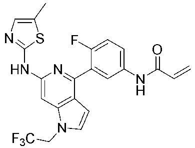 | *N*-(4-fluoro-3-(6-((5-methylthiazol-2-yl)amino)-1-(2,2,2-trifluoroethyl)-1*H-*pyrrolo[3,2-*c*]pyridin-4-yl)phenyl)acrylamide | ¹H NMR (500 MHz, DMSO-*d₆*) δ 10.88 (s, 1H), 10.36 (s, 1H), 8.23 (m, 1H), 7.80 (m, 1H), 7.42-7.35 (m, 2H), 7.17 (s, 1H), 6.98 (s, 1H), 6.45 (dd, *J* = 16.9, 10.1 Hz, 2H), 6.28 (d, *J* = 16.9 Hz, 1H), 5.78 (d, *J* = 10.2 Hz, 1H), 5.17 (dd, *J* = 18.1, 9.0 Hz, 2H), 2.27 (s, 3H).; 476 [M+H]⁺ |

### Example 79. Preparation of (S)-1-(3-((1-isopropyl-6-((5-methylthiazol-2-yl)amino)-1H-pyrrolo[3,2-c]pyridin-4-yl)oxy)pyrrolidin-1-yl)prop-2-en-1-one

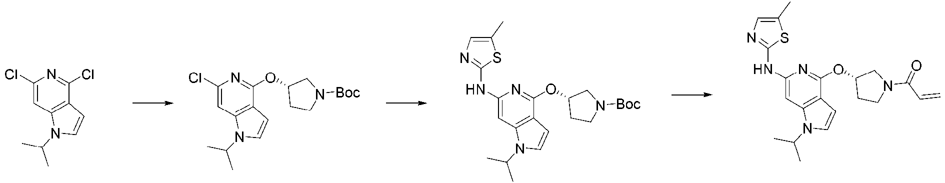

### Step 1. Preparation of tert-butyl (S)-3-((6-chloro-1-isopropyl-1H-pyrrolo[3,2-c]pyridin-4-yl)oxy)pyrrolidine-1-carboxylate

*tert*-Butyl (S)-3-hydroxypiperidine-1-carboxylate (307 mg, 1.64 mmol) was dissolved in DMF (5.0 mL), and then 60% NaH (66 mg, 1.64 mmol) was slowly added thereto at 0°C. The reaction mixture was stirred at 0°C for 30 minutes, and then 4,6-dichloro-1-isopropyl-1*H*-pyrrolo[3,2-c]pyridine (250 mg, 1.09 mmol) was added thereto, and allowed to react at 100°C for 2 hours. The reaction mixture was cooled to room temperature, and then diluted with ethyl acetate and washed with aqueous ammonium chloride solution. The organic layer was dried over MgSO₄, filtered, and concentrated. The resulting residue was purified by a silica gel chromatography (0-30% ethyl acetate/hexane) to synthesize *tert*-butyl (*S*)-3-((6-chloro-1-isopropyl-1*H*-pyrrolo[3,2-*c*]pyridin-4-yl)oxy)pyrrolidine-1-carboxylate as a white solid (242 mg, yield: 58%).
¹H NMR (500 MHz, CDCl₃) δ 7.08 (m, 1H), 6.94 (m, 1H), 6.55 (d, *J* = 2.8 Hz, 1H), 5.76 (d, *J* = 19.2 Hz, 1H), 4.51 (m, 1H), 3.77-3.47 (m, 4H), 2.20 (m, 2H), 1.54-1.43 (m, 15H).; LC-MS (ESI) *m*/*z :* 380 [M+H]⁺

### Step 2. Preparation of tert-butyl (S)-3-((1-isopropyl-6-((5-methylthiazol-2-yl)amino)-1H-pyrrolo[3,2-c]pyridin-4-yl)oxy)pyrrolidine-1-carboxylate

*tert*-butyl (*S*)-3-((1-isopropyl-6-((5-methylthiazol-2-yl)amino)-1*H*-pyrrolo[3,2-c]pyridin-4-yl)oxy)pyrrolidine-1-carboxylate (yield: 21%) was synthesized in the same manner as in step 2 of Example 1, except that tert-butyl (*S*)-3-((6-chloro-1-isopropyl-1*H-*pyrrolo[3,2-c]pyridin-4-yl)oxy)pyrrolidine-1-carboxylate was used instead of *tert*-butyl 4-(6-chloro-1-methyl-1*H*-pyrrolo[3,2-*c*]pyridin-4-yl)-3,6-dihydropyridine-1(2*H*)-carboxylate used in the synthesis step 2 of Example 1.
LC-MS (ESI) *m*/*z* : 458 [M+H]⁺

### Step 3. Preparation of (S)-1-(3-((1-isopropyl-6-((5-methylthiazol-2-yl)amino)-1H-pyrrolo[3,2-c]pyridin-4-yl)oxy)pyrrolidin-1-yl)prop-2-en-1-one

(*S*)-1-(3-((1-isopropyl-6-((5-methylthiazol-2-yl)amino)-1*H*-pyrrolo[3,2-*c*]pyridin-4-yl)oxy)pyrrolidin-1-yl)prop-2-en-1-one (yield: 69%) was synthesized in the same manner as in step 3 of Example 1, except that *tert*-butyl (*S*)-3-((1-isopropyl-6-((5-methylthiazol-2-yl)amino)-1*H*-pyrrolo[3,2-*c*]pyridin-4-yl)oxy)pyrrolidine-1-carboxylate was used instead of *tert*-butyl 4-(1-methyl-6-((5-methylthiazol-2-yl)amino)-1*H-*pyrrolo[3,2-c]pyridin-4-yl)-3,6-dihydropyridine-1(2*H*)-carboxylate used in the synthesis step 3 of Example 1.
¹H NMR (500 MHz, DMSO-*d₆*) δ 10.61 (s, 1H), 7.25 (t, *J* = 3.0 Hz, 1H), 6.97 (s, 1H), 6.72-6.50 (m, 2H), 6.37 (s, 1H), 6.14 (ddd, *J* = 16.7, 5.7, 2.0 Hz, 1H), 5.90 (d, *J =* 42.7 Hz, 1H), 5.66 (ddd, *J* = 26.2, 10.3, 1.9 Hz, 1H), 4.45 (m, 1H), 4.10-3.47 (m, 4H), 2.44-2.15 (m, 5H), 1.41 (d, *J* = 6.6 Hz, 6H).; LC-MS (ESI) *m*/*z* : 412 [M+H]⁺

Examples 80 to 204 were prepared in a similar manner to that of Example 79, and the structure and analysis results of the compounds are summarized in Table 3 below.

**[Table 3]**

| Exam ple | Structure | Compound name | ¹H NMR; LC-MS (ESI) *m*/*z* |
|---|---|---|---|
| 80 | 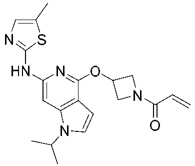 | 1-(3-((1-isopropyl-6-((5-methylthiazol-2-yl)amino)-1*H-*pyrrolo[3,2-*c*]pyridin-4-yl)oxy)azetidin-1-yl)prop-2-en-1-one | ¹H NMR (500 MHz, DMSO-*d₆*) δ 10.56 (s, 1H), 7.31 (d, *J* = 3.3 Hz, 1H), 6.97 (s, 1H), 6.75 (s, 1H), 6.46 (d, *J* = 3.2 Hz, 1H), 6.36 (dd, *J* = 17.0, 10.3 Hz, 1H), 6.13 (dd, *J* = 17.0, 1.9 Hz, 1H), 5.73 (dt, *J* = 10.0, 3.3 Hz, 1H), 5.68 (dd, *J* = 10.3, 1.9 Hz, 1H), 4.78 (dd, *J* = 9.4, 6.8 Hz, 1H), 4.54-4.42 (m, 2H), 4.25 (dd, *J* = 9.9, 2.8 Hz, 1H), 3.97 (dd, *J =* 11.2, 2.9 Hz, 1H), 2.33 (s, 3H), 1.43 (d, *J* = 6.6 Hz, 6H).; 398 [M+H]⁺ |
| 81 | 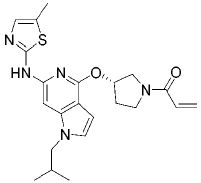 | (*S*)-1-(3-((1-isobutyl-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 7.90 (d, *J* = 49.0 Hz, 1H), 7.57 (s, 1H), 7.20 (d, *J* = 9.3 Hz, 1H), 7.01 (dd, *J* = 26.8, 5.2 Hz, 2H), 6.54-6.42 (m, 2H), 6.28 (dd, *J* = 16.6, 10.2 Hz, 1H), 5.79 (d, *J* = 10.2 Hz, 1H), 4.05-3.95 (m, 3H), 3.81-3.71 (m, 2H), 3.63 (dd, *J =* 8.9, 4.6 Hz, 1H), 2.82 (d, *J* = 6.7 Hz, 1H), 2.35 (s, 3H), 2.03 (dd, *J* = 13.4, 5.1 Hz, 2H), 1.26 (d, *J* = 10.5 Hz, 6H).; 426 [M+H]+ |
| 82 | 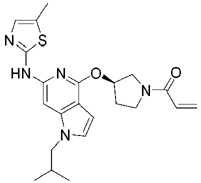 | (*R*)-1-(3-((1-isobutyl-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 7.02 (dd, *J* = 7.4, 1.1 Hz, 1H), 6.83 (dd, *J* = 7.8, 3.2 Hz, 1H), 6.57-6.33 (m, 4H), 6.03 (dd, *J* = 10.4, 8.2 Hz, 1H), 5.68 (ddd, *J* = 24.9, 9.6, 2.6 Hz, 1H), 4.05 (dd, *J* = 12.0, 4.9 Hz, 1H), 3.97-3.86 (m, 2H), 3.86-3.74 (m, 4H), 2.41-2.37 (m, 3H), 2.34-2.25 (m, 1H), 2.19-2.10 (m, 1H), 0.92 (dt, *J* = 25.3, 12.6 Hz, 6H).; 426 [M+H]⁺ |
| 83 | 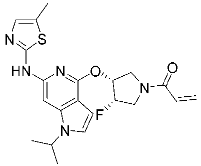 | 1-((3*S*,4*R*)-3-fluoro-4-((1-isopropyl-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 8.57 (s, 1H), 7.02 (m, 1H), 6.98 (s, 1H), 6.58 (m, 1H), 6.52-6.42 (m, 3H), 5.92-5.50 (m, 3H), 4.49 (m, 1H), 4.36 (m, 1H), 4.15-3.79 (m, 3H), 2.36 (s, 1H), 2.33 (s, 2H), 1.3-1.49, (m, 6H).; 430 [M+H]⁺ |
| 84 | 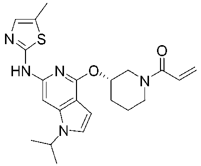 | (*S*)-1-(3-((1-isopropyl-6-((5-methylthiazol-2-yl)amino)-1*H-*pyrrolo[3,2-*c*]pyridin-4-yl)oxy)piperidin-1-yl)prop-2-en-1-one | ¹H NMR (500 MHz, CDCl₃) δ 8.25 (br s, 1H), 6.99-6.94 (m, 2H), 6.50-6.38 (m, 3H), 6.17 (d, *J* = 16.8 Hz, 1H), 5.55-5.43 (m, 2H), 4.48 (m, 1H), 3.94-3.36 (m, 4H), 2.36 (s, 3H), 2.21-1.54 (m, 4H), 1.49 (d, *J* = 6.7 Hz, 6H).; 426 [M+H]⁺ |
| 85 | 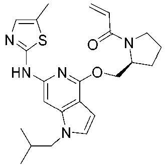 | (*S*)-1-(2-(((1-isobutyl-6-((5-methylthiazol-2-yl)amino)-1*H-*pyrrolo[3,2-*c*]pyridin-4-yl)oxy)methyl)pyrrolidin-1-yl)prop-2-en-1-one | ¹H NMR (500 MHz, CDCl₃) δ 9.19 (s, 1H), 6.96 (d, *J* = 14.5 Hz, 1H), 6.84 (d, *J* = 3.1 Hz, 1H), 6.57-6.32 (m, 4H), 5.65 (ddd, *J* = 33.0, 10.3, 1.7 Hz, 1H), 4.89-4.76 (m, 1H), 4.72-4.46 (m, 2H), 3.79 (d, *J* = 7.3 Hz, 2H), 3.76-3.60 (m, 2H), 2.37 (d, *J* = 7.2 Hz, 3H), 2.29 (dd, *J =* 19.5, 9.2 Hz, 1H), 2.19-1.96 (m, 6H), 0.91 (t, *J* = 8.3 Hz, 6H).; 440 [M+H]⁺ |
| 86 | 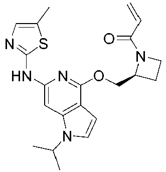 | (*S*)-1-(2-(((1-isopropyl-6-((5-methylthiazol-2-yl)amino)-1H-pyrrolo[3,2-*c*]pyridin-4-yl)oxy)methyl)azetidin-1-yl)prop-2-en-1-one | ¹H NMR (500 MHz, CDCl₃) δ 7.07-6.98 (m, 2H), 6.65-6.18 (m, 4H), 5.66 (dd, *J* = 26.1, 10.3 Hz, 1H), 5.08-4.81 (m, 3H), 4.55-4.07 (m, 3H), 2.69-2.17 (m, 5H), 1.52 (d, *J* = 3.7 Hz, 6H).; 412 [M+H]⁺ |
| 87 | 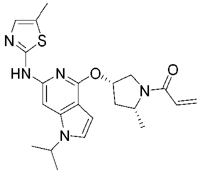 | 1-((2*R*,4*S*)-4-((1-isopropyl-6-((5-methylthiazol-2-yl)amino)-1*H-*pyrrolo[3,2-*c*]pyridin-4-yl)oxy)-2-methylpyrrolidin-1-yl)prop-2-en-1-one | ¹H NMR (500 MHz, CDCl₃) δ 7.06-6.97 (m, 2H), 6.58-6.33 (m, 4H), 6.07-5.95 (m, 1H), 5.67 (ddd, *J* = 29.7, 9.6, 2.6 Hz, 1H), 4.56-3.91 (m, 4H), 2.60-2.45 (m, 1H), 2.38 (s, 3H), 2.32-2.10 (m, 1H), 1.57-1.47 (m, 9H).; 426 [M+H]⁺ |
| 88 | 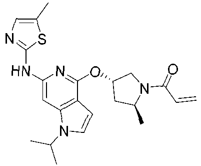 | 1-((2*S*,4*S*)-4-((1-isopropyl-6-((5-methylthiazol-2-yl)amino)-1*H-*pyrrolo[3,2-*c*]pyridin-4-yl)oxy)-2-methylpyrrolidin-1-yl)prop-2-en-1-one | ¹H NMR (500 MHz, CDCl₃) δ 7.05-6.95 (m, 2H), 6.57-6.32 (m, 4H), 6.07-5.99 (m, 1H), 5.75-5.60 (m, 1H), 4.56-3.81 (m, 4H), 2.60-2.13 (m, 5H), 1.54-1.36 (m, 9H).; 426 [M+H]⁺ |
| 89 | 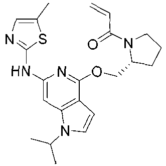 | (*R*)-1-(2-(((1-isopropyl-6-((5-methylthiazol-2-yl)amino)-1*H-*pyrrolo[3,2-*c*]pyridin-4-yl)oxy)methyl)pyrrolidin-1-yl)prop-2-en-1-one | ¹H NMR (500 MHz, CDCl₃) δ 7.03-6.94 (m, 2H), 6.73 (dd, *J* = 16.7, 10.3 Hz, 0.5H), 6.54-6.43 (m, 2.5H), 6.39 (td, *J* = 16.9, 1.7 Hz, 1H), 5.67 (dd, *J* = 10.2, 1.8 Hz, 0.5H), 5.61 (dd, *J* = 10.3, 1.7 Hz, 0.5H), 4.84 (m, 1H), 4.73-4.42 (m, 3H), 3.78-3.58 (m, 2H), 2.38 (s, 1.5H), 2.37 (s, 1.5H), 2.33-1.94 (m, 4H), 1.5.-1.46 (m, 6H).; 426 [M+H]⁺ |
| 90 | 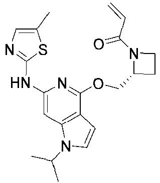 | (*R*)-1-(2-(((1-isopropyl-6-((5-methylthiazol-2-yl)amino)-1*H-*pyrrolo[3,2-c]pyridin-4-yl)oxy)methyl)azetidin-1-yl)prop-2-en-1-one | ¹H NMR (500 MHz, CDCl₃) δ 7.01-6.94 (m, 2H), 6.69-6.17 (m, 4H), 5.64 (dd, *J* = 26.0, 10.3 Hz, 1H), 5.07-4.77 (m, 3H), 4.49 (dt, *J* = 13.4, 6.7 Hz, 1H), 4.19 (ddd, *J* = 18.5, 14.7, 7.3 Hz, 2H), 2.64-2.42 (m, 2H), 2.39 (d, *J* = 14.4 Hz, 3H), 1.49 (d, *J* = 3.9 Hz, 6H).; 412 [M+H]⁺ |
| 91 | 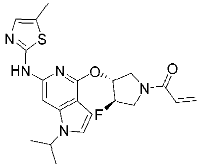 | 1-((3*R*,4*R*)-3-fluoro-4-((1-isopropyl-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 7.13-6.96 (m, 2H), 6.56-6.35 (m, 4H), 6.01 (ddd, *J* = 34.5, 7.8, 4.1 Hz, 1H), 5.73 (ddd, *J* = 19.2, 8.1, 4.1 Hz, 1H), 5.56-5.32 (m, 1H), 4.49 (m, 1H), 4.19-3.84 (m, 4H), 2.46-2.35 (m, 3H), 1.57-1.43 (m, 6H).; 430 [M+H]⁺ |
| 92 | 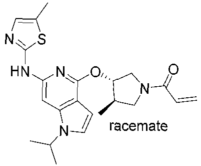 | *rac*-1-((3*S*,4*R*)-3-((1-isopropyl-6-((5-methylthiazol-2-yl)amino)-1*H-*pyrrolo[3,2-*c*]pyridin-4-yl)oxy)-4-methylpyrrolidin-1-yl)prop-2-en-1-one | ¹H NMR (500 MHz, CDCl₃) δ 9.77 (br s, 1H), 7.03 (dd, *J* = 6.4, 1.0 Hz, 1H), 6.98 (dd, *J* = 8.7, 3.2 Hz, 1H), 6.65-6.22 (m, 4H), 5.81-5.47 (m, 2H), 4.48 (m, 1H), 4.28-3.36 (m, 4H), 2.75-2.59 (m, 1H), 2.42-2.32 (m, 3H), 1.55-1.45 (m, 6H), 1.20 (dd, *J* = 7.1, 3.0 Hz, 3H).; 426 [M+H]⁺ |
| 93 | 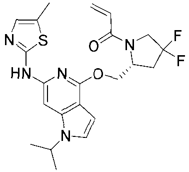 | (*R*)-1-(4,4-difluoro-2-(((1-isopropyl-6-((5-methylthiazol-2-yl)amino)-1*H-*pyrrolo[3,2-*c*]pyridin-4-yl)oxy)methyl)pyrrolidin-1-yl)prop-2-en-1-one | ¹H NMR (500 MHz, CDCl₃) δ 7.08-6.86 (m, 2H), 6.76-6.25 (m, 4H), 5.73 (dd, *J* = 29.6, 9.6 Hz, 1H), 5.03-3.83 (m, 6H), 2.80-2.55 (m, 2H), 2.38 (s, 1.5H), 2.35 (s, 1.5H), 1.49 (d, *J* = 6.6 Hz, 6H).; 462 [M+H]⁺ |
| 94 | 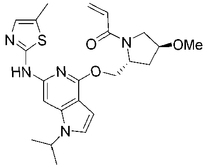 | 1-((2*R*,4*S*)-2-(((1-isopropyl-6-((5-methylthiazol-2-yl)amino)-1*H-*pyrrolo[3,2-c]pyridin-4-yl)oxy)methyl)-4-methoxypyrrolidin-1-yl)prop-2-en-1-one | ¹H NMR (500 MHz, CDCl₃) δ 7.02-6.96 (m, 2H), 6.68 (dd, *J* = 16.6, 10.3 Hz, 0.5H), 6.50-6.33 (m, 3.5H), 5.68 (d, *J* = 10.2 Hz, 0.5H), 5.64 (d, *J* = 10.4 Hz, 0.5H)., 4.92 (m, 0.5H), 4.81 (m, 1H), 4.62 (m, 1.5H), 4.47 (m, 1H), 4.33 (m, 0.5H), 4.18 (m, 0.5H), 4.03 (dd, *J* = 13.0, 2.9 Hz, 0.5H), 3.91 (dd, *J* = 10.6, 6.0 Hz, 0.5H), 3.65 (m, 1H), 3.34 (s, 1.5H), 3.33 (s, 1.5H), 2.39 (s, 1.5H), 2.38 (s, 1.5H), 2.34 (m, 2H), 2.15 (m, 1H), 1.50 (s, 3H), 1.49 (s, 3H).; 456 [M+H]⁺ |
| 95 | 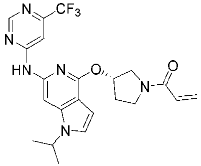 | (*S*)-1-(3-((1-isopropyl-6-((6-(trifluoromethyl)pyrimidin -4-yl)amino)-1*H-*pyrrolo[3,2-*c*]pyridin-4-yl)oxy)pyrrolidin-1-yl)prop-2-en-1-one | ¹H NMR (500 MHz, DMSO-*d₆*) δ 10.47 (s, 1H), 8.84 (s, 1H), 8.22-7.26 (m, 3H), 6.60 (ddd, *J* = 48.5, 16.7, 10.3 Hz, 1H), 6.45 (s, 1H), 6.15 (ddd, *J* = 16.7, 5.4, 2.2 Hz, 1H), 5.75-5.60 (m, 2H), 4.67-4.47 (m, 1H), 4.05-3.5 0(m, 4H), 2.39-2.13 (m, 2H), 1.45 (d, *J* = 6.5 Hz, 6H).; 461 [M+H]⁺ |
| 96 | 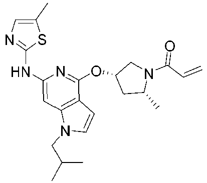 | 1-((2*R*,4*S*)-4-((1-isobutyl-6-((5-methylthiazol-2-yl)amino)-1*H-*pyrrolo[3,2-c]pyridin-4-yl)oxy)-2-methylpyrrolidin-1-yl)prop-2-en-1-one | ¹H NMR (500 MHz, CDCl₃) δ 7.01 (d, *J* = 11.9 Hz, 1H), 6.85 (dd, *J* = 6.7, 3.0 Hz, 1H), 6.62-6.29 (m, 4H), 5.99 (s, 1H), 5.68 (ddd, *J* = 29.7, 9.4, 2.5 Hz, 1H), 4.56-4.25 (m, 1H), 4.09-3.70 (m, 4H), 2.60-2.49 (m, 1H), 2.38 (s, 3H), 2.15 (d, *J* = 11.0 Hz, 2H), 1.52 (d, *J* = 6.1 Hz, 3H), 0.92 (dd, *J* = 14.9, 9.8 Hz, 6H).; 440 [M+H]⁺ |
| 97 | 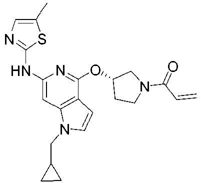 | (*S*)-1-(3-((1-(cyclopropylmethyl)-6-((5-methylthiazol-2-yl)amino)-1H-pyrrolo[3,2-*c*]pyridin-4-yl)oxy)pyrrolidin-1-yl)prop-2-en-1-one | ¹H NMR (500 MHz, CDCl₃) δ 7.08 (s, 1H), 6.94 (d, *J* = 9.0 Hz, 1H), 6.67-6.56 (m, 1H), 6.53 (dd, *J* = 17.9, 9.0 Hz, 1H), 6.42 (dd, *J* = 6.4, 4.3 Hz, 1H), 5.92 (d, *J* = 30.7 Hz, 1H), 5.75-5.65 (m, 1H), 4.06-3.99 (m, 2H), 3.96-3.81 (m, 5H), 2.38 (d, *J* = 3.1 Hz, 3H), 2.24-2.18 (m, 2H), 0.65 (d, *J* = 7.4 Hz, 2H), 0.37 (d, *J* = 3.9 Hz, 2H).; 424 [M+H]⁺ |
| 98 | 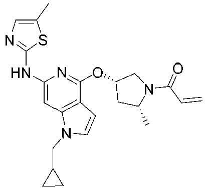 | 1-((2*R*,4*S*)-4-((1-(cyclopropylmethyl)-6-((5-methylthiazol-2-yl)amino)-1*H-*pyrrolo[3,2-c]pyridin-4-yl)oxy)-2-methylpyrrolidin-1-yl)prop-2-en-1-one | ¹H NMR (500 MHz, CDCl₃) δ 10.19 (s, 1H), 7.04 (d, *J* = 7.2 Hz, 1H), 6.97 (dd, *J* = 10.0, 3.1 Hz, 1H), 6.58-6.35 (m, 4H), 6.02 (dd, *J* = 9.8, 4.3 Hz, 1H), 5.67 (ddd, *J* = 30.2, 9.5, 2.5 Hz, 1H), 4.55-4.26 (m, 1H), 4.12 (dt, *J =* 12.0, 5.5 Hz, 1H), 3.98 (dd, *J =* 30.9, 13.3 Hz, 1H), 3.88-3.78 (m, 2H), 2.53 (dtt, *J* = 20.0, 17.0, 8.4 Hz, 1H), 2.39 (s, 3H), 2.31-2.07 (m, 2H), 1.52 (d, *J =* 6.3 Hz, 3H), 0.64 (t, *J* = 6.8 Hz, 2H), 0.39-0.32 (m, 2H).; 438 [M+H]⁺ |
| 99 | 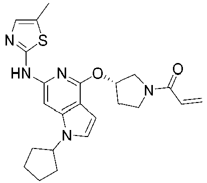 | (*S*)-1-(3-((1-cyclopentyl-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 10.46 (s, 1H), 7.02 (d, *J* = 10.3 Hz, 1H), 6.95 (dd, *J* = 5.5, 3.4 Hz, 1H), 6.55-6.35 (m, 4H), 6.01 (d, *J* = 21.5 Hz, 1H), 5.68 (ddd, *J* = 26.6, 9.3, 2.8 Hz, 1H), 4.59 (dd, *J* = 13.4, 6.8 Hz, 1H), 4.07-3.96 (m, 1H), 3.96-3.72 (m, 3H), 2.52-2.43 (m, 1H), 2.38 (d, *J =* 5.9 Hz, 3H), 2.33-2.24 (m, 1H), 2.18 (d, *J* = 6.9 Hz, 2H), 1.83 (d, *J* = 59.5 Hz, 6H).; 438 [M+H]⁺ |
| 100 | 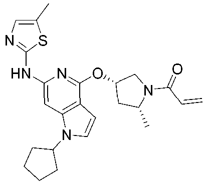 | 1-((2*R*,4*S*)-4-((1-cyclopentyl-6-((5-methylthiazol-2-yl)amino)-1*H-*pyrrolo[3,2-*c*]pyridin-4-yl)oxy)-2-methylpyrrolidin-1-yl)prop-2-en-1-one | ¹H NMR (500 MHz, CDCl₃) δ 10.64 (s, 1H), 7.05 (d, *J* = 7.0 Hz, 1H), 6.95 (dd, *J* = 10.3, 3.0 Hz, 1H), 6.59-6.33 (m, 4H), 6.03 (dd, *J* = 9.9, 4.9 Hz, 1H), 5.72-5.58 (m, 1H), 4.64-4.54 (m, 1H), 4.53-4.21 (m, 1H), 4.13 (dd, *J =* 12.6, 5.0 Hz, 1H), 4.07-3.91 (m, 1H), 2.53 (dddd, *J* = 29.2, 23.6, 14.6, 8.8 Hz, 1H), 2.39 (s, 3H), 2.32-2.13 (m, 3H), 1.84 (d, *J* = 63.4 Hz, 6H), 1.52 (dd, *J* = 6.3, 2.3 Hz, 3H).; 452 [M+H]⁺ |
| 101 | 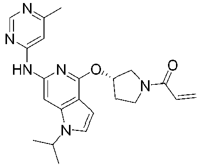 | (*S*)-1-(3-((1-isopropyl-6-((6-methylpyrimidin-4-yl)amino)-1*H-*pyrrolo[3,2-c]pyridin-4-yl)oxy)pyrrolidin-1-yl)prop-2-en-1-one | ¹H NMR (500 MHz, CDCl₃) δ 8.68 (d, *J* = 5.6 Hz, 1H), 7.45-7.30 (m, 2H), 7.12-7.01 (m, 2H), 6.56-6.35 (m, 3H), 5.80-5.61 (m, 2H), 4.65-4.51 (m, 1H), 4.04-3.72 (m, 4H), 2.44 (d, *J* = 5.4 Hz, 3H), 2.38-2.16 (m, 2H), 1.52 (d, *J* = 6.6 Hz, 6H).; 407 [M+H]⁺ |
| 102 | 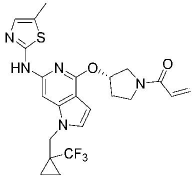 | (*S*)-1-(3-((6-((5-methylthiazol-2-yl)amino)-1-((1-(trifluoromethyl)cyclopro pyl)methyl)-1*H-*pyrrolo[3,2-*c*]pyridin-4-yl)oxy)pyrrolidin-1-yl)prop-2-en-1-one | ¹H NMR (500 MHz, CDCl₃) δ 9.47 (s, 1H), 7.03 (t, *J* = 7.5 Hz, 1H), 6.83 (dd, *J* = 5.2, 3.3 Hz, 1H), 6.62-6.33 (m, 4H), 6.00 (d, *J* = 23.0 Hz, 1H), 5.69 (ddd, *J =* 23.4, 9.4, 2.9 Hz, 1H), 4.39-4.29 (m, 2H), 4.07-4.01 (m, 1H), 3.95-3.74 (m, 3H), 2.50 (d, *J* = 11.3 Hz, 1H), 2.39 (d, *J* = 3.6 Hz, 3H), 2.29 (ddd, *J* = 45.3, 25.0, 4.6 Hz, 2H), 1.08-1.01 (m, 2H), 0.65 (s, 2H).; 492 [M+H]⁺ |
| 103 | 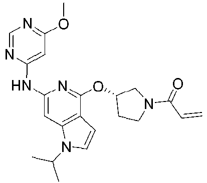 | (*S*)-1-(3-((1-isopropyl-6-((6-methoxypyrimidin-4-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 8.43 (s, 1H), 7.51 (br s, 1H), 7.04 (dd, *J* = 6.1, 3.2 Hz, 1H), 6.99-6.87 (m, 2H), 6.56-6.37 (m, 3H), 5.81-5.64 (m, 2H), 4.55 (m, 1H), 4.03-3.74 (m, 7H), 2.48-2.19 (m, 2H), 1.51 (d, *J =* 6.6 Hz, 6H).; 423 |
| 104 | 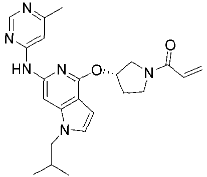 | (*S*)-1-(3-((1-isobutyl-6-((6-methylpyrimidin-4-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 8.67 (d, *J* = 4.7 Hz, 1H), 7.23 (d, *J* = 11.8 Hz, 1H), 7.08 (d, *J =* 14.9 Hz, 1H), 6.90 (dd, *J* = 7.1, 3.0 Hz, 1H), 6.56-6.37 (m, 3H), 5.81-5.63 (m, 2H), 4.04-3.92 (m, 1H), 3.92-3.75 (m, 5H), 2.46 (s, 1H), 2.43 (d, *J* = 5.1 Hz, 3H), 2.35-2.13 (m, 2H), 0.94 (dd, *J* = 6.4, 2.6 Hz, 6H).; 421 [M+H]⁺ |
| 105 | 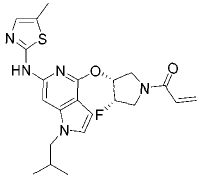 | 1-((3*S*,4*R*)-3-fluoro-4-((1-isobutyl-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 9.79 (s, 1H), 6.99 (s, 1H), 6.89 (d, *J* = 2.6 Hz, 1H), 6.62-6.36 (m, 4H), 5.78 (ddd, *J* = 22.3, 13.1, 12.1 Hz, 1H), 5.58 (d, *J* = 54.0 Hz, 1H), 4.37 (t, *J* = 8.0 Hz, 1H), 4.09 (dd, *J* = 24.4, 15.0 Hz, 1H), 3.93-3.71 (m, 4H), 2.32 (t, *J* = 17.8 Hz, 3H), 2.18-2.11 (m, 1H), 0.90 (dd, *J* = 29.2, 6.5 Hz, 6H). 444 [M+H]⁺ |
| 106 | 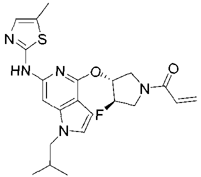 | 1-((3*R*,4*R*)-3-fluoro-4-((1-isobutyl-6-((5-methylthiazol-2-yl)amino)-1*H-*pyrrolo[3,2-*c*]pyridin-4-yl)oxy)pyrrolidin-1-yl)prop-2-en-1-one | 1H NMR (500 MHz, CDCl3) δ 11.14 (s, 1H), 7.06 (d, *J* = 5.5 Hz, 1H), 6.84 (dd, *J* = 5.4, 3.3 Hz, 1H), 6.55-6.31 (m, 4H), 6.02 (ddd, *J* = 34.3, 7.6, 3.9 Hz, 1H), 5.73 (ddd, *J* = 19.5, 8.2, 3.9 Hz, 1H), 5.46 (dd, *J* = 49.9, 35.6 Hz, 1H), 4.01 (dddd, *J* = 46.9, 28.5, 25.4, 13.7 Hz, 4H), 3.78 (d, *J =* 7.2 Hz, 2H), 2.49-2.33 (m, 3H), 2.15 (tt, *J* = 13.8, 6.8 Hz, 1H), 0.98-0.77 (m, 6H).; 444 [M+H]⁺ |
| 107 | 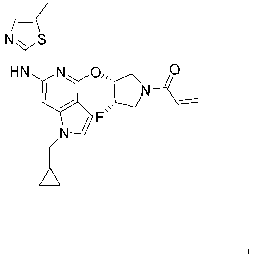 | 1-((3*R*,4*S*)-3-((1-(cyclopropylmethyl)-6-((5-methylthiazol-2-yl)amino)-1*H-*pyrrolo[3,2-*c*]pyridin-4-yl)oxy)-4-fluoropyrrolidin-1-yl)prop-2-en-1-one | ¹H NMR (500 MHz, CDCl₃) δ 10.97 (s, 1H), 7.06 (d, *J* = 6.0 Hz, 1H), 6.97 (dd, *J* = 6.6, 3.2 Hz, 1H), 6.53-6.37 (m, 4H), 6.01 (ddd, *J* = 34.5, 7.6, 3.9 Hz, 1H), 5.73 (ddd, *J* = 19.9, 7.8, 4.4 Hz, 1H), 5.44 (dd, *J* = 50.1, 33.6 Hz, 1H), 4.24-3.87 (m, 4H), 3.84 (d, *J* = 6.8 Hz, 2H), 2.41 (d, *J* = 6.0 Hz, 3H), 1.22 (dd, *J* = 11.7, 5.9 Hz, 1H), 0.64 (t, *J* = 6.6 Hz, 2H), 0.35 (d, *J* = 3.6 Hz, 2H).; 442 [M+H]⁺ |
| 108 | 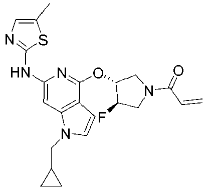 | 1-((3*R*,4*R*)-3-((1-(cyclopropylmethyl)-6-((5-methylthiazol-2-yl)amino)-1*H-*pyrrolo[3,2-*c*]pyridin-4-yl)oxy)-4-fluoropyrrolidin-1-yl)prop-2-en-1-one | ¹H NMR (500 MHz, CDCl₃) δ 10.53 (s, 1H), 7.01 (dd, *J* = 13.1, 9.8 Hz, 2H), 6.60-6.40 (m, 4H), 5.86 (ddd, *J* = 33.1, 20.1, 15.0 Hz, 1H), 5.80-5.49 (m, 2H), 4.44-4.32 (m, 1H), 4.15-3.75 (m, 5H), 2.36 (d, *J* = 15.7 Hz, 3H), 1.22 (d, *J* = 6.9 Hz, 1H), 0.69-0.58 (m, 2H), 0.36 (t, *J* = 4.9 Hz, 2H).; 442 [M+H]⁺ |
| 109 | 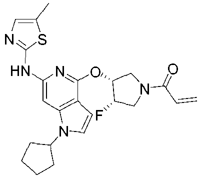 | 1-((3*R*,4*S*)-3-((1-cyclopentyl-6-((5-methylthiazol-2-yl)amino)-1*H-*pyrrolo[3,2-*c*]pyridin-4-yl)oxy)-4-fluoropyrrolidin-1-yl)prop-2-en-1-one | ¹H NMR (500 MHz, CDCl₃) δ 7.08-6.96 (m, 2H), 6.67-6.38 (m, 4H), 5.92-5.47 (m, 3H), 4.63 (t, *J* = 5.5 Hz, 1H), 4.36 (t, *J* = 8.6 Hz, 1H), 4.18-3.78 (m, 3H), 2.35 (s, 3H), 2.24-2.13 (m, 2H), 2.00-1.70 (m, 6H).; 456 [M+H]⁺ |
| 110 | 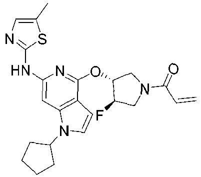 | 1-((3*R*,4*R*)-3-((1-cyclopentyl-6-((5-methylthiazol-2-yl)amino)-1*H-*pyrrolo[3,2-*c*]pyridin-4-yl)oxy)-4-fluoropyrrolidin-1-yl)prop-2-en-1-one | ¹H NMR (500 MHz, CDCl₃) δ 7.07-6.93 (m, 2H), 6.57-6.38 (m, 4H), 6.07-5.93 (m, 1H), 5.74 (ddd, *J* = 19.0, 7.9, 4.2 Hz, 1H), 5.55-5.34 (m, 1H), 4.64-4.55 (m, 1H), 4.22-3.82 (m, 4H), 2.40 (s, 3H), 2.25-2.12 (m, 2H), 1.97-1.70(m, 6H).; 456 |
| 111 | 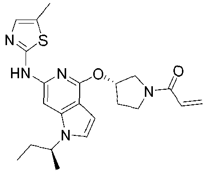 | 1-((*S*)-3-((1-((*S*)-*sec-*butyl)-6-((5-methylthiazol-2-yl)amino)-1*H-*pyrrolo[3,2-c]pyridin-4-yl)oxy)pyrrolidin-1-yl)prop-2-en-1-one | ¹H NMR (500 MHz, CDCl₃) δ 7.01 (dd, *J* = 8.9, 0.8 Hz, 1H), 6.95 (dd, *J* = 5.4, 3.4 Hz, 1H), 6.57-6.35 (m, 4H), 6.00 (m, 1H), 5.68 (ddd, *J* = 25.1, 9.5, 2.8 Hz, 1H), 4.26-4.16 (m, 1H), 4.08-3.74 (m, 4H), 2.55-2.24 (m, 5H), 1.85 (m, 2H), 1.48 (dd, *J* = 6.7, 3.5 Hz, 3H), 0.83 (t, *J* = 7.4 Hz, 3H).; 426 [M+H]⁺ |
| 112 | 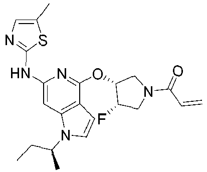 | 1-((3*R*,4*S*)-3-((1-((*S*)-*sec*-butyl)-6-((5-methylthiazol-2-yl)amino)-1*H-*pyrrolo[3,2-*c*]pyridin-4-yl)oxy)-4-fluoropyrrolidin-1-yl)prop-2-en-1-one | ¹H NMR (500 MHz, CDCl₃) δ 7.06-6.94 (m, 2H), 6.67-6.38 (m, 4H), 5.89-5.50 (m, 3H), 4.41-3.79 (m, 5), 2.357-2.32 (m, 3H), 1.90-1.81 (m, 2H), 1.48 (t, *J =* 3.3 Hz, 3H), 0.83 (t, *J =* 7.3 Hz, 3H).; 444 [M+H]⁺ |
| 113 | 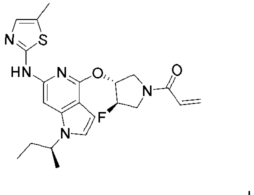 | 1-((3*R*,4*R*)-3-((1-((*S*)-sec-butyl)-6-((5-methylthiazol-2-yl)amino)-1*H-*pyrrolo[3,2-*c*]pyridin-4-yl)oxy)-4-fluoropyrrolidin-1-yl)prop-2-en-1-one | ¹H NMR (500 MHz, CDCl₃) δ 7.08-6.85 (m, 2H), 6.74-6.31 (m, 4H), 6.20-5.87 (m, 1H), 5.74 (ddd, *J* = 16.8, 7.7, 4.5 Hz, 1H), 5.59-5.26 (m, 1H), 4.44-3.74 (m, 5H), 2.39 (d, *J* = 3.9 Hz, 3H), 1.92-1.76 (m, 2H), 1.56-1.41 (m, 3H), 0.82 (t, *J* = 7.3 Hz, 3H).; 444 [M+H]⁺ |
| 114 | 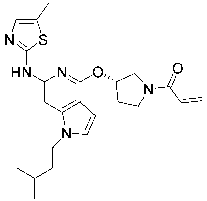 | (*S*)-1-(3-((1-isopentyl-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 11.24 (s, 1H), 7.09 (t, *J* = 10.4 Hz, 1H), 6.91-6.82 (m, 1H), 6.63-6.38 (m, 4H), 6.14-6.01 (m, 1H), 5.68 (ddd, *J* = 15.4, 12.2, 7.6 Hz, 1H), 3.93 (dddd, *J =* 39.9, 29.8, 18.8, 11.6 Hz, 6H), 2.69-2.19 (m, 6H), 1.61 (td, *J* = 12.7, 6.3 Hz, 1H), 0.99 (t, *J* = 9.0 Hz, 6H).; 440 [M+H]⁺ |
| 115 | 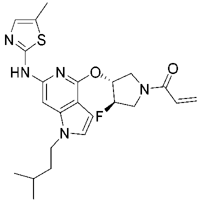 | 1-((3*R*,4*R*)-3-fluoro-4-((1-isopentyl-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 7.09 (d, *J* = 5.8 Hz, 1H), 6.87 (dd, *J* = 5.6, 3.1 Hz, 1H), 6.45 (td, *J* = 16.6, 5.1 Hz, 4H), 6.12-5.97 (m, 1H), 5.73 (ddd, *J =* 20.3, 8.1, 4.0 Hz, 1H), 5.46 (dd, *J* = 49.3, 35.0 Hz, 1H), 4.28-3.76 (m, 6H), 2.40 (d, *J* = 5.1 Hz, 3H), 1.80-1.51 (m, 3H), 0.98 (d, *J* = 6.5 Hz, 6H).; 458 [M+H]⁺ |
| 116 | 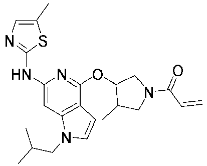 | 1-(3-((1-isobutyl-6-((5-methylthiazol-2-yl)amino)-1*H-*pyrrolo[3,2-*c*]pyridin-4-yl)oxy)-4-methylpyrrolidin-1-yl)prop-2-en-1-one | ¹H NMR (500 MHz, CDCl₃) δ 7.00 (d, *J* = 6.2 Hz, 1H), 6.76 (dd, *J* = 10.1, 2.8 Hz, 1H), 6.49-6.29 (m, 4H), 5.65-5.55 (m, 2H), 4.20 (m, 1H), 3.95-3.82 (m, 2H), 3.75-3.66 (m, 2H), 3.41 (m, 1H), 2.62 (m, 1H), 2.32 (s, 3H), 2.07 (m, 1H), 1.14 (dd, *J* = 6.9, 3.8 Hz, 3H), 0.89-0.84 (m, 6H).; 440 [M+H]⁺ |
| 117 | 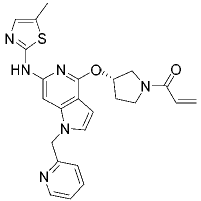 | (*S*)-1-(3-((6-((5-methylthiazol-2-yl)amino)-1-(pyridin-2-ylmethyl)-1*H*-pyrrolo[3,2-*c*]pyridin-4-yl)oxy)pyrrolidin-1-yl)prop-2-en-1-one | ¹H NMR (500 MHz, CDCl₃) δ 8.50 (m, 1H), 7.57 (t, *J* = 7.7 Hz, 1H), 7.20 (m, 1H), 6.98 (dd, *J =* 5.9, 3.2 Hz, 1H), 6.88 (s, 1H), 6.74 (dd, *J* = 17.6, 7.9 Hz, 1H), 6.58-6.35 (m, 4H), 6.01 (d, *J* = 20.2 Hz, 1H), 5.69 (ddd, *J =* 25.3, 9.3, 3.0 Hz, 1H), 5.33 (s, 2H), 4.08-3.75 (m, 4H), 2.55-2.26 (m, 5H).; 461 [M+H]⁺ |
| 118 | 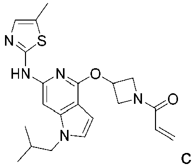 | 1-(3-((1-isobutyl-6-((5-methylthiazol-2-yl)amino)-1*H-*pyrrolo[3,2-*c*]pyridin-4-yl)oxy)azetidin-1-yl)prop-2-en-1-one | ¹H NMR (500 MHz, CDCl₃) δ 7.05 (s, 1H), 6.86 (d, *J* = 3.0 Hz, 1H), 6.51 (d, *J* = 3.0 Hz, 1H), 6.44 (s, 1H), 6.37 (dd, *J* = 16.9, 1.1 Hz, 1H), 6.23 (dd, *J* = 17.0, 10.3 Hz, 1H), 5.86 (m, 1H), 5.68 (dd, *J* = 10.4, 1.1 Hz, 1H), 4.82-4.61 (m, 2H), 4.30 (ddd, *J =* 14.8, 10.5, 3.2 Hz, 2H), 3.79 (d, *J* = 7.3 Hz, 2H), 2.43 (s, 3H), 2.16 (m, 1H), 0.94 (d, *J* = 6.5 Hz, 7H).412 [M+H]⁺ |
| 119 | 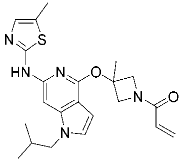 | 1-(3-((1-isobutyl-6-((5-methylthiazol-2-yl)amino)-1*H-*pyrrolo[3,2-*c*]pyridin-4-yl)oxy)-3-methylazetidin-1-yl)prop-2-en-1-one | ¹H NMR (500 MHz, CDCl₃) δ 6.98 (s, 1H), 6.87 (s, 1H), 6.70 (s, 1H), 6.48 (s, 1H), 6.39-6.18 (m, 2H), 5.67 (d, *J* = 10.3 Hz, 1H), 4.67-4.30 (m, 4H), 3.81 (d, *J* = 6.8 Hz, 2H), 2.40 (s, 3H), 2.18 (m, 1H), 2.01 (s, 3H), 0.93 (d, *J* = 5.7 Hz, 6H).; 426 [M+H]⁺ |
| 120 | 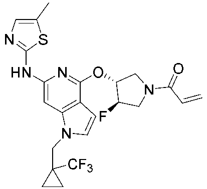 | 1-((3*R*,4*R*)-3-fluoro-4-((6-((5-methylthiazol-2-yl)amino)-1-((1-(trifluoromethyl)cyclopro pyl)methyl)-1*H-*pyrrolo[3,2-*c*]pyridin-4-yl)oxy)pyrrolidin-1-yl)prop-2-en-1-one | ¹H NMR (500 MHz, CDCl₃) δ 11.52 (s, 1H), 7.11 (d, *J* = 5.9 Hz, 1H), 6.81 (dd, *J* = 6.7, 2.8 Hz, 1H), 6.46 (dt, *J* = 18.2, 8.5 Hz, 4H), 6.09-5.94 (m, 1H), 5.74 (ddd, *J* = 19.2, 7.6, 4.4 Hz, 1H), 5.45 (dd, *J* = 49.3, 38.3 Hz, 1H), 4.43-3.76 (m, 7H), 2.41 (d, *J =* 5.6 Hz, 3H), 1.04 (d, *J* = 4.7 Hz, 2H), 0.62 (s, 2H).; 510 [M+H]⁺ |
| 121 | 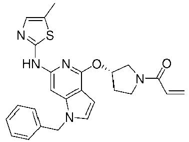 | (*S*)-1-(3-((1-benzyl-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 7.35-7.27 (m, 3H), 7.08 (t, *J =* 7.3 Hz, 2H), 6.92 (dd, *J* = 6.5, 3.2 Hz, 1H), 6.84 (s, 1H), 6.59-6.35 (m, 4H), 6.00 (d, *J* = 22.6 Hz, 1H), 5.74-5.61 (m, 1H), 5.21 (s, 2H), 4.11-3.72 (m, 4H), 2.56-2.23 (m, 5H).; 460 [M+H]⁺ |
| 122 | 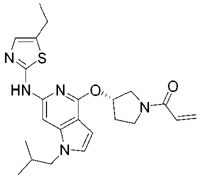 | (*S*)-1-(3-((6-((5-ethylthiazol-2-yl)amino)-1-isobutyl-1*H-*pyrrolo[3,2-*c*]pyridin-4-yl)oxy)pyrrolidin-1-yl)prop-2-en-1-one | ¹H NMR (500 MHz, CDCl₃) δ 7.02-6.84 (m, 2H), 6.57-6.37 (m, 4H), 5.94 (d, *J* = 29.8 Hz, 1H), 5.69 (ddd, *J* = 22.5, 9.1, 3.2 Hz, 1H), 4.06-3.70 (m, 6H), 2.80-2.72 (m, 2H), 2.60-2.09 (m, 3H), 1.32 (t, *J* = 7.5 Hz, 3H), 0.95-0.81 (m, 6H).; 440 [M+H]⁺ |
| 123 | 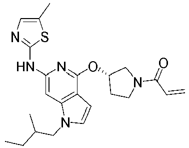 | 1-((3*S*)-3-((1-(2-methylbutyl)-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 7.06 (d, *J* = 8.3 Hz, 1H), 6.81 (dd, *J* = 7.4, 2.8 Hz, 1H), 6.56-6.34 (m, 4H), 6.03 (d, *J* = 15.4 Hz, 1H), 5.72-5.60 (m, 1H), 4.09-3.65 (m, 6H), 2.57-1.87 (m, 6H), 1.41 (m, 1H), 1.20 (m, 1H), 1.02-0.77 (m, 6H).; 440 [M+H]⁺ |
| 124 | 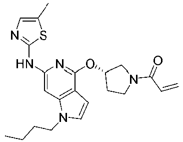 | (*S*)-1-(3-((1-butyl-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 10.39 (s, 1H), 7.04 (d, *J* = 8.6 Hz, 1H), 6.85 (dd, *J* = 7.4, 3.1 Hz, 1H), 6.62-6.31 (m, 4H), 6.03 (d, *J* = 17.2 Hz, 1H), 5.68 (ddd, *J* = 26.2, 9.6, 2.5 Hz, 1H), 4.15-3.68 (m, 6H), 2.67-2.19 (m, 5H), 1.86-1.69 (m, 2H), 1.38-1.28 (m, 2H), 0.94 (tt, *J* = 24.1, 12.1 Hz, 3H).; 426 [M+H]⁺ |
| 125 | 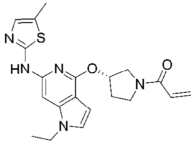 | (*S*)-1-(3-((1-ethyl-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, DMSO-*d₆*) δ 10.61 (s, 1H), 7.16 (t, *J* = 2.7 Hz, 1H), 6.97 (s, 1H), 6.70-6.51 (m, 2H), 6.35 (s, 1H), 6.14 (ddd, *J* = 16.7, 6.1, 2.0 Hz, 1H), 5.90 (d, *J* = 41.9 Hz, 1H), 5.66 (ddd, *J* = 26.2, 10.3, 2.0 Hz, 1H), 4.11-3.49 (m, 6H), 2.45-2.16 (m, 5H), 1.31 (t, *J* = 7.2 Hz, 3H).; 398 [M+H]⁺ |
| 126 | 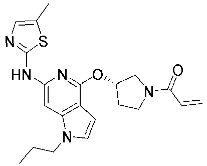 | (*S*)-1-(3-((6-((5-methylthiazol-2-yl)amino)-1-propyl-1*H-*pyrrolo[3,2-*c*]pyridin-4-yl)oxy)pyrrolidin-1-yl)prop-2-en-1-one | ¹H NMR (500 MHz, DMSO-*d₆*) δ 10.57 (s, 1H), 7.14 (t, *J* = 2.6 Hz, 1H), 6.96 (s, 1H), 6.70-6.50 (m, 2H), 6.34 (d, *J* = 2.7 Hz, 1H), 6.14 (ddd, *J* = 16.7, 6.6, 2.1 Hz, 1H), 5.90 (d, *J* = 42.0 Hz, 1H), 5.66 (ddd, *J* = 26.8, 10.3, 2.1 Hz, 1H), 4.13-3.48 (m, 6H), 2.45-2.15 (m, 5H), 1.78-1.65 (m, 2H), 0.81 (t, *J* = 7.3 Hz, 3H).; 412 [M+H]⁺ |
| 127 | 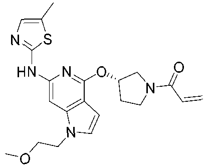 | (*S*)-1-(3-((1-(2-methoxyethyl)-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, DMSO-*d₆*) δ 10.60 (s, 1H), 7.13 (t, *J* = 2.9 Hz, 1H), 6.96 (s, 1H), 6.69-6.50 (m, 2H), 6.35 (s, 1H), 6.14 (ddd, *J* = 16.7, 6.3, 2.2 Hz, 1H), 5.90 (d, *J* = 42.0 Hz, 1H), 5.67 (ddd, *J* = 26.2, 10.3, 2.1 Hz, 1H), 4.15 (t, *J* = 5.1 Hz, 2H), 4.10-3.48 (m, 4H), 3.62 (t, *J* = 5.1 Hz, 2H), 3.21 (s, 3H), 2.45-2.15 (m, 5H).; 428 [M+H]⁺ |
| 128 | 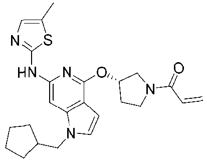 | (*S*)-1-(3-((1-(cyclopentylmethyl)-6-((5-methylthiazol-2-yl)amino)-1*H-*pyrrolo[3,2-*c*]pyridin-4-yl)oxy)pyrrolidin-1-yl)prop-2-en-1-one | ¹H NMR (500 MHz, CDCl₃) δ 10.93 (s, 4H), 7.09-7.03 (m, 1H), 6.85 (dd, *J* = 7.9, 3.2 Hz, 1H), 6.57-6.35 (m, 4H), 6.04 (dd, *J* = 10.0, 7.9 Hz, 1H), 5.67 (ddd, *J* = 26.2, 9.6, 2.7 Hz, 1H), 4.08-3.75 (m, 7H), 2.51-2.29 (m, 6H), 1.66 (dd, *J* = 30.8, 28.1 Hz, 7H).; 452 [M+H]⁺ |
| 129 | 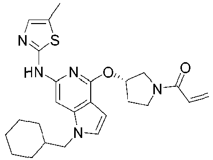 | (*S*)-1-(3-((1-(cyclohexylmethyl)-6-((5-methylthiazol-2-yl)amino)-1*H-*pyrrolo[3,2-c]pyridin-4-yl)oxy)pyrrolidin-1-yl)prop-2-en-1-one | ¹H NMR (500 MHz, CDCl₃) δ 10.14 (s, 1H), 7.04 (d, *J* = 8.0 Hz, 1H), 6.82 (dd, *J* = 7.4, 3.1 Hz, 1H), 6.63-6.32 (m, 4H), 6.03 (d, *J* = 16.7 Hz, 1H), 5.68 (ddd, *J* = 25.5, 9.6, 2.4 Hz, 1H), 4.13-3.73 (m, 6H), 2.60-2.27 (m, 5H), 1.84-1.57 (m, 6H), 1.08 (ddd, *J* = 34.7, 30.1, 12.5 Hz, 5H).; 466 [M+H]⁺ |
| 130 | 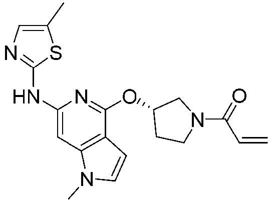 | (S)-1-(3-((1-methyl-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, CDCl₃) δ 7.26 (s, 1H), 7.01 (d, *J* = 12.1 Hz, 1H), 6.86-6.78 (m, 1H), 6.58-6.35 (m, 4H), 5.99 (d, *J =* 25.1 Hz, 1H), 5.76-5.61 (m, 1H), 4.09-3.98 (m, 1H), 3.98- 3.72 (m, 3H), 3.68 (a, 3H), 2.50-2.20 (m, 5H). 384 [M+H]⁺ |
| 131 | 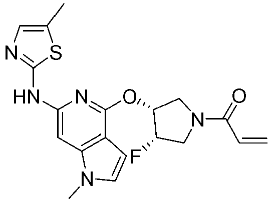 | 1-((3S,4R)-3-fluoro-4-((1-methyl-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, DMSO-*d₆*) δ 10.69 (s, 1H), 7.17 (s, 1H), 6.98 (s, 1H), 6.75 (s, 1H), 6.65 (dd, *J* = 16.5, 10.4 Hz, 1H), 6.40 (s, 1H), 6.22 (d, *J =* 16.7 Hz, 1H), 5.95-5.53 (m, 3H), 4.53-4.24 (m, 1H), 3.94-3.41 (m, 5H), 2.28 (d, *J* = 9.9 Hz, 3H). 402 [M+H]⁺ |
| 132 | 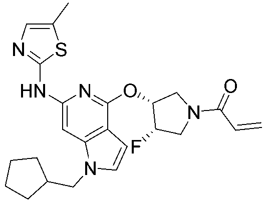 | 1-((3R,4S)-3-((1-(cyclopentylmethyl)-6-((5-methylthiazol-2-yl)amino)-1H-pyrrolo[3,2-c]pyridin-4-yl)oxy)-4-fluoropyrrolidin-1-yl)prop-2-en-1-one | ¹H NMR (500 MHz, CDCl₃) δ 9.68 (s, 1H), 7.02 (s, 1H), 6.91 (d, *J* = 3.0 Hz, 1H), 6.60 - 6.38 (m, 4H), 5.92 - 5.81 (m, 1H), 5.80 - 5.48 (m, 2H), 4.38 (t, *J* = 8.7 Hz, 1H), 4.15 - 3.76 (m, 5H), 2.40 (d, *J* = 7.4 Hz, 1H), 2.36 (d, *J =* 15.4 Hz, 3H), 1.76 - 1.53 (m, 6H), 1.32 - 1.23 (m, 2H). 470 [M+H]⁺ |
| 133 | 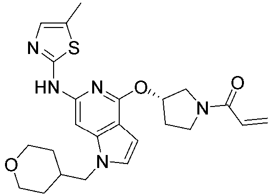 | (S)-1-(3-((6-((5-methylthiazol-2-yl)amino)-1-((tetrahydro-2H-pyran-4-yl)methyl)-1H-pyrrolo[3,2-c]pyridin-4-yl)oxy)pyrrolidin-1-yl)prop-2-en-1-one | ¹H NMR (500 MHz, CDCl₃) δ 9.26 (s, 1H), 7.01 (d, *J* = 8.4 Hz, 1H), 6.86 - 6.78 (m, *J* = 5.9, 3.1 Hz, 1H), 6.55 - 6.32 (m, J *=* 21.1, 4H), 6.01 (d, *J* = 19.6 Hz, 1H), 5.73 - 5.59 (m, 1H), 4.05 (d, *J* = 14.1 Hz, 1H), 3.96 - 3.87 (m, 2H), 3.82 (t, *J* = 11.1 Hz, 3H), 2.39 (s, 3H), 1.85 - 1.58 (m, 8H), 1.20 (d, *J* = 13.2 Hz, 3H). 468 [M+H]⁺ |
| 134 | 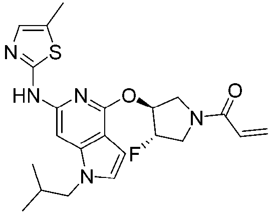 | 1-((3S,4S)-3-fluoro-4-((1-isobutyl-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, CDCl₃) δ 9.42 (s, 1H), 7.03 (d, *J* = 5.8 Hz, 1H), 6.90 - 6.79 (m, 1H), 6.53 - 6.29 (m, 4H), 6.11 - 5.89 (m, 1H), 5.78 - 5.66 (m, 1H), 5.45 (dd, *J* = 49.2, 36.9 Hz, 1H), 4.20 - 3.85 (m, 5H), 3.79 (d, *J =* 7.3 Hz, 1H), 2.40 (d, *J* = 4.2 Hz, 3H), 2.15 (dt, *J* = 13.4, 6.7 Hz, 1H), 0.93 (d, *J* = 6.3 Hz, 6H)., 444 [M+H]⁺ |
| 135 | 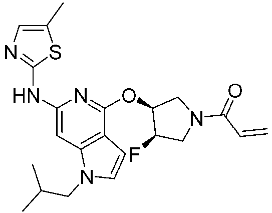 | 1-((3R,4S)-3-fluoro-4-((1-isobutyl-6-((5-methylthiazol-2-yl)amino)-1H-pyrrolo[3,2-c]pyridin-4-yl)oxy)pyrrolidin-1-yl)prop-2-en-1-one | 1H NMR (500 MHz, DMSO) δ 10.56 (s, 1H), 7.18 (t, J = 3.5 Hz, 1H), 6.95 (s, 1H), 6.76 (d, J = 3.1 Hz, 1H), 6.69 - 6.57 (m, 1H), 6.40 (t, J = 3.4 Hz, 1H), 6.21 (d, J = 16.7 Hz, 1H), 5.89 - 5.54 (m, 3H), 4.49 - 4.22 (m, 1H), 3.87 - 3.66 (m, 5H), 2.26 (d, J = 10.7 Hz, 3H), 2.09 (dt, J = 13.4, 6.7 Hz, 1H), 0.84 (d, J = 6.5 Hz, 6H), 444 [M+H]+ |
| 136 | 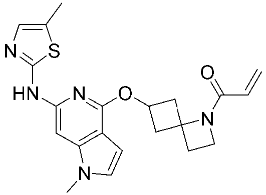 | 1-(6-((1-methyl-6-((5-methylthiazol-2-yl)amino)-1H-pyrrolo[3,2-c]pyridin-4-yl)oxy)-1-azaspiro[3.3]heptan-1-yl)prop-2-en-1-one | ¹H NMR (500 MHz, DMSO-*d₆*) δ 10.62 (s, 1H), 7.12 (s, 1H), 6.97 (s, 1H), 6.59 (s, 1H), 6.39 (s, 1H), 6.30-6.05 (m, 2H), 5.80-5.35(m, 2H), 4.16 (s, 2H), 3.65 (s, 3H), 3.15, s, 2H), 2.74 (s, 2H), 2.45 (s, 2H), 2.33 (s, 3H). 410 [M+H]⁺ |
| 137 | 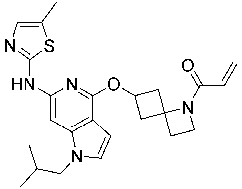 | 1-(6-((1-isobutyl-6-((5-methylthiazol-2-yl)amino)-1H-pyrrolo[3,2-c]pyridin-4-yl)oxy)-1-azaspiro[3.3]heptan-1-yl)prop-2-en-1-one | ¹H NMR (500 MHz, DMSO-*d₆*) δ 10.51 (s, 1H), 7.14 (d, *J* = 3.1 Hz, 1H), 6.96 (s, 1H), 6.66-6.56 (m, 1H), 6.44-6.38 (m, 1H), 6.24 (dd, *J* = 16.9, 10.2 Hz, 1H), 6.11 (dd, *J* = 16.9, 1.9 Hz, 1H), 4.22-3.78 (m, 4H), 3.15 (td, *J* = 8.6, 2.4 Hz, 2H), 2.80-2.70 (m, 2H), 2.47-2.41 (m, 2H), 2.33 (s, 3H), 2.12-2.04 (m, 1H), 0.84 (d, *J* = 6.6 Hz, 6H). 452 [M+H]⁺ |
| 138 | 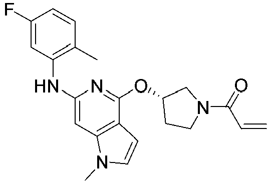 | (S)-1-(3-((6-((5-fluoro-2-methylphenyl)amino)-1-methyl-1H-pyrrolo[3,2-c]pyridin-4-yl)oxy)pyrrolidin-1-yl)prop-2-en-1-one | ¹H NMR (500 MHz, DMSO-*d₆*) δ 7.76 (d, *J* = 10.9 Hz, 1H), 7.73-7.57 (m, 1H), 7.13 (t, *J* = 7.6 Hz, 1H), 7.07 (t, *J* = 3.0 Hz, 1H), 6.70-6.47 (m, 3H), 6.31 (s, 1H), 6.21-6.10 (m, 1H), 5.67 (dd, *J =* 20.5, 10.4 Hz, 1H), 5.59 (d, *J =* 10.4 Hz, 1H), 3.97-3.47 (m, 7H), 2.34-2.11 (m, 5H). 395 [M+H]⁺ |
| 139 | 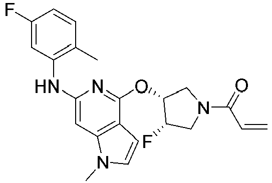 | 1-((3S,4R)-3-fluoro-4-((6-((5-fluoro-2-methylphenyl)amino)-1-methyl-1H-pyrrolo[3,2-c]pyridin-4-yl)oxy)pyrrolidin-1-yl)prop-2-en-1-one | ¹H NMR (500 MHz, DMSO-*d₆*) δ 7.82 (d, *J* = 20.4 Hz, 1H), 7.45 (t, *J* = 9.9 Hz, 1H), 7.19- 7.08 (m, 2H), 6.70-6.52 (m, 3H), 6.36 (d, *J =* 6.3 Hz, 1H), 6.18 (d, *J* = 16.7 Hz, 1H), 5.71 (t, *J* = 10.3 Hz, 1H), 5.56-5.37 (m, 2H), 4.35-3.52 (m, 7H), 2.22 (s, 3H). 413 [M+H]⁺ |
| 140 | 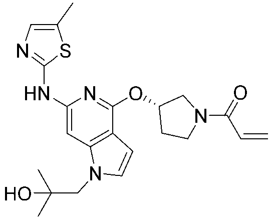 | (S)-1-(3-((1-(2-hydroxy-2-methylpropyl)-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, MeOD) δ 7.04 (t, *J* = 3.0 Hz, 1H), 6.90 (s, 1H), 6.73-6.53 (m, 2H), 6.43 (d, *J* = 3.0 Hz, 1H), 6.28 (ddd, *J =* 16.8, 9.1, 1.4 Hz, 1H), 6.10-5.98 (m, 1H), 5.74 (ddd, *J* = 24.7, 10.5, 1.4 Hz, 1H), 4.15-3.64 (m, 6H), 2.50-2.29 (m, 5H, 1.19 (s, 6H). 442 [M+H]⁺ |
| 141 | 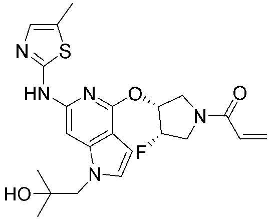 | 1-((3S,4R)-3-fluoro-4-((1-(2-hydroxy-2-methylpropyl)-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, MeOD) δ 7.08 (t, *J* = 2.9 Hz, 1H), 6.89 (s, 1H), 6.70-6.58 (m, 2H), 6.49 (t, *J* = 2.8 Hz, 1H), 6.33 (d, *J =* 16.8 Hz, 1H), 5.94-5.83 (m, 1H), 5.79 (ddd, *J* = 15.0, 10.5, 1.7 Hz, 1H), 5.69-5.51 (m, 1H), 4.55-3.58 (m, 6H), 2.30 (d, *J* = 12.6 Hz, 3H), 1.20 (s, 6H). 460 [M+H]⁺ |
| 142 | 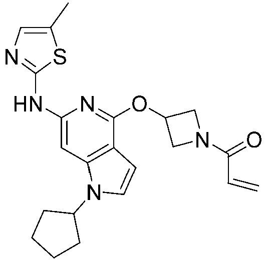 | 1-(3-((1-cyclopentyl-6-((5-methylthiazol-2-yl)amino)-1H-pyrrolo[3,2-c]pyridin-4-yl)oxy)azetidin-1-yl)prop-2-en-1-one | ¹H NMR (500 MHz, CDCl₃) δ 7.04 - 6.95 (m, 2H), 6.53 (s, 2H), 6.36 (d, *J* = 17.0 Hz, 1H), 6.24 (d, *J* = 10.3 Hz, 1H), 5.82 (s, 1H), 5.69 (d, *J* = 11.2 Hz, 1H), 4.77 - 4.57 (m, 3H), 4.36 - 4.21 (m, 2H), 2.41 (s, 3H), 2.24 - 2.13 (m, 3H), 1.90 (s, 5H). 424 [M+H]⁺ |
| 143 | 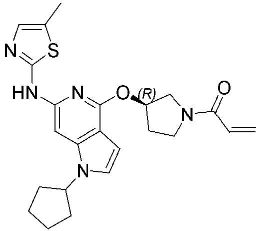 | (R)-1-(3-((1-cyclopentyl-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, CDCl₃) δ 7.06 - 6.88 (m, 2H), 6.64 - 6.32 (m, 4H), 5.98 (d, *J* = 24.5 Hz, 1H), 5.75 - 5.62 (m, 1H), 4.67 - 4.55 (m, 1H), 4.05 - 3.73 (m, 4H), 2.38 (d, *J* = 1.9 Hz, 3H), 2.24 - 2.14 (m, 2H), 1.83 (d, *J =* 55.8 Hz, 8H). 438[M+H]⁺ |
| 144 | 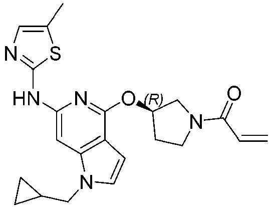 | (R)-1-(3-((1-(cyclopropylmethyl)-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, CDCl₃) δ 7.02 - 6.94 (m, 2H), 6.54 - 6.36 (m, 4H), 5.99 (d, *J* = 23.5 Hz, 1H), 5.74 - 5.59 (m, 1H), 4.04 (d, *J* = 15.5 Hz, 1H), 3.95 - 3.78 (m, 5H), 2.52 - 2.45 (m, 1H), 2.38 (d, *J* = 2.6 Hz, 3H), 0.87 (dd, *J* = 12.9, 6.0 Hz, 2H), 0.64 (dd, *J* = 8.2, 2.6 Hz, 2H), 0.38 - 0.33 (m, 2H). 424[M+H]⁺ |
| 145 | 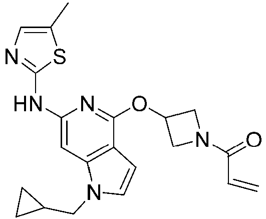 | 1-(3-((1-(cyclopropylmethyl)-6-((5-methylthiazol-2-yl)amino)-1H-pyrrolo[3,2-c]pyridin-4-yl)oxy)azetidin-1-yl)prop-2-en-1-one | ¹H NMR (500 MHz, CDCl₃) δ 7.06 - 6.97 (m, 2H), 6.58 - 6.46 (m, 2H), 6.41 - 6.18 (m, 2H), 5.88 - 5.75 (m, 1H), 5.69 (d, *J* = 10.4 Hz, 1H), 4.79 - 4.59 (m, 2H), 4.37 - 4.21 (m, 2H), 3.87 (d, *J* = 6.8 Hz, 2H), 2.42 (s, 3H), 0.84 (s, 1H), 0.66 - 0.62 (m, 2H), 0.36 (q, *J* = 5.0 Hz, 2H).410[M+H]⁺ |
| 146 | 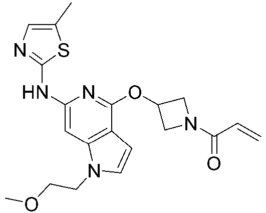 | 1-(3-((1-(2-methoxyethyl)-6-((5-methylthiazol-2-yl)amino)-1H-pyrrolo[3,2-c]pyridin-4-yl)oxy)azetidin-1-yl)prop-2-en-1-one | ¹H NMR (500 MHz, DMSO-*d₆*) δ 10.57 (s, 1H), 7.18 (d, *J* = 3.2 Hz, 1H), 6.96 (s, 1H), 6.73 (s, 1H), 6.44 (d, *J* = 3.1 Hz, 1H), 6.36 (dd, *J* = 17.0, 10.3 Hz, 1H), 6.13 (dd, *J* = 17.0, 2.0 Hz, 1H), 5.77-5.72 (m, 1H), 5.68 (dd, *J* = 10.3, 2.0 Hz, 1H), 4.79 (dd, *J* = 9.5, 6.7 Hz, 1H), 4.51 (dd, *J* = 11.0, 6.6 Hz, 1H), 4.25 (dd, *J* = 9.9, 2.8 Hz, 1H), 4.16 (t, *J* = 5.2 Hz, 2H), 3.97 (dd, *J* = 11.1, 3.0 Hz, 1H), 3.63 (t, *J* = 5.2 Hz, 2H), 3.22 (s, 3H), 2.34 (s, 3H). 414[M+H]⁺ |
| 147 | 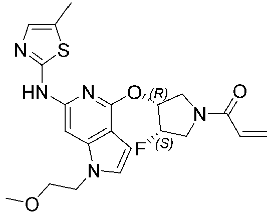 | 1-((3S,4R)-3-fluoro-4-((1-(2-methoxyethyl)-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, DMSO-*d₆*) δ 10.65 (s, 1H), 7.19 (t, *J* = 3.5 Hz, 1H), 6.96 (s, 1H), 6.78 (s, 1H), 6.64 (ddd, *J* = 16.4, 10.3, 3.2 Hz, 1H), 6.40 (t, *J* = 3.6 Hz, 1H), 6.21 (d, *J* = 16.7 Hz, 1H), 5.89-5.53 (m, 3H), 4.52-3.42 (m, 8H), 3.21 (s, 3H), 2.27 (d, *J =* 9.8 Hz, 3H). 446[M+H]⁺ |
| 148 | 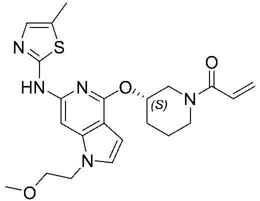 | (S)-1-(3-((1-(2-methoxyethyl)-6-((5-methylthiazol-2-yl)amino)-1H-pyrrolo[3,2-c]pyridin-4-yl)oxy)piperidin-1-yl)prop-2-en-1-one | ¹H NMR (500 MHz, DMSO-*d₆*) δ 10.60 (s, 1H), 7.11 (s, 1H), 6.95 (s, 1H), 6.91- 6.54 (m, 2H), 6.28 (d, *J* = 26.6 Hz, 1H), 6.04 (dd, *J* = 38.4, 16.6 Hz, 1H), 5.56 (dd, *J* = 112.4, 9.6 Hz, 2H), 4.14 (s, 2H), 3.98-3.40 (m, 6H), 3.21 (s, 3H), 2.34-1.48 (m, 7H). 442[M+H]⁺ |
| 149 | 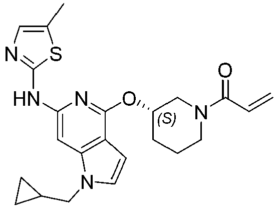 | (S)-1-(3-((1-(cyclopropylmethyl)-6-((5-methylthiazol-2-yl)amino)-1H-pyrrolo[3,2-c]pyridin-4-yl)oxy)piperidin-1-yl)prop-2-en-1-one | ¹H NMR (500 MHz, DMSO) δ 10.55 (s, 2H), 7.18 (s, 2H), 6.98 - 6.48 (m, 6H), 6.32 - 5.92 (m, 4H), 5.75 - 5.33 (m, 4H), 4.13 (d, *J =* 13.1 Hz, 3H), 3.88 (t, *J* = 11.0 Hz, 13H), 2.26 (d, *J* = 10.3 Hz, 7H), 2.15 - 1.47 (m, 10H), 1.20 (d, *J* = 41.0 Hz, 5H), 0.52 (s, 5H), 0.37 (s, 5H).438[M+H]⁺ |
| 150 | 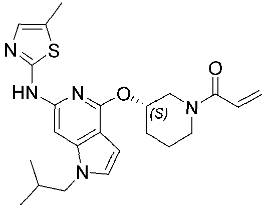 | (S)-1-(3-((1-isobutyl-6-((5-methylthiazol-2-yl)amino)-1H-pyrrolo[3,2-c]pyridin-4-yl)oxy)piperidin-1-yl)prop-2-en-1-one | ¹H NMR (500 MHz, DMSO) δ 10.50 (s, 1H), 7.10 (s, 1H), 6.99 - 6.79 (m, 1H), 6.62 - 6.49 (m, 1H), 6.28 (d, *J* = 28.0 Hz, 1H), 6.02 (dd, *J* = 49.8, 16.9 Hz, 1H), 5.55 (dd, *J* = 128.8, 10.5 Hz, 2H), 4.20 - 3.45 (m, 6H), 2.26 (d, *J* = 10.3 Hz, 3H), 1.86 (dd, *J* = 187.0, 85.0 Hz, 5H), 0.84 (s, 6H).440[M+H]⁺ |
| 151 | 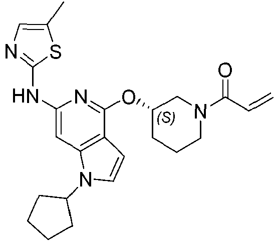 | (S)-1-(3-((1-cyclopentyl-6-((5-methylthiazol-2-yl)amino)-1H-pyrrolo[3,2-c]pyridin-4-yl)oxy)piperidin-1-yl)prop-2-en-1-one | ¹H NMR (500 MHz, DMSO) δ 10.56 (s, 1H), 7.19 (d, *J =* 3.2 Hz, 1H), 6.95 (s, 1H), 6.69 - 6.48 (m, 2H), 6.35 - 6.18 (m, 1H), 6.14 - 5.93 (m, 1H), 5.73 - 5.35 (m, 2H), 4.65 - 4.56 (m, 1H), 4.16 - 3.53 (m, 5H), 2.26 (d, *J =* 10.5 Hz, 3H), 2.10 (d, *J* = 5.7 Hz, 3H), 1.76 (d, *J* = 70.2 Hz, 9H). 452[M+H]⁺ |
| 152 | 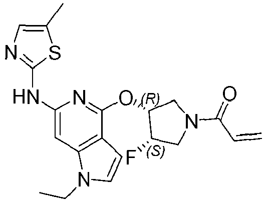 | 1-((3R,4S)-3-((1-ethyl-6-((5-methylthiazol-2-yl)amino)-1H-pyrrolo[3,2-c]pyridin-4-yl)oxy)-4-fluoropyrrolidin-1-yl)prop-2-en-1-one | ¹H NMR (500 MHz, CDCl₃) δ 7.02 (s, 1H), 6.90 (t, *J* = 3.9 Hz, 1H), 6.54 (d, *J* = 2.7 Hz, 1H), 6.51 - 6.40 (m, 3H), 5.92 - 5.80 (m, 1H), 5.79 - 5.70 (m, *J =* 12.1, 8.6, 3.5 Hz, 1H), 5.60 (dd, *J* = 53.8, 16.7 Hz, 1H), 4.37 (t, *J* = 8.5 Hz, 1H), 4.14 - 3.99 (m, 3H), 3.92 - 3.77 (m, 2H), 2.34 (d, *J =* 15.4 Hz, 3H), 1.43 (t, *J* = 7.2 Hz, 3H).;416[M+H]⁺ |
| 153 | 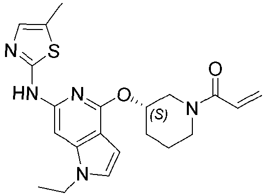 | (S)-1-(3-((1-ethyl-6-((5-methylthiazol-2-yl)amino)-1H-pyrrolo[3,2-c]pyridin-4-yl)oxy)piperidin-1-yl)prop-2-en-1-one | ¹H NMR (500 MHz, CDCl₃) δ 7.15 - 6.96 (m, 1H), 6.93 - 6.81 (m, 1H), 6.51 - 6.36 (m, 2H), 6.31 - 6.09 (m, 1H), 5.75 - 5.47 (m, 1H), 5.41 (dd, *J* = 21.3, 10.5 Hz, 1H), 4.02 (q, *J* = 6.7 Hz, 2H), 3.90 - 3.80 (m, 2H), 3.69 - 3.55 (m, 1H), 2.30 (d, *J =* 12.8 Hz, 3H), 2.21 - 2.11 (m, 1H), 2.04 - 1.87 (m, 1H), 1.73 - 1.57 (m, 1H), 1.42 (t, *J* = 7.1 Hz, 3H).; 412[M+H]⁺ |
| 154 | 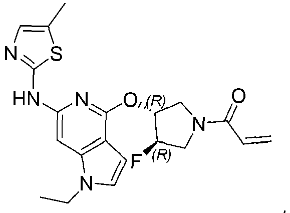 | 1-((3R,4R)-3-((1-ethyl-6-((5-methylthiazol-2-yl)amino)-1H-pyrrolo[3,2-c]pyridin-4-yl)oxy)-4-fluoropyrrolidin-1-yl)prop-2-en-1-one | ¹H NMR (500 MHz, CDCl₃) δ 7.02 (d, *J* = 6.1 Hz, 1H), 6.92 - 6.83 (m, 1H), 6.54 - 6.37 (m, 4H), 6.09 - 5.93 (m, 1H), 5.73 (ddd, *J* = 19.9, 8.1, 3.8 Hz, 1H), 5.44 (dd, *J* = 49.3, 33.7 Hz, 1H), 4.12 - 3.85 (m, 6H), 2.38 (d, *J =* 5.2 Hz, 3H), 1.43 (t, *J* = 6.7 Hz, 3H).; 416[M+H]⁺ |
| 155 | 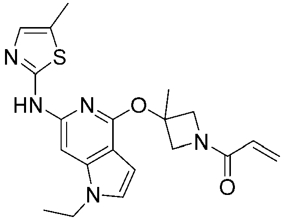 | 1-(3-((1-ethyl-6-((5-methylthiazol-2-yl)amino)-1H-pyrrolo[3,2-c]pyridin-4-yl)oxy)-3-methylazetidin-1-yl)prop-2-en-1-one | ¹H NMR (500 MHz, CDCl₃) δ 7.03 - 6.90 (m, 2H), 6.82 (s, 1H), 6.51 (s, 1H), 6.35 (d, *J =* 16.8 Hz, 1H), 6.22 (dd, J = 16.9, 10.2 Hz, 1H), 5.68 (d, *J* = 10.4 Hz, 1H), 4.61 (d, *J* = 9.5 Hz, 1H), 4.45 (dd, *J* = 21.8, 10.3 Hz, 2H), 4.33 (d, *J* = 11.0 Hz, 1H), 4.09 (q, *J* = 7.2 Hz, 2H), 2.39 (s, 3H), 1.99 (s, 3H), 1.47 - 1.41 (m, 3H).; 398[M+H]⁺ |
| 156 | 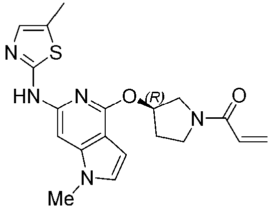 | (R)-1-(3-((1-methyl-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, DMSO-*d₆*) δ 10.85 (s, 1H), 7.11 (dd, J = 2.9, 1.7 Hz, 1H), 7.00 (s, 1H), 6.70-6.51 (m, 2H), 6.35 (d, *J* = 3.1 Hz, 1H), 6.14 (ddd, *J* = 16.7, 6.5, 2.3 Hz, 1H), 5.97-5.83 (m, 1H), 5.66 (ddd, *J* = 26.9, 10.3, 2.3 Hz, 1H), 4.13-3.44 (m, 7H), 2.45-2.17 (m, 5H). 384[M+H]⁺ |
| 157 | 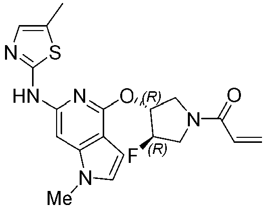 | 1-((3R,4R)-3-fluoro-4-((1-methyl-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, DMSO-*d₆*) δ 11.06 (s, 1H), 7.15 (d, *J* = 3.2 Hz, 1H), 7.04 (s, 1H), 6.71 (s, 1H), 6.63 (ddd, *J* = 16.7, 12.9, 10.4 Hz, 1H), 6.36 (d, *J* = 3.1 Hz, 1H), 6.25-6.15 (m, 1H), 5.90 (ddd, *J* = 52.8, 8.4, 3.9 Hz, 1H), 5.78-5.68 (m, 1H), 5.51 (dd, *J* = 48.9, 39.7 Hz, 1H), 4.28-3.69 (m, 7H), 2.31 (s, 3H). 402[M+H]⁺ |
| 158 | 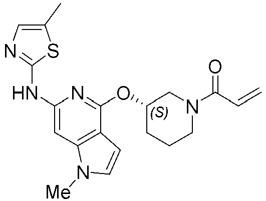 | (S)-1-(3-((1-methyl-6-((5-methylthiazol-2-yl)amino)-1H-pyrrolo[3,2-c]pyridin-4-yl)oxy)piperidin-1-yl)prop-2-en-1-one | ¹H NMR (500 MHz, DMSO-*d₆*) δ 10.80 (s, 1H), 7.09 (s, 1H), 6.98 (s, 1H), 6.90-6.51 (m, 2H), 6.35-6.22 (m, 1H), 6.03 (dd, *J* = 37.5, 16.6 Hz, 1H), 5.72-5.36 (m, 2H), 4.21-3.35 (m, 7H), 2.36-1.45 (m, 7H). 398[M+H]⁺ |
| 159 | 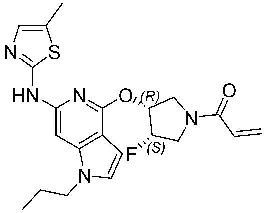 | 1-((3S,4R)-3-fluoro-4-((6-((5-methylthiazol-2-yl)amino)-1-propyl-1H-pyrrolo[3,2-c]pyridin-4-yl)oxy)pyrrolidin-1-yl)prop-2-en-1-one | ¹H NMR (500 MHz, DMSO) δ 10.60 (d, *J =* 11.3 Hz, 1H), 7.28 - 7.18 (m, 1H), 6.96 (s, 1H), 6.77 (s, 1H), 6.69 - 6.58 (m, 1H), 6.49 - 6.37 (m, 1H), 6.21 (d, *J =* 16.8 Hz, 1H), 5.89 - 5.54 (m, 3H), 4.50 - 4.25 (m, 1H), 4.00 (q, *J* = 6.9 Hz, 2H), 3.85 - 3.64 (m, 3H), 2.27 (d, *J* = 10.0 Hz, 2H), 1.75 (q, *J =* 7.1 Hz, 2H), 0.82 (t, *J* = 7.3 Hz, 3H).; 430[M+H]⁺ |
| 160 | 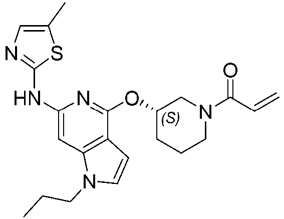 | (S)-1-(3-((6-((5-methylthiazol-2-yl)amino)-1-propyl-1H-pyrrolo[3,2-c]pyridin-4-yl)oxy)piperidin-1-yl)prop-2-en-1-one | ¹H NMR (500 MHz, CDCl₃) δ 7.01 (s, 1H), 6.87 - 6.78 (m, 1H), 6.50 - 6.34 (m, 3H), 6.15 (d, *J* = 16.5 Hz, 1H), 5.53 (s, 1H), 5.45 - 5.34 (m, 1H), 3.94 (dd, *J* = 13.2, 6.5 Hz, 2H), 3.85 (dd, J = 16.0, 4.5 Hz, 2H), 3.65 - 3.54 (m, 1H), 2.32 (d, *J* = 16.0 Hz, 3H), 2.20 - 2.11 (m, 1H), 2.01 - 1.93 (m, 1H), 1.81 (td, *J* = 14.3, 7.1 Hz, 2H), 1.71 - 1.58 (m, 1H), 0.92 (t, *J* = 7.3 Hz, 2H).; 426[M+H]⁺ |
| 161 | 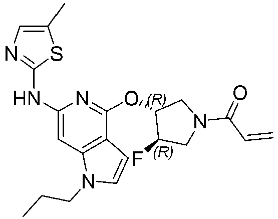 | 1-((3R,4R)-3-fluoro-4-((6-((5-methylthiazol-2-yl)amino)-1-propyl-1H-pyrrolo[3,2-c]pyridin-4-yl)oxy)pyrrolidin-1-yl)prop-2-en-1-one | ¹H NMR (500 MHz, DMSO) δ 10.63 (s, 1H), 7.18 (d, *J* = 2.6 Hz, 1H), 6.98 (s, 1H), 6.71 (s, 1H), 6.68 - 6.57 (m, 1H), 6.36 (d, *J* = 2.7 Hz, 1H), 6.19 (d, *J* = 15.5 Hz, 1H), 5.92 (d, *J* = 55.3 Hz, 1H), 5.72 (dd, *J* = 16.6, 11.7 Hz, 1H), 5.52 (dd, *J* = 48.0, 39.9 Hz, 1H), 4.23 - 3.63 (m, 6H), 2.30 (s, 3H), 1.73 (dd, *J* = 14.2, 7.1 Hz, 2H), 0.81 (t, *J* = 7.3 Hz, 3H).; 430[M+H]⁺ |
| 162 | 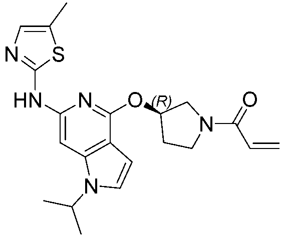 | (R)-1-(3-((1-isopropyl-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, CDCl₃) δ 9.75 (s, 1H), 7.05-6.95 (m, 2H), 6.58-6.35 (m, 4H), 5.99 (d, *J* = 23.1 Hz, 1H), 5.68 (ddd, *J =* 24.9, 9.4, 2.8 Hz, 1H), 4.50 (sep, *J* = 6.5 Hz, 1H), 4.07-3.74 (m, 4H), 3.70 (s, 3H), 2.56-2.22 (m, 5H), 1.50 (d, *J* = 6.5 Hz, 6H). 412[M+H]⁺ |
| 163 | 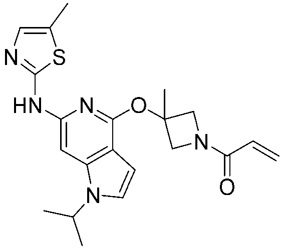 | 1-(3-((1-isopropyl-6-((5-methylthiazol-2-yl)amino)-1H-pyrrolo[3,2-c]pyridin-4-yl)oxy)-3-methylazetidin-1-yl)prop-2-en-1-one | ¹H NMR (500 MHz, CDCl₃) δ 8.78 (s, 1H), 7.00 (d, *J* = 3.0 Hz, 2H), 6.79 (d, *J* = 11.0 Hz, 1H), 6.49 (d, *J* = 3.2 Hz, 1H), 6.34 (dd, *J* = 16.9, 1.3 Hz, 1H), 6.22 (dd, *J* = 17.0, 10.3 Hz, 1H), 5.66 (d, *J =* 10.3 Hz, 1H), 4.61 (d, *J =* 9.5 Hz, 1H), 4.56-4.42 (m, 3H), 4.31 (d, *J* = 10.8 Hz, 1H), 2.40 (d, *J* = 0.8 Hz, 3H), 1.99 (s, 3H), 1.51 (d, *J* = 6.7 Hz, 6H). 412[M+H]⁺ |
| 164 | 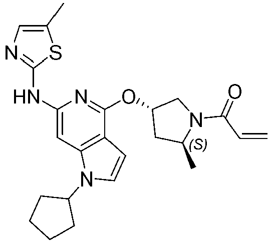 | 1-((2S,4S)-4-((1-cyclopentyl-6-((5-methylthiazol-2-yl)amino)-1H-pyrrolo[3,2-c]pyridin-4-yl)oxy)-2-methylpyrrolidin-1-yl)prop-2-en-1-one | ¹H NMR (500 MHz, DMSO) δ 10.60 (s, 1H), 7.21 (s, 1H), 6.97 (s, 1H), 6.73 - 6.48 (m, 2H), 6.32 (s, 1H), 6.20 - 6.06 (m, 1H), 5.97 - 5.84 (m, 1H), 5.75 - 5.55 (m, 1H), 4.67 - 4.57 (m, 1H), 4.45 - 4.10 (m, 2H), 3.90 - 3.74 (m, 2H), 2.44 - 2.35 (m, 1H), 2.30 (s, 3H), 2.20 - 2.05 (m, 3H), 1.76 (d, *J* = 69.9 Hz, 5H), 1.31 (t, *J* = 5.6 Hz, 3H).452[M+H]⁺ |
| 165 | 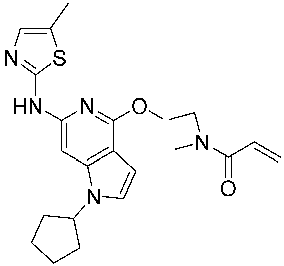 | 1-(3-((1-cyclopentyl-6-((5-methylthiazol-2-yl)amino)-1H-pyrrolo[3,2-c]pyridin-4-yl)oxy)-3-methylazetidin-1-yl)prop-2-en-1-one | ¹H NMR (500 MHz, DMSO) δ 10.33 (s, 1H), 7.27 - 7.11 (m, 2H), 6.96 (s, 1H), 6.44 - 6.30 (m, 2H), 6.11 (dd, *J* = 17.0, 1.9 Hz, 1H), 5.67 (dd, *J* = 10.4, 1.9 Hz, 1H), 4.66 - 4.59 (m, 1H), 4.55 - 4.47 (m, 2H), 4.30 - 4.15 (m, 2H), 2.33 (s, 3H), 2.11 (d, *J* = 6.3 Hz, 3H), 1.91 (s, 3H), 1.84 (s, 5H)..438[M+H]⁺ |
| 166 | 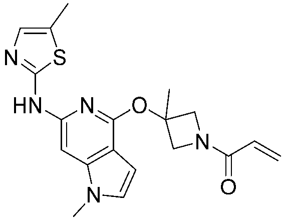 | 1-(3-methyl-3-((1-methyl-6-((5-methylthiazol-2-yl)amino)-1H-pyrrolo[3,2-c]pyridin-4-yl)oxy)azetidin-1-yl)prop-2-en-1-one | ¹H NMR (500 MHz, DMSO-*d₆*) δ 10.37 (s, 1H), 7.13 (d, *J* = 3.2 Hz, 1H), 7.09 (s, 1H), 6.97 (d, *J* = 1.2 Hz, 1H), 6.37 (d, *J* = 3.3 Hz, 1H), 6.36-6.30 (m, 1H), 6.11 (dd, *J =* 17.0, 2.0 Hz, 1H), 5.67 (dd, *J* = 10.4, 2.0 Hz, 1H), 4.51 (q, *J* = 9.8 Hz, 2H), 4.23 (dd, J *=* 38.2, 11.0 Hz, 2H), 3.66 (s, 3H), 2.33 (s, 3H), 1.91 (s, 3H). 384[M+H]⁺ |
| 167 | 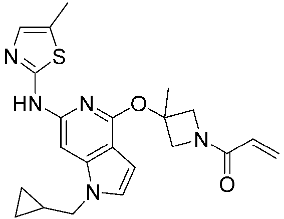 | 1-(3-((1-(cyclopropylmethyl)-6-((5-methylthiazol-2-yl)amino)-1H-pyrrolo[3,2-c]pyridin-4-yl)oxy)-3-methylazetidin-1-yl)prop-2-en-1-one | ¹H NMR (500 MHz, DMSO-*d₆*) δ 10.34 (s, 1H), 7.22 (d, *J* = 3.2 Hz, 1H), 7.14 (s, 1H), 6.97 (s, 1H), 6.38 (d, *J* = 3.1 Hz, 1H), 6.34 (dd, *J* = 17.0, 10.4 Hz, 1H), 6.11 (dd, *J* = 17.0, 1.9 Hz, 1H), 5.67 (dd, *J* = 10.4, 1.9 Hz, 1H), 4.52 (q, *J* = 9.9 Hz, 2H), 4.23 (dd, *J* = 36.4, 11.0 Hz, 2H), 3.88 (d, *J* = 6.9 Hz, 2H), 2.33 (s, 3H), 1.92 (s, 3H), 1.20-1.13 (m, 1H), 0.56-0.49 (m, 2H), 0.38 (q, *J* = 4.8 Hz, 2H). 424[M+H]⁺ |
| 168 | 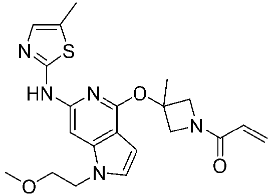 | 1-(3-((1-(2-methoxyethyl)-6-((5-methylthiazol-2-yl)amino)-1H-pyrrolo[3,2-c]pyridin-4-yl)oxy)-3-methylazetidin-1-yl)prop-2-en-1-one | ¹H NMR (500 MHz, DMSO-*d₆*) δ 12.22 (s, 1H), 7.51 (d, *J* = 3.2 Hz, 1H), 7.18-6.92 (m, 2H), 6.69 (d, *J* = 3.2 Hz, 1H), 6.19 (dd, *J =* 17.1, 10.1 Hz, 1H), 6.06 (dd, *J* = 17.1, 2.0 Hz, 1H), 5.59 (dd, *J =* 10.1, 2.0 Hz, 1H), 4.58-4.30 (m, 4H), 3.87-3.64 (m, 4H), 3.23 (s, 3H), 2.27 (s, 3H), 1.68 (s, 3H). 428[M+H]⁺ |
| 169 | 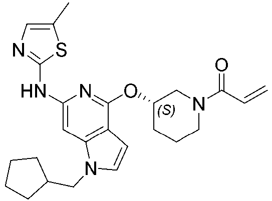 | (S)-1-(3-((1-(cyclopentylmethyl)-6-((5-methylthiazol-2-yl)amino)-1H-pyrrolo[3,2-c]pyridin-4-yl)oxy)piperidin-1-yl)prop-2-en-1-one | ¹H NMR (500 MHz, CDCl₃) δ 7.05 (s, 1H), 6.81 (s, 1H), 6.42 (s, 3H), 6.14 (d, *J* = 16.7 Hz, 1H), 5.54 (s, 1H), 5.41 (d, *J =* 10.2 Hz, 1H), 3.95 - 3.51 (m, 6H), 2.37 (s, 4H), 2.20 - 1.83 (m, 3H), 1.61 (d, *J* = 54.5 Hz, 7H), 1.29 - 1.23 (m, 2H).; 466[M+H]⁺ |
| 170 | 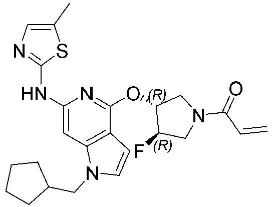 | 1-((3R,4R)-3-((1-(cyclopentylmethyl)-6-((5-methylthiazol-2-yl)amino)-1H-pyrrolo[3,2-c]pyridin-4-yl)oxy)-4-fluoropyrrolidin-1-yl)prop-2-en-1-one | ¹H NMR (500 MHz, CDCl₃) δ 7.06 (d, *J* = 5.8 Hz, 1H), 6.86 (dd, *J* = 5.6, 3.1 Hz, 1H), 6.55 - 6.35 (m, 4H), 6.09 - 5.93 (m, 1H), 5.72 (ddd, *J* = 19.7, 8.0, 4.1 Hz, 1H), 5.45 (dd, *J* = 49.4, 33.7 Hz, 1H), 4.21 - 3.82 (m, 6H), 2.38 (dd, *J* = 19.2, 6.3 Hz, 4H), 1.77 - 1.47 (m, 6H), 1.32 - 1.18 (m, 2H).; 470[M+H]⁺ |
| 171 | 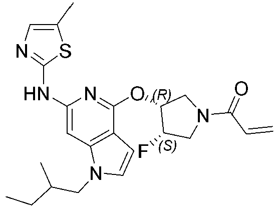 | 1-((3S,4R)-3-fluoro-4-((1-(2-methylbutyl)-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, CDCl₃) δ 7.05 (s, 1H), 6.86 (d, *J* = 2.9 Hz, 1H), 6.59 - 6.39 (m, 4H), 5.88 (dt, *J* = 14.9, 8.5 Hz, 1H), 5.82 - 5.71 (m, 1H), 5.62 (dd, *J* = 53.8, 17.8 Hz, 1H), 4.37 (dd, *J* = 18.0, 9.0 Hz, 1H), 4.15 - 3.79 (m, 4H), 3.73 (dd, *J* = 14.0, 8.0 Hz, 1H), 2.46 - 2.26 (m, 3H), 1.94 (d, *J* = 6.0 Hz, 1H), 1.41 (dd, *J* = 12.6, 5.8 Hz, 1H), 1.27 - 1.13 (m, 1H), 1.00 - 0.91 (m, 3H), 0.86 (t, *J* = 18.1 Hz, 3H).; 458[M+H]⁺ |
| 172 | 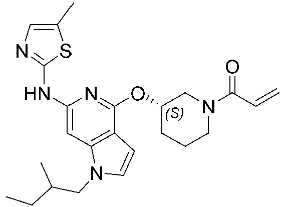 | 1-((3S)-3-((1-(2-methylbutyl)-6-((5-methylthiazol-2-yl)amino)-1H-pyrrolo[3,2-c]pyridin-4-yl)oxy)piperidin-1-yl)prop-2-en-1-one | ¹H NMR (500 MHz, CDCl₃) δ 7.06 (s, 1H), 6.78 (s, 1H), 6.48 - 6.33 (m, 3H), 6.14 (d, *J* = 16.7 Hz, 1H), 5.55 (s, 1H), 5.40 (d, *J* = 10.6 Hz, 1H), 3.93 - 3.78 (m, 3H), 3.69 (dd, *J* = 14.1, 7.9 Hz, 1H), 3.64 - 3.51 (m, 1H), 2.36 (s, 3H), 2.22 - 2.13 (m, 1H), 2.12 - 2.05 (m, 1H), 1.96 - 1.87 (m, 1H), 1.71 - 1.61 (m, 1H), 1.46 - 1.34 (m, 1H), 1.21 - 1.11 (m, 1H), 0.92 (t, *J* = 7.4 Hz, 3H), 0.85 (d, *J* = 6.6 Hz, 3H).; 454[M+H]⁺ |
| 173 | 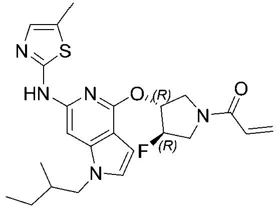 | 1-((3R,4R)-3-fluoro-4-((1-(2-methylbutyl)-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, CDCl₃) δ 6.87 (dd, *J* = 10.8, 3.8 Hz, 2H), 6.60 (s, 1H), 6.50 - 6.32 (m, 3H), 5.78 (dd, *J* = 28.9, 22.3 Hz, 1H), 5.74 - 5.66 (m, 1H), 5.38 (dd, *J* = 49.3, 37.4 Hz, 1H), 4.13 - 3.67 (m, 6H), 2.30 (d, *J* = 6.1 Hz, 3H), 1.86 (dd, *J* = 12.4, 6.3 Hz, 1H), 1.40 - 1.25 (m, 1H), 1.17 - 1.06 (m, 1H), 0.89 (t, *J* = 7.3 Hz, 3H), 0.81 (d, *J* = 6.3 Hz, 3H).; 458[M+H]⁺ |
| 174 | 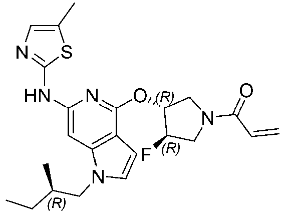 | 1-((3R,4R)-3-fluoro-4-((1-((R)-2-methylbutyl)-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, DMSO) δ 10.60 (s, 1H), 7.15 (d, *J* = 3.2 Hz, 1H), 6.97 (s, 1H), 6.72 (s, 1H), 6.69 - 6.57 (m, 1H), 6.36 (d, *J* = 3.1 Hz, 1H), 6.19 (ddd, *J* = 16.8, 4.2, 2.3 Hz, 1H), 5.91 (ddd, *J* = 55.0, 8.9, 3.8 Hz, 1H), 5.73 (ddd, *J* = 16.8, 10.3, 2.2 Hz, 1H), 5.52 (dd, *J* = 49.1, 41.5 Hz, 1H), 4.20 - 3.67 (m, 6H), 2.34 - 2.26 (m, 3H), 1.92 - 1.82 (m, 1H), 1.35 - 1.06 (m, 2H), 0.89 (t, *J* = 7.4 Hz, 3H), 0.79 (d, *J* = 6.6 Hz, 3H).; 458[M+H]⁺ |
| 175 | 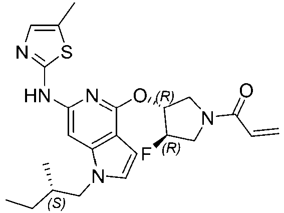 | 1-((3R,4R)-3-fluoro-4-((1-((S)-2-methylbutyl)-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, DMSO) δ 10.59 (s, 1H), 7.15 (d, *J* = 3.1 Hz, 1H), 6.97 (s, 1H), 6.72 (s, 1H), 6.63 (ddd, *J* = 16.7, 12.8, 10.4 Hz, 1H), 6.36 (d, *J* = 3.0 Hz, 1H), 6.19 (ddd, *J* = 16.7, 4.0, 2.3 Hz, 1H), 5.91 (ddd, *J* = 54.9, 8.7, 3.9 Hz, 1H), 5.72 (ddd, *J* = 17.0, 10.3, 2.0 Hz, 1H), 5.51 (dd, *J* = 49.4, 39.7 Hz, 1H), 4.21 - 3.67 (m, 6H), 1.86 (dq, *J* = 13.4, 6.8 Hz, 1H), 1.35 - 1.25 (m, 1H), 1.18 - 1.05 (m, 1H), 0.88 (t, *J* = 7.4 Hz, 3H), 0.79 (d, *J* = 6.7 Hz, 3H).; 458[M+H]⁺ |
| 176 | 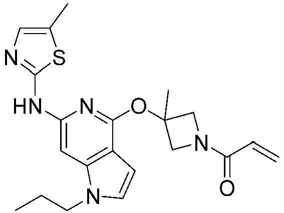 | 1-(3-methyl-3-((6-((5-methylthiazol-2-yl)amino)-1-propyl-1H-pyrrolo[3,2-c]pyridin-4-yl)oxy)azetidin-1-yl)prop-2-en-1-one | ¹H NMR (500 MHz, DMSO-*d₆*) δ 10.32 (s, 1H), 7.17 (d, *J* = 3.2 Hz, 1H), 7.08 (s, 1H), 6.97 (d, *J* = 1.1 Hz, 1H), 6.38 (d, *J* = 3.1 Hz, 1H), 6.34 (dd, *J* = 17.0, 10.4 Hz, 1H), 6.11 (dd, *J* = 17.0, 2.0 Hz, 1H), 5.67 (dd, *J* = 10.4, 2.0 Hz, 1H), 4.52 (q, *J* = 9.8 Hz, 2H), 4.23 (dd, *J* = 37.4, 11.0 Hz, 2H), 3.98 (t, *J* = 6.9 Hz, 2H), 2.33 (s, 3H), 1.92 (s, 3H), 1.81-1.68 (m, 2H), 0.82 (t, *J* = 7.4 Hz, 3H). 412[M+H]⁺ |
| 177 | 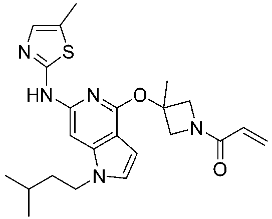 | 1-(3-((1-isopentyl-6-((5-methylthiazol-2-yl)amino)-1H-pyrrolo[3,2-c]pyridin-4-yl)oxy)-3-methylazetidin-1-yl)prop-2-en-1-one | ¹H NMR (500 MHz, DMSO-*d₆*) δ 10.35 (s, 1H), 7.18 (d, *J* = 3.2 Hz, 1H), 7.10 (s, 1H), 6.96 (d, *J* = 1.2 Hz, 1H), 6.40-6.30 (m, 2H), 6.11 (dd, *J* = 17.0, 2.0 Hz, 1H), 5.67 (dd, *J* = 10.4, 2.0 Hz, 1H), 4.52 (q, *J* = 9.8 Hz, 2H), 4.23 (dd, *J* = 38.4, 11.0 Hz, 2H), 4.03 (t, *J* = 7.2 Hz, 2H), 2.33 (s, 3H), 1.92 (s, 3H), 1.61 (dd, *J* = 14.2, 7.0 Hz, 2H), 1.47 (tt, *J* = 13.3, 6.6 Hz, 1H), 0.92 (d, *J* = 6.6 Hz, 6H). 440[M+H]⁺ |
| 178 | 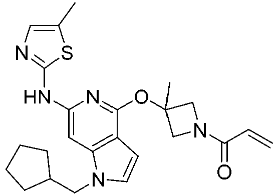 | 1-(3-((1-(cyclopentylmethyl)-6-((5-methylthiazol-2-yl)amino)-1H-pyrrolo[3,2-c]pyridin-4-yl)oxy)-3-methylazetidin-1-yl)prop-2-en-1-one | ¹H NMR (500 MHz, DMSO-*d₆*) δ 10.31 (s, 1H), 7.18 (d, *J* = 3.2 Hz, 1H), 7.08 (s, 1H), 6.96 (d, *J* = 1.0 Hz, 1H), 6.41-6.30 (m, 2H), 6.11 (dd, *J* = 17.0, 1.9 Hz, 1H), 5.67 (dd, *J* = 10.4, 2.0 Hz, 1H), 4.52 (q, *J* = 9.8 Hz, 2H), 4.23 (dd, *J* = 38.2, 11.0 Hz, 2H), 3.92 (d, *J* = 7.5 Hz, 2H), 2.40-2.29 (m, 4H), 1.92 (s, 3H), 1.69-1.45 (m, 6H), 1.30-1.19 (m, 2H). 452[M+H]⁺ |
| 179 | 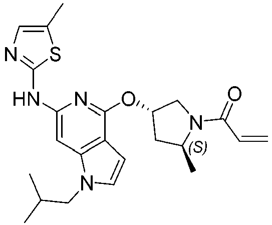 | 1-((2S,4S)-4-((1-isobutyl-6-((5-methylthiazol-2-yl)amino)-1H-pyrrolo[3,2-c]pyridin-4-yl)oxy)-2-methylpyrrolidin-1-yl)prop-2-en-1-one | ¹H NMR (500 MHz, CDCl₃) δ 7.05 (d, *J* = 8.0 Hz, 1H), 6.84 - 6.78 (m, *J =* 5.9, 3.0 Hz, 1H), 6.47 - 6.32 (m, 4H), 6.10 - 6.01 (m, 1H), 5.74 - 5.59 (m, 1H), 4.56 - 4.32 (m, 1H), 4.18 - 4.06 (m, 1H), 3.90 - 3.74 (m, 3H), 2.50 - 2.43 (m, 1H), 2.39 (d, 3H), 2.21 - 2.12 (m, 2H), 1.46 - 1.39 (m, 3H), 0.93 (d, *J =* 6.4 Hz, 6H).440[M+H]⁺ |
| 180 | 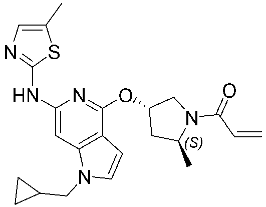 | 1-((2S,4S)-4-((1-(cyclopropylmethyl)-6-((5-methylthiazol-2-yl)amino)-1H-pyrrolo[3,2-c]pyridin-4-yl)oxy)-2-methylpyrrolidin-1-yl)prop-2-en-1-one | ¹H NMR (500 MHz, CDCl₃) δ 7.08 - 6.99 (m,1H), 6.89 (d, *J* = 14.5 Hz, 1H), 6.67 - 6.32 (m, 4H), 5.85 (d, *J* = 24.9 Hz, 1H), 5.75 - 5.58 (m, 1H), 4.55 - 4.30 (m, 1H), 4.18 - 4.02 (m, 1H), 3.97 - 3.77 (m, 3H), 2.58 - 2.38 (m, 1H), 2.31 (d, *J* = 10.8 Hz, 3H), 2.26 - 2.06 (m, 1H), 1.46 - 1.32 (m, 3H), 1.21 - 1.07 (m, 1H), 0.67 - 0.54 (m, 2H), 0.41 - 0.27 (m, 2H).438[M+H]⁺ |
| 181 | 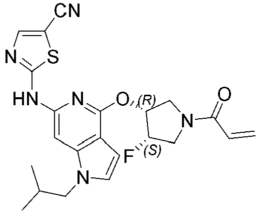 | 2-((4-(((3R,4S)-1-acryloyl-4-fluoropyrrolidin-3-yl)oxy)-1-isobutyl-1H-pyrrolo[3,2-c]pyridin-6-yl)amino)thiazole-5-carbonitrile | ¹H NMR (500 MHz, DMSO) δ 8.18 (s, 1H), 7.30 (s, 1H), 6.81 (s, 1H), 6.65 (dd, *J* = 16.1, 10.7 Hz, 1H), 6.48 (s, 1H), 6.21 (d, *J* = 16.4 Hz, 1H), 5.90 (s, 1H), 5.78 - 5.69 (m, 1H), 5.69 - 5.51 (m, 2H), 4.29 (d, *J* = 92.1 Hz, 1H), 4.16 - 3.99 (m, 1H), 3.94 - 3.71 (m, 4H), 2.14 - 2.05 (m, 1H), 0.85 (d, *J* = 5.4 Hz, 6H).; 455[M+H]⁺ |
| 182 | 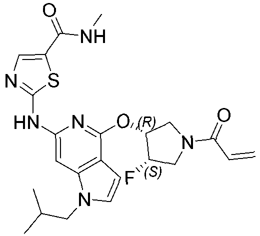 | 2-((4-(((3R,4S)-1-acryloyl-4-fluoropyrrolidin-3-yl)oxy)-1-isobutyl-1H-pyrrolo[3,2-c]pyridin-6-yl)amino)-N-methylthiazole-5-carboxamide | ¹H NMR (500 MHz, DMSO) δ 11.19 (s, 1H), 8.20 (d, *J* = 4.4 Hz, 1H), 7.88 (s, 1H), 7.25 (s, 1H), 6.79 (s, 1H), 6.73 - 6.58 (m, 1H), 6.46 (dd, *J* = 8.2, 2.5 Hz, 1H), 6.22 (dd, *J* = 16.0, 5.0 Hz, 1H), 6.01 - 5.82 (m, 1H), 5.81 - 5.60 (m, 2H), 4.42 - 4.17 (m, 1H), 4.14 - 4.01 (m, 1H), 3.93 - 3.73 (m, 3H), 3.56 - 3.48 (m, 1H), 2.73 (d, *J* = 3.8 Hz, 2H), 2.16 - 2.04 (m, 1H), 0.85 (d, *J* = 6.4 Hz, 6H).; 487[M+H]⁺ |
| 183 | 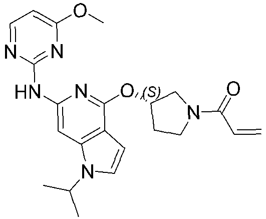 | (S)-1-(3-((1-isopropyl-6-((4-methoxypyrimidin-2-yl)amino)-1 H-pyrrolo[3,2-c]pyridin-4-yl)oxy)pyrrolidin-1-yl)prop-2-en-1-one | ¹H NMR (500 MHz, CDCl₃) δ 8.19 (d, *J =* 5.7 Hz, 1H), 8.00 (s, 1H), 7.04-6.97 (m, 1H), 6.52-6.32(m, 3H), 6.24 (t, *J* = 6.2 Hz, 1H), 5.79-5.63 (m, 2H), 4.59 (sep, *J* = 6.6 Hz, 1H), 4.04 (s, 3H), 3.99-3.73 (m, 4H), 2.45-2.20 (m, 2H), 1.52 (d, *J* = 6.6 Hz, 6H). 423[M+H]⁺ |
| 184 | 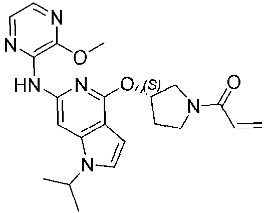 | (S)-1-(3-((1-isopropyl-6-((3-methoxypyrazin-2-yl)amino)-1H-pyrrolo[3,2-c]pyridin-4-yl)oxy)pyrrolidin-1-yl)prop-2-en-1-one | ¹H NMR (500 MHz, CDCl₃) δ 8.17 (d, *J* = 6.9 Hz, 1H), 7.78 (dd, *J* = 5.2, 3.1 Hz, 1H), 7.64 (d, *J* = 12.2 Hz, 1H), 7.50 (dd, *J* = 5.1, 3.1 Hz, 1H), 7.02 (dd, *J =* 5.7, 3.3 Hz, 1H), 6.56-6.37 (m, 3H), 5.89-5.75 (m, 1H), 5.68 (ddd, *J* = 19.8, 9.3, 2.9 Hz, 1H), 4.67 (sep, *J* = 6.6 Hz, 1H), 4.09 (s, 3H), 4.01-3.74 (m, 4H), 2.46-2.15 (m, 2H), 1.53 (d, *J* = 6.6 Hz, 6H). 436[M+Hl⁺ |
| 185 | 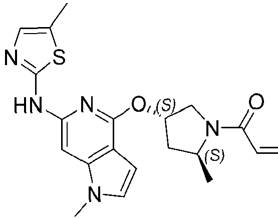 | 1-((2S,4S)-2-methyl-4-((1-methyl-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, DMSO-*d₆*) δ 10.69 (s, 1H), 7.10 (d, *J =* 1.0 Hz, 1H), 6.98 (s, 1H), 6.72-6.47 (m, 2H), 6.30 (s, 1H), 6.14 (dd, *J* = 24.7, 16.3 Hz, 1H), 5.92 (dd, *J* = 10.4, 4.6 Hz, 1H), 5.65 (dd, *J* = 40.9, 9.7 Hz, 1H), 4.44-3.75 (m, 3H), 3.35 (s, 3H), 2.45-2.12 (m, 5H), 1.30 (t, *J =* 6.7 Hz, 3H). 398[M+H]⁺ |
| 186 | 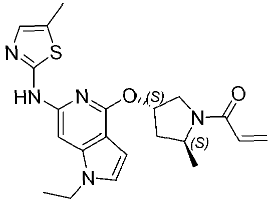 | 1-((2S,4S)-4-((1-ethyl-6-((5-methylthiazol-2-yl)amino)-1H-pyrrolo[3,2-c]pyridin-4-yl)oxy)-2-methylpyrrolidin-1-yl)prop-2-en-1-one | ¹H NMR (500 MHz, DMSO-*d₆*) δ 10.63 (s, 1H), 7.16 (s, 1H), 6.97 (s, 1H), 6.75-6.49 (m, 2H), 6.31 (s, 1H), 6.14 (dd, *J* = 24.0, 16.7 Hz, 1H), 5.97-5.85 (m, 1H), 5.66 (dd, *J* = 40.5, 10.3 Hz, 1H), 4.47-3.75 (m, 5H), 2.44-2.13 (m, 5H), 1.35-1.18 (m, 6H). 412[M+H]⁺ |
| 187 | 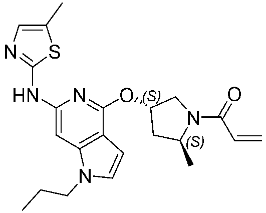 | 1-((2S,4S)-2-methyl-4-((6-((5-methylthiazol-2-yl)amino)-1-propyl-1H-pyrrolo[3,2-c]pyridin-4-yl)oxy)pyrrolidin-1-yl)prop-2-en-1-one | ¹H NMR (500 MHz, DMSO-*d₆*) δ 10.65 (s, 1H), 7.15 (s, 1H), 6.98 (s, 1H), 6.77-6.48 (m, 2H), 6.31 (s, 1H), 6.14 (dd, *J* = 24.6, 16.7 Hz, 1H), 5.99-5.87 (m, 1H), 5.66 (dd, *J* = 41.2, 10.2 Hz, 1H), 4.48-3.74 (m, 5H), 2.45-2.12 (m, 5H), 1.73 (dd, *J* = 13.3, 6.6 Hz, 2H), 1.31 (t, *J* = 5.1 Hz, 3H), 0.81 (t, *J* = 6.8 Hz, 3H). 426[M+H]⁺ |
| 188 | 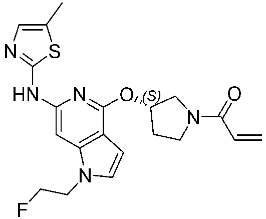 | (S)-1-(3-((1-(2-fluoroethyl)-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, OMSO-*d₆*) δ 10.65 (s, 1H), 7.16 (s, 1H), 6.97 (s, 1H), 6.71-6.50 (m, 2H), 6.40 (s, 1H), 6.15 (dd, *J =* 16.7, 6.5 Hz, 1H), 5.91 (d, *J* = 42.3 Hz, 1H), 5.67 (dd, *J* = 26.5, 10.3 Hz, 1H), 4.71 (d, *J* = 47.3 Hz, 2H), 4.33 (d, *J* = 28.0 Hz, 2H), 4.13-3.48 (m, 4H), 2.46-2.16 (m, 5H). 416[M+H]⁺ |
| 189 | 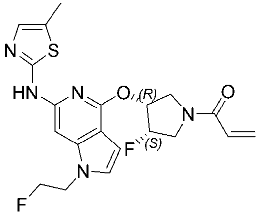 | 1-((3S,4R)-3-fluoro-4-((1-(2-fluoroethyl)-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, DMSO-*d₆*) δ 10.68 (s, 1H), 7.22 (t, *J* = 3.7 Hz, 1H), 6.97 (s, 1H), 6.78 (s, 1H), 6.64 (ddd, *J* = 16.5, 10.3, 4.1 Hz, 1H), 6.45 (t, *J* = 3.7 Hz, 1H), 6.21 (dd, *J* = 16.7, 1.8 Hz, 1H), 5.90-5.54 (m, 3H), 4.73 (dt, *J* = 47.3, 4.5 Hz, 2H), 4.52-3.43 (m, 6H), 2.27 (d, *J* = 9.9 Hz, 3H). 434[M+H]⁺ |
| 190 | 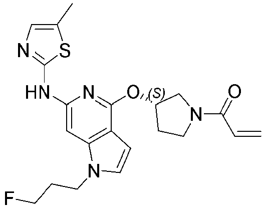 | (S)-1-(3-((1-(3-fluoropropyl)-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, DMSO-*d₆*) δ 10.63 (s, 1H), 7.15 (t, *J =* 3.2 Hz, 1H), 6.97 (s, 1H), 6.71- 6.50 (m, 2H), 6.38 (s, 1H), 6.15 (ddd, *J* = 16.7, 5.8, 2.3 Hz, 1H), 5.91 (d, *J* = 42.5 Hz, 1H), 5.66 (ddd, *J* = 25.8, 10.3, 2.3 Hz, 1H), 4.39 (dt, *J* = 47.3, 5.2 Hz, 2H), 4.22-3.48 (m, 6H), 2.45-2.03 (m, 7H). 430[M+H]⁺ |
| 191 | 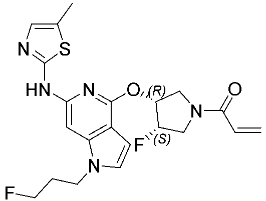 | 1-((3S,4R)-3-fluoro-4-((1-(3-fluoropropyl)-6-((5-methylthiazol-2-yl)amino)-1H-pyrrolo[3,2-c]pyridin-4-yl)oxy)pyrrolidin-1-yl)prop-2-en-1-one | ¹H NMR (500 MHz, DMSO-*d₆*) δ 11.21 (s, 1H), 7.27 (d, *J =* 2.9 Hz, 1H), 7.08 (d, *J* = 9.7 Hz, 1H), 6.81 (s, 1H), 6.64 (ddd, *J* = 16.6, 10.2, 6.3 Hz, 1H), 6.47 (dd, *J* = 8.3, 3.0 Hz, 1H), 6.21 (d, *J =* 16.7 Hz, 1H), 5.90-5.51 (m, 3H), 4.50-3.44 (m, 10H), 2.28 (d, *J* = 9.7 Hz, 3H), 2.20-2.03 (m, 2H). 448[M+H]⁺ |
| 192 | 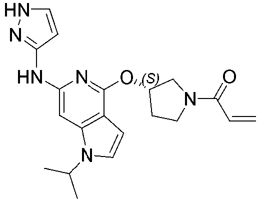 | (S)-1-(3-((6-((1H-pyrazol-3-yl)amino)-1-isopropyl-1H-pyrrolo[3,2-c]pyridin-4-yl)oxy)pyrrolidin-1-yl)prop-2-en-1-one | ¹H NMR (500 MHz, DMSO-*d₆*) δ 11.99 (s, 1H), 8.61 (d, *J =* 4.9 Hz, 1H), 7.54 (s, 1H), 7.17 (s, 1H), 7.14 (s, 1H), 6.60 (ddd, *J* = 45.7, 16.7, 10.3 Hz, 1H), 6.29 (s, 1H), 6.18-6.08 (m, 2H), 5.67 (dd, *J* = 20.8, 11.4 Hz, 2H), 4.44 (sep, *J* = 6.6 Hz, 1H), 4.09-3.45 (m, 4H), 2.38-2.09 (m, 2H), 1.41 (d, *J* = 6.6 Hz, 6H). 381[M+H]⁺ |
| 193 | 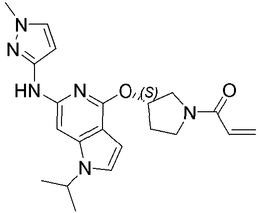 | (S)-1-(3-((1-isopropyl-6-((1-methyl-1H-pyrazol-3-yl)amino)-1H-pyrrolo[3,2-c]pyridin-4-yl)oxy)pyrrolidin-1-yl)prop-2-en-1-one | ¹H NMR (500 MHz, DMSO-*d₆*) δ 8.61 (d, *J* = 6.3 Hz, 1H), 7.48 (s, 1H), 7.13 (t, *J* = 3.2 Hz, 1H), 7.02 (d, *J* = 11.2 Hz, 1H), 6.59 (ddd, *J* = 45.8, 16.7, 10.3 Hz, 1H), 6.29 (s, 1H), 6.18-6.12 (m, 2H), 5.74-5.60 (m, 2H), 4.44 (sep, *J* = 6.6 Hz, 1H), 4.04-3.48 (m, 7H), 2.38-2.09 (m, 2H), 1.41 (d, *J* = 6.6 Hz, 6H). 395[M+H]⁺ |
| 194 | 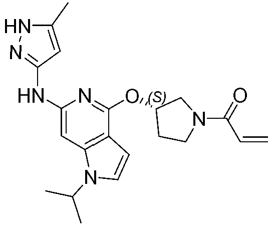 | (S)-1-(3-((1-isopropyl-6-((5-methyl-1H-pyrazol-3-yl)amino)-1H-pyrrolo[3,2-c]pyridin-4-yl)oxy)pyrrolidin-1-yl)prop-2-en-1-one | ¹H NMR (500 MHz, DMSO-*d₆*) δ 11.86 (s, 1H), 8.58 (d, *J =* 12.1 Hz, 1H), 7.22-7.05 (m, 2H), 6.60 (ddd, *J* = 44.5, 16.7, 10.3 Hz, 1H), 6.29 (d, *J* = 2.7 Hz, 1H), 6.15 (d, *J =* 16.7 Hz, 1H), 5.89 (d, *J* = 12.2 Hz, 1H), 5.74-5.60 (m, 2H), 4.44 (sep, *J* = 6.6 Hz, 1H), 4.07-3.47 (m, 4H), 2.39-2.09 (m, 5H), 1.41 (d, *J* = 6.6 Hz, 6H). 395[M+H]⁺ |
| 195 | 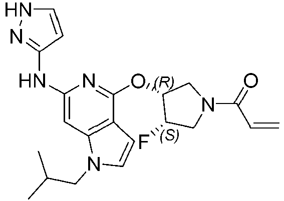 | 1-((3R,4S)-3-((6-((1H-pyrazol-3-yl)amino)-1-isobutyl-1H-pyrrolo[3,2-c]pyridin-4-yl)oxy)-4-fluoropyrrolidin-1-yl)prop-2-en-1-one | ¹H NMR (500 MHz, DMSO-*d₆*) δ 12.08 (s, 1H), 8.70 (s, 1H), 7.57 (d, *J =* 1.9 Hz, 1H), 7.17 (d, *J* = 4.9 Hz, 1H), 7.06 (t, *J* = 2.8 Hz, 1H), 6.64 (dt, *J* = 16.8, 9.7 Hz, 1H), 6.38-6.30 (m, 1H), 6.21 (dd, *J* = 16.7, 2.1 Hz, 1H), 6.08-6.00 (m, 1H), 5.75-5.44 (m, 3H), 4.49-3.21 (m, 6H), 2.17-2.04 (m, 1H), 0.85 (d, *J* = 6.6 Hz, 6H). 413[M+H]⁺ |
| 196 | 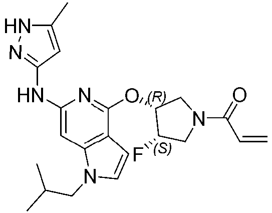 | 1-((3S,4R)-3-fluoro-4-((1-isobutyl-6-((5-methyl-1H-pyrazol-3-yl)amino)-1H-pyrrolo[3,2-c]pyridin-4-yl)oxy)pyrrolidin-1-yl)prop-2-en-1-one | ¹H NMR (500 MHz, DMSO-*d₆*) δ 11.91 (s, 1H), 8.62 (s, 1H), 7.17 (d, *J* = 2.2 Hz, 1H), 7.05 (t, *J =* 2.7 Hz, 1H), 6.64 (dt, *J* = 16.8, 10.3 Hz, 1H), 6.41-6.29 (m, 1H), 6.21 (dd, *J* = 16.7, 1.7 Hz, 1H), 5.86-5.47 (m, 4H), 4.48-3.22 (m, 6H), 2.18 (d, *J* = 2.4 Hz, 3H), 2.15-2.06 (m, 1H), 0.84 (d, *J* = 6.6 Hz, 6H). 427[M+H]⁺ |
| 197 | 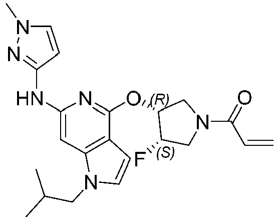 | 1-((3S,4R)-3-fluoro-4-((1-isobutyl-6-((1-methyl-1H-pyrazol-3-yl)amino)-1H-pyrrolo[3,2-c]pyridin-4-yl)oxy)pyrrolidin-1-yl)prop-2-en-1-one | ¹H NMR (500 MHz, DMSO-*d₆*) δ 8.65 (s, 1H), 7.49 (t, *J* = 2.1 Hz, 1H), 7.12 (d, *J* = 3.4 Hz, 1H), 7.05 (t, *J* = 3.0 Hz, 1H), 6.70-6.58 (m, 1H), 6.36-6.28 (m, 1H), 6.21 (dd, *J* = 16.7, 1.9 Hz, 1H), 6.01 (dd, *J* = 3.4, 2.3 Hz, 1H), 5.75-5.41 (m, 3H), 4.43-3.31 (m, 9H), 2.15-2.06 (m, 1H), 0.86 (d, *J* = 6.6 Hz, 6H). 427[M+H]⁺ |
| 198 | 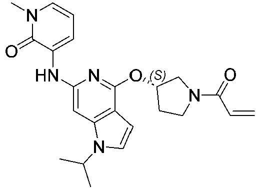 | (S)-3-((4-((1-acryloylpyrrolidin-3-yl)oxy)-1-isopropyl-1H-pyrrolo[3,2-c]pyridin-6-yl)amino)-1-methylpyridin-2(1H)-one | ¹H NMR (500 MHz, DMSO-*d₆*) δ 8.10 (ddd, *J =* 19.7, 7.4, 1.3 Hz, 1H), 8.04 (d, *J* = 6.7 Hz, 1H), 7.23 (t, *J* = 3.0 Hz, 1H), 7.14 (dd, *J =* 6.7, 1.3 Hz, 1H), 6.94 (s, 1H), 6.60 (ddd, *J* = 47.2, 16.7, 10.3 Hz, 1H), 6.35 (s, 1H), 6.26 (t, *J =* 7.1 Hz, 1H), 6.14 (ddd, *J* = 16.7, 6.4, 2.3 Hz, 1H), 5.74-5.61 (m, 2H), 4.60-4.51 (m, 1H), 4.04-3.45 (m, 7H), 2.41-2.15 (m, 2H), 1.41 (d, *J* = 6.7 Hz, 6H). 422 [M+H]⁺ |
| 199 | 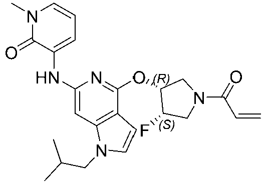 | 3-((4-(((3R,4S)-1-acryloyl-4-fluoropyrrolidin-3-yl)oxy)-1-isobutyl-1H-pyrrolo[3,2-c]pyridin-6-yl)amino)-1-methylpyridin-2(1H)-one | ¹H NMR (500 MHz, DMSO-*d₆*) δ 8.05 (d, *J* = 2.2 Hz, 1H), 7.95 (dd, *J =* 13.5, 7.4 Hz, 1H), 7.14 (dd, *J* = 8.4, 5.2 Hz, 2H), 6.99 (s, 1H), 6.67-6.57 (m, 1H), 6.42-6.33 (m, 1H), 6.27-6.13 (m, 2H), 5.76-5.45 (m, 3H), 4.35-3.41 (m, 6H), 3.53 (s, 3H), 2.15-2.05 (m, 1H), 0.84 (d, *J* = 6.6 Hz, 6H). 454 [M+H]⁺ |
| 200 | 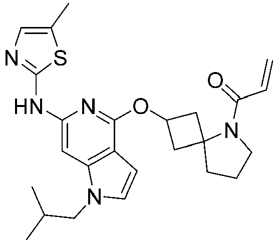 | 1-(2-((1-isobutyl-6-((5-methylthiazol-2-yl)amino)-1H-pyrrolo[3,2-c]pyridin-4-yl)oxy)-5-azaspiro[3.4]octan-5-yl)prop-2-en-1-one | ¹H NMR (500 MHz, DMSO) δ 7.19 - 7.15 (m, 1H), 7.04 (d, *J* = 24.0 Hz, 1H), 6.67 - 6.55 (m, 2H), 6.46 - 6.40 (m, 1H), 6.27 - 6.10 (m, 1H), 6.04 - 5.95 (m, 1H), 5.73 - 5.60 (m, 1H), 3.84 (t, *J* = 7.7 Hz, 3H), 2.32 (s, 3H), 2.23 - 2.17 (m, 2H), 2.13 - 2.05 (m, 4H), 1.85 - 1.72 (m, 3H), 0.85 (d, *J* = 6.6 Hz, 6H).466 [M+H]⁺ |
| 201 | 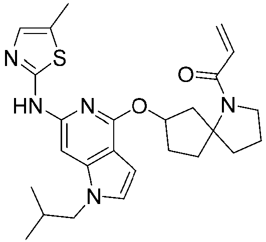 | 1-(7-((1-isobutyl-6-((5-methylthiazol-2-yl)amino)-1H-pyrrolo[3,2-c]pyridin-4-yl)oxy)-1-azaspiro[4.4]nonan-1-yl)prop-2-en-1-one | ¹H NMR (500 MHz, DMSO) δ 7.12 (d, *J* = 3.2 Hz, 1H), 6.96 (s, 1H), 6.57 (s, 1H), 6.38 (d, *J =* 3.1 Hz, 1H), 6.12 (d, *J* = 7.7 Hz, 2H), 5.68 (d, *J* = 8.9 Hz, 2H), 3.87 (d, *J* = 7.4 Hz, 1H), 3.80 (d, *J* = 7.2 Hz, 3H), 2.36 (s, 1H), 2.30 (s, 3H), 2.19 - 2.01 (m, 5H), 1.85 (d, *J* = 12.1 Hz, 2H), 1.60 - 1.49 (m, 2H), 1.18 (s, 1H), 0.88 - 0.76 (m, 6H). 480 [M+H]⁺ |
| 202 | 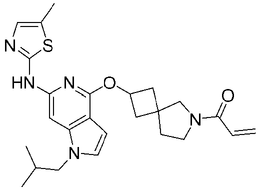 | 1-(2-((1-isobutyl-6-((5-methylthiazol-2-yl)amino)-1H-pyrrolo[3,2-c]pyridin-4-yl)oxy)-6-azaspiro[3.4]octan-6-yl)prop-2-en-1-one | ¹H NMR (500 MHz, DMSO) δ 10.50 (s, 1H), 7.13 (s, 1H), 6.95 (d, *J* = 8.1 Hz, 1H), 6.62 - 6.47 (m, 2H), 6.37 (s, 1H), 6.13 (t, *J* = 14.6 Hz, 1H), 5.65 (d, *J* = 12.9 Hz, 2H), 3.81 (d, *J* = 6.9 Hz, 3H), 3.71 - 3.52 (m, 5H), 2.33 (d, *J* = 7.7 Hz, 3H), 2.23 - 1.91 (m, 6H), 0.84 (d, *J* = 6.3 Hz, 6H).; 466[M+H]⁺ |
| 203 | 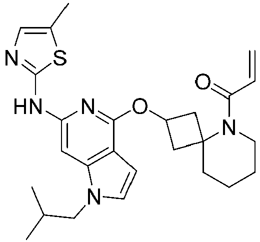 | 1-(2-((1-isobutyl-6-((5-methylthiazol-2-yl)amino)-1H-pyrrolo[3,2-c]pyridin-4-yl)oxy)-5-azaspiro[3.5]nonan-5-yl)prop-2-en-1-one | ¹H NMR (500 MHz, CDCl₃) δ 6.95 (s, 1H), 6.87 (d, *J* = 2.8 Hz, 1H), 6.56 - 6.45 (m, 2H), 6.41 (s, 1H), 6.28 (d, *J* = 16.7 Hz, 1H), 5.68 - 5.54 (m, 2H), 3.79 (d, *J* = 7.3 Hz, 2H), 3.44 (s, 2H), 2.94 (dd, *J* = 14.3, 6.9 Hz, 2H), 2.52 (d, *J* = 13.2 Hz, 2H), 2.41 - 2.32 (m, 3H), 2.17 - 2.06 (m, 3H), 1.70 (s, 2H), 1.43 (s, 3H), 0.95 - 0.86 (m, 6H).; 480[M+H]⁺ |
| 204 | 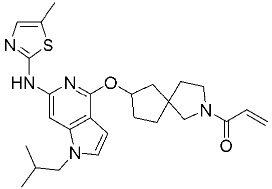 | 1-(7-((1-isobutyl-6-((5-methylthiazol-2-yl)amino)-1H-pyrrolo[3,2-c]pyridin-4-yl)oxy)-2-azaspiro[4.4]nonan-2-yl)prop-2-en-1-one | ¹H NMR (500 MHz, CDCl₃) δ 6.94 (d, *J* = 5.4 Hz, 1H), 6.83 (d, *J* = 2.9 Hz, 1H), 6.50 - 6.33 (m, 4H), 5.86 (s, 1H), 5.67 (dd, *J =* 9.6, 2.8 Hz, 1H), 3.78 (d, *J* = 7.3 Hz, 2H), 3.64 - 3.44 (m, 4H), 2.39 (d, *J* = 13.1 Hz, 2H), 2.29 - 2.09 (m, 5H), 2.04 (t, *J* = 13.8 Hz, 3H), 1.99 - 1.87 (m, 2H), 0.92 (d, *J* = 6.6 Hz, 6H).; 480 [M+H]⁺ |

### Example 205. Preparation of (S)-1-(3-((6-((5-methylthiazol-2-yl)amino)-1-(2,2,2-trifluoroethyl)-1H-pyrrolo[3,2-c]pyridin-4-yl)oxy)pyrrolidin-1-yl)prop-2-en-1-one

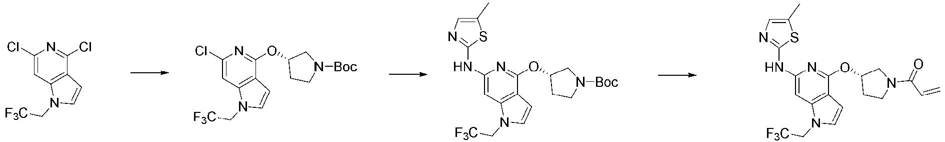

### Step 1. Preparation of tert-butyl (S)-3-((6-chloro-1-(2,2,2-trifluoroethyl)-1H-pyrrolo[3,2-c]pyridin-4-yl)oxy)pyrrolidine-1-carboxylate

4,6-Dichloro-1-(2,2,2-trifluoroethyl)-1*H*-pyrrolo[3,2-*c*]pyridine (269 mg, 1.0 mmol) was dissolved in 1,4-dioxane (4.0 mL), and then *tert*-butyl (*S*)-3-hydroxypyrrolidine-1-carboxylate (225 mg, 1.2 mmol), Pd₂(dba)₃ (46 mg, 0.05 mmol), (*S*)-(-)-BINAP (62 mg, 0.1 mmol) and Cs₂CO₃ (651 mg, 2.0 mmol) were added thereto at room temperature. The reaction mixture was reacted at 130°C for 1 hour using a microwave reactor. The mixture was diluted with ethyl acetate and then washed with distilled water. The organic layer was dried over MgSO₄, filtered, and concentrated. The resulting residue was purified by a silica gel chromatography (0~30% ethyl acetate/hexane) to synthesize tert-butyl (*S*)-3-((6-chloro-1-(2,2,2-trifluoroethyl)-1*H*-pyrrolo[3,2-*c*]pyridin-4-yl)oxy)pyrrolidine-1-carboxylate as a white solid (268 mg, yield: 64%).
¹H NMR (500 MHz, CDCl₃) δ 7.02-6.91 (m, 2H), 6.65 (d, *J* = 2.9 Hz, 1H), 5.76 (d, *J* = 18.8 Hz, 2H), 4.56 (q, *J =* 8.4 Hz, 2H), 3.77-3.48 (m, 4H), 2.22 (br s, 2H), 1.47 (d, *J* = 7.3 Hz, 9H).; LC-MS (ESI) *m*/*z :* 420 [M+H]⁺

### Step 2. Preparation of tert-butyl (S)-3-((6-((5-methylthiazol-2-yl)amino)-1-(2,2,2-trifluoroethyl)-1H-pyrrolo[3,2-c]pyridin-4-yl)oxy)pyrrolidine-1-carboxylate

*tert*-butyl (*S*)-3-((6-((5-methylthiazol-2-yl)amino)-1-(2,2,2-trifluoroethyl)-1*H-*pyrrolo[3,2-*c*]pyridin-4-yl)oxy)pyrrolidine-1-carboxylate (yield: 39%) was synthesized in the same manner as in step 2 of Example 1, except that *tert*-butyl (*S*)-3-((6-chloro-1-(2,2,2-trifluoroethyl)-1*H*-pyrrolo[3,2-c]pyridin-4-yl)oxy)pyrrolidine-1-carboxylate was used instead of *tert*-butyl 4-(6-chloro-1-methyl-1*H*-pyrrolo[3,2-c]pyridin-4-yl)-3,6-dihydropyridine-1(2*H*)-carboxylate used in the synthesis step 2 of Example 1.
¹H NMR (500 MHz, CDCl₃) δ 8.96 (s, 1H), 7.00 (d, *J =* 6.0 Hz, 1H), 6.87 (s, 1H), 6.59 (s, 1H), 6.40 (d, *J =* 13.6 Hz, 1H), 5.94 (d, *J =* 18.0 Hz, 1H), 4.52 (q, *J =* 8.4 Hz, 2H), 3.85-3.50 (m, 4H), 2.44-2.20 (m, 5H), 1.48 (d, *J* = 11.4 Hz, 8H).; LC-MS (ESI) *m*/*z :* 498 [M+H]⁺

### Step 3. Preparation of (S)-1-(3-((6-((5-methylthiazol-2-yl)amino)-1-(2,2,2-trifluoroethyl)-1H-pyrrolo[3,2-c]pyridin-4-yl)oxy)pyrrolidin-1-yl)prop-2-en-1-one

(*S*)-1-(3-((6-((5-methylthiazol-2-yl)amino)-1-(2,2,2-trifluoroethyl)-1*H*-pyrrolo[3,2-c]pyridin-4-yl)oxy)pyrrolidin-1-yl)prop-2-en-1-one (yield: 64%) was synthesized in the same manner as in step 3 of Example 1, except that *tert*-butyl (*S*)-3-((6-((5-methylthiazol-2-yl)amino)-1-(2,2,2-trifluoroethyl)-1*H*-pyrrolo[3,2-*c*]pyridin-4-yl)oxy)pyrrolidine-1-carboxylate was used instead of *tert*-butyl 4-(1-methyl-6-((5-methylthiazol-2-yl)amino)-1*H*-pyrrolo[3,2-c]pyridin-4-yl)-3,6-dihydropyridine-1(2*H*)-carboxylate used in the synthesis step 3 of Example 1.
¹H NMR (500 MHz, DMSO-*d₆*) δ 10.73 (s, 1H), 7.17 (t, *J* = 2.6 Hz, 1H), 6.98 (s, 1H), 6.72 (d, *J* = 4.4 Hz, 1H), 6.60 (ddd, *J* = 50.6, 16.7, 10.3 Hz, 1H), 6.47 (s, 1H), 6.15 (ddd, *J* = 16.7, 7.3, 2.2 Hz, 1H), 5.91 (d, *J* = 42.8 Hz, 1H), 5.67 (ddd, *J* = 27.1, 10.3, 2.2 Hz, 1H), 5.05 (q, *J* = 9.1 Hz, 2H), 4.11-3.48 (m, 4H), 2.44-2.16 (m, 5H).; LC-MS (ESI) *m*/*z* : 452 [M+H]⁺

Examples 206 to 223 were prepared in a similar manner to that of Example 205, and the structure and analysis results of the compounds are summarized in Table 4 below.

**[Table 4]**

| Example | Structure | Compound name | ¹H NMR; LC-MS (ESI) *m*/*z* |
|---|---|---|---|
| 206 | 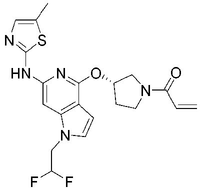 | (*S*)-1-(3-((1-(2,2-difluoroethyl)-6-((5-methylthiazol-2-yl)amino)-1*H*-pyrrolo[3,2-*c*]pyridin-4-yl)oxy)pyrrolidin-1-yl)prop-2-en-1-one | ¹H NMR (500 MHz, CDCl₃) δ 8.79 (s, 1H), 7.03-6.99 (m, 1H), 6.87 (dd, *J* = 7.3, 3.2 Hz, 1H), 6.65-6.33 (m, 4H), 6.00 (ddd, *J* = 58.9, 44.5, 4.0 Hz, 2H), 5.69 (ddd, *J =* 23.9, 9.4, 2.9 Hz, 1H), 4.35 (dd, *J* = 30.0, 14.5 Hz, 2H), 4.07-4.00 (m, 1H), 3.97-3.69 (m, 3H), 2.52-2.25 (m, 5H).; 434 [M+H]⁺ |
| 207 | 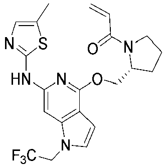 | (*R*)-1-(2-(((6-((5-methylthiazol-2-yl)amino)-1-(2,2,2-trifluoroethyl)-1*H-*pyrrolo[3,2-*c*]pyridin-4-yl)oxy)methyl)pyrrolidin-1-yl)prop-2-en-1-one | ¹H NMR (500 MHz, DMSO-*d*₆) δ 10.68 (d, *J* = 12.5 Hz, 1H), 7.19 (d, *J* = 3.2 Hz, 1H), 6.96 (s, 1H), 6.83-6.60 (m, 2H), 6.45 (dd, *J* = 20.2, 3.0 Hz, 1H), 6.14 (m, 1H), 5.62 (dd, *J* = 56.6, 12.1 Hz, 1H), 5.06 (dd, *J* = 18.2, 8.9 Hz, 2H), 4.75-4.44 (m, 3H), 3.76- 3.42 (m, 2H), 2.29 (s, 3H), 2.20-1.87 (m, 4H).; 466 [M+H]⁺ |
| 208 | 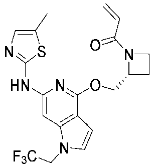 | (*R*)-1-(2-(((6-((5-methylthiazol-2-yl)amino)-1-(2,2,2-trifluoroethyl)-1*H-*pyrrolo[3,2-c]pyridin-4-yl)oxy)methyl)azetidin-1-yl)prop-2-en-1-one | ¹H NMR (500 MHz, DMSO-*d*₆) δ 10.70 (d, *J* = 14.2 Hz, 1H), 7.20 (m, 1H), 6.97 (s, 1H), 6.72 (d, *J* = 10.1 Hz, 1H), 6.64-6.28 (m, 2H), 6.09 (d, *J =* 16.7 Hz, 1H), 5.63 (dd, *J* = 36.9, 10.3 Hz, 1H), 5.11-4.73 (m, 5H), 4.32-3.83 (m, 2H), 2.38-1.93 (m, 5H).; 452 [M+H]⁺ |
| 209 | 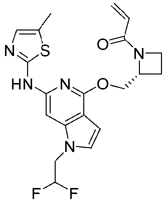 | (*R*)-1-(2-(((1-(2,2-difluoroethyl)-6-((5-methylthiazol-2-yl)amino)-1*H*-pyrrolo[3,2-*c*]pyridin-4-yl)oxy)methyl)azetidin-1-yl)prop-2-en-1-one | ¹H NMR (500 MHz, CDCl₃) δ 9.46 (s, 1H), 7.01 (d, *J =* 15.1 Hz, 1H), 6.91-6.83 (m, 1H), 6.60-6.19 (m, 4H), 6.14-5.84 (m, 1H), 5.65 (dd, *J* = 30.5, 10.1 Hz, 1H), 5.07-4.78 (m, 3H), 4.42-4.06 (m, 4H), 2.66-2.34 (m, 5H).; 434 [M+H]⁺ |
| 210 | 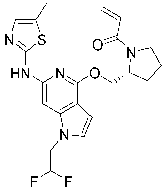 | (*R*)-1-(2-(((1-(2,2-difluoroethyl)-6-((5-methylthiazol-2-yl)amino)-1*H*-pyrrolo[3,2-c]pyridin-4-yl)oxy)methyl)pyrrolidin-1-yl)prop-2-en-1-one, | ¹H NMR (500 MHz, CDCl₃) δ 7.01 (d, *J* = 12.6 Hz, 1H), 6.89-6.80 (m, 1H), 6.75-6.25 (m, 4H), 5.97 (td, *J* = 55.1, 4.1 Hz, 1H), 5.64 (ddd, *J* = 37.5, 10.3, 1.3 Hz, 1H), 4.84 (t, *J* = 7.2 Hz, 1H), 4.72-4.45 (m, 2H), 4.30 (td, *J* = 13.9, 6.8 Hz, 2H), 3.82-3.56 (m, 2H), 2.33 (d, *J =* 39.8 Hz, 3H), 2.21-2.06 (m, 1H). ; 448 [M+H]⁺ |
| 211 | 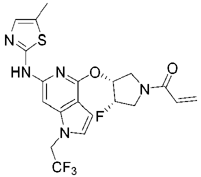 | 1-((3*S*,4*R*)-3-fluoro-4-((6-((5-methylthiazol-2-yl)amino)-1-(2,2,2-trifluoroethyl)-1*H-*pyrrolo[3,2-c]pyridin-4-yl)oxy)pyrrolidin-1-yl)prop-2-en-1-one, | ¹H NMR (500 MHz, DMSO-*d*₆) δ 10.77 (s, 0.6H), 10.75 (s, 0.4H), 7.28-7.16 (m, 11H), 6.98 (s, 9H), 6.84 (d, *J* = 3.2 Hz, 9H), 6.64 (ddd, *J* = 16.7, 10.3, 2.7 Hz, 12H), 6.56-6.45 (m, 9H), 6.21 (d, *J* = 16.8 Hz, 10H), 5.89-5.54 (m, 30H), 5.17-5.01 (m, 18H), 4.49-4.42 (m, 6H), 4.28 (dd, *J* = 11.8, 7.6 Hz, 5H), 4.10-3.99 (m, 10H), 3.78 (ddd, *J* = 33.8, 22.2, 12.1 Hz, 25H), 3.51-3.43 (m, 14H), 2.28 (s, 9H), 2.26 (s, 13H).; 470 [M+H]⁺ |
| 212 | 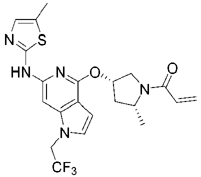 | 1-((2*R*,4*S*)-2-methyl-4-((6-((5-methylthiazol-2-yl)amino)-1-(2,2,2-trifluoroethyl)-1*H-*pyrrolo[3,2-c]pyridin-4-yl)oxy)pyrrolidin-1-yl)prop-2-en-1-one | ¹H NMR (500 MHz, CDCl₃) δ 7.02 (d, *J* = 8.1 Hz, 1H), 6.88 (dd, *J* = 8.5, 3.2 Hz, 1H), 6.63-6.38 (m, 3H), 6.00 (m, 1H), 5.68 (ddd, *J* = 28.6, 9.4, 2.8 Hz, 3H), 4.57-4.47 (m, 2H), 4.36-4.08 (m, 2H), 3.98 (dd, *J* = 28.6, 13.3 Hz, 1H), 2.55 (m, 1H), 2.38 (s, 3H), 2.20 (m, 1H), 1.51 (d, *J* = 6.5 Hz, 3H).; 466 [M+H]⁺ |
| 213 | 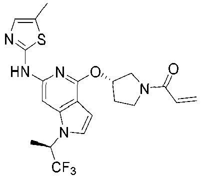 | 1-((*S*)-3-((6-((5-methylthiazol-2-yl)amino)-1-((*R*)-1,1,1-trifluoropropan-2-yl)-1*H*-pyrrolo[3,2-*c*]pyridin-4-yl)oxy)pyrrolidin-1-yl)prop-2-en-1-one | ¹H NMR (500 MHz, CDCl₃) δ 7.06-6.95 (m, 2H), 6.59 (m, 1H), 6.56-6.36 (m, 3H), 6.02 (m, 1H), 5.69 (ddd, *J =* 21.5, 9.4, 2.9 Hz, 2H), 4.77 (m, 1H), 4.05 (m, 1H), 3.97-3.73 (m, 3H), 2.54-2.25 (m, 5H), 1.76 (dd, *J =* 7.0, 3.6 Hz, 3H).; 466 [M+H]⁺ |
| 214 | 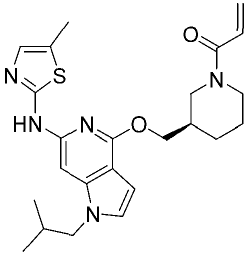 | (R)-1-(3-(((1-isobutyl-6-((5-methylthiazol-2-yl)amino)-1H-pyrrolo[3,2-c]pyridin-4-yl)oxy)methyl)piperidin-1-yl)prop-2-en-1-one | ¹H NMR (500 MHz, DMSO) δ 10.49 (s, 1H), 7.13 (s, 1H), 6.94 (s, 1H), 6.86 - 6.68 (m, 1H), 6.61 (s, 1H), 6.40 (d, *J* = 17.7 Hz, 1H), 6.03 (t, *J =* 14.8 Hz, 1H), 5.68 - 5.47 (m, *J* = 36.9, 10.4 Hz, 1H), 4.56 - 4.36 (m, 2H), 4.03 - 3.77 (m, 4H), 3.17 - 3.05 (m, 1H), 2.34 (d, *J* = 23.5 Hz, 3H), 2.14 - 2.03 (m, 2H), 1.90 (s, 1H), 1.72 (s, 1H), 1.60 - 1.36 (m, *J* = 46.6, 19.8 Hz, 3H), 0.85 (d, *J* = 6.4 Hz, 6H). 454[M+H]⁺ |
| 215 | 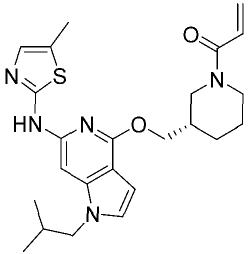 | (S)-1-(3-(((1-isobutyl-6-((5-methylthiazol-2-yl)amino)-1H-pyrrolo[3,2-c]pyridin-4-yl)oxy)methyl)piperidin-1-yl)prop-2-en-1-one | ¹H NMR (500 MHz, DMSO) δ 10.49 (s, 1H), 7.13 (s, 1H), 6.94 (s, 1H), 6.83 - 6.68 (m, 1H), 6.61 (s, 1H), 6.40 (d, *J* = 17.3 Hz, 1H), 6.03 (t, *J* = 14.8 Hz, 1H), 5.68 - 5.47 (m, 1H), 4.56 - 4.40 (m, 2H), 3.98 - 3.80 (m, 4H), 3.16 - 3.08 (m, 1H), 2.31 (s, 3H), 2.13 - 2.02 (m, 2H), 1.90 (s, 1H), 1.77 - 1.35 (m, 4H), 0.85 (d, *J* = 6.4 Hz, 6H).454[M+H]⁺ |
| 216 | 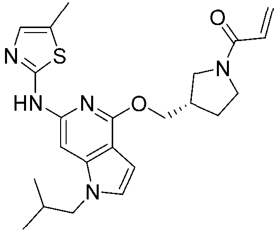 | (S)-1-(3-(((1-isobutyl-6-((5-methylthiazol-2-yl)amino)-1H-pyrrolo[3,2-c]pyridin-4-yl)oxy)methyl)pyrrolidin-1-yl)prop-2-en-1-one | ¹H NMR (500 MHz, DMSO) δ 10.50 (s, 1H), 7.13 (s, 1H), 6.94 (s, 1H), 6.66 - 6.53 (m, 2H), 6.39 (d, *J* = 8.0 Hz, 1H), 6.12 (d, *J* = 16.8 Hz, 1H), 5.69 - 5.58 (m, 1H), 4.64 - 4.50 (m, 2H), 3.82 (d, *J* = 7.1 Hz, 2H), 3.72 (s, 1H), 3.67 - 3.52 (m, 3H), 2.30 (s, 3H), 2.18 - 1.76 (m, 4H), 0.84 (d, *J* = 6.2 Hz, 6H).440[M+H]⁺ |
| 217 | 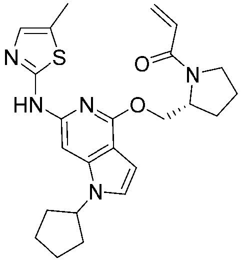 | 1-(2-(((1-cyclopentyl-6-((5-methylthiazol-2-yl)amino)-1H-pyrrolo[3,2-c]pyridin-4-yl)oxy)methyl)pyrrolidin-1-yl)prop-2-en-1-one | ¹H NMR (500 MHz, CDCl₃) δ 7.00 (d, *J* = 10.1 Hz, 1H), 6.96 - 6.91 (m, 1H), 6.55 - 6.34 (m, 4H), 5.70 - 5.56 (m, 1H), 4.90 - 4.80 (m, 1H), 4.72 - 4.45 (m, 3H), 3.81 - 3.55 (m, 2H), 2.37 (d, *J* = 7.2 Hz, 3H), 2.33 - 2.23 (m, 1H), 2.22 - 2.05 (m, 5H), 1.83 (d, *J* = 61.2 Hz, 6H).452[M+H]⁺ |
| 218 | 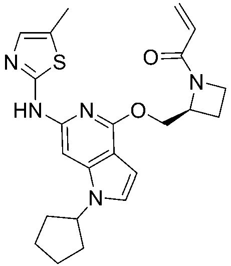 | 1-(2-(((1-cyclopentyl-6-((5-methylthiazol-2-yl)amino)-1H-pyrrolo[3,2-c]pyridin-4-yl)oxy)methyl)azetidin-1-yl)prop-2-en-1-one | ¹H NMR (500 MHz, CDCl₃) δ 7.08 - 6.89 (m, 2H), 6.62 - 6.18 (m, 4H), 5.75 - 5.52 (m, 1H), 5.09 - 4.80 (m, 3H), 4.64 - 4.51 (m, 1H), 4.36 - 4.04 (m, 2H), 2.64 - 2.42 (m, 2H), 2.38 (s, 3H), 2.18 (d, *J* = 6.2 Hz, 3H), 1.83 (d, *J* = 61.2Hz,5H). 438[M+H]⁺ |
| 219 | 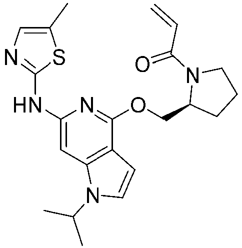 | (S)-1-(2-(((1-isopropyl-6-((5-methylthiazol-2-yl)amino)-1H-pyrrolo[3,2-c]pyridin-4-yl)oxy)methyl)pyrrolidin-1-yl)prop-2-en-1-one | ¹H NMR (500 MHz, CDCl₃) δ 9.91 (s, 1H), 7.03-6.91 (m, 2H), 6.78-6.46 (m, 3H), 5.74-5.59 (m, 4H), 4.82 (ddd, *J* = 15.5, 10.6, 3.8 Hz, 1H), 4.73-4.45 (m, 3H), 3.78-3.59 (m, 2H), 2.41-1.94 (m, 7H), 1.49 (s, 6H). 426[M+H]⁺ |
| 220 | 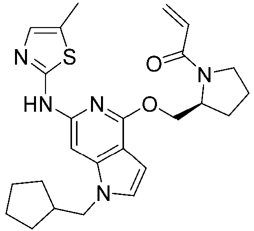 | (S)-1-(2-(((1-(cyclopentylmethyl)-6-((5-methylthiazol-2-yl)amino)-1H-pyrrolo[3,2-c]pyridin-4-yl)oxy)methyl)pyrrolidin-1-yl)prop-2-en-1-one | ¹H NMR (500 MHz, CDCl₃) δ 7.02 (d, *J* = 9.5 Hz, 3H), 6.85 (d, *J* = 3.1 Hz, 3H), 6.62 (ddd, *J* = 102.4, 16.7, 10.3 Hz, 4H), 6.47 - 6.31 (m, 9H), 5.63 (dd, *J* = 37.1, 10.4 Hz, 3H), 4.91 - 4.79 (m, 3H), 4.73 - 4.45 (m, 7H), 3.92 - 3.83 (m, 6H), 3.79 - 3.57 (m, 7H), 2.38 (d, *J* = 8.4 Hz, 11H), 2.30 (dd, *J* = 19.5, 8.8 Hz, 3H), 2.13 (ddd, *J* = 30.5, 20.7, 7.8 Hz, 9H), 2.01 - 1.89 (m, 4H), 1.68 (s, 14H), 1.57 (s, 7H), 1.25 (s, 8H).; 466[M+H]⁺ |
| 221 | 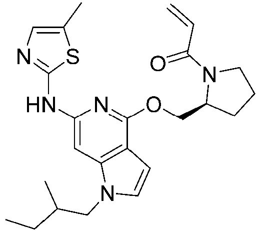 | 1-((2S)-2-(((1-(2-methylbutyl)-6-((5-methylthiazol-2-yl)amino)-1H-pyrrolo[3,2-c]pyridin-4-yl)oxy)methyl)pyrrolidin-1-yl)prop-2-en-1-one | ¹H NMR (500 MHz, CDCl₃) δ 7.01 (d, *J* = 10.6 Hz, 1H), 6.82 (s, 1H), 6.61 (ddd, *J =* 102.3, 16.6, 10.3 Hz, 1H), 6.46 - 6.31 (m, 3H), 5.64 (dd, *J* = 37.6, 10.2 Hz, 1H), 4.90 - 4.78 (m, 1H), 4.73 - 4.45 (m, 2H), 3.96 - 3.85 (m, 1H), 3.80 - 3.56 (m, 3H), 2.38 (d, *J* = 8.1 Hz, 3H), 2.30 (dd, *J* = 18.4, 9.1 Hz, 1H), 2.17 - 2.07 (m, 2H), 2.02 - 1.86 (m, 2H), 1.48 - 1.34 (m, 1H), 1.19 (d, *J* = 6.7 Hz, 1H), 0.97 - 0.80 (m, 6H).; 454[M+H]⁺ |
| 222 | 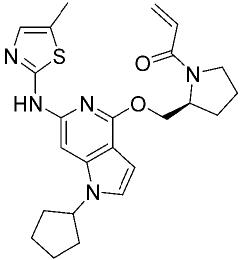 | (S)-1-(2-(((1-cyclopentyl-6-((5-methylthiazol-2-yl)amino)-1H-pyrrolo[3,2-c]pyridin-4-yl)oxy)methyl)pyrrolidin-1-yl)prop-2-en-1-one | ¹H NMR (500 MHz, CDCl₃) δ 7.02 (d, *J* = 8.8 Hz, 1H), 6.93 (dd, *J* = 6.6, 3.2 Hz, 1H), 6.75 - 6.35 (m, 4H), 5.70 - 5.57 (m, 1H), 4.89 - 4.81 (m, 1H), 4.71 - 4.47 (m, 3H), 3.77 - 3.58 (m, 2H), 2.38 (d, *J* = 7.5 Hz, 3H), 2.31 - 2.09 (m, 6H), 1.91 - 1.73 (m, 6H).452[M+H]⁺ |
| 223 | 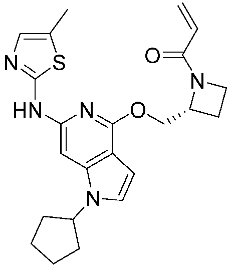 | (R)-1-(2-(((1-cyclopentyl-6-((5-methylthiazol-2-yl)amino)-1H-pyrrolo[3,2-c]pyridin-4-yl)oxy)methyl)azetidin-1-yl)prop-2-en-1-one | ¹H NMR (500 MHz, CDCl₃) δ 7.03 (d, *J* = 12.5 Hz, 1H), 6.92 (d, *J* = 8.7 Hz, 1H), 6.64 - 6.15 (m, 4H), 5.69 - 5.53 (m, 1H), 5.05 - 4.82 (m, 3H), 4.62 - 4.06 (m, 3H), 2.37 (s, 3H), 2.19 (d, *J* = 45.4 Hz, 3H), 1.81 (d, *J* = 63.4 Hz, 7H).438[M+H]⁺ |
| 214 | 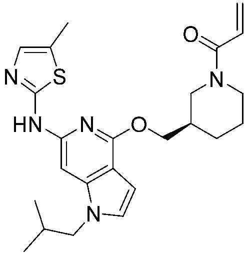 | (R)-1-(3-(((1-isobutyl-6-((5-methylthiazol-2-yl)amino)-1H-pyrrolo[3,2-c]pyridin-4-yl)oxy)methyl)piperidin-1-yl)prop-2-en-1-one | ¹H NMR (500 MHz, DMSO) δ 10.49 (s, 1H), 7.13 (s, 1H), 6.94 (s, 1H), 6.86 - 6.68 (m, 1H), 6.61 (s, 1H), 6.40 (d, *J* = 17.7 Hz, 1H), 6.03 (t, *J* = 14.8 Hz, 1H), 5.68 - 5.47 (m, *J* = 36.9, 10.4 Hz, 1H), 4.56 - 4.36 (m, 2H), 4.03 - 3.77 (m, 4H), 3.17 - 3.05 (m, 1H), 2.34 (d, *J* = 23.5 Hz, 3H), 2.14 - 2.03 (m, 2H), 1.90 (s, 1H), 1.72 (s, 1H), 1.60 - 1.36 (m, *J* = 46.6, 19.8 Hz, 3H), 0.85 (d, *J* = 6.4 Hz, 6H). 454[M+H]⁺ |
| 215 | 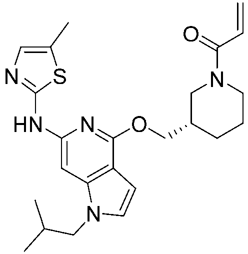 | (S)-1-(3-(((1-isobutyl-6-((5-methylthiazol-2-yl)amino)-1H-pyrrolo[3,2-c]pyridin-4-yl)oxy)methyl)piperidin-1-yl)prop-2-en-1-one | ¹H NMR (500 MHz, DMSO) δ 10.49 (s, 1H), 7.13 (s, 1H), 6.94 (s, 1H), 6.83 - 6.68 (m, 1H), 6.61 (s, 1H), 6.40 (d, *J* = 17.3 Hz, 1H), 6.03 (t, *J* = 14.8 Hz, 1H), 5.68 - 5.47 (m, 1H), 4.56 - 4.40 (m, 2H), 3.98 - 3.80 (m, 4H), 3.16 - 3.08 (m, 1H), 2.31 (s, 3H), 2.13 - 2.02 (m, 2H), 1.90 (s, 1H), 1.77 - 1.35 (m, 4H), 0.85 (d, *J* = 6.4 Hz, 6H).454[M+H]⁺ |
| 216 | 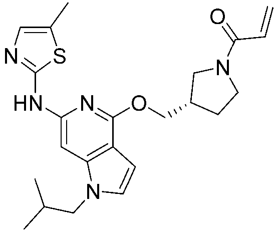 | (S)-1-(3-(((1-isobutyl-6-((5-methylthiazol-2-yl)amino)-1H-pyrrolo[3,2-c]pyridin-4-yl)oxy)methyl)pyrrolidin-1-yl)prop-2-en-1-one | ¹H NMR (500 MHz, DMSO) δ 10.50 (s, 1H), 7.13 (s, 1H), 6.94 (s, 1H), 6.66 - 6.53 (m, 2H), 6.39 (d, *J* = 8.0 Hz, 1H), 6.12 (d, *J* = 16.8 Hz, 1H), 5.69 - 5.58 (m, 1H), 4.64 - 4.50 (m, 2H), 3.82 (d, *J* = 7.1 Hz, 2H), 3.72 (s, 1H), 3.67 - 3.52 (m, 3H), 2.30 (s, 3H), 2.18 - 1.76 (m, 4H), 0.84 (d, *J* = 6.2 Hz, 6H).440[M+H]⁺ |
| 217 | 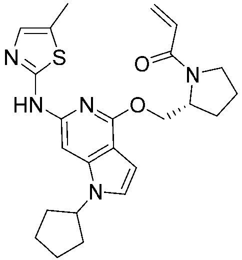 | 1-(2-(((1-cyclopentyl-6-((5-methylthiazol-2-yl)amino)-1H-pyrrolo[3,2-c]pyridin-4-yl)oxy)methyl)pyrrolidin-1-yl)prop-2-en-1-one | ¹H NMR (500 MHz, CDCl₃) δ 7.00 (d, *J* = 10.1 Hz, 1H), 6.96 - 6.91 (m, 1H), 6.55 - 6.34 (m, 4H), 5.70 - 5.56 (m, 1H), 4.90 - 4.80 (m, 1H), 4.72 - 4.45 (m, 3H), 3.81 - 3.55 (m, 2H), 2.37 (d, *J* = 7.2 Hz, 3H), 2.33 - 2.23 (m, 1H), 2.22 - 2.05 (m, 5H), 1.83 (d, *J* = 61.2 Hz, 6H).452[M+H]⁺ |
| 218 | 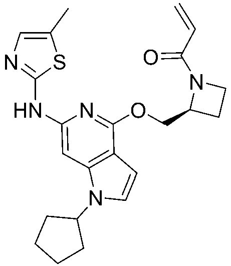 | 1-(2-(((1-cyclopentyl-6-((5-methylthiazol-2-yl)amino)-1H-pyrrolo[3,2-c]pyridin-4-yl)oxy)methyl)azetidin-1-yl)prop-2-en-1-one | ¹H NMR (500 MHz, CDCl₃) δ 7.08 - 6.89 (m, 2H), 6.62 - 6.18 (m, 4H), 5.75 - 5.52 (m, 1H), 5.09 - 4.80 (m, 3H), 4.64 - 4.51 (m, 1H), 4.36 - 4.04 (m, 2H), 2.64 - 2.42 (m, 2H), 2.38 (s, 3H), 2.18 (d, *J* = 6.2 Hz, 3H), 1.83 (d, *J* = 61.2Hz,5H). 438[M+H]⁺ |
| 219 | 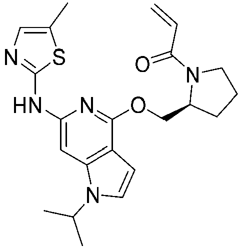 | (S)-1-(2-(((1-isopropyl-6-((5-methylthiazol-2-yl)amino)-1H-pyrrolo[3,2-c]pyridin-4-yl)oxy)methyl)pyrrolidin-1-yl)prop-2-en-1-one | ¹H NMR (500 MHz, CDCl₃) δ 9.91 (s, 1H), 7.03-6.91 (m, 2H), 6.78-6.46 (m, 3H), 5.74-5.59 (m, 4H), 4.82 (ddd, *J* = 15.5, 10.6, 3.8 Hz, 1H), 4.73-4.45 (m, 3H), 3.78-3.59 (m, 2H), 2.41-1.94 (m, 7H), 1.49 (s, 6H). 426[M+H]⁺ |
| 220 | 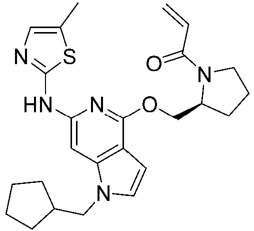 | (S)-1-(2-(((1-(cyclopentylmethyl)-6-((5-methylthiazol-2-yl)amino)-1H-pyrrolo[3,2-c]pyridin-4-yl)oxy)methyl)pyrrolidin-1-yl)prop-2-en-1-one | ¹H NMR (500 MHz, CDCl₃) δ 7.02 (d, *J* = 9.5 Hz, 3H), 6.85 (d, *J* = 3.1 Hz, 3H), 6.62 (ddd, *J* = 102.4, 16.7, 10.3 Hz, 4H), 6.47 - 6.31 (m, 9H), 5.63 (dd, *J* = 37.1, 10.4 Hz, 3H), 4.91 - 4.79 (m, 3H), 4.73 - 4.45 (m, 7H), 3.92 - 3.83 (m, 6H), 3.79 - 3.57 (m, 7H), 2.38 (d, *J* = 8.4 Hz, 11H), 2.30 (dd, *J* = 19.5, 8.8 Hz, 3H), 2.13 (ddd, *J* = 30.5, 20.7, 7.8 Hz, 9H), 2.01 - 1.89 (m, 4H), 1.68 (s, 14H), 1.57 (s, 7H), 1.25 (s, 8H).; 466[M+H]⁺ |
| 221 | 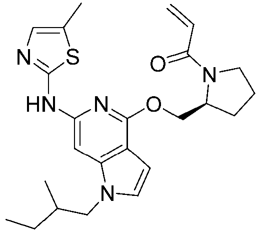 | 1-((28)-2-(((1-(2-methylbutyl)-6-((5-methylthiazol-2-yl)amino)-1H-pyrrolo[3,2-c]pyridin-4-yl)oxy)methyl)pyrrolidin-1-yl)prop-2-en-1-one | ¹H NMR (500 MHz, CDCl₃) δ 7.01 (d, *J* = 10.6 Hz, 1H), 6.82 (s, 1H), 6.61 (ddd, *J =* 102.3, 16.6, 10.3 Hz, 1H), 6.46 - 6.31 (m, 3H), 5.64 (dd, *J* = 37.6, 10.2 Hz, 1H), 4.90 - 4.78 (m, 1H), 4.73 - 4.45 (m, 2H), 3.96 - 3.85 (m, 1H), 3.80 - 3.56 (m, 3H), 2.38 (d, *J* = 8.1 Hz, 3H), 2.30 (dd, *J* = 18.4, 9.1 Hz, 1H), 2.17 - 2.07 (m, 2H), 2.02 - 1.86 (m, 2H), 1.48 - 1.34 (m, 1H), 1.19 (d, *J* = 6.7 Hz, 1H), 0.97 - 0.80 (m, 6H).; 454[M+H]⁺ |
| 222 | 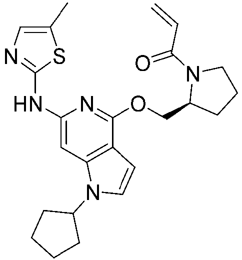 | (S)-1-(2-(((1-cyclopentyl-6-((5-methylthiazol-2-yl)amino)-1H-pyrrolo[3,2-c]pyridin-4-yl)oxy)methyl)pyrrolidin-1-yl)prop-2-en-1-one | ¹H NMR (500 MHz, CDCl₃) δ 7.02 (d, *J* = 8.8 Hz, 1H), 6.93 (dd, *J* = 6.6, 3.2 Hz, 1H), 6.75 - 6.35 (m, 4H), 5.70 - 5.57 (m, 1H), 4.89 - 4.81 (m, 1H), 4.71 - 4.47 (m, 3H), 3.77 - 3.58 (m, 2H), 2.38 (d, *J* = 7.5 Hz, 3H), 2.31 - 2.09 (m, 6H), 1.91 - 1.73 (m, 6H).452[M+H]⁺ |
| 223 | 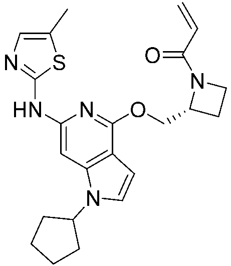 | (R)-1-(2-(((1-cyclopentyl-6-((5-methylthiazol-2-yl)amino)-1H-pyrrolo[3,2-c]pyridin-4-yl)oxy)methyl)azetidin-1-yl)prop-2-en-1-one | ¹H NMR (500 MHz, CDCl₃) δ 7.03 (d, *J* = 12.5 Hz, 1H), 6.92 (d, *J* = 8.7 Hz, 1H), 6.64 - 6.15 (m, 4H), 5.69 - 5.53 (m, 1H), 5.05 - 4.82 (m, 3H), 4.62 - 4.06 (m, 3H), 2.37 (s, 3H), 2.19 (d, *J* = 45.4 Hz, 3H), 1.81 (d, *J =* 63.4 Hz, 7H).438[M+H]⁺ |

### Example 224. (S)-2-fluoro-1-(3-((1-isobutyl-6-((5-methylthiazol-2-yl)amino)-1H-pyrrolo[3,2-c]pyridin-4-yl)oxy)pyrrolidin-1-yl)prop-2-en-1-one

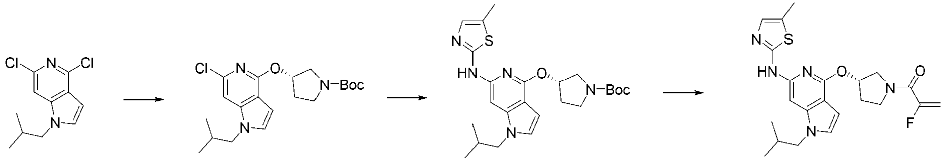

### Step 1. Preparation of tert-butyl (S)-3-((6-chloro-1-isopropyl-1H-pyrrolo[3,2-c]pyridin-4-yl)oxy)pyrrolidine-1-carboxylate

*tert*-Butyl (*S*)-3-hydroxypyrrolidine-1-carboxylate (1.76 g, 9.4 mmol) was dissolved in THF(10.0 mL), and then 60% NaH (469 mg, 11.2 mmol) was slowly added thereto at 0°C. The reaction mixture was stirred at 0°C for 30 minutes, and then 4,6-dichloro-1-isopropyl-1*H*-pyrrolo[3,2-c]pyridine (1.90 g, 7.8 mmol) was added thereto, and allowed to react at 60°C for 6 hours. The reaction mixture was cooled to room temperature, diluted with ethyl acetate, and washed with an aqueous ammonium chloride solution. The organic layer was dried over MgSO₄, filtered, and concentrated. The resulting residue was purified by a silica gel chromatography (0~30% ethyl acetate/hexane) to synthesize tert-butyl (*S*)-3-((6-chloro-1-isopropyl-1*H*-pyrrolo[3,2-*c*]pyridin-4-yl)oxy)pyrrolidine-1-carboxylate as a white solid (2.00 g, yield: 65%).
¹H NMR (500 MHz, DMSO-*d₆*) δ 7.40-7.27 (m, 2H), 6.48 (s, 1H), 5.57 (d, *J =* 23.4 Hz, 1H), 3.96 (d, *J* = 7.3 Hz, 2H), 3.70-3.56 (m, 2H), 3.45 (d, *J* = 9.1 Hz, 2H), 2.26-2.00 (m, 3H), 1.39 (d, *J* = 12.5 Hz, 9H), 0.81 (d, *J* = 6.6 Hz, 6H).; LC-MS (ESI) m/z : 394 [M+H]⁺

### Step 2. Preparation of tert-butyl (S)-3-((1-isopropyl-6-((5-methylthiazol-2-yl)amino)-1H-pyrrolo[3,2-c]pyridin-4-yl)oxy)pyrrolidine-1-carboxylate

*tert*-Butyl (*S*)-3-((1-isopropyl-6-((5-methylthiazol-2-yl)amino)-1*H*-pyrrolo[3,2-*c*]pyridin-4-yl)oxy)pyrrolidine-1-carboxylate (yield: 42%) was synthesized in the same manner as in step 2 of Example 1, except that *tert*-butyl (*S*)-3-((6-chloro-1-isobutyl-1*H-*pyrrolo[3,2-*c*]pyridin-4-yl)oxy)pyrrolidine-1-carboxylate was used instead of *tert*-butyl 4-(6-chloro-1-methyl-1*H*-pyrrolo[3,2-*c*]pyridin-4-yl)-3,6-dihydropyridine-1(2*H*)-carboxylate used in the synthesis step 2 of Example 1.
LC-MS (ESI) m/z : 472 [M+H]⁺

### Step 3. Preparation of (S)-2-fluoro-1-(3-((1-isobutyl-6-((5-methylthiazol-2-yl)amino)-1H-pyrrolo[3,2-c]pyridin-4-yl)oxy)pyrrolidin-1-yl)prop-2-en-1-one

tert-Butyl (*S*)-3-((1-isopropyl-6-((5-methylthiazol-2-yl)amino)-1*H*-pyrrolo[3,2-*c*]pyridin-4-yl)oxy)pyrrolidine-1-carboxylate (118 mg, 0.25 mmol) was dissolved in CH₂Cl₂(2.0 mL), and then TFA (1.5 mL) was added thereto, and stirred at room temperature for 1 hour. After completion of the reaction, the mixture was concentrated under reduced pressure to synthesize a salt compound. The resulting salt compound was dissolved in THF (2.0 mL), and then DIPEA (0.28 mL, 1.6 mmol), 2-fluoroacrylic acid (34 mg, 0.38 mmol), and HATU (115 mg, 0.30 mmol) were added thereto, and stirred at room temperature for 15 hours. The reaction product was diluted with ethyl acetate and washed with distilled water and salt water. The organic layer was dried over MgSO₄, filtered, and concentrated. The resulting residue was purified by a silica gel chromatography (70% ethyl acetate/hexane) to synthesize (*S*)-2-fluoro-1-(3-((1-isobutyl-6-((5-methylthiazol-2-yl)amino)-1*H*-pyrrolo[3,2-*c*]pyridin-4-yl)oxy)pyrrolidin-1-yl)prop-2-en-1-one as a while solid (45 mg, yield: 41%).
¹H NMR (500 MHz, CDCl₃) δ 7.05 (d, *J* = 4.5 Hz, 1H), 6.83 (s, 1H), 6.48-6.43 (m, 1H), 6.41 (d, *J* = 5.1 Hz, 1H), 6.02 (d, *J* = 18.7 Hz, 1H), 5.56 (dd, *J* = 46.8, 2.9 Hz, 1H), 5.13 (ddd, *J =* 22.1, 16.1, 2.8 Hz, 1H), 4.18-3.67 (m, 6H), 2.53-2.22 (m, 5H), 2.15 (m, 1H), 0.93 (dd, *J* = 6.3, 1.8 Hz, 6H).; LC-MS (ESI) *m*/*z :* 444 [M+H]⁺

Examples 225 to 231 were prepared in a similar manner to that of Example 224, and the structure and analysis results of the compounds are summarized in Table 5 below.

**[Table 5]**

| Example | Structure | Compound name | ¹H NMR; LC-MS (ESI) *m*/*z* |
|---|---|---|---|
| 225 | 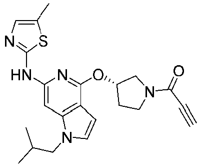 | ((*S*)-1-(3-((1-isobutyl-6-((5-methylthiazol-2-yl)amino)-1*H*-pyrrolo[3,2*-c*]pyridin-4-yl)oxy)pyrrolidin-1-yl)prop-2-yn-1-one | 1H NMR (500 MHz, CDCl₃) δ 7.04 (d, *J* = 9.2 Hz, 1H), 6.85 (dd, *J* = 5.8, 3.2 Hz, 1H), 6.47 (t, *J* = 3.3 Hz, 1H), 6.42 (d, *J* = 8.8 Hz, 1H), 6.02 (m, 1H), 4.15-3.69 (m, 6H), 3.08 (s, 0.5H), 3.01 (s, 0.5 H), 2.50-2.30 (m, 5H), 2.15 (m, 1H), 0.93 (t, *J* = 5.7 Hz, 6H).; 424 [M+H]⁺ |
| 226 | 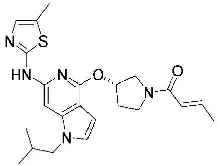 | (*S,E*)-1-(3-((1-isobutyl-6-((5-methylthiazol-2-yl)amino)-1*H*-pyrrolo[3,2-*c*]pyridin-4-yl)oxy)pyrrolidin-1-yl)but-2-en-1-one | ¹H NMR (500 MHz, CDCl₃) δ 7.05-6.81 (m, 3H), 6.53-6.40 (m, 2H), 6.15 (dd, *J* = 55.4, 15.0 Hz, 1H), 5.99 (m, 1H), 4.09-3.69 (m, 6H), 2.58-2.08 (m, 6H), 1.88 (dd, *J* = 25.1, 6.6 Hz, 3H), 0.97-0.87 (m, 6H).; 440 [M+H]⁺ |
| 227 | 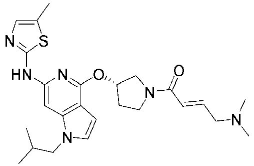 | (*S,E*)-4-(dimethylamino)-1-(3-((1-isobutyl-6-((5-methylthiazol-2-yl)amino)-1*H*-pyrrolo[3,2-*c*]pyridin-4-yl)oxy)pyrrolidin-1-yl)but-2-en-1-one | ¹H NMR (500 MHz, CDCl₃) δ 10.60 (br s, 1H), 7.02 (d, *J* = 12.7 Hz, 1H), 6.94 (m, 1H), 6.82 (m, 1H), 6.50-6.41 (m, 2H), 6.31 (dd, *J* = 60.6, 15.2 Hz, 1H), 6.03 (m, 1H), 4.07-3.50 (m, 6H), 3.09 (dd, *J* = 26.3, 5.9 Hz, 2H), 2.55-2.10 (m, 12H), 0.97-0.89 (m, 6H).; 483 [M+H]⁺ |
| 228 | 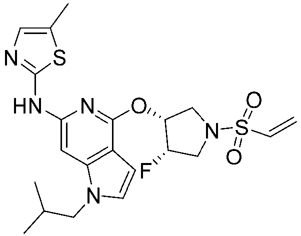 | N-(4-(((3R,4S)-4-fluoro-1-(vinylsulfonyl)pyrrolidin-3-yl)oxy)-1-isobutyl-1H-pyrrolo[3,2-c]pyridin-6-yl)-5-methylthiazol-2-amine | 1H NMR (500 MHz, DMSO) δ 10.57 (s, 1H), 7.18 (d, J = 3.1 Hz, 1H), 7.02 - 6.94 (m, J = 17.0, 9.5 Hz, 2H), 6.76 (s, 1H), 6.40 (d, J = 3.0 Hz, 1H), 6.23 - 6.14 (m, 2H), 5.81 - 5.68 (m, 1H), 5.60 (d, J = 54.3 Hz, 1H), 4.07 - 4.00 (m, 1H), 3.84 (d, J = 7.2 Hz, 2H), 3.74 - 3.59 |
| | | | (m, 2H), 3.38 (d, J = 9.4 Hz, 1H), 2.32 (s, 2H), 2.12 - 2.04 (m, 1H), 0.84 (d, J = 6.5 Hz, 6H).480[M+H]+ |
| 229 | 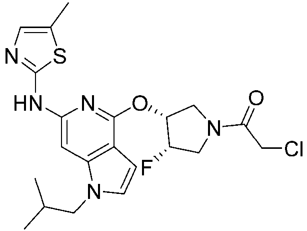 | 2-chloro-1-((3S,4R)-3-fluoro-4-((1-isobutyl-6-((5-methylthiazol-2-yl)amino)-1H-pyrrolo[3,2-c]pyridin-4-yl)oxy)pyrrolidin-1-yl)ethan-1-one | 1H NMR (500 MHz, DMSO) δ 10.82 (s, 1H), 7.21 (s, 1H), 7.01 (s, 1H), 6.79 (d, J = 4.4 Hz, 1H), 6.42 (s, 1H), 5.92 - 5.55 (m, 2H), 4.45 - 4.23 (m, 3H), 4.08 - 3.95 (m, 1H), 3.88 - 3.67 (m, 4H), 2.30 (s, 3H), 2.12 - 2.04 (m, J = 13.2, 6.6 Hz, 1H), 0.85 (d, J = 6.4 Hz, 6H).466[M+H]+ |
| 230 | 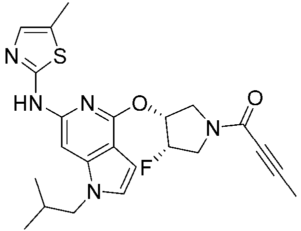 | 1-((3S,4R)-3-fluoro-4-((1-isobutyl-6-((5-methylthiazol-2-yl)amino)-1H-pyrrolo[3,2-c]pyridin-4-yl)oxy)pyrrolidin-1-yl)but-2-yn-1-one | 1H NMR (500 MHz, DMSO) δ 10.56 (d, J = 12.1 Hz, 1H), 7.18 (s, 1H), 6.97 (s, 1H), 6.76 (s, 1H), 6.40 (s, 1H), 5.84 - 5.55 (m, 2H), 4.57 - 4.42 (m, 1H), 4.25 - 3.98 (m, 2H), 3.87 - 3.61 (m, 6H), 2.29 (s, 3H), 2.11 - 2.06 (m, 1H), 0.85 (d, J = 6.5 Hz, 6H).456[M+H]+ |
| 231 | 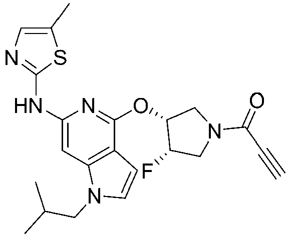 | 1-((3S,4R)-3-fluoro-4-((1-isobutyl-6-((5-methylthiazol-2-yl)amino)-1H-pyrrolo[3,2-c]pyridin-4-yl)oxy)pyrrolidin-1-yl)prop-2-yn-1-one | 1H NMR (500 MHz, DMSO) δ 10.91 (s, 1H), 7.22 (d, J = 2.9 Hz, 1H), 7.03 (d, J = 3.4 Hz, 1H), 6.79 (d, J = 7.4 Hz, 1H), 6.43 (s, 1H), 5.88 - 5.54 (m, 3H), 4.66 - 4.49 (m, 2H), 4.37 (s, 1H), 3.86 (d, J = 7.3 Hz, 3H), 2.30 (s, 3H), 2.14 - 2.04 (m, J = 13.4, 6.7 Hz, 1H), 0.85 (d, J = 6.6 Hz, 6H).442[M+H]+ |

### Experimental Example: Inhibitory activity against BTK and ITK

Inhibitory activities against BTK and ITK were measured for the compounds prepared in the above Examples as follows.

The inhibitory activities against BTK and ITK were evaluated using 'ADP-Glo^{™} Kinase system' (Promega Corporation). In the case of this evaluation method, activation was measured by quantifying the amount of ADP produced during the kinase reaction, which was utilized to measure the inhibitory activities against BTK and ITK.

Specifically, the inhibitory activities against BTK and ITK were measured using BTK kinase (Signalchem, B10-10H), ITK kinase (Signalchem, I13-11G-10), and ADP-Glo kinase assay kit (Promega, V9102). Recombinant purified human BTK and ITK were diluted with 1 x kinase reaction buffer (40 mM Tris-Cl, pH 7.5, 20 mM MgCl₂, 0.1 mg/mL BSA, 2 mM MnCl₂ and 50 uM DTT) and added to a 96 well plate (ITK: final concentration of 4 ng per reaction/BTK: final concentration of 10 ng per reaction). The compounds prepared in the previous Examples were treated so as to be finally a 1% DMSO aqueous solution, and a substrate cocktail containing ATP (1mM or Km ATP (BTK: 10 uM /ITK: 25uM)) and 0.2 ug/ul of Poly (Glu4,Tyr1) peptide (BTK and ITK final concentration) in the total 25 uL reactants was added to a 96-well plate to initiate enzymatic reaction. After incubation (30°C) for 1 hour, equivalent volume (25 uL per reaction) of ADP Glo was added, and incubated (30°C) for 40 minutes at room temperature. Then, a kinase detection reagent (45 uL per reaction) was added, and incubated (30°C) for 30 minutes at room temperature. The kinase activity was measured by chemiluminescence according to the instructions of ADP Glo kinase assay kit, and the inhibitory activity of the compounds according to the present disclosure was calculated. For the analysis of the results of each compound, Microsoft Excel was used, and IC₅₀ values were calculated by GraphPad Prism software.

According to the range of derived IC₅₀ values, the inhibitory activity of each compound was classified into A, B, C, and D as follows, and these are shown in Table 6 below.
A: When the derived IC₅₀ value is 20 nM or less
B: When the derived IC₅₀ value is more than 20 nM and 100 nM or less
C: When the derived IC₅₀ value is more than 100 nM and 500 nM or less
D: When the derived IC₅₀ value is more than 500 nM

**[Table 6]**

| Exam ple | ITK IC₅₀(nM) | | BTK IC₅₀(nM) | | Exam ple | ITK IC₅₀(nM) | | BTK IC₅₀(nM) | |
|---|---|---|---|---|---|---|---|---|---|
| | Km ATP | 1mM ATP | Km ATP | 1mM ATP | | Km ATP | 1mM ATP | Km ATP | 1mM ATP |
| 1 | A | - | A | - | 117 | A | - | A | - |
| 2 | A | B | A | B | 118 | B | - | B | - |
| 3 | C | - | C | - | 119 | A | - | A | - |
| 4 | A | B | A | B | 120 | A | - | A | - |
| 5 | A | - | A | - | 121 | A | - | A | - |
| 6 | A | C | A | B | 122 | A | - | A | - |
| 7 | A | - | A | - | 123 | A | - | A | - |
| 8 | A | - | A | - | 124 | A | - | A | - |
| 9 | A | - | A | - | 125 | A | - | A | - |
| 10 | B | - | A | - | 126 | - | A | - | A |
| 11 | A | - | A | - | 127 | - | A | - | A |
| 12 | A | A | A | A | 128 | - | A | - | A |
| 13 | A | - | A | - | 129 | - | A | - | A |
| 14 | A | - | A | - | 130 | - | A | - | A |
| 15 | A | - | A | - | 131 | - | A | - | A |
| 16 | A | - | A | - | 132 | - | A | - | A |
| 17 | A | - | A | - | 133 | - | A | - | A |
| 18 | A | - | B | - | 134 | - | D | - | D |
| 19 | A | C | A | B | 135 | - | D | - | D |
| 20 | A | - | A | - | 136 | - | A | - | A |
| 21 | A | - | A | - | 137 | - | A | - | A |
| 22 | A | - | A | - | 138 | - | D | - | D |
| 23 | A | - | B | - | 139 | - | D | - | D |
| 24 | - | B | - | A | 140 | - | A | - | A |
| 25 | - | D | - | D | 141 | - | A | - | A |
| 26 | A | - | A | - | 142 | - | A | - | A |
| 27 | A | - | A | - | 143 | - | D | - | D |
| 28 | A | A | A | A | 144 | - | D | - | D |
| 29 | A | - | A | - | 145 | - | A | - | A |
| 30 | - | A | - | A | 146 | - | A | - | A |
| 31 | A | - | A | - | 147 | - | A | - | A |
| 32 | A | A | A | A | 148 | - | A | - | A |
| 33 | - | A | - | A | 149 | - | A | - | A |
| 34 | A | B | A | A | 150 | - | B | - | A |
| 35 | - | A | - | A | 151 | - | B | - | A |
| 36 | - | A | - | A | 152 | - | A | - | A |
| 37 | - | A | - | A | 153 | - | B | - | A |
| 38 | - | A | - | A | 154 | - | A | - | A |
| 39 | - | A | - | A | 155 | - | B | - | A |
| 40 | D | - | C | - | 156 | - | D | - | D |
| 41 | D | - | B | - | 157 | - | A | - | A |
| 42 | D | - | D | - | 158 | - | B | - | A |
| 43 | - | B | - | A | 159 | - | A | - | A |
| 44 | - | C | - | B | 160 | - | C | - | C |
| 45 | - | C | - | B | 161 | - | A | - | A |
| 46 | - | C | - | A | 162 | - | D | - | D |
| 47 | - | B | - | A | 163 | - | A | - | A |
| 48 | - | B | - | B | 164 | - | C | - | B |
| 49 | B | C | B | C | 165 | - | A | - | A |
| 50 | B | - | A | - | 166 | - | A | - | A |
| 51 | A | - | A | - | 167 | - | A | - | A |
| 52 | A | - | A | - | 168 | - | D | - | D |
| 53 | C | - | A | - | 169 | - | B | - | A |
| 54 | B | - | A | - | 170 | - | A | - | A |
| 55 | A | - | A | - | 171 | - | A | - | A |
| 56 | A | D | A | A | 172 | - | B | - | A |
| 57 | A | - | A | - | 173 | - | A | - | A |
| 58 | - | A | - | A | 174 | - | A | - | A |
| 59 | - | A | - | A | 175 | - | A | - | A |
| 60 | A | A | A | A | 176 | - | A | - | A |
| 61 | A | - | A | - | 177 | - | A | - | A |
| 62 | - | B | - | A | 178 | - | A | - | A |
| 63 | B | C | A | B | 179 | - | A | - | A |
| 64 | A | - | A | - | 180 | - | A | - | A |
| 65 | B | - | A | - | 181 | - | A | - | A |
| 66 | A | B | A | A | 182 | - | A | - | A |
| 67 | A | B | A | A | 183 | - | D | - | D |
| 68 | - | B | - | A | 184 | - | D | - | D |
| 69 | - | B | - | C | 185 | - | A | - | A |
| 70 | - | B | - | A | 186 | - | A | - | A |
| 71 | A | - | A | - | 187 | - | A | - | A |
| 72 | A | - | A | - | 188 | - | A | - | A |
| 73 | A | - | A | - | 189 | - | A | - | A |
| 74 | A | - | A | - | 190 | - | A | - | A |
| 75 | A | C | A | B | 191 | - | A | - | A |
| 76 | B | - | A | - | 192 | - | C | - | A |
| 77 | - | B | - | A | 193 | - | D | - | D |
| 78 | - | B | - | A | 194 | - | B | - | A |
| 79 | A | A | A | A | 195 | - | C | - | B |
| 80 | A | A | A | A | 196 | - | B | - | B |
| 81 | - | A | - | A | 197 | - | D | - | D |
| 82 | - | D | - | D | 198 | - | D | - | D |
| 83 | - | A | - | A | 199 | - | D | - | D |
| 84 | - | B | - | A | 200 | - | C | - | B |
| 85 | - | D | - | B | 201 | - | D | - | C |
| 86 | - | B | - | A | 202 | - | C | - | C |
| 87 | - | A | - | A | 203 | - | D | - | D |
| 88 | - | A | - | A | 204 | - | D | - | D |
| 89 | - | B | - | A | 205 | - | A | - | A |
| 90 | - | A | - | A | 206 | - | A | - | A |
| 91 | - | A | - | A | 207 | - | A | - | A |
| 92 | - | A | - | A | 208 | - | A | - | A |
| 93 | - | A | - | A | 209 | - | A | - | A |
| 94 | - | A | - | A | 210 | - | A | - | A |
| 95 | - | C | - | A | 211 | - | A | - | A |
| 96 | - | A | - | A | 212 | - | A | - | A |
| 97 | - | A | - | A | 213 | - | A | - | A |
| 98 | - | A | - | A | 214 | - | D | - | B |
| 99 | - | A | - | A | 215 | - | C | - | A |
| 100 | - | A | - | A | 216 | - | C | - | B |
| 101 | - | B | - | A | 217 | - | B | - | A |
| 102 | - | A | - | A | 218 | - | A | - | A |
| 103 | - | B | - | A | 219 | - | D | - | B |
| 104 | - | B | - | B | 220 | - | B | - | A |
| 105 | - | A | - | A | 221 | - | B | - | A |
| 106 | - | A | - | A | 222 | - | B | - | A |
| 107 | - | A | - | A | 223 | - | A | - | A |
| 108 | - | A | - | A | 224 | - | D | - | D |
| 109 | - | A | - | A | 225 | - | A | - | A |
| 110 | - | A | - | A | 226 | - | D | - | D |
| 111 | - | A | - | A | 227 | - | D | - | D |
| 112 | - | A | - | A | 228 | - | A | - | A |
| 113 | - | A | - | A | 229 | - | A | - | B |
| 114 | - | A | - | A | 230 | - | D | - | D |
| 115 | A | - | A | - | 231 | - | A | - | A |
| 116 | A | - | A | - | | | | | |

## Claims

1. A compound represented by the following Chemical Formula 1, or a pharmaceutically acceptable salt thereof: 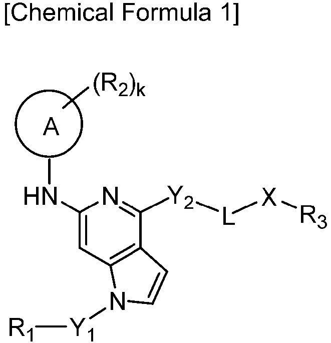 in Chemical Formula 1,
Y₁ is a single bond, a C₁₋₄ alkylene, or a C₁₋₄ haloalkylene,
R₁ is a C₁₋₁₀ alkyl, a C₁₋₁₀ alkoxy, a C₃₋₁₀ cycloalkyl, a C₆₋₂₀ aryl, a N-containing 6-membered heteroaryl, tetrahydrofuranyl, or tetrahydropyranyl,
wherein R₁ is unsubstituted, or substituted with one or more substituents selected from the group consisting of halogen, cyano, hydroxy, a C₁₋₄ alkyl and a C₁₋₄ haloalkyl,
A is benzene ring; or a 5- or 6-membered heterocycle containing 1 to 3 heteroatoms selected from the group consisting of N, O and S substituted or unsubstituted with oxo(=O), provided that the 5-membered or 6-membered heterocycle contains at least one N,
R₂ is each independently hydrogen, halogen, cyano, a C₁₋₄ alkyl, a C₁₋₄ haloalkyl, a C₁₋₄ alkoxy, a C₁₋₄ haloalkoxy, or -CONH(C₁₋₄ alkyl),
k is 1 or 2,
Y₂ is a single bond, -O-, or -O-(C₁₋₄ alkylene),
L is any one of the linking groups represented by the following Chemical Formulas 2a to 2k, 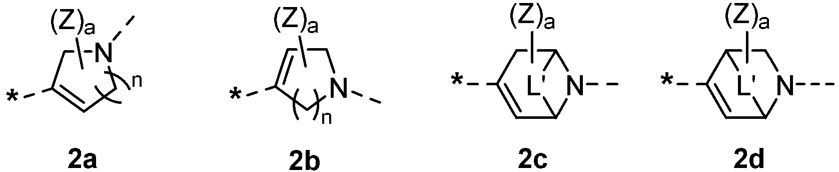 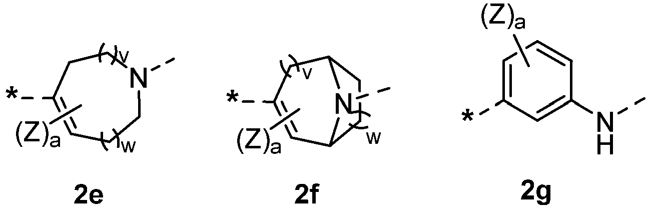 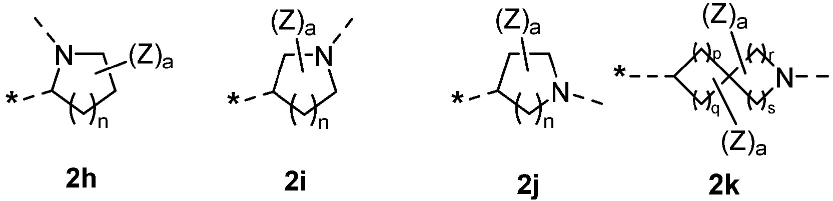
in Chemical Formulas 2a to 2k,
n is 0, 1, 2, or 3,
Z is each independently halogen, a C₁₋₄ alkyl, a C₁₋₄ haloalkyl, a C₁₋₄ alkoxy, or a C₁₋₄ haloalkoxy,
a is each independently 0, 1, or 2,
L' is methylene, ethylene, or methylene-O-methylene,
v and w are each independently 0, 1, or 2,
provided that v+w is an integer from 0 to 3,
p to s are each independently 0, 1, 2, 3, or 4,
provided that p+q and r+s are each independently an integer from 2 to 4,
* means a point linked to Y₂ in Chemical Formula 1,
X is CO or SO₂, and
R₃ is a C₁₋₄ alkyl, a C₂₋₄ alkenyl, or a C₂₋₄ alkynyl,
wherein R₃ is unsubstituted, or substituted with one or more substituents selected from the group consisting of halogen, NH₂, NH(C₁₋₄ alkyl) and N(C₁₋₄ alkyl)₂.

2. The compound or a pharmaceutically acceptable salt thereof as claimed in claim 1, wherein:
Y₁ is a single bond, methylene, or ethylene.

3. The compound or a pharmaceutically acceptable salt thereof as claimed in claim 1, wherein:
R₁ is methyl, ethyl, n-propyl, isopropyl, n-butyl, isobutyl, sec-butyl, tert-butyl, n-pentyl, isopentyl, n-hexyl, isohexyl, methoxy, ethoxy, propoxy, cyclopropyl, cyclobutyl, cyclopentyl, cyclohexyl, phenyl, pyridinyl, tetrahydrofuranyl, or tetrahydropyranyl,
wherein R₁ is unsubstituted, or substituted with 1 to 3 substituents selected from the group consisting of chloro, fluoro, cyano, hydroxy, methyl, monofluoromethyl, difluoromethyl and trifluoromethyl.

4. The compound or a pharmaceutically acceptable salt thereof as claimed in claim 1, wherein:
A is benzene, thiazole, thiadiazole, imidazole, pyrazole, pyrazine, pyridine, pyrimidine, or pyridin-2(1H)-one ring.

5. The compound or a pharmaceutically acceptable salt thereof as claimed in claim 1, wherein:
R₂ is hydrogen, chloro, fluoro, cyano, methyl, ethyl, monofluoromethyl, difluoromethyl, trifluoromethyl, methoxy, or -CONH(methyl).

6. The compound or a pharmaceutically acceptable salt thereof as claimed in claim 1, wherein:
Y₂ is a single bond, -O-, or -O-(methylene).

7. The compound or a pharmaceutically acceptable salt thereof as claimed in claim 1, wherein:
when Y₂ is a single bond, L is any one of the linking groups represented by the Chemical Formulas 2a to 2g.

8. The compound or a pharmaceutically acceptable salt thereof as claimed in claim 1, wherein:
when Y₂ is -O- or -O-(C₁₋₄ alkylene), L is any one of the linking groups represented by Chemical Formulas 2h to 2k.

9. The compound or a pharmaceutically acceptable salt thereof as claimed in claim 1, wherein:
L is any one linking group selected from the group represented by the following: 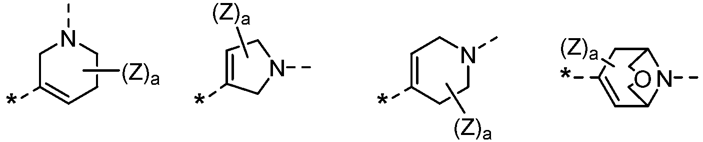 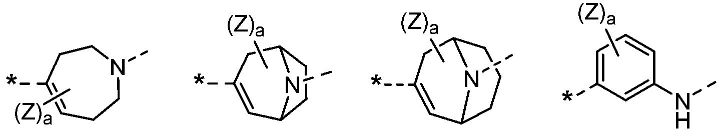 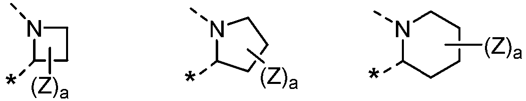 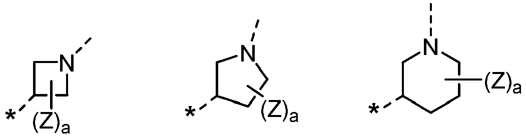 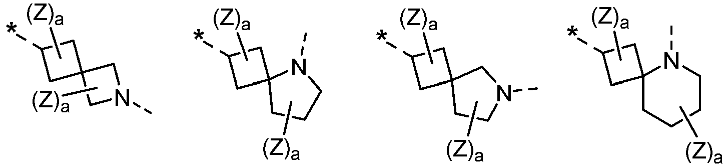
wherein,
Z and a are as defined in claim 1, and
* means a point linked to Y₂ in Chemical Formula 1.

10. The compound or a pharmaceutically acceptable salt thereof as claimed in claim 1, wherein:
Z is chloro, fluoro, methyl, or methoxy.

11. The compound or a pharmaceutically acceptable salt thereof as claimed in claim 1, wherein:
R₃ is -CH₃, -CH=CH₂, -CH=CHCH₃, -C≡CH, or -C≡CCH₃,
wherein R₃ is unsubstituted, or substituted with one or two substituents selected from the group consisting of chloro, fluoro, NH₂, NH(methyl) and N(methyl)₂.

12. The compound or a pharmaceutically acceptable salt thereof as claimed in claim 1, wherein:
the compound represented by Chemical Formula 1 is represented by any one of the following Chemical Formulas 1-1 to 1-3: 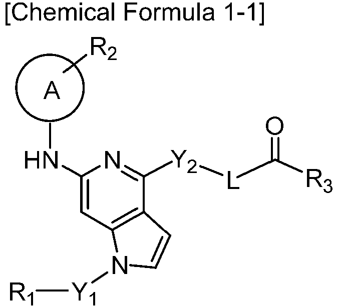 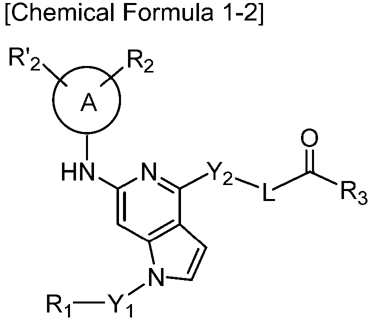 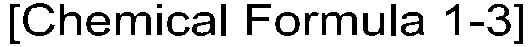 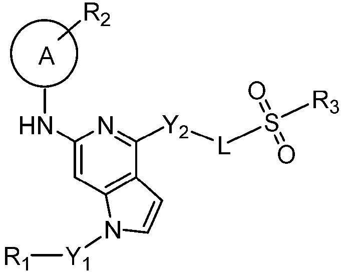
in Chemical Formulas 1-1 to 1-3,
R'₂ is halogen, cyano, a C₁₋₄ alkyl, a C₁₋₄ haloalkyl, a C₁₋₄ alkoxy, a C₁₋₄ haloalkoxy, or -CONH(C₁₋₄ alkyl),
Y₁, R₁, R₂, Y₂, L and R₃ are as defined in claim 1.

13. The compound or a pharmaceutically acceptable salt thereof as claimed in claim 1, wherein:
the compound represented by Chemical Formula 1 is any one selected from the group consisting of:
1) 1-(4-(1-methyl-6-((5-methylthiazol-2-yl)amino)-1H-pyrrolo[3,2-c]pyridin-4-yl)-3,6-dihydropyridin-1(2H)-yl)prop-2-en-1-one,
2) 1-(4-(1-isopropyl-6-((5-methylthiazol-2-yl)amino)-1H-pyrrolo[3,2-c]pyridin-4-yl)-3,6-dihydropyridin-1(2H)-yl)prop-2-en-1-one,
3) 1-(3-(1-methyl-6-((5-methylthiazol-2-yl)amino)-1H-pyrrolo[3,2-c]pyridin-4-yl)-2,5-dihydro-1H-pyrrol-1-yl)prop-2-en-1-one,
4) 1-(4-(1-(cyclopropylmethyl)-6-((5-methylthiazol-2-yl)amino)-1H-pyrrolo[3,2-c]pyridin-4-yl)-3,6-dihydropyridin-1(2H)-yl)prop-2-en-1-one,
5) 1-(4-(1-cyclopentyl-6-((5-methylthiazol-2-yl)amino)-1H-pyrrolo[3,2-c]pyridin-4-yl)-3,6-dihydropyridin-1(2H)-yl)prop-2-en-1-one,
6) 1-(4-(1-isobutyl-6-((5-methylthiazol-2-yl)amino)-1H-pyrrolo[3,2-c]pyridin-4-yl)-3,6-dihydropyridin-1(2H)-yl)prop-2-en-1-one,
7) 1-(4-(1-(cyclobutylmethyl)-6-((5-methylthiazol-2-yl)amino)-1H-pyrrolo[3,2-c]pyridin-4-yl)-3,6-dihydropyridin-1(2H)-yl)prop-2-en-1-one,
8) 1-(4-(6-((5-methylthiazol-2-yl)amino)-1-((tetrahydrofuran-3-yl)methyl)-1H-pyrrolo[3,2-c]pyridin-4-yl)-3,6-dihydropyridin-1(2H)-yl)prop-2-en-1-one,
9) 1-(4-(1-(cyclohexylmethyl)-6-((5-methylthiazol-2-yl)amino)-1H-pyrrolo[3,2-c]pyridin-4-yl)-3,6-dihydropyridin-1(2H)-yl)prop-2-en-1-one,
10) 1-(4-(1-(cyclopentylmethyl)-6-((5-methylthiazol-2-yl)amino)-1H-pyrrolo[3,2-c]pyridin-4-yl)-3,6-dihydropyridin-1(2H)-yl)prop-2-en-1-one,
11) 1-(4-(1-((4,4-difluorocyclohexyl)methyl)-6-((5-methylthiazol-2-yl)amino)-1 H-pyrrolo[3,2-c]pyridin-4-yl)-3,6-dihydropyridin-1 (2H)-yl)prop-2-en-1-one,
12) 1-(4-(1-(2,2-difluoroethyl)-6-((5-methylthiazol-2-yl)amino)-1H-pyrrolo[3,2-c]pyridin-4-yl)-3,6-dihydropyridin-1(2H)-yl)prop-2-en-1-one,
13) (S)-1-(4-(1-(sec-butyl)-6-((5-methylthiazol-2-yl)amino)-1H-pyrrolo[3,2-c]pyridin-4-yl)-3,6-dihydropyridin-1(2H)-yl)prop-2-en-1-one,
14) (R)-1-(4-(1-(sec-butyl)-6-((5-methylthiazol-2-yl)amino)-1H-pyrrolo[3,2-c]pyridin-4-yl)-3,6-dihydropyridin-1(2H)-yl)prop-2-en-1-one,
15) 1-(4-(1-benzyl-6-((5-methylthiazol-2-yl)amino)-1H-pyrrolo[3,2-c]pyridin-4-yl)-3,6-dihydropyridin-1(2H)-yl)prop-2-en-1-one,
16) 1-(4-(6-((5-methylthiazol-2-yl)amino)-1-phenethyl-1H-pyrrolo[3,2-c]pyridin-4-yl)-3,6-dihydropyridin-1(2H)-yl)prop-2-en-1-one,
17) 1-(4-(6-((5-methylthiazol-2-yl)amino)-1-((1-(trifluoromethyl)cyclopropyl)methyl)-1H-pyrrolo[3,2-c]pyridin-4-yl)-3,6-dihydropyridin-1 (2H)-yl)prop-2-en-1-one,
18) 1-(4-(1-(3,3-dimethylbutyl)-6-((5-methylthiazol-2-yl)amino)-1H-pyrrolo[3,2-c]pyridin-4-yl)-3,6-dihydropyridin-1(2H)-yl)prop-2-en-1-one,
19) 1-(4-(6-((5-methylthiazol-2-yl)amino)-1-(2,2,2-trifluoroethyl)-1H-pyrrolo[3,2-c]pyridin-4-yl)-3,6-dihydropyridin-1(2H)-yl)prop-2-en-1-one,
20) 1-(4-(1-(2,2-difluorocyclopropyl)-6-((5-methylthiazol-2-yl)amino)-1H-pyrrolo[3,2-c]pyridin-4-yl)-3,6-dihydropyridin-1(2H)-yl)prop-2-en-1-one,
21) 1-(4-(1-(difluoromethyl)-6-((5-methylthiazol-2-yl)amino)-1H-pyrrolo[3,2-c]pyridin-4-yl)-3,6-dihydropyridin-1(2H)-yl)prop-2-en-1-one,
22) 2-(4-(1-acryloyl-1,2,3,6-tetrahydropyridin-4-yl)-6-((5-methylthiazol-2-yl)amino)-1H-pyrrolo[3,2-c]pyridin-1-yl)-2-methylpropanenitrile,
23) 2-(4-(1-acryloyl-1,2,5,6-tetrahydropyridin-3-yl)-6-((5-methylthiazol-2-yl)amino)-1H-pyrrolo[3,2-c]pyridin-1-yl)-2-methylpropanenitrile,
24) (R)-1-(4-(6-((5-methylthiazol-2-yl)amino)-1-(1,1,1-trifluoropropan-2-yl)-1 H-pyrrolo[3,2-c]pyridin-4-yl)-3,6-dihydropyridin-1 (2H)-yl)prop-2-en-1-one,
25) 1-(4-(1-(2,2-difluoroethyl)-6-((5-methylthiazol-2-yl)amino)-1H-pyrrolo[3,2-c]pyridin-4-yl)-3-methyl-3,6-dihydropyridin-1(2H)-yl)prop-2-en-1-one,
26) 1-(4-(1-isopropyl-6-((5-methylthiazol-2-yl)amino)-1H-pyrrolo[3,2-c]pyridin-4-yl)-2,3,6,7-tetrahydro-1H-azepin-1-yl)prop-2-en-1-one,
27) 1-(7-(1-isopropyl-6-((5-methylthiazol-2-yl)amino)-1H-pyrrolo[3,2-c]pyridin-4-yl)-3-oxa-9-azabicyclo[3.3.1]non-6-en-9-yl)prop-2-en-1-one,
28) 1-(3-(1-methyl-6-((5-methylthiazol-2-yl)amino)-1H-pyrrolo[3,2-c]pyridin-4-yl)-8-azabicyclo[3.2.1]oct-2-en-8-yl)prop-2-en-1-one,
29) 1-(3-(1-isopropyl-6-((5-methylthiazol-2-yl)amino)-1H-pyrrolo[3,2-c]pyridin-4-yl)-8-azabicyclo[3.2.1]oct-2-en-8-yl)prop-2-en-1-one,
30) 1-(3-(6-((5-methylthiazol-2-yl)amino)-1-((1-(trifluoromethyl)cyclopropyl)methyl)-1H-pyrrolo[3,2-c]pyridin-4-yl)-8-azabicyclo[3.2.1]oct-2-en-8-yl)prop-2-en-1-one,
31) 1-(3-(1-(2,2-difluorocyclopropyl)-6-((5-methylthiazol-2-yl)amino)-1H-pyrrolo[3,2-c]pyridin-4-yl)-8-azabicyclo[3.2.1]oct-2-en-8-yl)prop-2-en-1-one,
32) 1-(3-(1-isobutyl-6-((5-methylthiazol-2-yl)amino)-1H-pyrrolo[3,2-c]pyridin-4-yl)-8-azabicyclo[3.2.1]oct-2-en-8-yl)prop-2-en-1-one,
33) 1-(3-(1-cyclopentyl-6-((5-methylthiazol-2-yl)amino)-1H-pyrrolo[3,2-c]pyridin-4-yl)-8-azabicyclo[3.2.1]oct-2-en-8-yl)prop-2-en-1-one,
34) 1-(3-(1-(2,2-difluoroethyl)-6-((5-methylthiazol-2-yl)amino)-1H-pyrrolo[3,2-c]pyridin-4-yl)-8-azabicyclo[3.2.1]oct-2-en-8-yl)prop-2-en-1-one,
35) 1-(3-(6-((5-methylthiazol-2-yl)amino)-1-(2,2,2-trifluoroethyl)-1H-pyrrolo[3,2-c]pyridin-4-yl)-8-azabicyclo[3.2.1]oct-2-en-8-yl)prop-2-en-1-one,
36) 1-(3-(1-methyl-6-((5-methylthiazol-2-yl)amino)-1H-pyrrolo[3,2-c]pyridin-4-yl)-9-azabicyclo[3.3.1]non-2-en-9-yl)prop-2-en-1-one,
37) 1-(3-(1-isopropyl-6-((5-methylthiazol-2-yl)amino)-1H-pyrrolo[3,2-c]pyridin-4-yl)-9-azabicyclo[3.3.1]non-2-en-9-yl)prop-2-en-1-one,
38) 1-(3-(1-isobutyl-6-((5-methylthiazol-2-yl)amino)-1H-pyrrolo[3,2-c]pyridin-4-yl)-9-azabicyclo[3.3.1]non-2-en-9-yl)prop-2-en-1-one,
39) 1-(3-(1-(cyclopropylmethyl)-6-((5-methylthiazol-2-yl)amino)-1H-pyrrolo[3,2-c]pyridin-4-yl)-8-azabicyclo[3.2.1]oct-2-en-8-yl)prop-2-en-1-one,
40) 1-(4-(6-((1H-imidazol-2-yl)amino)-1-methyl-1H-pyrrolo[3,2-c]pyridin-4-yl)-3,6-dihydropyridin-1(2H)-yl)prop-2-en-1-one,
41) 1-(4-(1-isopropyl-6-((5-methyl-1,3,4-thiadiazol-2-yl)amino)-1H-pyrrolo[3,2-c]pyridin-4-yl)-3,6-dihydropyridin-1(2H)-yl)prop-2-en-1-one,
42) 1-(4-(1-isopropyl-6-((4-methylthiazol-2-yl)amino)-1H-pyrrolo[3,2-c]pyridin-4-yl)-3,6-dihydropyridin-1(2H)-yl)prop-2-en-1-one,
43) 1-(4-(1-isopropyl-6-(pyrimidin-4-ylamino)-1H-pyrrolo[3,2-c]pyridin-4-yl)-3,6-dihydropyridin-1(2H)-yl)prop-2-en-1-one,
44) 1-(4-(1-isopropyl-6-((6-methylpyrimidin-4-yl)amino)-1H-pyrrolo[3,2-c]pyridin-4-yl)-3,6-dihydropyridin-1(2H)-yl)prop-2-en-1-one,
45) 1-(4-(1-isopropyl-6-((6-(trifluoromethyl)pyrimidin-4-yl)amino)-1H-pyrrolo[3,2-c]pyridin-4-yl)-3,6-dihydropyridin-1(2H)-yl)prop-2-en-1-one,
46) 1-(4-(1-isopropyl-6-((6-methoxypyrimidin-4-yl)amino)-1H-pyrrolo[3,2-c]pyridin-4-yl)-3,6-dihydropyridin-1(2H)-yl)prop-2-en-1-one,
47) 1-(4-(6-((4-fluoropyridin-2-yl)amino)-1-isopropyl-1H-pyrrolo[3,2-c]pyridin-4-yl)-3,6-dihydropyridin-1(2H)-yl)prop-2-en-1-one,
48) 1-(4-(1-isopropyl-6-((4-(trifluoromethyl)pyridin-2-yl)amino)-1H-pyrrolo[3,2-c]pyridin-4-yl)-3,6-dihydropyridin-1(2H)-yl)prop-2-en-1-one,
49) 1-(4-(1-isopropyl-6-((4-methylpyridin-2-yl)amino)-1H-pyrrolo[3,2-c]pyridin-4-yl)-3,6-dihydropyridin-1(2H)-yl)prop-2-en-1-one,
50) N-(3-(1-methyl-6-((5-methylthiazol-2-yl)amino)-1H-pyrrolo[3,2-c]pyridin-4-yl)phenyl)acrylamide,
51) N-(2-fluoro-3-(1-methyl-6-((5-methylthiazol-2-yl)amino)-1H-pyrrolo[3,2-c]pyridin-4-yl)phenyl)acrylamide,
52) N-(2-methoxy-3-(1-methyl-6-((5-methylthiazol-2-yl)amino)-1H-pyrrolo[3,2-c]pyridin-4-yl)phenyl)acrylamide,
53) N-(3-methyl-5-(1-methyl-6-((5-methylthiazol-2-yl)amino)-1H-pyrrolo[3,2-c]pyridin-4-yl)phenyl)acrylamide,
54) N-(2-fluoro-5-(1-methyl-6-((5-methylthiazol-2-yl)amino)-1H-pyrrolo[3,2-c]pyridin-4-yl)phenyl)acrylamide,
55) N-(2-methyl-5-(1-methyl-6-((5-methylthiazol-2-yl)amino)-1H-pyrrolo[3,2-c]pyridin-4-yl)phenyl)acrylamide,
56) N-(2,6-dimethyl-3-(1-methyl-6-((5-methylthiazol-2-yl)amino)-1H-pyrrolo[3,2-c]pyridin-4-yl)phenyl)acrylamide,
57) N-(2,6-difluoro-3-(1-methyl-6-((5-methylthiazol-2-yl)amino)-1H-pyrrolo[3,2-c]pyridin-4-yl)phenyl)acrylamide,
58) N-(3-(1-isopropyl-6-((5-methylthiazol-2-yl)amino)-1H-pyrrolo[3,2-c]pyridin-4-yl)-2-methylphenyl)acrylamide,
59) N-(4-fluoro-3-(1-isopropyl-6-((5-methylthiazol-2-yl)amino)-1H-pyrrolo[3,2-c]pyridin-4-yl)phenyl)acrylamide,
60) N-(2,4-difluoro-5-(1-isopropyl-6-((5-methylthiazol-2-yl)amino)-1H-pyrrolo[3,2-c]pyridin-4-yl)phenyl)acrylamide,
61) N-(3-(1-(cyclopropylmethyl)-6-((5-methylthiazol-2-yl)amino)-1H-pyrrolo[3,2-c]pyridin-4-yl)-4-fluorophenyl)acrylamide,
62) N-(3-(1-isobutyl-6-((5-methylthiazol-2-yl)amino)-1H-pyrrolo[3,2-c]pyridin-4-yl)-2-methylphenyl)acrylamide,
63) N-(4-fluoro-3-(1-isobutyl-6-((5-methylthiazol-2-yl)amino)-1H-pyrrolo[3,2-c]pyridin-4-yl)phenyl)acrylamide,
64) N-(5-(1-(cyclopropylmethyl)-6-((5-methylthiazol-2-yl)amino)-1H-pyrrolo[3,2-c]pyridin-4-yl)-2-fluorophenyl)acrylamide,
65) N-(5-(1-(cyclopropylmethyl)-6-((5-methylthiazol-2-yl)amino)-1H-pyrrolo[3,2-c]pyridin-4-yl)-2-methylphenyl)acrylamide,
66) N-(5-(1-(cyclopropylmethyl)-6-((5-methylthiazol-2-yl)amino)-1H-pyrrolo[3,2-c]pyridin-4-yl)-2,4-difluorophenyl)acrylamide,
67) N-(2,4-difluoro-5-(1-isobutyl-6-((5-methylthiazol-2-yl)amino)-1H-pyrrolo[3,2-c]pyridin-4-yl)phenyl)acrylamide,
68) (S)-N-(3-(1-(sec-butyl)-6-((5-methylthiazol-2-yl)amino)-1H-pyrrolo[3,2-c]pyridin-4-yl)-4-fluorophenyl)acrylamide,
69) N-(5-(1-(3,3-dimethylbutyl)-6-((5-methylthiazol-2-yl)amino)-1H-pyrrolo[3,2-c]pyridin-4-yl)-2,4-difluorophenyl)acrylamide,
70) N-(3-(1-cyclopentyl-6-((5-methylthiazol-2-yl)amino)-1H-pyrrolo[3,2-c]pyridin-4-yl)-4-fluorophenyl)acrylamide,
71) N-(4-fluoro-3-(6-((5-methylthiazol-2-yl)amino)-1-((tetrahydrofuran-3-yl)methyl)-1H-pyrrolo[3,2-c]pyridin-4-yl)phenyl)acrylamide,
72) N-(2-fluoro-5-(6-((5-methylthiazol-2-yl)amino)-1-((tetrahydrofuran-3-yl)methyl)-1H-pyrrolo[3,2-c]pyridin-4-yl)phenyl)acrylamide,
73) N-(2-fluoro-3-(6-((5-methylthiazol-2-yl)amino)-1-((tetrahydro-2H-pyran-4-yl)methyl)-1H-pyrrolo[3,2-c]pyridin-4-yl)phenyl)acrylamide,
74) N-(5-(1-(2,2-difluoroethyl)-6-((5-methylthiazol-2-yl)amino)-1H-pyrrolo[3,2-c]pyridin-4-yl)-2-methylphenyl)acrylamide,
75) N-(3-(1-(2,2-difluoroethyl)-6-((5-methylthiazol-2-yl)amino)-1H-pyrrolo[3,2-c]pyridin-4-yl)-4-fluorophenyl)acrylamide,
76) N-(5-(1-(2,2-difluoroethyl)-6-((5-methylthiazol-2-yl)amino)-1H-pyrrolo[3,2-c]pyridin-4-yl)-2-fluorophenyl)acrylamide,
77) N-(2,4-difluoro-5-(6-((5-methylthiazol-2-yl)amino)-1-(2,2,2-trifluoroethyl)-1H-pyrrolo[3,2-c]pyridin-4-yl)phenyl)acrylamide,
78) N-(4-fluoro-3-(6-((5-methylthiazol-2-yl)amino)-1-(2,2,2-trifluoroethyl)-1H-pyrrolo[3,2-c]pyridin-4-yl)phenyl)acrylamide,
79) (S)-1-(3-((1-isopropyl-6-((5-methylthiazol-2-yl)amino)-1H-pyrrolo[3,2-c]pyridin-4-yl)oxy)pyrrolidin-1-yl)prop-2-en-1-one,
80) 1-(3-((1-isopropyl-6-((5-methylthiazol-2-yl)amino)-1H-pyrrolo[3,2-c]pyridin-4-yl)oxy)azetidin-1-yl)prop-2-en-1-one,
81) (S)-1-(3-((1-isobutyl-6-((5-methylthiazol-2-yl)amino)-1H-pyrrolo[3,2-c]pyridin-4-yl)oxy)pyrrolidin-1-yl)prop-2-en-1-one,
82) (R)-1-(3-((1-isobutyl-6-((5-methylthiazol-2-yl)amino)-1H-pyrrolo[3,2-c]pyridin-4-yl)oxy)pyrrolidin-1-yl)prop-2-en-1-one,
83) 1-((3S,4R)-3-fluoro-4-((1-isopropyl-6-((5-methylthiazol-2-yl)amino)-1H-pyrrolo[3,2-c]pyridin-4-yl)oxy)pyrrolidin-1-yl)prop-2-en-1-one,
84) (S)-1-(3-((1-isopropyl-6-((5-methylthiazol-2-yl)amino)-1H-pyrrolo[3,2-c]pyridin-4-yl)oxy)piperidin-1-yl)prop-2-en-1-one,
85) (S)-1-(2-(((1-isobutyl-6-((5-methylthiazol-2-yl)amino)-1H-pyrrolo[3,2-c]pyridin-4-yl)oxy)methyl)pyrrolidin-1-yl)prop-2-en-1-one,
86) (S)-1-(2-(((1-isopropyl-6-((5-methylthiazol-2-yl)amino)-1H-pyrrolo[3,2-c]pyridin-4-yl)oxy)methyl)azetidin-1-yl)prop-2-en-1-one,
87) 1-((2R,4S)-4-((1-isopropyl-6-((5-methylthiazol-2-yl)amino)-1H-pyrrolo[3,2-c]pyridin-4-yl)oxy)-2-methylpyrrolidin-1-yl)prop-2-en-1-one,
88) 1-((2S,4S)-4-((1-isopropyl-6-((5-methylthiazol-2-yl)amino)-1H-pyrrolo[3,2-c]pyridin-4-yl)oxy)-2-methylpyrrolidin-1-yl)prop-2-en-1-one,
89) (R)-1-(2-(((1-isopropyl-6-((5-methylthiazol-2-yl)amino)-1H-pyrrolo[3,2-c]pyridin-4-yl)oxy)methyl)pyrrolidin-1-yl)prop-2-en-1-one,
90) (R)-1-(2-(((1-isopropyl-6-((5-methylthiazol-2-yl)amino)-1H-pyrrolo[3,2-c]pyridin-4-yl)oxy)methyl)azetidin-1-yl)prop-2-en-1-one,
91) 1-((3R,4R)-3-fluoro-4-((1-isopropyl-6-((5-methylthiazol-2-yl)amino)-1H-pyrrolo[3,2-c]pyridin-4-yl)oxy)pyrrolidin-1-yl)prop-2-en-1-one,
92) rac-1-((3S,4R)-3-((1-isopropyl-6-((5-methylthiazol-2-yl)amino)-1H-pyrrolo[3,2-c]pyridin-4-yl)oxy)-4-methylpyrrolidin-1-yl)prop-2-en-1-one,
93) (R)-1-(4,4-difluoro-2-(((1-isopropyl-6-((5-methylthiazol-2-yl)amino)-1H-pyrrolo[3,2-c]pyridin-4-yl)oxy)methyl)pyrrolidin-1-yl)prop-2-en-1-one,
94) 1-((2R,4S)-2-(((1-isopropyl-6-((5-methylthiazol-2-yl)amino)-1H-pyrrolo[3,2-c]pyridin-4-yl)oxy)methyl)-4-methoxypyrrolidin-1-yl)prop-2-en-1-one,
95) (S)-1-(3-((1-isopropyl-6-((6-(trifluoromethyl)pyrimidin-4-yl)amino)-1H-pyrrolo[3,2-c]pyridin-4-yl)oxy)pyrrolidin-1-yl)prop-2-en-1-one,
96) 1-((2R,4S)-4-((1-isobutyl-6-((5-methylthiazol-2-yl)amino)-1H-pyrrolo[3,2-c]pyridin-4-yl)oxy)-2-methylpyrrolidin-1-yl)prop-2-en-1-one,
97) (S)-1-(3-((1-(cyclopropylmethyl)-6-((5-methylthiazol-2-yl)amino)-1H-pyrrolo[3,2-c]pyridin-4-yl)oxy)pyrrolidin-1-yl)prop-2-en-1-one,
98) 1-((2R,4S)-4-((1-(cyclopropylmethyl)-6-((5-methylthiazol-2-yl)amino)-1H-pyrrolo[3,2-c]pyridin-4-yl)oxy)-2-methylpyrrolidin-1-yl)prop-2-en-1-one,
99) (S)-1-(3-((1-cyclopentyl-6-((5-methylthiazol-2-yl)amino)-1H-pyrrolo[3,2-c]pyridin-4-yl)oxy)pyrrolidin-1-yl)prop-2-en-1-one,
100) 1-((2R,4S)-4-((1-cyclopentyl-6-((5-methylthiazol-2-yl)amino)-1H-pyrrolo[3,2-c]pyridin-4-yl)oxy)-2-methylpyrrolidin-1-yl)prop-2-en-1-one,
101) (S)-1-(3-((1-isopropyl-6-((6-methylpyrimidin-4-yl)amino)-1H-pyrrolo[3,2-c]pyridin-4-yl)oxy)pyrrolidin-1-yl)prop-2-en-1-one,
102) (S)-1-(3-((6-((5-methylthiazol-2-yl)amino)-1-((1-(trifluoromethyl)cyclopropyl)methyl)-1H-pyrrolo[3,2-c]pyridin-4-yl)oxy)pyrrolidin-1-yl)prop-2-en-1-one,
103) (S)-1-(3-((1-isopropyl-6-((6-methoxypyrimidin-4-yl)amino)-1H-pyrrolo[3,2-c]pyridin-4-yl)oxy)pyrrolidin-1-yl)prop-2-en-1-one,
104) (S)-1-(3-((1-isobutyl-6-((6-methylpyrimidin-4-yl)amino)-1H-pyrrolo[3,2-c]pyridin-4-yl)oxy)pyrrolidin-1-yl)prop-2-en-1-one,
105) 1-((3S,4R)-3-fluoro-4-((1-isobutyl-6-((5-methylthiazol-2-yl)amino)-1H-pyrrolo[3,2-c]pyridin-4-yl)oxy)pyrrolidin-1-yl)prop-2-en-1-one,
106) 1-((3R,4R)-3-fluoro-4-((1-isobutyl-6-((5-methylthiazol-2-yl)amino)-1H-pyrrolo[3,2-c]pyridin-4-yl)oxy)pyrrolidin-1-yl)prop-2-en-1-one,
107) 1-((3R,4S)-3-((1-(cyclopropylmethyl)-6-((5-methylthiazol-2-yl)amino)-1H-pyrrolo[3,2-c]pyridin-4-yl)oxy)-4-fluoropyrrolidin-1-yl)prop-2-en-1-one,
108) 1-((3R,4R)-3-((1-(cyclopropylmethyl)-6-((5-methylthiazol-2-yl)amino)-1H-pyrrolo[3,2-c]pyridin-4-yl)oxy)-4-fluoropyrrolidin-1-yl)prop-2-en-1-one,
109) 1-((3R,4S)-3-((1-cyclopentyl-6-((5-methylthiazol-2-yl)amino)-1H-pyrrolo[3,2-c]pyridin-4-yl)oxy)-4-fluoropyrrolidin-1-yl)prop-2-en-1-one,
110) 1-((3R,4R)-3-((1-cyclopentyl-6-((5-methylthiazol-2-yl)amino)-1H-pyrrolo[3,2-c]pyridin-4-yl)oxy)-4-fluoropyrrolidin-1-yl)prop-2-en-1-one,
111) 1-((S)-3-((1-((S)-sec-butyl)-6-((5-methylthiazol-2-yl)amino)-1H-pyrrolo[3,2-c]pyridin-4-yl)oxy)pyrrolidin-1-yl)prop-2-en-1-one,
112) 1-((3R,4S)-3-((1-((S)-sec-butyl)-6-((5-methylthiazol-2-yl)amino)-1H-pyrrolo[3,2-c]pyridin-4-yl)oxy)-4-fluoropyrrolidin-1-yl)prop-2-en-1-one,
113) 1-((3R,4R)-3-((1-((S)-sec-butyl)-6-((5-methylthiazol-2-yl)amino)-1H-pyrrolo[3,2-c]pyridin-4-yl)oxy)-4-fluoropyrrolidin-1-yl)prop-2-en-1-one,
114) (S)-1-(3-((1-isopentyl-6-((5-methylthiazol-2-yl)amino)-1H-pyrrolo[3,2-c]pyridin-4-yl)oxy)pyrrolidin-1-yl)prop-2-en-1-one,
115) 1-((3R,4R)-3-fluoro-4-((1-isopentyl-6-((5-methylthiazol-2-yl)amino)-1H-pyrrolo[3,2-c]pyridin-4-yl)oxy)pyrrolidin-1-yl)prop-2-en-1-one,
116) 1-(3-((1-isobutyl-6-((5-methylthiazol-2-yl)amino)-1H-pyrrolo[3,2-c]pyridin-4-yl)oxy)-4-methylpyrrolidin-1-yl)prop-2-en-1-one,
117) (S)-1-(3-((6-((5-methylthiazol-2-yl)amino)-1-(pyridin-2-ylmethyl)-1H-pyrrolo[3,2-c]pyridin-4-yl)oxy)pyrrolidin-1-yl)prop-2-en-1-one,
118) 1-(3-((1-isobutyl-6-((5-methylthiazol-2-yl)amino)-1H-pyrrolo[3,2-c]pyridin-4-yl)oxy)azetidin-1-yl)prop-2-en-1-one,
119) 1-(3-((1-isobutyl-6-((5-methylthiazol-2-yl)amino)-1H-pyrrolo[3,2-c]pyridin-4-yl)oxy)-3-methylazetidin-1-yl)prop-2-en-1-one,
120) 1-((3R,4R)-3-fluoro-4-((6-((5-methylthiazol-2-yl)amino)-1-((1-(trifluoromethyl)cyclopropyl)methyl)-1H-pyrrolo[3,2-c]pyridin-4-yl)oxy)pyrrolidin-1-yl)prop-2-en-1-one,
121) (S)-1-(3-((1-benzyl-6-((5-methylthiazol-2-yl)amino)-1H-pyrrolo[3,2-c]pyridin-4-yl)oxy)pyrrolidin-1-yl)prop-2-en-1-one,
122) (S)-1-(3-((6-((5-ethylthiazol-2-yl)amino)-1-isobutyl-1H-pyrrolo[3,2-c]pyridin-4-yl)oxy)pyrrolidin-1-yl)prop-2-en-1-one,
123) 1-((3S)-3-((1-(2-methylbutyl)-6-((5-methylthiazol-2-yl)amino)-1H-pyrrolo[3,2-c]pyridin-4-yl)oxy)pyrrolidin-1-yl)prop-2-en-1-one,
124) (S)-1-(3-((1-butyl-6-((5-methylthiazol-2-yl)amino)-1H-pyrrolo[3,2-c]pyridin-4-yl)oxy)pyrrolidin-1-yl)prop-2-en-1-one,
125) (S)-1-(3-((1-ethyl-6-((5-methylthiazol-2-yl)amino)-1H-pyrrolo[3,2-c]pyridin-4-yl)oxy)pyrrolidin-1-yl)prop-2-en-1-one,
126) (S)-1-(3-((6-((5-methylthiazol-2-yl)amino)-1-propyl-1H-pyrrolo[3,2-c]pyridin-4-yl)oxy)pyrrolidin-1-yl)prop-2-en-1-one,
127) (S)-1-(3-((1-(2-methoxyethyl)-6-((5-methylthiazol-2-yl)amino)-1H-pyrrolo[3,2-c]pyridin-4-yl)oxy)pyrrolidin-1-yl)prop-2-en-1-one,
128) (S)-1-(3-((1-(cyclopentylmethyl)-6-((5-methylthiazol-2-yl)amino)-1H-pyrrolo[3,2-c]pyridin-4-yl)oxy)pyrrolidin-1-yl)prop-2-en-1-one,
129) (S)-1-(3-((1-(cyclohexylmethyl)-6-((5-methylthiazol-2-yl)amino)-1H-pyrrolo[3,2-c]pyridin-4-yl)oxy)pyrrolidin-1-yl)prop-2-en-1-one,
130) (S)-1-(3-((1-methyl-6-((5-methylthiazol-2-yl)amino)-1H-pyrrolo[3,2-c]pyridin-4-yl)oxy)pyrrolidin-1-yl)prop-2-en-1-one,
131) 1-((3S,4R)-3-fluoro-4-((1-methyl-6-((5-methylthiazol-2-yl)amino)-1H-pyrrolo[3,2-c]pyridin-4-yl)oxy)pyrrolidin-1-yl)prop-2-en-1-one,
132) 1-((3R,4S)-3-((1-(cyclopentylmethyl)-6-((5-methylthiazol-2-yl)amino)-1H-pyrrolo[3,2-c]pyridin-4-yl)oxy)-4-fluoropyrrolidin-1-yl)prop-2-en-1-one,
133) (S)-1-(3-((6-((5-methylthiazol-2-yl)amino)-1-((tetrahydro-2H-pyran-4-yl)methyl)-1H-pyrrolo[3,2-c]pyridin-4-yl)oxy)pyrrolidin-1-yl)prop-2-en-1-one,
134) 1-((3S,43)-3-fluoro-4-((1-isobutyl-6-((5-methylthiazol-2-yl)amino)-1H-pyrrolo[3,2-c]pyridin-4-yl)oxy)pyrrolidin-1-yl)prop-2-en-1-one,
135) 1-((3R,4S)-3-fluoro-4-((1-isobutyl-6-((5-methylthiazol-2-yl)amino)-1H-pyrrolo[3,2-c]pyridin-4-yl)oxy)pyrrolidin-1-yl)prop-2-en-1-one,
136) 1-(6-((1-methyl-6-((5-methylthiazol-2-yl)amino)-1H-pyrrolo[3,2-c]pyridin-4-yl)oxy)-1-azaspiro[3.3]heptan-1-yl)prop-2-en-1-one,
137) 1-(6-((1-isobutyl-6-((5-methylthiazol-2-yl)amino)-1H-pyrrolo[3,2-c]pyridin-4-yl)oxy)-1-azaspiro[3.3]heptan-1-yl)prop-2-en-1-one,
138) (S)-1-(3-((6-((5-fluoro-2-methylphenyl)amino)-1-methyl-1H-pyrrolo[3,2-c]pyridin-4-yl)oxy)pyrrolidin-1-yl)prop-2-en-1-one,
139) 1-((3S,4R)-3-fluoro-4-((6-((5-fluoro-2-methylphenyl)amino)-1-methyl-1H-pyrrolo[3,2-c]pyridin-4-yl)oxy)pyrrolidin-1-yl)prop-2-en-1-one,
140) (S)-1-(3-((1-(2-hydroxy-2-methylpropyl)-6-((5-methylthiazol-2-yl)amino)-1H-pyrrolo[3,2-c]pyridin-4-yl)oxy)pyrrolidin-1-yl)prop-2-en-1-one,
141) 1-((3S,4R)-3-fluoro-4-((1-(2-hydroxy-2-methylpropyl)-6-((5-methylthiazol-2-yl)amino)-1H-pyrrolo[3,2-c]pyridin-4-yl)oxy)pyrrolidin-1-yl)prop-2-en-1-one,
142) 1-(3-((1-cyclopentyl-6-((5-methylthiazol-2-yl)amino)-1H-pyrrolo[3,2-c]pyridin-4-yl)oxy)azetidin-1-yl)prop-2-en-1-one,
143) (R)-1-(3-((1-cyclopentyl-6-((5-methylthiazol-2-yl)amino)-1H-pyrrolo[3,2-c]pyridin-4-yl)oxy)pyrrolidin-1-yl)prop-2-en-1-one,
144) (R)-1-(3-((1-(cyclopropylmethyl)-6-((5-methylthiazol-2-yl)amino)-1H-pyrrolo[3,2-c]pyridin-4-yl)oxy)pyrrolidin-1-yl)prop-2-en-1-one,
145) 1-(3-((1-(cyclopropylmethyl)-6-((5-methylthiazol-2-yl)amino)-1H-pyrrolo[3,2-c]pyridin-4-yl)oxy)azetidin-1-yl)prop-2-en-1-one,
146) 1-(3-((1-(2-methoxyethyl)-6-((5-methylthiazol-2-yl)amino)-1H-pyrrolo[3,2-c]pyridin-4-yl)oxy)azetidin-1-yl)prop-2-en-1-one,
147) 1-((3S,4R)-3-fluoro-4-((1-(2-methoxyethyl)-6-((5-methylthiazol-2-yl)amino)-1H-pyrrolo[3,2-c]pyridin-4-yl)oxy)pyrrolidin-1-yl)prop-2-en-1-one,
148) (S)-1-(3-((1-(2-methoxyethyl)-6-((5-methylthiazol-2-yl)amino)-1H-pyrrolo[3,2-c]pyridin-4-yl)oxy)piperidin-1-yl)prop-2-en-1-one,
149) (S)-1-(3-((1-(cyclopropylmethyl)-6-((5-methylthiazol-2-yl)amino)-1H-pyrrolo[3,2-c]pyridin-4-yl)oxy)piperidin-1-yl)prop-2-en-1-one,
150) (S)-1-(3-((1-isobutyl-6-((5-methylthiazol-2-yl)amino)-1H-pyrrolo[3,2-c]pyridin-4-yl)oxy)piperidin-1-yl)prop-2-en-1-one,
151) (S)-1-(3-((1-cyclopentyl-6-((5-methylthiazol-2-yl)amino)-1H-pyrrolo[3,2-c]pyridin-4-yl)oxy)piperidin-1-yl)prop-2-en-1-one,
152) 1-((3R,4S)-3-((1-ethyl-6-((5-methylthiazol-2-yl)amino)-1H-pyrrolo[3,2-c]pyridin-4-yl)oxy)-4-fluoropyrrolidin-1-yl)prop-2-en-1-one,
153) (S)-1-(3-((1-ethyl-6-((5-methylthiazol-2-yl)amino)-1H-pyrrolo[3,2-c]pyridin-4-yl)oxy)piperidin-1-yl)prop-2-en-1-one,
154) 1-((3R,4R)-3-((1-ethyl-6-((5-methylthiazol-2-yl)amino)-1H-pyrrolo[3,2-c]pyridin-4-yl)oxy)-4-fluoropyrrolidin-1-yl)prop-2-en-1-one,
155) 1-(3-((1-ethyl-6-((5-methylthiazol-2-yl)amino)-1H-pyrrolo[3,2-c]pyridin-4-yl)oxy)-3-methylazetidin-1-yl)prop-2-en-1-one,
156) (R)-1-(3-((1-methyl-6-((5-methylthiazol-2-yl)amino)-1H-pyrrolo[3,2-c]pyridin-4-yl)oxy)pyrrolidin-1-yl)prop-2-en-1-one,
157) 1-((3R,4R)-3-fluoro-4-((1-methyl-6-((5-methylthiazol-2-yl)amino)-1H-pyrrolo[3,2-c]pyridin-4-yl)oxy)pyrrolidin-1-yl)prop-2-en-1-one,
158) (S)-1-(3-((1-methyl-6-((5-methylthiazol-2-yl)amino)-1H-pyrrolo[3,2-c]pyridin-4-yl)oxy)piperidin-1-yl)prop-2-en-1-one,
159) 1-((3S,4R)-3-fluoro-4-((6-((5-methylthiazol-2-yl)amino)-1-propyl-1H-pyrrolo[3,2-c]pyridin-4-yl)oxy)pyrrolidin-1-yl)prop-2-en-1-one,
160) (S)-1-(3-((6-((5-methylthiazol-2-yl)amino)-1-propyl-1H-pyrrolo[3,2-c]pyridin-4-yl)oxy)piperidin-1-yl)prop-2-en-1-one,
161) 1-((3R,4R)-3-fluoro-4-((6-((5-methylthiazol-2-yl)amino)-1-propyl-1H-pyrrolo[3,2-c]pyridin-4-yl)oxy)pyrrolidin-1-yl)prop-2-en-1-one,
162) (R)-1-(3-((1-isopropyl-6-((5-methylthiazol-2-yl)amino)-1H-pyrrolo[3,2-c]pyridin-4-yl)oxy)pyrrolidin-1-yl)prop-2-en-1-one,
163) 1-(3-((1-isopropyl-6-((5-methylthiazol-2-yl)amino)-1H-pyrrolo[3,2-c]pyridin-4-yl)oxy)-3-methylazetidin-1-yl)prop-2-en-1-one,
164) 1-((2S,45)-4-((1-cyclopentyl-6-((5-methylthiazol-2-yl)amino)-1H-pyrrolo[3,2-c]pyridin-4-yl)oxy)-2-methylpyrrolidin-1-yl)prop-2-en-1-one,
165) 1-(3-((1-cyclopentyl-6-((5-methylthiazol-2-yl)amino)-1H-pyrrolo[3,2-c]pyridin-4-yl)oxy)-3-methylazetidin-1-yl)prop-2-en-1-one,
166) 1-(3-methyl-3-((1-methyl-6-((5-methylthiazol-2-yl)amino)-1H-pyrrolo[3,2-c]pyridin-4-yl)oxy)azetidin-1-yl)prop-2-en-1-one,
167) 1-(3-((1-(cyclopropylmethyl)-6-((5-methylthiazol-2-yl)amino)-1H-pyrrolo[3,2-c]pyridin-4-yl)oxy)-3-methylazetidin-1-yl)prop-2-en-1-one,
168) 1-(3-((1-(2-methoxyethyl)-6-((5-methylthiazol-2-yl)amino)-1H-pyrrolo[3,2-c]pyridin-4-yl)oxy)-3-methylazetidin-1-yl)prop-2-en-1-one,
169) (S)-1-(3-((1-(cyclopentylmethyl)-6-((5-methylthiazol-2-yl)amino)-1H-pyrrolo[3,2-c]pyridin-4-yl)oxy)piperidin-1-yl)prop-2-en-1-one,
170) 1-((3R,4R)-3-((1-(cyclopentylmethyl)-6-((5-methylthiazol-2-yl)amino)-1H-pyrrolo[3,2-c]pyridin-4-yl)oxy)-4-fluoropyrrolidin-1-yl)prop-2-en-1-one,
171) 1-((3S,4R)-3-fluoro-4-((1-(2-methylbutyl)-6-((5-methylthiazol-2-yl)amino)-1H-pyrrolo[3,2-c]pyridin-4-yl)oxy)pyrrolidin-1-yl)prop-2-en-1-one,
172) 1-((3S)-3-((1-(2-methylbutyl)-6-((5-methylthiazol-2-yl)amino)-1H-pyrrolo[3,2-c]pyridin-4-yl)oxy)piperidin-1-yl)prop-2-en-1-one,
173) 1-((3R,4R)-3-fluoro-4-((1-(2-methylbutyl)-6-((5-methylthiazol-2-yl)amino)-1H-pyrrolo[3,2-c]pyridin-4-yl)oxy)pyrrolidin-1-yl)prop-2-en-1-one,
174) 1-((3R,4R)-3-fluoro-4-((1-((R)-2-methylbutyl)-6-((5-methylthiazol-2-yl)amino)-1H-pyrrolo[3,2-c]pyridin-4-yl)oxy)pyrrolidin-1-yl)prop-2-en-1-one,
175) 1-((3R,4R)-3-fluoro-4-((1-((S)-2-methylbutyl)-6-((5-methylthiazol-2-yl)amino)-1H-pyrrolo[3,2-c]pyridin-4-yl)oxy)pyrrolidin-1-yl)prop-2-en-1-one,
176) 1-(3-methyl-3-((6-((5-methylthiazol-2-yl)amino)-1-propyl-1H-pyrrolo[3,2-c]pyridin-4-yl)oxy)azetidin-1-yl)prop-2-en-1-one,
177) 1-(3-((1-isopentyl-6-((5-methylthiazol-2-yl)amino)-1H-pyrrolo[3,2-c]pyridin-4-yl)oxy)-3-methylazetidin-1-yl)prop-2-en-1-one,
178) 1-(3-((1-(cyclopentylmethyl)-6-((5-methylthiazol-2-yl)amino)-1H-pyrrolo[3,2-c]pyridin-4-yl)oxy)-3-methylazetidin-1-yl)prop-2-en-1-one,
179) 1-((2S,4S)-4-((1-isobutyl-6-((5-methylthiazol-2-yl)amino)-1H-pyrrolo[3,2-c]pyridin-4-yl)oxy)-2-methylpyrrolidin-1-yl)prop-2-en-1-one,
180) 1-((2S,4S)-4-((1-(cyclopropylmethyl)-6-((5-methylthiazol-2-yl)amino)-1H-pyrrolo[3,2-c]pyridin-4-yl)oxy)-2-methylpyrrolidin-1-yl)prop-2-en-1-one,
181) 2-((4-(((3R,4S)-1-acryloyl-4-fluoropyrrolidin-3-yl)oxy)-1-isobutyl-1H-pyrrolo[3,2-c]pyridin-6-yl)amino)thiazole-5-carbonitrile,
182) 2-((4-(((3R,4S)-1-acryloyl-4-fluoropyrrolidin-3-yl)oxy)-1-isobutyl-1H-pyrrolo[3,2-c]pyridin-6-yl)amino)-N-methylthiazole-5-carboxamide,
183) (S)-1-(3-((1-isopropyl-6-((4-methoxypyrimidin-2-yl)amino)-1H-pyrrolo[3,2-c]pyridin-4-yl)oxy)pyrrolidin-1-yl)prop-2-en-1-one,
184) (S)-1-(3-((1-isopropyl-6-((3-methoxypyrazin-2-yl)amino)-1H-pyrrolo[3,2-c]pyridin-4-yl)oxy)pyrrolidin-1-yl)prop-2-en-1-one,
185) 1-((2S,4S)-2-methyl-4-((1-methyl-6-((5-methylthiazol-2-yl)amino)-1H-pyrrolo[3,2-c]pyridin-4-yl)oxy)pyrrolidin-1-yl)prop-2-en-1-one,
186) 1-((2S,4S)-4-((1-ethyl-6-((5-methylthiazol-2-yl)amino)-1H-pyrrolo[3,2-c]pyridin-4-yl)oxy)-2-methylpyrrolidin-1-yl)prop-2-en-1-one,
187) 1-((2S,4S)-2-methyl-4-((6-((5-methylthiazol-2-yl)amino)-1-propyl-1H-pyrrolo[3,2-c]pyridin-4-yl)oxy)pyrrolidin-1-yl)prop-2-en-1-one,
188) (S)-1-(3-((1-(2-fluoroethyl)-6-((5-methylthiazol-2-yl)amino)-1H-pyrrolo[3,2-c]pyridin-4-yl)oxy)pyrrolidin-1-yl)prop-2-en-1-one,
189) 1-((3S,4R)-3-fluoro-4-((1-(2-fluoroethyl)-6-((5-methylthiazol-2-yl)amino)-1H-pyrrolo[3,2-c]pyridin-4-yl)oxy)pyrrolidin-1-yl)prop-2-en-1-one,
190) (S)-1-(3-((1-(3-fluoropropyl)-6-((5-methylthiazol-2-yl)amino)-1H-pyrrolo[3,2-c]pyridin-4-yl)oxy)pyrrolidin-1-yl)prop-2-en-1-one,
191) 1-((3S,4R)-3-fluoro-4-((1-(3-fluoropropyl)-6-((5-methylthiazol-2-yl)amino)-1H-pyrrolo[3,2-c]pyridin-4-yl)oxy)pyrrolidin-1-yl)prop-2-en-1-one,
192) (S)-1-(3-((6-((1H-pyrazol-3-yl)amino)-1-isopropyl-1H-pyrrolo[3,2-c]pyridin-4-yl)oxy)pyrrolidin-1-yl)prop-2-en-1-one,
193) (S)-1-(3-((1-isopropyl-6-((1-methyl-1H-pyrazol-3-yl)amino)-1H-pyrrolo[3,2-c]pyridin-4-yl)oxy)pyrrolidin-1-yl)prop-2-en-1-one,
194) (S)-1-(3-((1-isopropyl-6-((5-methyl-1H-pyrazol-3-yl)amino)-1H-pyrrolo[3,2-c]pyridin-4-yl)oxy)pyrrolidin-1-yl)prop-2-en-1-one,
195) 1-((3R,4S)-3-((6-((1H-pyrazol-3-yl)amino)-1-isobutyl-1H-pyrrolo[3,2-c]pyridin-4-yl)oxy)-4-fluoropyrrolidin-1-yl)prop-2-en-1-one,
196) 1-((3S,4R)-3-fluoro-4-((1-isobutyl-6-((5-methyl-1H-pyrazol-3-yl)amino)-1H-pyrrolo[3,2-c]pyridin-4-yl)oxy)pyrrolidin-1-yl)prop-2-en-1-one,
197) 1-((3S,4R)-3-fluoro-4-((1-isobutyl-6-((1-methyl-1H-pyrazol-3-yl)amino)-1H-pyrrolo[3,2-c]pyridin-4-yl)oxy)pyrrolidin-1-yl)prop-2-en-1-one,
198) (S)-3-((4-((1-acryloylpyrrolidin-3-yl)oxy)-1-isopropyl-1H-pyrrolo[3,2-c]pyridin-6-yl)amino)-1-methylpyridin-2(1H)-one,
199) 3-((4-(((3R,4S)-1-acryloyl-4-fluoropyrrolidin-3-yl)oxy)-1-isobutyl-1H-pyrrolo[3,2-c]pyridin-6-yl)amino)-1-methylpyridin-2(1H)-one,
200) 1-(2-((1-isobutyl-6-((5-methylthiazol-2-yl)amino)-1H-pyrrolo[3,2-c]pyridin-4-yl)oxy)-5-azaspiro[3.4]octan-5-yl)prop-2-en-1-one,
201) 1-(7-((1-isobutyl-6-((5-methylthiazol-2-yl)amino)-1H-pyrrolo[3,2-c]pyridin-4-yl)oxy)-1-azaspiro[4.4]nonan-1-yl)prop-2-en-1-one,
202) 1-(2-((1-isobutyl-6-((5-methylthiazol-2-yl)amino)-1H-pyrrolo[3,2-c]pyridin-4-yl)oxy)-6-azaspiro[3.4]octan-6-yl)prop-2-en-1-one,
203) 1-(2-((1-isobutyl-6-((5-methylthiazol-2-yl)amino)-1H-pyrrolo[3,2-c]pyridin-4-yl)oxy)-5-azaspiro[3.5]nonan-5-yl)prop-2-en-1-one,
204) 1-(7-((1-isobutyl-6-((5-methylthiazol-2-yl)amino)-1H-pyrrolo[3,2-c]pyridin-4-yl)oxy)-2-azaspiro[4.4]nonan-2-yl)prop-2-en-1-one,
205) (S)-1-(3-((6-((5-methylthiazol-2-yl)amino)-1-(2,2,2-trifluoroethyl)-1H-pyrrolo[3,2-c]pyridin-4-yl)oxy)pyrrolidin-1-yl)prop-2-en-1-one,
206) (S)-1-(3-((1-(2,2-difluoroethyl)-6-((5-methylthiazol-2-yl)amino)-1H-pyrrolo[3,2-c]pyridin-4-yl)oxy)pyrrolidin-1-yl)prop-2-en-1-one,
207) (R)-1-(2-(((6-((5-methylthiazol-2-yl)amino)-1-(2,2,2-trifluoroethyl)-1H-pyrrolo[3,2-c]pyridin-4-yl)oxy)methyl)pyrrolidin-1-yl)prop-2-en-1-one,
208) (R)-1-(2-(((6-((5-methylthiazol-2-yl)amino)-1-(2,2,2-trifluoroethyl)-1H-pyrrolo[3,2-c]pyridin-4-yl)oxy)methyl)azetidin-1-yl)prop-2-en-1-one,
209) (R)-1-(2-(((1-(2,2-difluoroethyl)-6-((5-methylthiazol-2-yl)amino)-1H-pyrrolo[3,2-c]pyridin-4-yl)oxy)methyl)azetidin-1-yl)prop-2-en-1-one,
210) (R)-1-(2-(((1-(2,2-difluoroethyl)-6-((5-methylthiazol-2-yl)amino)-1H-pyrrolo[3,2-c]pyridin-4-yl)oxy)methyl)pyrrolidin-1-yl)prop-2-en-1-one,
211) 1-((3S,4R)-3-fluoro-4-((6-((5-methylthiazol-2-yl)amino)-1-(2,2,2-trifluoroethyl)-1H-pyrrolo[3,2-c]pyridin-4-yl)oxy)pyrrolidin-1-yl)prop-2-en-1-one,
212) 1-((2R,4S)-2-methyl-4-((6-((5-methylthiazol-2-yl)amino)-1-(2,2,2-trifluoroethyl)-1H-pyrrolo[3,2-c]pyridin-4-yl)oxy)pyrrolidin-1-yl)prop-2-en-1-one,
213) 1-((S)-3-((6-((5-methylthiazol-2-yl)amino)-1-((R)-1,1,1-trifluoropropan-2-yl)-1H-pyrrolo[3,2-c]pyridin-4-yl)oxy)pyrrolidin-1-yl)prop-2-en-1-one,
214) (R)-1-(3-(((1-isobutyl-6-((5-methylthiazol-2-yl)amino)-1H-pyrrolo[3,2-c]pyridin-4-yl)oxy)methyl)piperidin-1-yl)prop-2-en-1-one,
215) (S)-1-(3-(((1-isobutyl-6-((5-methylthiazol-2-yl)amino)-1H-pyrrolo[3,2-c]pyridin-4-yl)oxy)methyl)piperidin-1-yl)prop-2-en-1-one,
216) (S)-1-(3-(((1-isobutyl-6-((5-methylthiazol-2-yl)amino)-1H-pyrrolo[3,2-c]pyridin-4-yl)oxy)methyl)pyrrolidin-1-yl)prop-2-en-1-one,
217) 1-(2-(((1-cyclopentyl-6-((5-methylthiazol-2-yl)amino)-1H-pyrrolo[3,2-c]pyridin-4-yl)oxy)methyl)pyrrolidin-1-yl)prop-2-en-1-one,
218) 1-(2-(((1-cyclopentyl-6-((5-methylthiazol-2-yl)amino)-1H-pyrrolo[3,2-c]pyridin-4-yl)oxy)methyl)azetidin-1-yl)prop-2-en-1-one,
219) (S)-1-(2-(((1-isopropyl-6-((5-methylthiazol-2-yl)amino)-1H-pyrrolo[3,2-c]pyridin-4-yl)oxy)methyl)pyrrolidin-1-yl)prop-2-en-1-one,
220) (S)-1-(2-(((1-(cyclopentylmethyl)-6-((5-methylthiazol-2-yl)amino)-1H-pyrrolo[3,2-c]pyridin-4-yl)oxy)methyl)pyrrolidin-1-yl)prop-2-en-1-one,
221) 1-((2S)-2-(((1-(2-methylbutyl)-6-((5-methylthiazol-2-yl)amino)-1H-pyrrolo[3,2-c]pyridin-4-yl)oxy)methyl)pyrrolidin-1-yl)prop-2-en-1-one,
222) (S)-1-(2-(((1-cyclopentyl-6-((5-methylthiazol-2-yl)amino)-1H-pyrrolo[3,2-c]pyridin-4-yl)oxy)methyl)pyrrolidin-1-yl)prop-2-en-1-one,
223) (R)-1-(2-(((1-cyclopentyl-6-((5-methylthiazol-2-yl)amino)-1H-pyrrolo[3,2-c]pyridin-4-yl)oxy)methyl)azetidin-1-yl)prop-2-en-1-one,
224) (S)-2-fluoro-1-(3-((1-isobutyl-6-((5-methylthiazol-2-yl)amino)-1H-pyrrolo[3,2-c]pyridin-4-yl)oxy)pyrrolidin-1-yl)prop-2-en-1-one,
225) (S)-1-(3-((1-isobutyl-6-((5-methylthiazol-2-yl)amino)-1H-pyrrolo[3,2-c]pyridin-4-yl)oxy)pyrrolidin-1-yl)prop-2-yn-1-one,
226) (S,E)-1-(3-((1-isobutyl-6-((5-methylthiazol-2-yl)amino)-1H-pyrrolo[3,2-c]pyridin-4-yl)oxy)pyrrolidin-1-yl)but-2-en-1-one,
227) (S,E)-4-(dimethylamino)-1-(3-((1-isobutyl-6-((5-methylthiazol-2-yl)amino)-1H-pyrrolo[3,2-c]pyridin-4-yl)oxy)pyrrolidin-1-yl)but-2-en-1-one,
228) N-(4-(((3R,4S)-4-fluoro-1-(vinylsulfonyl)pyrrolidin-3-yl)oxy)-1-isobutyl-1H-pyrrolo[3,2-c]pyridin-6-yl)-5-methylthiazol-2-amine,
229) 2-chloro-1-((3S,4R)-3-fluoro-4-((1-isobutyl-6-((5-methylthiazol-2-yl)amino)-1H-pyrrolo[3,2-c]pyridin-4-yl)oxy)pyrrolidin-1-yl)ethan-1-one,
230) 1-((3S,4R)-3-fluoro-4-((1-isobutyl-6-((5-methylthiazol-2-yl)amino)-1H-pyrrolo[3,2-c]pyridin-4-yl)oxy)pyrrolidin-1-yl)but-2-yn-1-one, and
231) 1-((3S,4R)-3-fluoro-4-((1-isobutyl-6-((5-methylthiazol-2-yl)amino)-1H-pyrrolo[3,2-c]pyridin-4-yl)oxy)pyrrolidin-1-yl)prop-2-yn-1-one.

14. A pharmaceutical composition comprising the compound as claimed in any one of claims 1 to 13 or a pharmaceutically acceptable salt thereof as an active ingredient.

15. A pharmaceutical composition for the prevention or treatment of inflammatory diseases, autoimmune diseases, or cancers, comprising the compound as claimed in any one of claims 1 to 13 or a pharmaceutically acceptable salt thereof as an active ingredient.
